# EUROPEAN PATENT SPECIFICATION

(11) **EP 1 496 060 B1**
(45) Date of publication and mention of the grant of the patent: **01.08.2007**
(21) Application number: 04010399.6
(22) Date of filing: 15.05.2001
(51) Int. Cl.: C07D 515/22, C07D 491/22, C07D 471/18, A61K 35/00, C07D 317/00, C07D 291/00, C07D 241/00, C07D 221/00

(54) **Antitumoral analogs of ET-743**
ET-743 Analoga als Antitumoralverbindungen
Analogues antitumoraux de ET-743

(30) Priority: 15.05.2000 WO PCT/GB00/01852
(43) Date of publication of application: 12.01.2005
(62) Divisional of application: 01929813.2
(73) Proprietor: PHARMA MAR, S.A., 28760 Tres Cantos, Madrid (ES)
(72) Inventor: Manzanares, Ignacio, Tres Cantos 28760 Madrid (ES); Martin, Maria Jesus, Pharma Mar, S.A.U., 28770 Colmenar Viejo Madrid (ES); Rodrigues, Alberto, Pharma Mar, S.A.U., 28770 Colmenar Viejo Madrid (ES); Munt, Simon, Pharma Mar, S.A.U., 28770 Colmenar Viejo Madrid (ES); Cuevas, Carmen, Pharma Mar, S.A.U., 28770 Colmenar Viejo Madrid (ES); Perez, Marta, Pharma Mar, S.A.U., 28770 Colmenar Viejo Madrid (ES)
(74) Representative: Ruffles, Graham Keith

(56) References cited:
- US-A- 5 721 362
- FUKUYAMA, LIHU YANG, KAREN L.AJECK: "Total Synthesis of(+)-Saframycic" J.AM.CHEM.SOC.,, vol. 112, 1990, pages 3713-3715, XP002925425
- J.W.LOWN, ALUMMOOTTIL V.JOSHUA ET AL.: "Molecular Mechanisms of Binding and Single-Strand Scission of Deoxyribonucleic Acid by the Antitumor Antibiotics saframycic A and C" BIOCHEMISTRY, vol. 21, no. 3, 1982, XP002925424
- RYUICHI SAKAI ET AL.: "Ecteinascidins: Putative Biosynthetic Precursors and Absolute Stereochemistry" J.AM.CHEM.SOC., vol. 118, 1996, pages 9017-9023, XP002925426

## Description

The present invention relates to antitumoral compounds, and in particular to antitumoral analogs of ecteinascidin 743, ET-743.

### BACKGROUND OF THE INVENTION

European Patent 309,477 relates to ecteinascidins 729, 743, 745, 759A, 759B and 770. The ecteinascidin compounds are disclosed to have antibacterial and other useful properties. Ecteinascidin 743 is now undergoing clinical trials as an antitumour agent.

Ecteinascidin 743 has a complex tris(tetrahydroisoquinolinephenol) structure of the following formula (I):

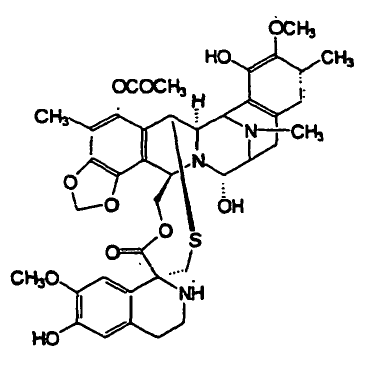

In ecteinascidin 743, the 1,4 bridge has the structure of formula (IV):

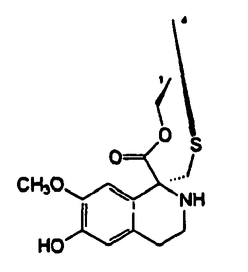

Other known ecteinascidins include compounds with a different bridged cyclic ring system, such as occurs in ecteinascidin 722 and 736, where the bridge has the structure of formula (V): 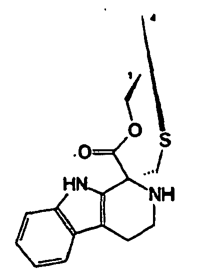 ecteinascidins 583 and 597, where the bridge has the structure of formula (VI): 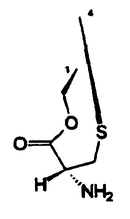 and ecteinascidin 594 and 596, where the bridge has the structure of formula (VII):

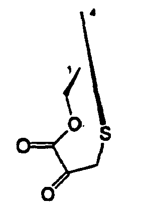

The complete structure for these and related compounds is given in J. Am. Chem. Soc. (1996) 118, 9017-9023. This article is incorporated by reference.

The ecteinascidins are currently prepared by isolation from extracts of the marine tunicate *Ecteinascidin turbinata.* The yield is low, and alternative preparative processes have been sought.

A synthetic process for producing ecteinascidin compounds is described in US Patent 5,721,362, see also WO 9812198. The claimed method is long and complicated. By way of illustration, there are 38 Examples each describing one or more steps in the synthetic sequence to arrive at ecteinascidin 743.

Claim 25 of US 5,721,362 is directed at an intermediate phenol compound of a given formula (11), which we refer to also as Intermediate 11 or Int-11. It has the following bis(tetrahydroisoquinolinephenol) structure (II): 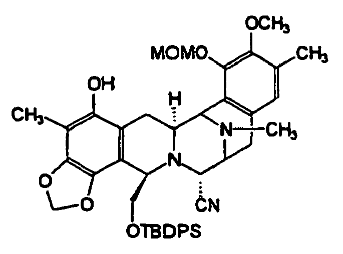 where MOM is a methoxymethyl substituent and TBDPS is a tert-butyldiphenylsilyl substituent.

From Intermediate 11 it is possible to synthesise another interesting antitumour agent, phthalascidin, see Proc. Natl. Acad. Sci. USA, 96,3496-3501,1999. Phthalascidin is a bis(tetrahydroisoquinolinephenol) derivative of formula (III):

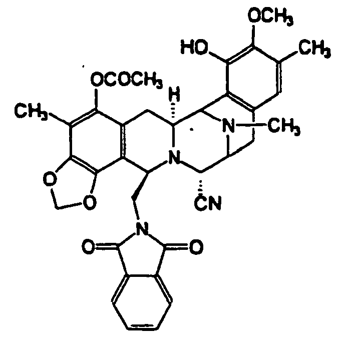

More generally, phthalascidin and related compounds are described in WO 0018233. Claim 1 is directed at compounds of formula: 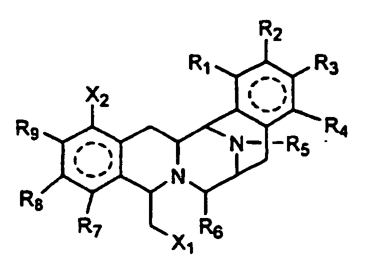 wherein the substituent groups defined by R₁, R₂, R₃, R₄, R₅, R₆, R₇, R₈ and R₉ are each independently selected from the group consisting of H, OH, OR', SH, SR', SOR', SO₂R', NO₂, NH₂, NHR, N(R')₂, NHC(O)R', CN, halogen,=O, C(=O)H, C(=O)R', CO₂H, CO₂R', C₁₋C₁₂ alkyl, C₂-C₁₂ alkenyl, C₂-C₁₂ alkynyl, substituted or unsubstituted aryl, substituted or unsubstituted aralkyl, and substituted or unsubstituted heteroaromatic;
wherein each of the R' groups is independently selected from the group consisting of H, OH, NO₂, NH₂, SH, CN, halogen, =O, C(=O)H, C(=O)CH₃, CO₂H, CO₂CH₃, C₁-C₁₂ alkyl, C₂-C₁₂ alkenyl, C₂-C₁₂ alkynyl, aryl, aralkyl, and heteroaromatic;
wherein each dotted circle represents one, two or three optional double bonds;
wherein R₇ and R₈ may be joined into a carbocyclic or heterocyclic ring system; and wherein X₁ and X₂ are each independently defined as above for R₁-R₈ and further include various permitted definitions.

Further naturally occuring compounds are known which lack a bridged cyclic ring system. They include the bis(tetrahydroisoquinolinequinone) antitumor-antimicrobial antibiotics safracins and saframycins, and the marine natural products renieramicins and xestomycin isolated from cultured microbes or sponges. They all have a common dimeric tetrahydroisoquinoline carbon framework. These compounds can be classified into four types, types I to IV, with respect to the oxidation pattern of the aromatic rings.

Type I, dimeric isoquinolinequinones, is a system of formula (VIII) most commonly occurring in this class of compounds, see the following table I.

**Table I**

| | | | | | | |
|---|---|---|---|---|---|---|
| Structure of Type I Saframycin Antibiotics. | | | | | | |
| 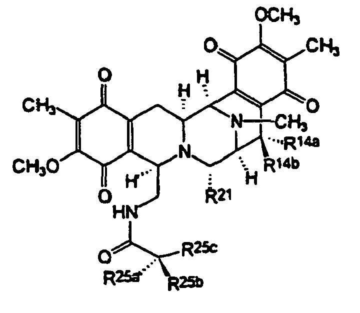 | | | | | | |

| | Substituents | | | | | |
|---|---|---|---|---|---|---|
| Compound | R^{14a} | R^{14b} | R²¹ | R^{25a} | R^{25b} | R^{25c} |
| saframycin A | H | H | CN | O | O | CH₃ |
| saframycin B | H | H | H | O | O | CH₃ |
| saframycin C | H | OCH₃ | H | O | O | CH₃ |
| saframycin G | H | OH | CN | O | O | CH₃ |
| saframycin H | H | H | CN | OH | CH₂COCH₃ | CH₃ |
| saframycin S | H | H | OH | O | O | CH₃ |
| saframycin Y₃ | H | H | CN | NH₂ | H | CH₃ |
| saframycin Yd₁ | H | H | CN | NH₂ | H | C₂H₅ |
| saframycin Ad₁ | H | H | CN | O | O | C₂H₅ |
| saframycin Yd₂ | H | H | CN | NH₂ | H | H |
| saframycin Y_{2b} | H | Q*^{b}* | CN | NH₂ | H | CH₃ |
| saframycin Y_{2b-d} | H | Q*^{b}* | CN | NH₂ | H | C₂H₅ |
| saframycin AH₂ | H | H | CN | H*^{a}* | OH*^{a}* | CH₃ |
| saframycin AH₂Ac | H | H | CN | H | OAc | CH₃ |
| saframycin AH₁ | H | H | CN | OH*^{a}* | H*^{a}* | CH₃ |
| saframycin AH₁Ac | H | H | CN | OAc | H | CH₃ |
| saframycin AR₃ | H | H | H | H | OH | CH₃ |

| | | | | | | |
|---|---|---|---|---|---|---|
| *^{a}* assignments are interchangeable. *^{b}* where the group Q is of formula (IX): | | | | | | |

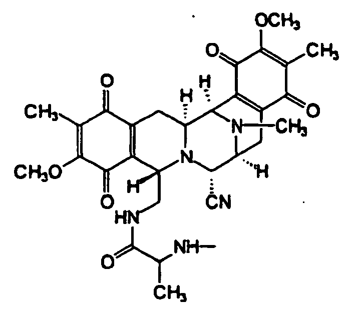

Type I aromatic rings are seen in saframycins A, B and C; G and H; and S isolated from *Streptomyces lavendulae* as minor components. A cyano derivative of saframycin A, called cyanoquinonamine, is known from Japanese Kokai JP-A2 59/225189 and 60/084288. Saframycins Y₃, Yd₁, Ad₁, and Yd₂ were produced by *S. lavendulae* by directed biosynthesis, with appropriate supplementation of the culture medium. Saframycins Y_{2b} and Y_{2b-d} dimers formed by linking the nitrogen on the C-25 of one unit to the C-14 of the other, have also been produced in supplemented culture media of *S. lavendulae.* Saframycins AR₁(=AH₂,), a microbial reduction product of saframycin A at C-25 produced by *Rhodococcus amidophilus,* is also prepared by nonstereoselective chemical reduction of saframycin A by sodium borohydride as a 1:1 mixture of epimers followed by chromatographic separation [the other isomer AH₁ is less polar]. The further reduction product saframycin AR₃, 21-decyano-25-dihydro-saframycin A. (= 25-dihydrosaframycin B) was produced by the same microbial conversion. Another type of microbial conversion of saframycin A using a *Nocardia* species produced saframycin B and further reduction by a *Mycobacterium* species produced saframycin AH¹ Ac. The 25-*O*-acetates of saframycin AH₂ and AH₁ have also been prepared chemically for biological studies.

Type I compounds of formula (X) have also been isolated from marines sponges, see Table II.

**Table II**

| | | | | |
|---|---|---|---|---|
| Structures of Type I Compounds from Marine Sponges. | | | | |
| 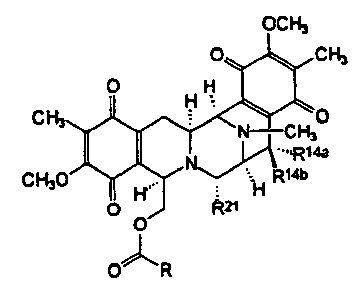 | | | | |

| | Substituents | | | |
|---|---|---|---|---|
| | R^{14a} | R^{14b} | R²¹ | R |
| renieramycin A | OH | H | H | -C(CH₃)=CH-CH₃ |
| renieramycin B | OC₂H₅ | H | H | -C(CH₃)=CH-CH₃ |
| renieramycin C | OH | O | O | -C(CH₃)=CH-CH₃ |
| renieramycin D | OC₂H₅ | O | O | -C(CH₃)=CH-CH₃ |
| renieramycin E | H | H | OH | -C(CH₃)=CH-CH₃ |
| renieramycin F | OCH₃ | H | OH | -C(CH₃)=CH-CH₃ |
| xestomycin | OCH₃ | H | H | -CH₃ |

Renieramycins A-D were isolated from the antimicrobial extract of a sponge, a *Reniera* species collected in Mexico, along with the biogenetically related monomeric isoquinolines renierone and related compounds. The structure of renieramycin A was initially assigned with inverted stereochemistry at C-3, C-11, and C-13. However, careful examination of the ¹H NMR data for new, related compounds renieramycins E and F, isolated from the same sponge collected in Palau, revealed that the ring junction of renieramycins was identical to that of saframycins. This result led to the conclusion that the formerly assigned stereochemistry of renieramycins A to D must be the same as that of saframycins.

Xestomycin was found in a sponge, a *Xestospongia* species collected from Sri Lancan waters. Type II compounds of formula (XI) with a reduced hydroquinone ring include saframycins D and F, isolated from *S. lavendulae,* and saframycins Mx-1 and Mx-2, isolated from *Myxococcus xanthus.* See table III.

**Table III**

| | | | | | | |
|---|---|---|---|---|---|---|
| Type II Compounds | | | | | | |
| 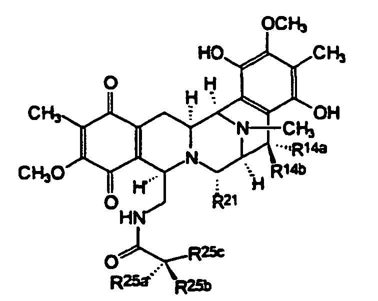 | | | | | | |

| | Substituents | | | | | |
|---|---|---|---|---|---|---|
| Compound | R^{14a} | R^{14b} | R²¹ | R^{25a} | R^{25b} | R^{25c} |
| saframycin D | O | O | H | O | O | CH₃ |
| saftamycin F | O | O | CN | O | O | CH₃ |
| saframycin Mx-1 | H | OCH₃ | OH | H | CH₃ | NH₂ |
| saframycin Mx-2 | H | OCH₃ | H | H | CH₃ | NH₂ |

The type III skeleton is found in the antibiotics safracins A and B, isolated from cultured *Pseudomonas fluorescens.* These antibiotics of formula (XII) consist of a tetrahydroisoquinoline-quinone subunit and a tetrahydroisoquninolinephenol subunit. 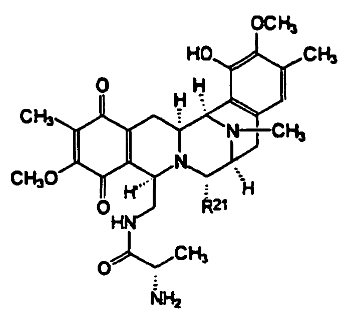 where R²¹ is -H in safracin A and is -OH in safracin B.

Saframycin R, the only compound classified as the Type IV skeleton, was also isolated from *S. lavendulae.* This compound of formula (XIII), consisting of a hydroquinone ring with a glycolic ester sidechain on one of the phenolic oxygens, is conceivably a pro-drug of saframycin A because of its moderate toxicity.

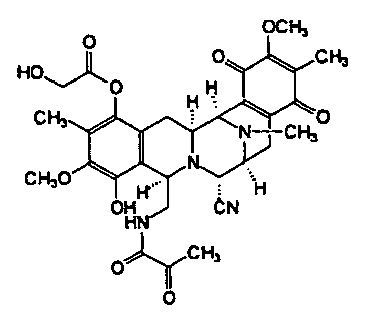

All these known compounds have a fused system of five rings (*A*) to (*E*) as shown in the following structure of formula (XIV):

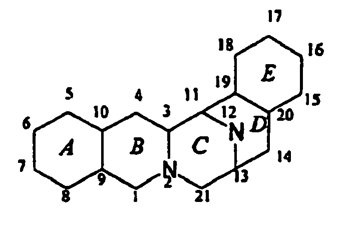

The rings *A* and *E* are phenolic in the ecteinascidins and some other compounds, while in other compounds, notably the saframycins, the rings *A* and *E* are quinolic. In the known compounds, the rings *B* and *D* are tetrahydro, while ring *C* is perhydro.

### SUMMARY OF THE INVENTION

According to this invention there are provided compounds of the formula: 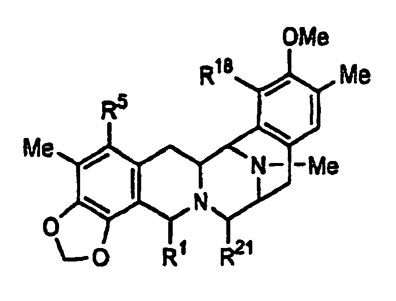 wherein:
- R¹: is selected from the group consisting of -CH₂-NHR^{a} and -CH₂-OR^{a}, where R^{a} is selected from the group consisting of alkyl-CO-; haloalkyl-CO-; haloalkyl-O-CO-; arylalkenyl-CO- wherein the aryl group is selected from phenyl, biphenyl and naphthyl; heteroaryl-CO- wherein the heteroaryl contains one, two or three heteroatoms selected from nitrogen, sulphur and oxygen; alkenyl-CO-; and amino acid acyl;
- R⁵: is OH; acetyloxy or other acyloxy group up to 4 carbon atoms;
- R¹⁸: is -OH; and
- R²¹: is -CN.

Further, according to this invention there are provided compounds of the formula : 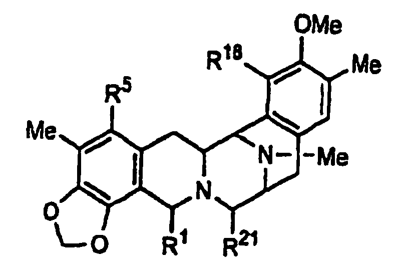 wherein:
- R¹: is -CH₂-OR^{a}, where R^{a} is selected from the group consisting of alkyl-CO-; haloalkyl-CO-; cycloalkylalkyl-CO-; haloalkyl-O-CO-; arylalkenyl-CO- wherein the aryl group is selected from phenyl, biphenyl and naphthyl; heteroaryl-CO- where the heteroaryl contains one, two or three heteroatoms selected from nitrogen, sulphur and oxygen; alkenyl-CO-; alkenyl; and amino acid acyl;
- R⁵: is -OR", where R" is selected from the group consisting of H; alkyl-CO-; cycloalkyl-CO-; and haloalkyl-CO-; R¹⁸ is -OR, where R is selected from the group consisting of H, alkyl-CO-; cycloalkylalkyl-CO-; and
- R²¹: is-CN.

The present invention provides new compounds with the fused system of five rings (A) to (E). In particular, it provides new compounds which can be made from intermediates described in WO 9812198 or by a new process which is part of this invention. In this latter respect, we refer to our WO 0069862 published 23 November 2000, and which relates to hemisynthetic methods and new compounds. The present application claims priority from that PCT filing, and we incorporate that text by reference to the extent that there is disclosure therein which is not in the present specification.

In WO 0069862, various routes are described for the preparation of ecteinascidin compounds, including ecteinascidin 743, as well as ecteinascidin analogs including phthaliscidin. The present invention is founded partly on the use of intermediates of WO 0069862 to prepare further analogs of the ecteinasacidins.

### PREFERRED EMBODIMENTS

We have found that compounds of the invention have exceptional activity in the treatment of cancers, such as leukaemias, lung cancer, colon cancer, kidney cancer and melanoma.

Thus, the present invention provides compounds for use in a method of treating any mammal, notably a human, affected by cancer which comprises administering to the affected individual a therapeutically effective amount of a compound of the invention, or a pharmaceutical composition thereof.

The present invention also relates to pharmaceutical preparations, which contain as active ingredient a compound or compounds of the invention, as well as the processes for their preparation.

Examples of pharmaceutical compositions include any solid (tablets, pills, capsules, granules, etc.) or liquid (solutions, suspensions or emulsions) with suitable composition or oral, topical or parenteral administration, and they may contain the pure compound or in combination with any carrier or other pharmacologically active compounds. These compositions may need to be sterile when administered parenterally.

Administration of the compounds or compositions of the present invention may be by any suitable method, such as intravenous infusion, oral preparations, intraperitoneal and intravenous administration. We prefer that infusion times of up to 24 hours are used, more preferably 2-12 hours, with 2-6 hours most preferred. Short infusion times which allow treatment to be carried out without an overnight stay in hospital are especially desirable. However, infusion may be 12 to 24 hours or even longer if required. Infusion may be carried out at suitable intervals of say 2 to 4 weeks. Pharmaceutical compositions containing compounds of the invention may be delivered by liposome or nanosphere encapsulation, in sustained release formulations or by other standard delivery means.

The correct dosage of the compounds will vary according to the particular formulation, the mode of application, and the particular *situs,* host and tumour being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

The compounds and compositions of this invention may be used with other drugs to provide a combination therapy. The other drugs may form part of the same composition, or be provided as a separate composition for administration at the same time or a different time. The identity of the other drug is not particularly limited, and suitable candidates include:
a) drugs with antimitotic effects, especially those which target cytoskeletal elements, including microtubule modulators such as taxane drugs (such as taxol, paclitaxel, taxotere, docetaxel), podophylotoxins or vinca alkaloids (vincristine, vinblastine);
b) antimetabolite drugs such as 5-fluorouracil, cytarabine, gemcitabine, purine analogues such as pentostatin, methotrexate);
c) alkylating agents such as nitrogen mustards (such as cyclophosphamide or ifosphamide);
d) drugs which target DNA such as the antracycline drugs adriamycin, doxorubicin, pharmorubicin or epirubicin;
e) drugs which target topoisomerases such as etoposide;
f) hormones and hormone agonists or antagonists such as estrogens, antiestrogens (tamoxifen and related compounds) and androgens, flutamide, leuprorelin, goserelin, cyprotrone or octreotide;
g) drugs which target signal transduction in tumour cells including antibody derivatives such as herceptin;
h) alkylating drugs such as platinum drugs (cis-platin, carbonplatin, oxaliplatin, paraplatin) or nitrosoureas;
i) drugs potentially affecting metastasis of tumours such as matrix metalloproteinase inhibitors;
j) gene therapy and antisense agents;
k) antibody therapeutics;
l) other bioactive compounds of marine origin, notably the didemnins such as aplidine;
m) steroid analogues, in particular dexamethasone;
n) anti-inflammatory drugs, in particular dexamethasone;
o) anti-emetic drugs, in particular dexamethasone;
p) skeletal muscle protectors, such as L-carnitine or precursor amino acids.

The present invention also extends to the compounds of the invention for use in a method of treatment, and to the use of the compounds in the preparation of a composition for treatment of cancer.

The compounds of this invention include compounds which do not have a hydroxy group at the C-18 position. Furthermore, the compounds of this invention include compounds which do not have a dicarboximidomethyl substituent, such as phthalimidomethyl, at the C-1 position. In particular, we provide active compounds where the subsituent X₁ is not as shown in the penultimate line at page 19 of WO0018233.

In one aspect, the analogs of this invention are typically of the formula (XVIIb): 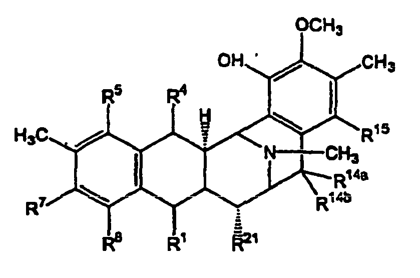 where
R¹ is an optionally protected or derivatised aminomethylene group, an optionally protected or derivatised hydroxymethylene group;
R⁴ is -H;
R⁵ is or -OH ;
R⁷ and R⁸ together form a group -O-CH₃-O-;
R^{14a} and R^{14b} are both -H ; and
R¹⁵ is -H;
R²¹ is -CN;
and derivatives including acyl derivatives thereof especially where R⁵ is acetyloxy or other acyloxy group of up to 4 carbon atoms.

In the present invention, a key class of products has the general formula (XX): 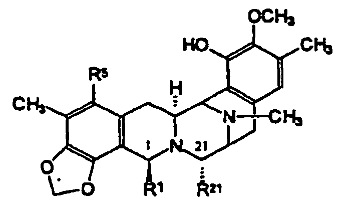 where R¹ is an amidomethylene group; R⁵ is a small oxy-sidechain; and R²¹ is a cyano group. R⁵ an acetoxy group; and R²¹ is a cyano group. R¹ includes mono- and di-N-substituted amidomethylenes as well as other cyclic amidomethylenes, and other groups for R⁵ include further C₁-C₄ acyl groups, as well as C₁-C₄ alkyl groups.

In the present invention, a key class of intermediates and analogs includes Intermediate 11 and has the general formula (XXI): 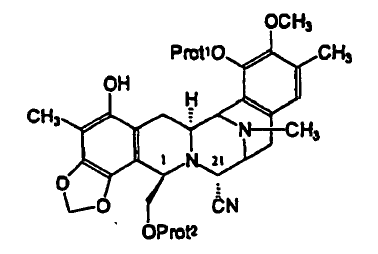 where Prot¹ and Prot² are hydroxy protecting groups, preferably different. For Intermediate 11 itself, the group Prot¹ is a methoxymethyl group, and Prot² is a t-butyldiphenylsilyl group.

In the light of the preceding explanations, it can be seen that the present invention provides novel analogs and novel intermediate compounds. Depending on ring A, the compounds include those of formula (XXIIb): 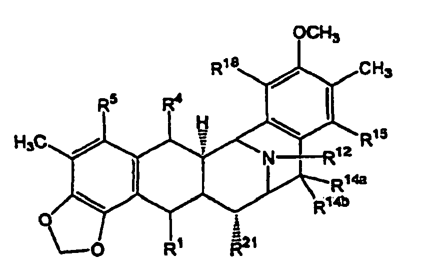 where:
R¹ is a protected or derivatised version of -CH₂NH₂ or -CH₂OH and R⁴ is-H;
R⁵ is -OH or a protected or derivatised version of such a group;
R^{14a} and R^{14b} are both -H
R¹² is -CH₃-;
R¹⁵ is -H,
R¹⁸ is -OH or a protected or derivatised version of such a group; and R²¹ is -CN.
Respectively, each derivatised version of the groups is as defined.

In one embodiment, preferably at least of R¹, R⁵ or R¹⁸ is a protected or derivatised group.

In one variation of this invention, the group R¹ is not a tert-butyldiphenylsilyl substituent and/or the group R¹⁸ is not a methoxymethyloxy group.

One preferred class of intermediates includes the compound which we identify as compound 25, of formula:

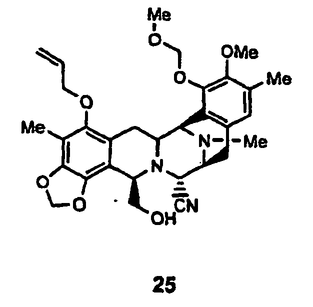

The preferred class is thus of the general formula where the group MOM is replaced by any other protecting group, and/or the allyl is replaced by any other protecting group.

Other preferred intermediates includes the compounds which we identify as compounds **17,43** and **45**. 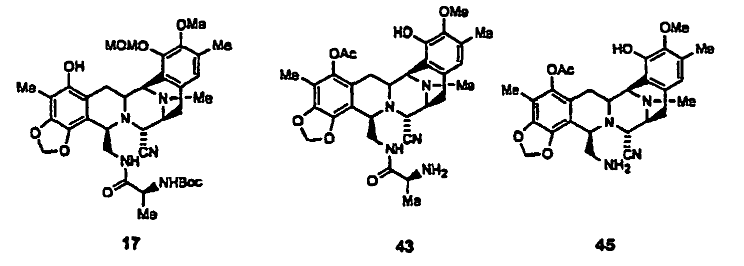 Other N-acyl derivatives may readily be made from compound **45** and are an important part of this invention. Suitable acyl groups include those previously mentioned. The corresponding 21-hydroxy compounds are also useful and are among the active compounds which we have found.

From the activity data and other considerations, it can be seen that the active compounds of this invention include a preferred class of compounds of the general formula (XXIII): 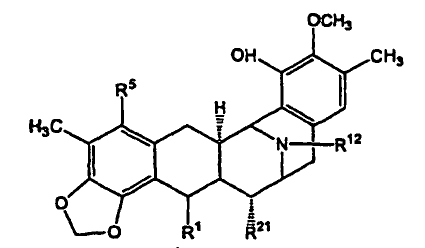 where R¹ is as previously defined and is preferably a derivatised aminomethylene group of moderate bulk;
R⁵ is as previously defined and is preferably a derivatised hydroxy group of low bulk;
R¹² is -CH₃- ; and
R²¹ is cyano group.

R¹ is suitably a hydrophobic group and which thus lacks free amino, hydroxy or other hydrophilic function. Typically R¹ is a group -CH₂-NH₂-CO-R^{a}, where R^{a} is as defined but preferably has a linear chain length of less than 20 atoms, more preferably less than 15 or 10 atoms, where a 1,4-phenyl is counted as a chain length of four atoms and similar considerations apply to other cyclic groups (for example, 1,2-cyclohexyl is chain length of two), and the linear chain of less than 10, 15 or 20 atoms can itself be substituted. In particular, the data suggests there is a balance to be achieved between having no such group R^{a}-CO- and having a large, bulky group.

In one variation, we prefer that R¹ is free from cyclic groups, especially aromatic groups. In a related variation, the present invention does not prepare the compounds which are described in the article Proc. Natl. Acad. Sci. USA, 96, 3496-3501, 1999, incorporated by reference. Our preferred groups for R¹ exclude the corresponding substituents CH₂R₂ shown in Table 1 of that article, specifically the groups A, B, C and D for R₂.

R⁵ is preferably an acetyl group.

In particularly preferred compounds, the group R¹ is acylated on an -NH₂ group, and for example N-acyl derivatives can be formed from groups -CH₂NH₂ and -CH₂-NH-aa. The acyl derivatives can be N-acyl or N-thioacyl derivatives thereof. The acyl groups can be of formula -CO-R^{a}, where R^{a} is as defined and is chosen to meet the indicated criteria. Suitable acyl groups include alanyl, arginyl, aspartyl, asparagyl, cystyl, glutamyl, glutaminyl, glycyl, histidyl, hydroxyprolyl., isoleucyl, leucyl, lysyl, methionyl, phenylalanyl, prolyl, seryl, threonyl, thyronyl, tryptophyl, tyrosyl, valyl, as well as other amino acid acyl groups, which may be L- or D-. Such amino acid acyl groups are preferred derivatised on the amino group to give hydrophobicity.

In a variation, the group R¹ is a derivatised hydroxymethylene group. Similar considerations apply as with the derivatised aminomethylene group.

The invention extends to compounds where the various substituents around the ring are as defined in the WO 0018233. Thus, as appropriate, substituents in the present compounds can be chosen, among other possibilites from H, OH, OR', SH, SR', SOR', SO₂R', NO₂, NH₂, NHR', N(R)₂, NHC(O)R', CN, halogen, =O, C₁-C₆ alkyl, substituted or unsubstituted aryl, substituted or unsubstituted aralkyl, and substituted or unsubstituted heteroaromatic;
wherein each of the R' groups is independently selected from the group consisting of H, OH, NO₂, NH₂, SH, CN, halogen, =O, C(=O)H, C(=O)CH₃, CO₂H, CO₂CH₃, C₁-C₆ alkyl, phenyl, benzyl and heteroaromatic.

Suitable halogen substituents in the compounds of the present invention include F, Cl, Br and I.

Alkyl groups preferably have from 1 to about 12 carbon atoms, more preferably 1 to about 8 carbon atoms, still more preferably 1 to about 6 carbon atoms, and most prefereably 1, 2, 3 or 4 carbon atoms. Methyl, ethyl and propyl including isopropyl are particularly preferred alkyl groups in the compounds of the present invention. As used herein, the term alkyl, unless otherwise modified, refers to both cyclic and noncyclic groups, although cyclic groups will comprise at least three carbon ring members.

Preferred alkenyl and alkynyl groups in the compounds of the present invention have one or more unsaturated linkages and from 2 to about 12 carbon atoms, more preferably 2 to about 8 carbon atoms, still more prefereably 2 to about 6 carbon atoms, even more preferably 2, 3 or 4 carbon atoms. The terms alkenyl and alkynyl as used herein refere to both cyclic and moncyclic groups, although-straight or branched noncyclic groups are generally more preferred.

Preferred alkoxy groups in the compounds of the present invention include groups having one or more oxygem linkages and from 1 to about 12 carbon atoms, more preferably from 1 to about 8 carbon atoms, and still more preferably 1 to about 6 carbon atoms, and most preferably 1, 2, 3 or 4 carbon atoms.

Preferred alkylthio groups in the compounds of the present invention have one or more thioether linkages and from 1 to about 12 carbon atoms, more prefereably from 1 to about 8 carbon atoms, and still more preferably 1 to about 6 carbon atoms. Alkylthio groups having 1, 2, 3 or 4 carbon atoms are particularly preferred.

Preferred alkylsulfinyl groups in the compounds of the present invention include those groups having one or more sulfoxide (SO) groups and from 1 to about 12 carbon atoms, more preferably from 1 to about 8 carbon atoms, and still more preferably 1 to about 6 carbon atoms. Alkylsulfinyl groups having 1, 2, 3 or 4 carbon atoms are particularly preferred.

Preferred alkylsulfonyl groups in the compounds of the present invention include those groups having one or more sulfonyl (SO₂) groups and from 1 to about 12 carbon atoms, more preferably from 1 to about 8 carbon atoms, and still more preferably 1 to about 6 carbon atoms. Alkylsulfonyl groups having 1,2,3 or 4 carbon atoms are particularly preferred.

Preferred aminoalkyl groups include those groups having one or more primary, secondary and/or tertiary amine groups, and from 1 to about 12 carbon atoms, more preferably 1 to about 8 carbon atoms, still more preferably 1 to about 6 carbon atoms, even more preferably 1, 2, 3 or 4 carbon atoms. Secondary and tertiary amine groups are generally more preferred than primary amine moieties.

Suitable heteroaromatic groups in the compounds of the present invention contain one, two or three heteroatoms selected from N, O or S atoms and include, e.g., coumarinyl including 8-coumarinyl, quinolinyl including 8-quinolinyl, pyridyl, pyrazinyl, pyrimidyl, furyl, pyrrolyl, thienyl, thiazolyl, oxazolyl, imidazolyl, indolyl, benzofuranyl and benzothiazol. Suitable heteroalicyclic groups in the compounds of the present invention contain one, two or three heteroatoms selected from N, O or S atoms and include, e.g., tetrahydrofuranyl, tetrahydropyranyl, piperidinyl, morpholino and pyrrolindinyl groups.

Suitable carbocyclic aryl groups in the compounds of the present invention include single and multiple ring compounds, including multiple ring compounds that contain separate and/or fused aryl groups. Typical carbocyclic aryl groups contain 1 to 3 separate or fused rings and from 6 to about 18 carbon ring atoms. Specifically preferred carbocyclic arykl groups include phenyl including substituted phenyl, such as 2-substituted phenyl, 3-substituted phenyl, 2,3-substituted phenyl, 2,5-substituted phenyl, 2.3.5-substituted and 2,4,5-substituted phenyl, including where one or more of the phenyl substituents is an electron-withdrawing group such as halogen, cyano, nitro, alkanoyl, sulfinyl, sulfonyl and the like; naphthyl including 1-naphthyl and 2-naphthyl; biphenyl; phenanthryl: and anthracyl.

Any references herein to substituted groups in the compounds of the present invention refer to the specified moiety that may be substituted at one or more available positions by one or more suitable groups, e.g., halogen such as fluoro, chloro, bromo and iodide; cyano; hydroxyl; nitro; azido; alkanoyl such as a C1-6 alkanoyl group such as acyl and the like; carboxamido; alkyl groups including those groups having 1 to about °2 carbon atoms or from 1 to about 6 carbon atoms and more preferably 1-3 carbon atoms; alkenyl and alkynyl groups including groups having one or more unsaturated linkages and from 2 to about 12 carbon or from 2 to about 6 carbon atoms; alkoxy groups having those having one or more oxygen linkages and from 1 to about 12 carbon atoms or 1 to about 6 carbon atoms; aryloxy such as phenoxy; alkylthio groups including those moieties having one or more thioether linkages and from 1 to about 12 carbon atoms or from 1 to about 6 carbo atoms; alkylsulfinyl groups including those moieties having one or more sulfinyl linkages and from 1 to about 12 carbon atoms or from 1 to about 6 carbon atoms; alkylsulfinyl groups including those moieties having one or more sulfonyl linkages and from 1 to about 12 carbon atoms or from 1 to about 6 carbon atoms; aminoalkyl groups such as groups having one or more N atoms and from 1 to about 12 carbon atoms or from 1 to about 6 carbon atoms; carbocyclic aryl having 6 or more carbons, particularly phenyl (e.g., R being a substituted or unsubstituted biphenyl moiety); and aralkyl such as benzyl.

Without being exhaustive, in terms of a formula: 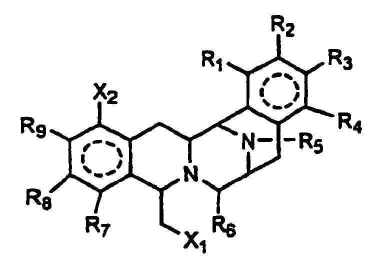 preferred compounds of this invention have one or more of the following definitions:
R₁ is -OR, where R is H, alkyl-CO- especially acetyl (alkyl being up to about 20 carbon atoms, more preferably from 1 to about 12 carbon atoms, and especially an odd number of carbon atoms such as 3, 5, 7 and 9), cycloalkyl-alkyl-CO- and especially alkyl groupings with a terminal cyclohexyl group and up to six additional carbon atoms in the sidechain, or a protecting group, especially methoxymethyl, and R₁ is more especially OH.
R₂ is methoxy.
R₃ is methyl.
R₄ is hydrogen.
R₅ is methyl.
R₆ is -CN.
X₁ is -NHR', -NH-aa-R' or -OR' where aa is an optionally protected amino acid acyl group, especially alanine, phenylalanine, cysteine, proline, valine, arginine, tryptophan or other amino acid. Other possibilities for X₁ include -N(R')₂, -N(R')-aa-R', and -N-(aa-R')₂. In the case of any group -aa-R', the R' is usually on the amino group of the amino acid, and there may be two such substituents. R' is preferably H; alkyl-CO- (alkyl being up to 25 carbon atoms, such as up to 17, 19 or 21 carbon atoms and preferably an odd number of carbon atoms corresponding to a fatty acid carboxylic acid of even number of carbon atoms or else a low number of carbon atoms such as 1 to 6), especially CH₃-(CH₂)n-CO- where n is for example 1, 2, 4, 12 or 16; alkenyl, especially allyl; haloalkyl-CO-, especially CF₃-CO-; cycloalkyl-alkyl-CO-, preferably alkyl groupings with a terminal cyclohexyl group and up to six additional carbon atoms in the sidechain, especially cyclohexyl-(CH₂)ₙ-CO- where n is for example 1 or 2; haloalkyl-O-CO-, especially trichloroethoxycarbonyl; arylalkyl-CO- or arylalkenyl-CO- especially phenyl-methyl/ethyl/vinyl-CO-, where aryl may be substituted as in trifluoromethylcinnamoyl; optionally substituted heteroaryl-CO-, where the substituents and heterocyclic group are as elsewhere discussed, as in 2-chloronicotinoyl; alkenyl-CO-especially crotonyl; opitionally subsituted aminoalkyl-CO-, particularly amino acid acyl, especially alanine, phenylalanine, cysteine, proline, valine, arginine, tryptophan or other amino acid, or a derivative thereof, as in Boc- phenylalanine, valine, proline, arginine or tryptophan, or as in phenethylalanine, trifluoroathylacetylalanine, trifluorodiacetylalanine and isomers thereof, or diacetyl- or dipropionyl- trifluoroacetyl, or as in or as in Cbz-Val- or a group notionally derived from cysteine and being of general formula Prot^{SH}-S-CH₂-C(=NOProt^{OH})-CO- or Prot^{SH}-S-CH=C(-OProt^{OH})-CO-, where Prot^{SH} and Prot^{OH} are protecting groups for thiol and for hydroxy, especially where Prot^{SH} is Fm and Prot^{OH} is methoxy for the first formulaor MOM for the second formula;or other possibilities such as a protecting group as in an alkoxycarbonyl such as Boc, or PhNR'CS. The various groups may be susbtituted as indicated elsewhere in this specification.
R₇ and R₈ are -O-CH₂-O-.
R₉ is methyl.
X₂ is -OR", where R" is preferably H; alkyl-CO-, especially acetyl; alkenyl especially allyl; alkenyl-O-CO-, especially allyl-O-CO-; haloalkyl-CO-, especially trifluoromethylcarbonyl or chloromethylcarbonyl or 2-chloroethylcarbonyl or perfluoropropylcarbonyl.

Of special interest are compounds wherein:
R₁ is -OR, where R is H or acetyl, alkyl-CO-, especially n-propyl-CO-, and R₁ is more especially OH.
R₂ is methoxy.
R₃ is methyl.
R₄ is hydrogen.
R₅ is methyl.
R₆ is -CN.
X₁ is -NHR', where R' is preferably alkenyl, especially allyl, alkyl-CO- (alkyl being 1 to 6 carbon atoms, especially CH₃-(CH₂)n-CO- where n is for example 1 to 6, and more especially 1 to 4); cycloalkyl-alkyl-CO-, especially cyclohexyl-(CH₂)ₙ-CO where n is 1 or 2; arylalkyl-CO- or arylalkenyl-CO- especially phenethylcarbonyl, phenylvinylcarbonyl or benzylcarbonyl, alkenyl-CO- especially CH₃-CH=CH-CO-; amino acid acyl, especially Cbz-Val-; optionally substituted heteroaryl-CO-, especially 2-chloropyridinylcarbonyl;
or X₁ is -NH-aa-R' where aa is alanine, phenylalanine, tryptophan or valine; R' is an amino subsituent and is arylalkyl-CO- especially phenethylcarbonyl or benzylcarbonyl; alkyl-CO-(alkyl being 1 to 6 carbon atoms, especially CH₃-(CH₂)n-CO- where n is for example 1 to 6 and more especially 1, 2 or 4; alkenyl-CO- especially CH₃-CH=CH-CO-; or protecting group especially alkyloxy-CO as in Boc;
or X₁ is -OR' where R' is preferably alkyl-CO- (alkyl being 1 to 6 carbon atoms, especially CH₃-(CH₂)n-CO- where n is for example 1 to 6, and more especially 2; arylalkyl-CO- or arylalkenyl-CO- especially phenethylcarbonyl, phenylvinylcarbonyl or trifluoromethylcinnamoyl.
R₇ and R₈ are -O-CH₂-O-.
R₉ is methyl.
X₂ is -OR", where R" is H; acetyl, allyloxycarbonyl, chloromethylcarbonyl or perfluoropropylcarbonyl; and R" is more especially H; acetyl or allyloxycarbonyl.

Especially preferred embodiments of the present invention are the novel ecteinascidin-like compounds with the following general structures I, II, III, IV and V that have been prepared from compounds 17, 25, 43 and 45 derived from cyanosafracin B. Compound 25 corresponds to the synthetic intermediate 3 described in US patent No 6,124,292.

Preferred embodiments include a compound of a formula selected from the following general structure I: 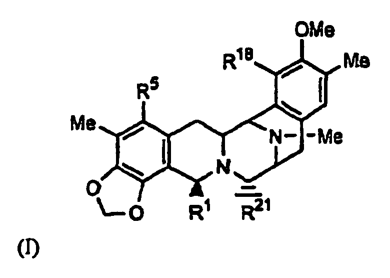 wherein R^{a}, R⁵, R¹⁸, R¹, and R²¹ are each independently selected from the groups defined below:

| R^{a} | R⁵ | R¹⁸ | R¹ | R²¹ |
|---|---|---|---|---|
| | OH | OH | CH₂NHR^{a} | CN |
| COCH₂CH₂CH₃ | OAc | | CH₂OR^{a} | |
| COCH=CHPh | | | | |
| 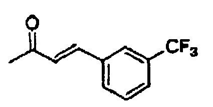 | | | | |
| COCH(CH₃)NHCOCH₂CH₂Ph | | | | |
| CO-(S)-CH(CH₃)NHCOCF₃ | | | | |
| CO-(R)-CH(CH₃)NHCOCF₃ | | | | |
| CO-(S)-CH(NHCbz)CH(CH₃)₂ | | | | |
| CSNHPh | | | | |
| 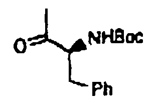 | | | | |
| 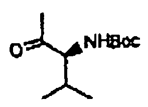 | | | | |
| 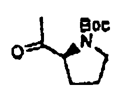 | | | | |
| 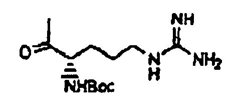 | | | | |
| 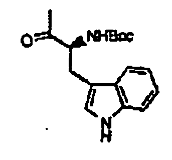 | | | | |

Preferred embodiments also include a compound of a formula selected from the following general structure (II): 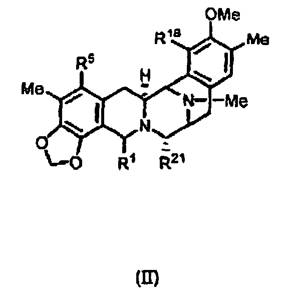 wherein R⁸, R⁵, R¹⁸, R¹, and R²¹ are each independently selected from the groups defined below:

| R^{a} | R⁵ | R¹⁸ | R¹ | R²¹ |
|---|---|---|---|---|
| COCH₂CH₂CH₃ | OH | OH | CH₂NHR^{a} | CN |
| COCH=CHPh | OAc | | CH₂OR^{a} | |
| 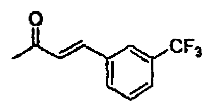 | | | | |
| COCH(CH₃)NHCOCH₂CH₂Ph | | | | |
| CO-(S)-CH(CH₃)NHCOCF₃ | | | | |
| CO-(R)-CH(CH₃)NHCOCF₃ | | | | |
| CO-(S)-CH(NHCbz)CH(CH₃)₂ | | | | |
| CSNHPh | | | | |
| 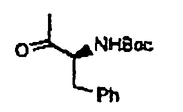 | | | | |
| 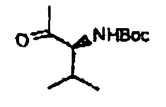 | | | | |
| 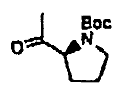 | | | | |
| 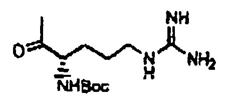 | | | | |
| 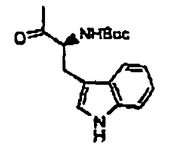 | | | | |

Preferred embodiments include a compound of a formula of the following general structure I: 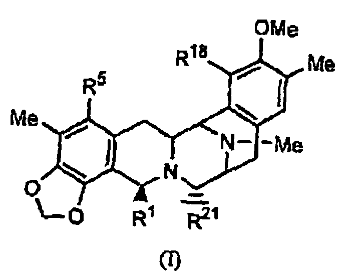 wherein R^{b}, R⁵, R¹, R¹⁸ and R²¹ are each independently selected from the groups defined below:
R¹ is -CH₂-NH-CO-CHCH₃-NHR^{b}

| R^{b} | R⁵ | R¹⁸ | R²¹ |
|---|---|---|---|
| H | OH | OH | CN |
| | OAc | | |
| COCH₂CH₂CH₃ | | | |
| COCH₂Ph | | | |
| COCH₂CH₂Ph | | | |
| COCH=CHPh | | | |
| 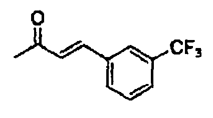 | | | |
| Boc | | | |
| CSNHPh | | | |

Preferred embodiments also include a compound of a formula of the following general structure II: 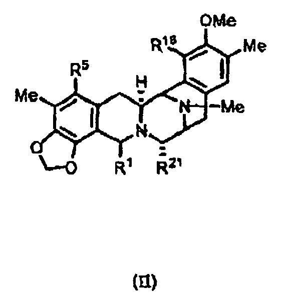 wherein R^{b}, R⁵, R¹, R¹⁸ and R²¹ are each independently selected from the groups defined below:
R¹ is -CH₂-NH-CO-CHCH₃-NHR^{b}

| | | | |
|---|---|---|---|
| R^{b} | R⁵ | R¹⁸ | R²¹ |
| H | OH | OH | CN |
| COCH₂CH₂CH₃ | OAc | | |
| COCH₂Ph | | | |
| COCH₂CH₂Ph | | | |
| COCH=CHPh | | | |
| 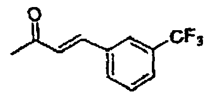 | | | |
| Boc | | | |
| CSNHPh | | | |

Preferred embodiments also include a compound of a formula selected from the following general structures (III) and (IV): 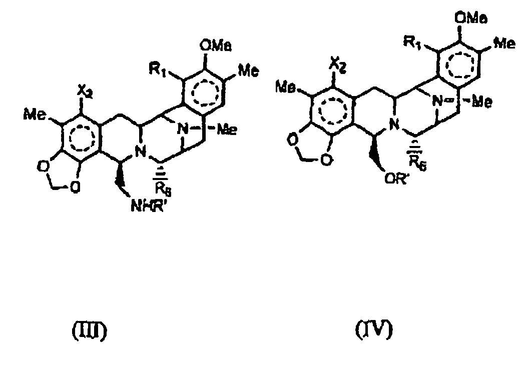 wherein R', X₂, R₁, and R₆ are each independently selected from the groups defined

| R' | X₂ | R₁ | R₆ |
|---|---|---|---|
| CH₂CH=CH₂ | OH | OH | CN |
| COCH₂CH₃ | OAc | OAc | |
| CO(CH₂)₄CH₃ CO(CH₂)₁₂CH₃ | OCOOCH₂CH=CH₂ OCOCF₃ | OCOCH₂C₆H₁₁ OCOCH₂CH₂C₆H₁₁ | |
| CO(CH₂)₁₆CH₃ | OCOCH₂Cl | OCOCH₂CH₂CH₃ | |
| COCH₂C₆H₁₁ | OCOCH₂CH₂Cl | OCO(CH₂)₄CH₃ | |
| COCH₂CH₂C₆H₁₁ | OCOCF₂CF₂CF₃ | OCO(CH₂)₈CH₃ | |
| | | OCO(CH₂)₁₆CH₃ | |
| COCH=CHCH₃ | | | |
| COCH(CH₃)NHCOCH₂CH₂Ph | | | |
| CO-(S)-CH(NHCbz)CH(CH₃)₂ | | | |
| 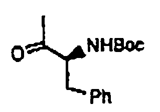 | | | |
| 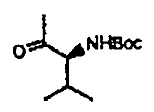 | | | |
| 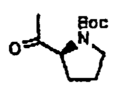 | | | |
| 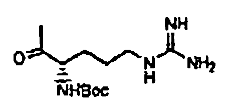 | | | |
| 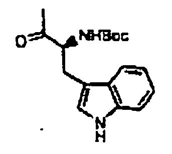 | | | |
| 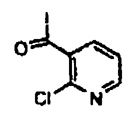 | | | |

Preferred embodiments also include a compound of a formula of the following general structure V: 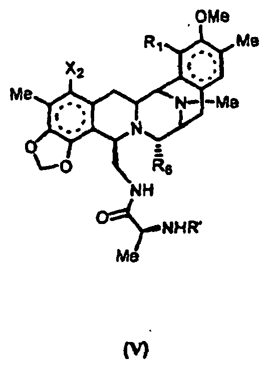 wherein X₂, R₁, R₆, and R' are each independently selected from the groups defined below:

| R' | X₂ | R₁ | R₆ |
|---|---|---|---|
| - | OH | OH | CN |
| CH₂CH=CH₂ | OAc | OAc | |
| COCH₂CH₃ | OCOOCH₂CH=CH₂ | OCOCH₂C₆H₁₁ | |
| CO(CH₂)₄CH₃ | OCOCF₃ | OCOCH₂CH₂C₆H₁₁ | |
| CO(CH₂)₁₂CH₃ | OCOCH₂Cl | OCOCH₂CH₂CH₃ | |
| CO(CH₂)₁₆CH₃ | OCOCH₂CH₂Cl | OCO(CH₂)₄CH₃ | |
| COCH₂C₆H₁₁ | OCOCF₂CF₂CF₃ | OCO(CH₂)₈CH₃ | |
| COCH₂CH₂C₆H₁₁ | | OCO(CH₂)₁₆CH₃ | |
| COCH₂Ph | | | |
| COCH₂CH₂Ph | | | |
| COCH=CHCH₃ | | | |
| COCH(CH₃)NHCOCH₂CH₂Ph | | | |
| CO-(S)-CH(CH₃)NHCOCF₃ | | | |
| CO-(R)-CH(CH₃)NHCOCF₃ | | | |
| CO-(S)-CH(NHCbz)CH(CH₃)₂ | | | |
| Boc | | | |
| 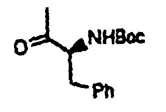 | | | |
| 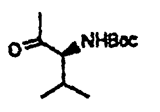 | | | |
| 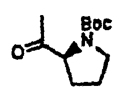 | | | |
| 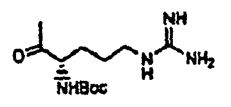 | | | |
| 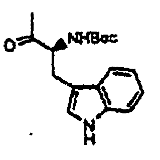 | | | |
| 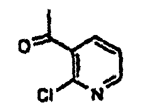 | | | |

In the compounds of this invention, R¹ is typically aminomethylene, amidomethylene or R¹ with R⁴ forms a group (IV) or (V). Suitable amidomethylene groups include those of formula -CH₂-NH-CO-CHCH₃-NH₂ derived from alanine, and similar groups derived from other amino acids, notably, both D and L, glycine, valine, leucine, isoleucine, phenylalanine, tyrosine, tryptophan, methionine, cysteine, aspartate, asparagine, glutamatic acid, glutamine, lysine, arginine, proline, serine, threonine, histidine and hydroxyproline. A general formula for the group R¹ is then -CH₂-NH -aa, where aa indicates an acyl amino acid group.

The group R¹ can be acylated on an -NH₂ group, and for example N-acyl derivatives can be formed from groups -CH₂NH₂ and -CH₂NH-aa. The acyl derivatives can be N-acyl or N-thioacyl derivatives thereof, as well as cyclic amides. The acyl groups can illustratively be alkanoyl, haloalkanoyl, arylalkanoyl, alkenoyl, heterocyclylacyl, aroyl, arylaroyl, haloaroyl, nitroaroyl, or other acyl groups. The acyl groups can be of formula - CO-R^{a}, where R^{a} can be various groups such as alkyl, alkoxy, alkylene, arylalkyl, arylalkylene, amino acid acyl, or heterocyclyl, each optionally substituted with halo, cyano, nitro, carboxyalkyl, alkoxy, aryl, aryloxy, heterocyclyl, heterocyclyloxy, alkyl, amino or substituted amino. Other acylating agents include isothiocyanates, such as aryl isothiocyanates, notably phenyl isocyanate. The alkyl, alkoxy or alkylene groups of R^{a} suitably have 1 to 6 or 12 carbon atoms, and can be linear, branched or cyclic. Aryl groups are typically phenyl, biphenyl or naphthyl. Heterocyclyl groups can be aromatic or partially or completely unsaturated and suitably have 4 to 8 ring atoms, more preferably 5 or 6 ring atoms, with one or more heteroatoms selected from nitrogen, sulphur and oxygen.

Without being exhaustive, typical R^{a} groups include alkyl, haloalkyl, alkoxyalkyl, haloalkoxyalkyl, arylalkylene, haloalkylarylakylene, acyl, haloacyl, arlyalkyl, alkenyl and amino acid. For example, R^{a}-CO- can be acetyl, trifluoroacetyl, 2,2,2-trichloroethoxycarbonyl, isovalerylcarbonyl, trans-3-(trifluoromethyl)cinnamoylcarbonyl, heptafluorobutyrylcarbonyl, decanoylcarbonyl, trans-cinnamoylcarbonyl, butyrylcarbonyl, 3-chloropropyonylcarbonyl, cinnamoylcarbonyl, 4-methylcinnamoylcarbonyl, hydrocinnamoylcarbonyl, or trans-hexenoylcarbonyl, or alanyl, arginyl, aspartyl, asparagyl, cystyl, glutamyl, glutaminyl, glycyl, histidyl, hydroxyprolyl., isoleucyl, leucyl, lysyl, methionyl, phenylalanyl, prolyl, seryl, threonyl, thyronyl, tryptophyl, tyrosyl, valyl, as well as other less common amino acid acyl groups, as well as phthalimido and other cyclic amides. Other examples may be found among the listed protecting groups. Compounds wherein -CO-R^{a} is derived from an amino acid and include an amino group can themselves form acyl derivatives. Suitable N-acyl commands include dipeptides which in turn can form N-acyl derivatives.

In an important aspect of this invention, there are provided preferred compounds of the formula: 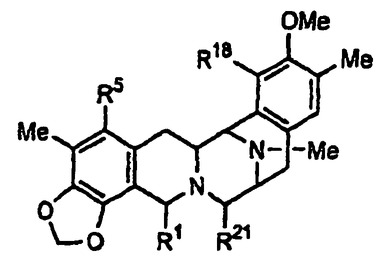 wherein:
R¹ is selected from the group consisting of -CH₂-NHR⁸ and -CH₂-OR^{a},
   where R^{a} is selected from the group consisting of alkyl-CO-; haloalkyl-CO-; haloalkyl-O-CO-; arylalkenyl-CO- wherein the aryl group is selected from phenyl, biphenyl and naphthyl; heteroaryl-CO- wherein the heteroaryl contains one, two or three heteroatoms selected from nitrogen, sulphur and oxygen; alkenyl-CO-; and amino acid acyl;
R⁵ is OH; acetyloxy or other acyloxy group up to 4 carbon atoms;
R¹⁸ is -OH; and
R²¹ is-CN.

In a further important aspect of this invention, there are provided preferred compounds of the formula : 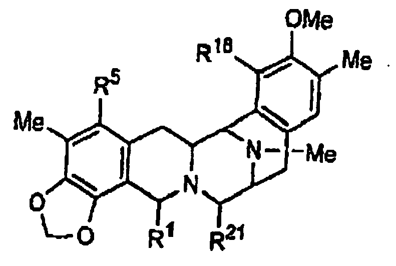 wherein:
R¹ is -CH₂-OR^{a},
   where R^{a} is selected from the group consisting of alkyl-CO-; haloalkyl-CO-; cycloalkylallryl-CO-; haloalkyl-O-CO-; arylalkenyl-CO- wherein the aryl group is selected from phenyl, biphenyl and naphthyl; heteroaryl-CO- where the heteroaryl contains one, two or three heteroatoms selected from nitrogen, sulphur and oxygen; alkenyl-CO-; alkenyl; and amino acid acyl;
R⁵ is -OR", where R" is selected from the group consisting of H; alkyl-CO-; cycloalkyl-CO-; and haloalkyl-CO-; R¹⁸ is -OR, where R is selected from the group consisting of H, alkyl-CO-; cycloalkylalkyl-CO-; and
R²¹ is -CN.

Typically such a compound of this invention is of the formula: 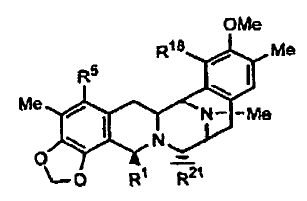 wherein R¹, R⁵, R¹⁸, and R²¹ are as defined.

In such preferred compounds of this invention, R¹ can be -CH₂-NHR^{a}.

R^{a} can be -aa-R^{b} where aa is amino acid acyl and R^{b} is as defined for R^{a}. The amino acid acyl is optionally further substituted with one or more R^{a} groups.

In further preferred compounds, R¹ is -CH₂-NH-aa-R^{b} where aa is an amino acid and R^{b} is hydrogen; protecting group; arylalkenyl-CO-; haloalkyl-CO-; alkyl-CO-; arylalkyl-CO-; or amino acid acyl, Such compounds include those wherein R¹ is -CH₂-NH-aa-R^{b} where aa is alanine and R⁶ is hydrogen, Boc, PhNHCS-, CF₃CO-, PhNAcCS-, trifluorocinnamoyl, cinnamoyl, C₃F₇CO-, butyryl, 3-chloroproprionoyl, hydrocinnamoyl, hexanoyl, phenylacetyl. Cbz-val or acetyl; -CH₂-aa-R^{b} where aa is valine and R^{b} is Cbz or Boc; -CH₂-aa-R^{b} where aa is phenylalanine and R^{b} is Boc; -CH₂-aa-R^{b} where aa is proline and R^{b} is Boc: -CH₂-aa-R^{b} where aa is arginine and R^{b} is Boc; or -CH₂-aa-R^{b} where aa is tryptophan and R^{b} is Boc.

R¹ can be -CH₂-NR^{a}-aa-R^{b} where aa is an amino acid, R^{a} is alkyl-CO- and R^{b} is haloalkyl-CO-, Such compounds include those wherein R¹ is -CH₂-NR^{a}-aa-R^{b} where aa is acetylalanine, R^{a} is acetyl or butyryl, and R^{b} is CF₃-CO-.

R¹ can be -CH₂-NHR^{a} where R^{a} is hydrogen, protecting group, alkyl-CO-; alkenyl-CO-; arylalkenyl-CO-; arylalkyl-CO-; heteroaryl-CO-; cycloalkylalkyl-CO-; or alkenyl. Such compounds include those wherein R¹ is -CH₂-NHR^{a} where R^{a} is hydrogen. Troc, acetyl; isovaleroyl, decanoyl, cinnamoyl, hydrocinnamoyl, phenylacetyl, propionyl, myristoyl, stearoyl, hexanoyl, crotonyl, chloronicotinoyl cyclohexylacetyl, cyclohexylpropionyl or allyl.

R¹ can be -CH₂-OR^{a} where R^{a} is hydrogen; a protected cysteine; a cysteine derivative of the formula Prot^{SH}-S-CH₂-C(NHProt^{NH})-CO-, where Prot^{SH} and Prot^{NH} are protecting groups for thiol and for amino; a protecting group; alkyl-CO-; arylalkyl-CO-; arylalkenyl-CO-;
a cysteine derivative of the formula Prot^{SH}-S-CH₂-C(=NOProt^{OH})-CO- where Prot^{SH} and prot^{OH} are protecting groups for thiol and for hydroxy; or a cysteine derivative of formula Prot^{SH}-S-CH=C(-OProt^{OH})-CO-, where Prot^{SH} and Prot^{OH} are protecting groups for thiol and for hydroxy. Such compounds include those wherein R¹ is -CH₂-OR^{a} where R² is hydrogen;
S-Fm-O-TBDMS-cysteines; a cysteine derivative of the formula Prot^{SH}-S-CH₂-C(NHProt^{NH})-CO., where Prot^{SH} is Fm and Prot^{OH} is Troc ; TBDPS; butyryl; trifluormethylcinnamoyl ; cinnamoyl; hydrocinnamoyl; a cysteine derivative of the formula Prot^{SH}-S-CH₂-C(=NOProt^{OH})-CO- where Prot^{SH} is Fm and Prot^{OH} is methoxy; or a cysteine derivative of formula Prot^{SH}-S-CH=C(-OProt^{OH})-CO-, where Prot^{SH} is Fm and Prot^{OH} is MOM.

In these preferred compounds, R⁵ is suitably -OR", where R" is H; alkyl-CO where the alkyl has an odd number of carbon atoms, ω-cyclohexylalkyl-CO-; or a protecting group.

In these preferred compounds, R¹⁸ is suitably -OR, where R is H, alkyl-CO-; or a protecting group;

In one variation which relates to intermediate products, the ring A is modified to incorporate the substructure shown as formula (XX) or (XXI), discussed later.

In another variation relating to intermediates, the group R¹ can be -CH₂O-CO-CFu-CH₂-S-Prot³, derived from a compound of formula (XIX), where Prot³ and Fu have the indicated meanings. In such a case, R⁷ and R⁸ from the oxymethyleneoxy group. The group R¹⁸ is usually protected. Usually R²¹ is cyano.

Preferably R¹⁵ is hydrogen.The O-acyl derivatives are suitably aliphatic O-acyl derivatives, especially acyl derivatives of 1 to 4 carbon atoms, and typically an O-acetyl group, notably at the 5-position.

Suitable protecting groups for phenols and hydroxy groups include ethers and esters, such as alkyl, alkoxyalkyl, aryloxyalkyl, alkoxyalkoxyalkyl, alkylsilylalkoxyalkyl, alkylthioalkyl, arylthioalkyl, azidoalkyl, cyanoalkyl, chloroalkyl, heterocyclic, arylacyl, haloarylacyl, cycloalkylalkyl, alkenyl, cycloalkyl, alyklarylalkyl, alkoxyarylalkyl, nitroarylalkyl, haloarylalkyl, alkylaminocarbonylarylalkyl, alkylsulfinylarylalky, alkylsilyl and other ethers, and arylacyl, aryl alkyl carbonate, aliphatic carbonate, alkylsulfinylarlyalkyl carbonate, alkyl carbonate, aryl haloalkyl carbonate, aryl alkenyl carbonate, aryl carbamate, alkyl phosphinyl, alkylphosphinothioyl, aryl phosphinothioyl, aryl alkyl sulphonate and other esters. Such groups may optionally be substituted with the previously mentioned groups in R¹.

Suitable protecting groups for amines include carbamates, amides, and other protecting groups, such as alkyl, arylalkyl, sulpho- or halo- arylalkyl, haloalkyl, alkylsilylalkyl, arylalkyl, cycloalkylalkyl, alkylarylalkyl, heterocyclylalkyl, nitroarylalkyl, acylaminoalkyl, nitroaryldithioarylalkyl, dicycloalkylcarboxamidoalkyl, cycloalkyl, alkenyl, arylalkenyl, nitroarylalkenyl, heterocyclylalkenyl, heterocyclyl, hydroxyheterocyclyl, alkyldithio, alkoxy- or halo- or alkylsulphinyl arylalkyl, hetercyclylacyl, and other carbamates, and alkanoyl, haloalkanoyl, arylalkanoyl, alkenoyl, heterocyclylacyl, aroyl, arylaroyl, haloaroyl, nitroaroyl, and other amides, as well as alkyl, alkenyl, alkylsilylalkoxyalkyl, alkoxyalkyl, cyanoalkyl, heterocyclyl, alkoxyarylalkyl, cycloalkyl, nitroaryl, arylalkyl, alkoxy- or hydroxy- arylalkyl, and many other groups. Such groups may optionally be substituted with the previously mentioned groups in R¹.

Examples of such protecting groups are given in the following tables. protection for -OH group

| ethers | abbreviation |
|---|---|
| methyl | |
| methoxymethyl | MOM |
| benzyloxymethyl | BOM |
| methoxyethoxymethyl | MEM |
| 2-(trimethylsilyl)ethoxymethyl | SEM |
| methylthiomethyl | MTM |
| phenylthiomethyl | PTM |
| azidomethyl | |
| cyanomethyl | |
| 2,2-dichloro-1,1-difluoroethyl | |
| 2-chloroethyl | |
| 2-bromoethyl | |
| tetrahydropyranyl | THP |
| 1-ethoxyethyl | EE |
| phenacyl | |
| 4-bromophenacyl | |
| cyclopropylmethyl | |
| allyl | |
| propargyl | |
| isopropyl | |
| cyclohexyl | |
| *t*-butyl | |
| benzyl | |
| 2,6-dimethylbenzyl | |
| 4-methoxybenzyl | MPM or PMB |
| *o*-nitrobenzyl | |
| 2,6-dichlorobenzyl | |
| 3,4-dichlorobenzyl | |
| 4-(dimethylamino)carbonylbenzyl | |
| 4-methylsuflinylbenzyl | Msib |
| 9-anthrylmethyl | |
| 4-picolyl | |
| heptafluoro-*p*-tolyl | |
| tetrafluoro-4-pyridyl | |
| trimethylsilyl | TMS |
| *t*-butyldimethylsilyl | TBDMS |
| *t*-butyldiphenylsilyl | TBDPS |
| triisopropylsilyl | TIPS |
| esters | |
| aryl formate | |
| aryl acetate | |
| aryl levulinate | |
| aryl pivaloate | ArOPv |
| aryl benzoate | |
| aryl 9-fluorocarboxylate | |
| aryl methyl carbonate | |
| 1-adamantyl carbonate | |
| *t*-butyl carbonate | BOC-OAr |
| 4-methylsulfinylbenzyl carbonate | Msz-Oar |
| 2,4-dimethylpent-3-yl carbonate | Doc-Oar |
| aryl 2,2,2-trichloroethyl carbonate | |
| aryl vinyl carbonate | |
| aryl benzyl carbonate | |
| aryl carbamate | |
| dimethylphosphinyl | Dmp-OAr |
| dimethylphosphinothioyl | Mpt-OAr |
| diphenylphosphinothioyl | Dpt-Oar |
| aryl methanesulfonate | |
| aryl toluenesulfonate | |
| aryl 2-formylbenzenesulfonate | |
| protection for the -NH₂ group | |
| carbamates | abbreviation |
| methyl | |
| ethyl | |
| 9-fluomnylmethyl | Fmoc |
| 9-(2-sulfo)fluroenylmethyl | |
| 9-(2,7-dibromo)fluorenylmethyl | |
| 17-tetrabenzo[a,c,g,i]fluorenylmethyl | Tbfmoc |
| 2-chloro-3-indenylmethyl | Climoc |
| benz[*f*]inden-3-ylmethyl | Bimoc |
| 2,7-di-t-butyl[9-(10,10-dioxo-10,10,10,10-tetrahydrothioxanthyl)]methyl | DBD-Tmoc |
| 2,2,2-trichloroethyl | Troc |
| 2-trimethylsilylethyl | Teoc |
| 2-phenylethyl | hZ |
| 1-(1-adamantyl)-l-methylethyl | Adpoc |
| 2-chlooethyl | |
| 1,1-dimethyl-2-chloroethyl | |
| 1,1-dimethyl-2-bromoethyl | |
| 1,1-dimethyl-2,2-dibromoethyl | DB-*t*-BOC |
| 1,1-dimethyl-2,2,2-trichlomethyl | TCBOC |
| 1-methyl-1-(4-biphenyl)ethyl | Bpoc |
| 1-(3,5-di-*t*-butylphenyl)- 1-methylethyl | *t*-Burmeoc |
| 2-(2'-and 4'-pyridyl)ethyl | Pyoc |
| 2,2-bis(4'-nitrophenyl)ethyl | Bnpeoc |
| *n*-(2-pivaloylamino)-1,1-dimethylethyl | |
| 2-[(2-nitrophenyl)dithio]-1-phenylethyl | NpSSPeoc |
| 2-(*n,n*-dicyclohexylcarboxamido)ethyl | |
| *t*-butyl | BOC |
| 1-adamantyl | 1-Adoc |
| 2-adamantyl | 2-Adoc |
| vinyl | Voc |
| allyl | Aloc or Alloc |
| 1-isopropylallyl | Ipaoc |
| cinnamyl | Coc |
| 4-nitrocinnamyl | Noc |
| 3-(3'-pyridyl)prop-2-enyl | Paloc |
| 8-quinolyl | |
| n-hydroxypiperidinyl | |
| alkyldithio | |
| benzyl | Cbz or Z |
| *p*-methoxybenzyl | Moz |
| *p*-nitrobenzyl | PNZ |
| *p*-bromobenzyl | |
| *p*-chlorobenzyl | |
| 2,4-dichlorobenzyl | |
| 4-methylsulfinylbenzyl | Msz |
| 9-anthrylmethyl | |
| diphenylmethyl | |
| phenothiazinyl-(10)-carbonyl | |
| *n*'*-p*-toluenesulfonylaminocarbonyl | |
| *n*'-phenylaminothiocarbonyl | |
| amides | |
| formamide | |
| acetamide | |
| chloroacetamide | |
| trifluoroacetamide | TFA |
| phenyl acetamide | |
| 3-phenylpropanamide | |
| pent-4-enamide | |
| picolinamide | |
| 3-pyridylcarboxamide | |
| benzamide | |
| *p-*phenylbenzamide | |
| *n*-phthalimide | |
| *n*-tetrachlorophthalimide | TCP |
| 4-nitro-*n*-phthalimide | |
| *n*-dithiasuccinimide | Dts |
| *n*-2,3-diphenylmaleimide | |
| *n*-2,5-dimethylpyrrole | |
| *n*-2,5-bis(triisopropylsiloxyl)pyrrole | BIPSOP |
| *n*-1,1,4,4-tetramethyldisiliazacyclopentante adduct | STABASE |
| 1,1,3,3-tetramethyl-1,3-disilaisoindoline | BSB |
| special -NH protective groups | |
| *n*-methylamine | |
| *n*-*t*-butylamine | |
| *n*-allylamine | |
| *n*-[2-trimethylsilyl)ethoxy]methylamine | SEM |
| *n*-3-acetoxypropylamine | |
| *n*-cyanomethylamine | |
| *n*-(1-isopropyl-4-nitro-2-oxo-3-pyrrolin-3-yl)amine *n*-2,4-dimethoxybenrylamine | Dmb |
| 2-azanorbomenes | |
| *n*-2,4-dinitrophenylamine | |
| n-benzylamine | Bn |
| n-4-methoxybenzylamine | MPM |
| n-2,4-dimethoxybenzylamine | DMPM |
| *n*-2-hydroxybenrylamine | Hbn |
| n-(diphenylmethyl)amino | DPM |
| *n*-bis(4-methoxyphenyl)methylamine | |
| *n*-5-dibenzosuberylamine | DBS |
| *n*-triphenylmethylamine | Tr |
| *n*-[(4-methoxyphenyl)diphenylmethyl]amino | MMTr |
| *n*-9-phenylflurenytamine | Pf |
| *n*-ferrocenylmethylamine | Fcm |
| *n*-2-picolylamine *n'*-oxide | |
| *n*-1,1-dimethylthiomethyleneamine | |
| *n*-benzylideneamine | |
| *n-p*-methoxybenzylideneamine | |
| *n*-diphenylmethyleneamine | |
| *n*-(5,5-dimethyl-3-oxo-1-cyclohexenyl)amine | |
| *n*-nitroamine | |
| *n*-nitrosoamine | |
| diphenylphosphinamide | Dpp |
| dimethylthiophosphinamide | Mpt |
| diphenylthiophosphinamide | Ppt |
| dibenzyl phosphoramidate | |
| 2-nitrobenzenesulfenamide | Nps |
| *n*-1-(2,2,2-trifluoro-1,1-diphenyl)ethylsufenamide | TDE |
| 3-nitro-2-pyndinesulfenamide | Npys |
| *p*-toluenesulfonamide | Ts |
| benzenesulfonamide | |

Examples of preferred methods of this invention will firstly be considered with reference to starting compunds 45, 43 and 25. It will be appreicated that the particular substituents, notably at positions C-5 and C-18, can be varied in the light of the present disclosure.

The preferred methods of producing the compounds of formula I, II, III, IV and V are described below in the following reaction schemes with examples of typical substituent groups.

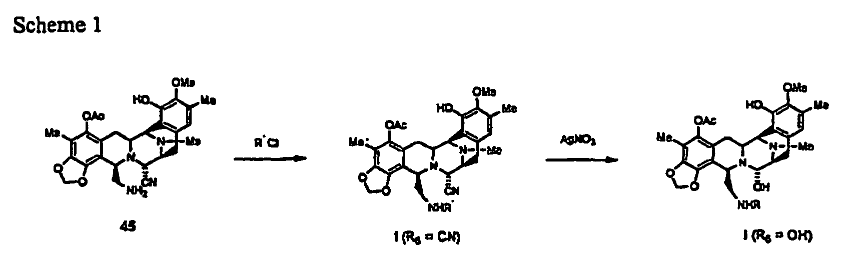

As illustrated in Scheme I the first step for producing the preferred compounds (I) (where R₁ = OH, X₂ = OAc and R₆ = CN or OH) of the present invention from compound 45 is the high yielding conversion of the amino group to the amide group.

After acylation of the amino group the second step is the transformation of the CN group into an OH group by reaction with silver nitrate in AcCN/H₂O.

The preparation of other compounds of the general formula I of the present invention starting from compound 17 is described below (Scheme 4).

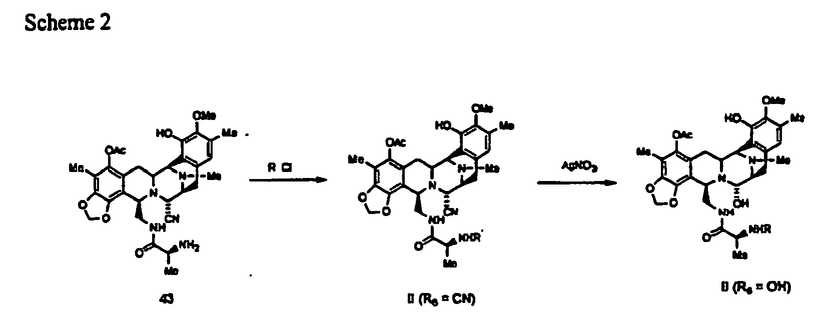

As illustrated in Scheme 2 another group of interesting derivatives with formula II (where R₁ = OH, X₂ = OAc and R₆ = CN or OH) can be obtained from compound 43 using the following sequence. Acylation of the amino group to provide the corresponding amide and transformation of the CN group into an OH group by reaction with silver nitrate in AcCN/H2O.

The preparation of other compounds of the general formula II of the present invention starting from compound 17 is described below (Scheme 4).

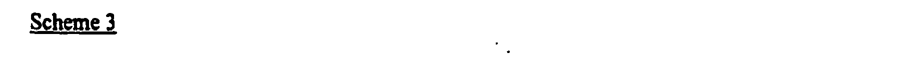

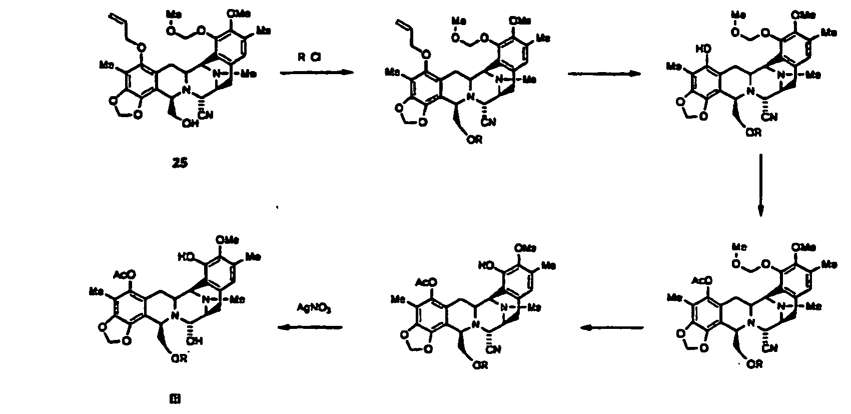

The preferred procedure for producing compounds of formula III is the transformation of compound 25 into the corresponding ester derivatives by acylation of the OH group, deprotection of the phenol group followed by acetylation and deprotection of the MOM group to provide the corresponding ester followed by transformation of the CN group to the OH group by reaction with silver nitrate in AcCN/H₂O to give the compound of formula III (where R₁ = OH, X₂ = OAc and R₆ = CN or OH).

Other compounds of the general formulae I and II of the present invention can be prepared from compound 17 via the amine intermediate 120 as described in Scheme 4.

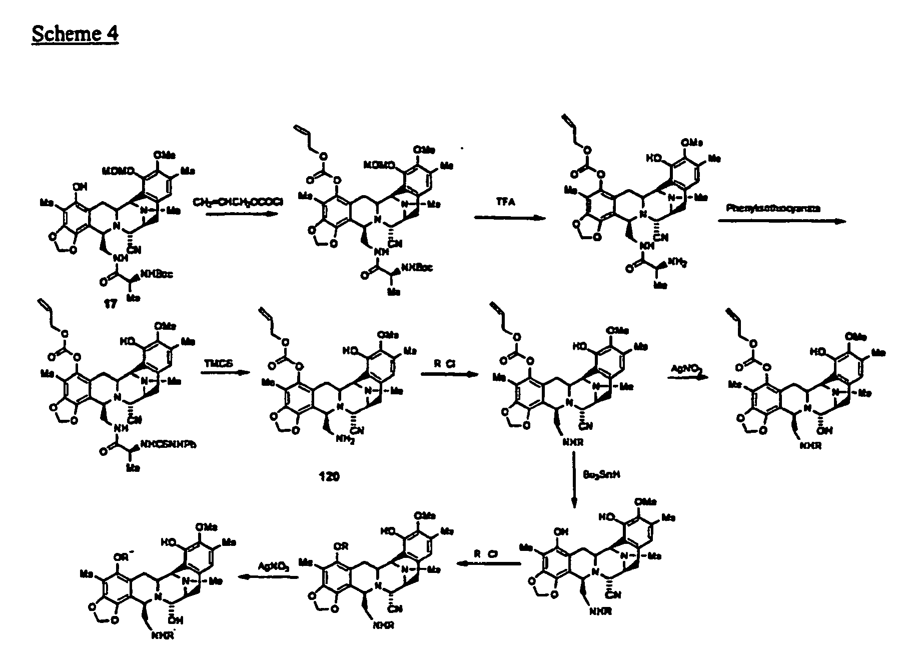

The following additional compounds of the present invention (including for example 140 and 141) have been prepared starting from cyanosafracin B (2) as described in detail in the examples (Scheme 5).

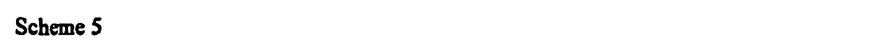

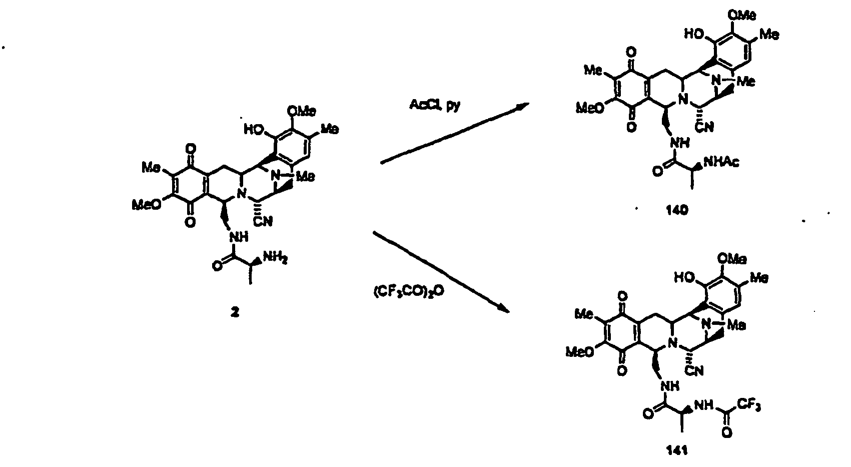

As the skilled artisan will readily appreciate, the reaction schemes described herein may be modified and/or combined in various ways, and the compounds generated therefore are to be considered as being part of this invention. In particular the starting material and/or reagents and reactions can be varied to suit other combinations of the substituent groups in the formulae I, II and III.

In a related aspect, a known compound, safracin B, also referred to as quinonamine, is used in hemisynthetic synthesis to give the present compound

More generally, such hemisynthetic process permits the formation of intermediates, derivatives and related structures of ecteinascidin or other tetrahydroisoquinolinephenol compounds starting from natural bis(tetrahydroisoquinoline) alkaloids. Suitable starting materials for the hemi-synthetic process include the classes of saframycin and safracin antibiotics available from different culture broths, and also the classes of reineramicin and xestomycin compounds available from marine sponges.

A general formula (XV) for the starting compounds is as follows: 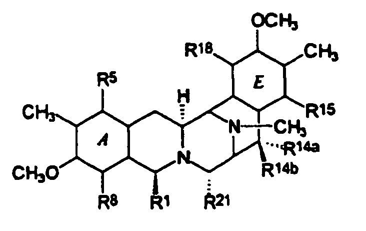 where:
R¹ is an amidomethylene group such as -CH₂-NH-CO-CR^{25a}R^{25b}R^{25c} where R^{25a} and R^{25b} form a keto group or one is -OH, -NH₂ or -OCOCH₃ and the other is -CH₂COCH₃, -H, -OH or -OCOCH₃, provided that when R^{25a} is -OH or -NH₂ then R^{25b} is not -OH, and R^{25c} is -H, - CH₃ or -CH₂CH₃, or R¹ is an acyloxymethylene group such as -CH₂-O-CO-R, where R is - C(CH₃)=CH-CH₃ or -CH₃;
R⁵ and R⁸ are independently chosen from -H, -OH or -OCOCH₂OH, or R⁵ and R⁸ are both keto and the ring *A* is a p-benzoquinone ring;
R^{14a} and R^{14b} are both -H or one is -H and the other is -OH, -OCH₃ or -OCH₂CH₃, or R^{14a} and R^{14b} together form a keto group;
R¹⁵ and R¹⁸ are independently chosen from -H or -OH, or R⁵ and R⁸ are both keto and the ring *A* is a p-benzoquinone ring; and
R²¹ is -OH or -CN.

A more general formula for these class of compounds is provided below: 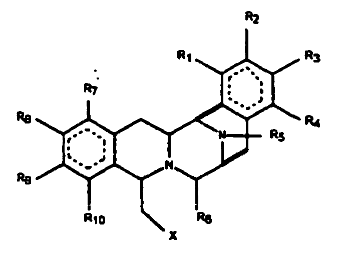 wherein the substituent groups defined by R₁, R₂, R₃, R₄, R₅, R₆, R₇, R₈, R₉, R₁₀ are each independently selected from the group consisting of H, OH, OCH₃, CN, =O, CH₃ ;
wherein X are the different amide or ester functionalities contained in the mentioned natural products;
wherein each dotted circle represents one, two or three optional double bonds.

Thus, for the compounds according to the present invention, we now describe hemisynthetic routes for the production of intermediates including Intermediate 11 and thus for the production of the ecteinascidin compounds as well as phthalascidin and additional compounds. The hemisynthetic routes each comprise a number of transformation steps to arrive at the desired product. The invention is not limited to the compounds made by the routes that are exemplified, and alternative routes may be provided by, for example, changing the order of the transformation steps, as appropriate.

In particular, this hemisynthesis involves the provision of a 21-cyano starting material of general formula (XVI): 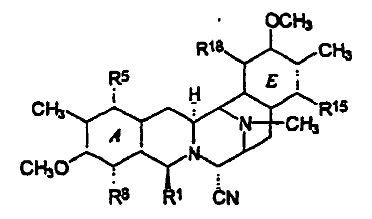 where R¹, R⁵, R⁸, R^{14a} R^{14b}, R¹⁵ and R¹⁸ are as defined. Other compounds of formula (XVI) with different substituents at the 21-position may also represent possible starting materials. In general, any derivative capable of production by nucleophilic displacement of the 21-hydroxy group of compounds of formula (XV) wherein R²¹ is a hydroxy group cis a candidate. Examples of suitable 21-substituents include but are not limited to:
a mercapto group;
an alkylthio group (the alkyl group having from 1 to 6 carbon atoms);
an arylthio group (the aryl group having from 6 to 10 carbon atoms and being unsubstituted or substituted by from 1 to 5 substituents selected from, for example, alkyl group having from 1 to 6 carbon atoms, alkoxy groups having from 1 to 6 carbon atoms, halogen atoms, mercapto groups and nitro groups);
an amino group;
a mono-or dialkylamino (the or each alkyl group having from 1 to 6 carbon atoms):
a mono-or diarylamino group (the or each aryl group being as defined above in relation to arylthio groups);
an α-carbonylalkyl group of formula -C(R^{a})(R^{b})-C(=O)R^{c}, where
R^{a} and R^{b} are selected from hydrogen atoms, alkyl groups having from 1 to 20 carbon atoms, aryl groups (as defined above in relation to arylthio groups) and aralkyl groups (in which an alkyl group having from 1 to 4 carbon atoms is substituted by an aryl group a defined above in relation to arylthio groups), with the proviso that one of R^{a} and R^{b} is a hydrogen atom;
R^{c} is selected from a hydrogen atom, an alkyl group having from 1 to 20 carbon atoms, aryl groups (as defined above in relation to arylthio groups), an aralkyl group (in which an alkyl group having from 1 to 4 carbon atoms is substituted by an aryl group a defined above in relation to arylthio groups), an alkoxy group having from 1 to 6 carbon atoms, an amino group or a mono- or dialkylamino group as defined above.

Thus, in a more general aspect, the hemisynthesis relates to processes where the first step is to form a 21 derivative using a nucleophilic reagent. We refer to such compounds as 21-Nuc compounds.

The presence of the 21-cyano group is required for some of the end-products, notably ecteinascidin 770 and phthalascidin, while for other end-products it acts as a protecting group which can readily be converted to another substituent, such as the 21-bydroxy group of octeinascidin 743 or of 21-hydroxyphthalascidin. The adoption of the 21-cyano compound as the starting material effectively stabilises the molecule during the ensuing synthetic steps, until it is optionally removed. Other 21-Nuc compounds can offer this and other advantages.

In one important aspect, the present invention consists in the use of a 21-cyano compound of the general formula (XVI) in the preparation of a bis- or tris-(tetrahydroisoquinolinephenol) compounds. Products which may be prepared include intermediates such as Intermediate 11, and the ecteinascidins and phthalascidin, as well as new and known compounds of related structure.

Preferred starting materials include those compounds of formula (XV) or (XVI) where R^{14a} and R^{14b} are both hydrogen. Preferred starting materials also include compounds of formula (XV) or (XVI) where R¹⁵ is hydrogen. Furthermore, the preferred starting materials include compounds of formula (XV) or (XVI) where ring *E* is a phenolic ring. Preferred starting materials further include compounds of formula (XV) or (XVI) where at least one, better at least two or three of R⁵, R⁸, R¹⁵ and R¹⁸ is not hydrogen.

Examples of suitable starting materials for this invention include saframycin A, saframycin B, saframycin C, saframycin G, saframycin H, saframycin S, saframycin Y₃, saframycin Yd₁, saframycin Ad₁, saframycin Yd₂, saframycin AH₂, saframycin AH₂Ac, saframycin AH₁, saframycin AH₁Ac, saframycin AR₃, renieramycin A, renieramycin B, renieramycin C, renieramycin D. renieramycin E, renieramycin F, xestomycin, saframycin D, saframycin F, saframycin Mx-1, saframycin Mx-2, safracin A, safracin B and saframycin R. Preferred starting materials have a cyano group in position 21, for the group R²¹.

In a particularly preferred aspect, for the hemisynthetic process wherein the transformation steps are applied to safracin B:

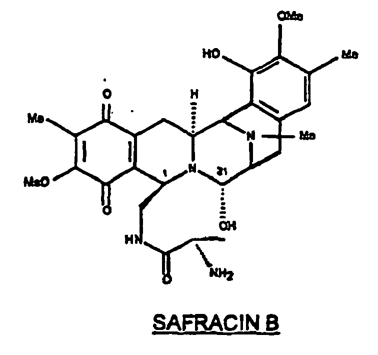

Safracin B presents a ring system closely related to the ecteinascidins. This compound has the same pentacycle structure and the same substitution pattern in the righthand aromatic ring, ring *E*. Also, safracin B presents very close similarities to some of the synthetic intermediates in the total synthesis of ET-743, particularly to the intermediate 11. Such intermediate can be transformed into Et-743 using a well established method. Synthetic conversion of safracin B into intermediate 11 will therefore provide an hemisynthetic method to obtain ET-743.

Intermediate 11 can be made from this compound safracin B, and compounds derived from Intermediate 11, particularly ecteinascidin compounds. We further provide phthalascidin made from safracin B. The invention also relates to use of safracin B in the production of Intermediate 11, phthalascidin, ecteinascidin compounds and the other intermediates of the invention. The invention also relates to compounds described herein derived from the other suggested starting materials, and use of those compounds in the production of such compounds.

The more preferred starting materials of this invention have a 21-cyano group. The currently most preferred compound of the present invention is the compound of Formula **2**. This compound is obtained directly from safracin B and is considered a key intermediate in the hemisynthetic process.

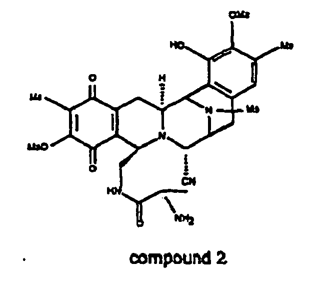

In a related aspect, we provide cyanosafracin B by fermentation of a safracin B-producing strain of *Pseudomonas fluorescens,* and working up the cultured broth using cyanide ion. The preferred strain of *Pseudomonas fluorescens* is strain A2-2, FERM BP-14, which is employed in the procedure of EP 055,299. A suitable source of cyanide ion is potassium cyanide. In a typical work-up, the broth is filtered and excess cyanide ion is added. After an appropriate interval of agitation, such as 1 hour, the pH is rendered alkaline, say pH 9.5, and an organic extraction gives a crude extract which can be further purified to give the cyanosafracin B.

Safracin B includes an alanyl sidechain. In one aspect of the invention, we have found that protection of the free amino group with a Boc group can give strong advantages.

In general, the conversion of the 21-cyano starting compound to an ecteinascidin analog of this invention an be carried out in accordance with our copending PCT patent application published as WO 0187895, which also claims priority from the PCT filing published as WO 0069862 published 23 November 2000, and which relates to hemisynthetic methods and new compounds.

Typically the hemisynthesis of an analog of this invention involves:
a) conversion if necessary of a quinone system for the ring *E* into the phenol system
b) conversion if necessary of a quinone system for the ring *A* into the phenol system;
c) conversion of the phenol system for the ring *A* into the methylenedioxyphenol ring; and
d) derivatisation as appropriate, such as acylation.

Step (a), conversion if necessary of a quinone system for the ring *E* into the phenol system, can be effected by conventional reduction procedures. A suitable reagent system is hydrogen with a palladium-carbon catalyst, though other reducing systems can be employed.

Step (b), conversion if necessary of a quinone system for the ring *A* into the phenol system is analogous to step (a), and more detail is not needed.

Step (c), conversion of the phenol system for the ring *A* into the methylenedioxyphenol ring, can be effected in several ways, possibly along with step (b). For example, a quinone ring A can be demethylated in the methoxy substituent at the 7-position and reduced to a dihydroquinone and trapped with a suitable electrophilic reagent such as CH₂Br₂, BrCH₂Cl, or a similar divalent reagent directly yielding the methylenedioxy ring system, or with a divalent reagent such as thiocarbonyldiimidazol which yields a substituted methylenedioxy ring system which can be converted to the desired ring.

Derivatisation in step (d) can include acylation, for instance with a group R^{a}-CO- as well as conversion of the 12-NCH₃ group to 12-NH or 12-NCH₂CH₃. Such conversion can be effected before or after the other steps, using available methods.

By way of illustration, it is now feasible to transform cyanosafracin B in a shorter and more straightforward way to make new analogs. Cyanosafracin B can be transformed into Intermediate **25;** 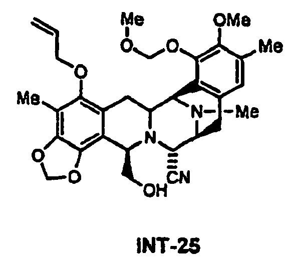 and from this derivative it is possible to introduce further analogs of this invention.

One method transforms cyanosafracin B into intermediate **25** through a sequence of reactions that involves essentially (1) removal of methoxy group placed in ring A, (2) reduction of ring A and formation of methylene-dioxy group in one pot, (3) hydrolysis of amide function placed over carbon 1, (4) transformation of the resulting amine group into hydroxyl group.

The conversion of the **2**-cyano compound into Intermediate **25** usually involves the following steps (see scheme II):
formation of the protected compound of Formula **14** by reacting **2** with *tert*-butoxycarbonyl anhydride;
converting of **14** into the di-protected compound of Formula **15** by reacting with bromomethylmethyl ether and diisopropylethylamine in acetonitrile;
selectively elimination of the methoxy group of the quinone system in **15** to obtain the compound of Formula **16** by reacting with a methanolic solution of sodium hydroxide;
transforming of **16** into the methylene-dioxy compound of Formula **18** by employing the next preferred sequence: (1) quinone group of compound **16** is reduced with 10% Pd/C under hydrogen atmosphere; (2) the hydroquinone intermediate is converted into the methylenedioxy compound of Formula 17 by reacting with bromochloromethane and caesium carbonate under hydrogen atmosphere; (3) **17** is transformed into the compound of Formula **18** by protecting the free hydroxyl group as a OCH₂R group. This reaction is carried out with BrCH₂R and caesium carbonate, where R can be aryl, CH=CH₂, OR' etc.
elimination of the *tert*-butoxycarbonyl and the methyloxymethyl protecting groups of **18** to afford the compound of Formula **19** by reacting with a solution of HCl in dioxane. Also this reaction is achieved by mixing **18** with a solution of trifluoroacetic acid in dichloromethane;
formation of the thiourea compound of Formula **20** by reacting **19** with phenylisothiocyanate;
converting compound of Formula **20** into the amine compound of Formula **21** by reacting with a solution of hydrogen chloride in dioxane;
transforming compound of Formula **21** into the *N*-Troc derivative **22** by reacting with trichloroethyl chloroformate and pyridine;
formation of the protected hydroxy compound of Formula **23** by reacting **22** with bromomethylmethyl ether and diisopropylethylamine;
transforming compound of Formula **23** into the *N*-H derivative **24** by reacting with acetic acid and zinc;
conversion of compound of Formula **24** into the hydroxy compound of Formula **25** by reaction with sodium nitrite in acetic acid. Alternatively, it can be used nitrogen tetroxide in a mixture of acetic acid and acetonitrile followed by treatment with sodium hydroxide.
Also, it can be used sodium nitrite in a mixture of acetic anhydride-acetic acid, followed by treatment with sodium hydroxide.

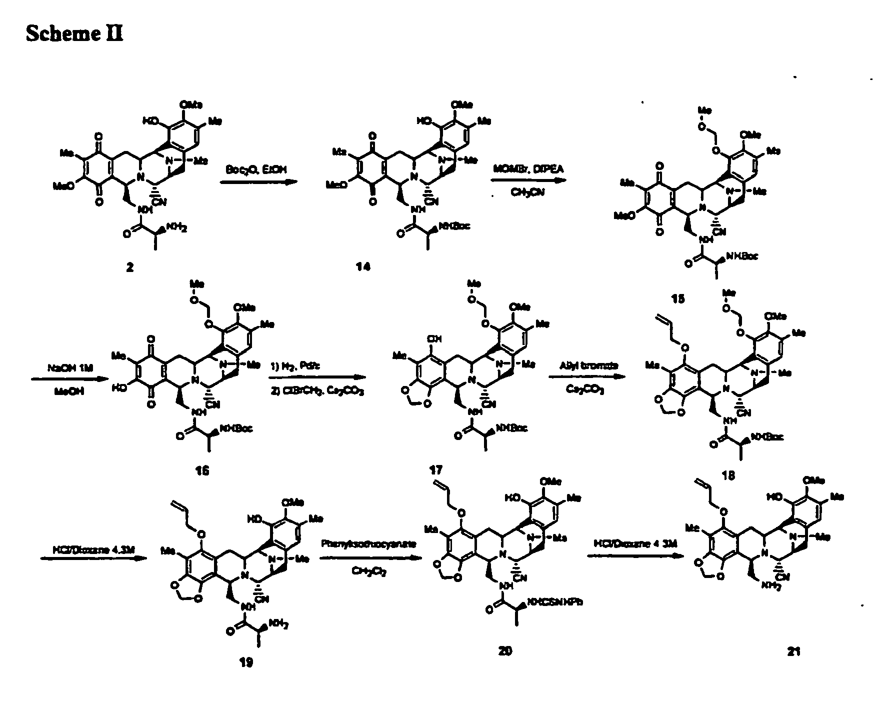

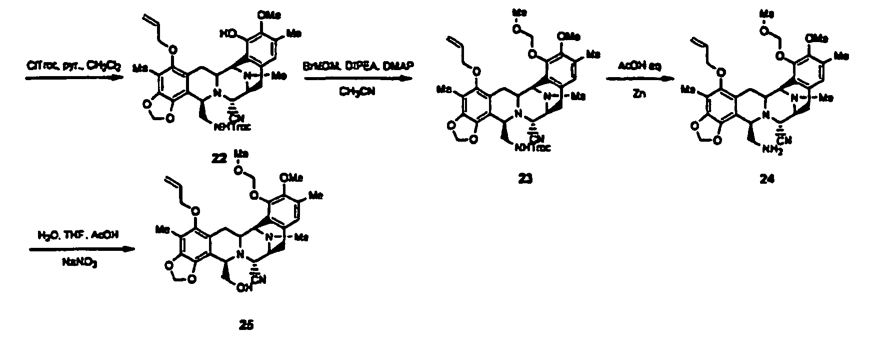

The conversion of the Intermediate **25** compound into other analogs of this invention is then readily achieved, as illustrated for example in Scheme III, which usually involves the following steps:
transforming compound of formula **24** into the derivative **30** by protecting the primary hydroxyl function with (S)-N-2,2.2-tricloroethoxycarbonyl-S-(9H-fluoren-9-ylmethyl)cysteine **29**;
converting the protected compound of formula **30** into the phenol derivative **31** by cleavage of the allyl group with tributyltin hydride and dichloropalladium-bis (triphenylphosphine). transforming the phenol compound of Formula **31** into compound of formula **32** by oxidation with benzeneseleninic anhydride at low temperature;

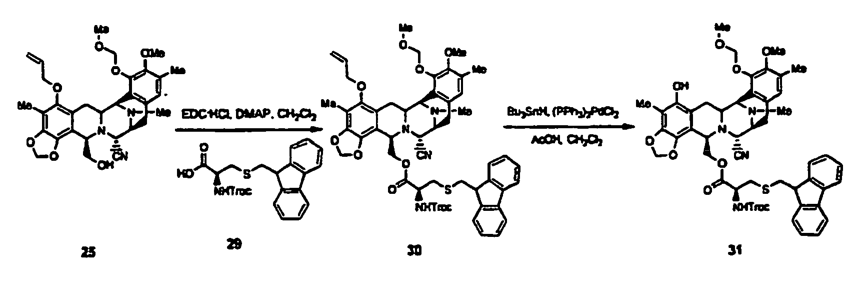

The route described above to transform Intermediate **25** can be conveniently modified to form other derivatives.

In more detail, the conversion of the starting 21-cyano compound to a related product of this invention, such as one of formula (XX), usually involves the following steps:
a) conversion if necessary of a quinone system for the ring *E* into the phenol system
b) formation of the -R⁵ group at the 5-position in ring *A*;
c) formation of the R¹ group at the 1-position in ring *B*; and
d) conversion if necessary of a quinone system for the ring *A* into the phenol system;
e) conversion of the phenol system for the ring *A* into the methylenedioxyphenol ring.

These steps have many similarities with the steps given previously. Step (c) typically involves forming a group -CH₂NH₂ at the 1-position and acylating it.

Phthlascidin can be made using Intermediates described in the conversion of cyanosafracin B into Intermediate **25**. For example, Intermediates **21** and **17** are suitable starting materials to make Phthlascidin and other analogs of this invention.

As shown in scheme V, the process for the synthetic formation of phthlascidin starting from Intermediate **21** comprises the sequential steps of:
transforming of **21** into the compound of Formula **27** by reaction with phthalic anhydride in dichloromethane and carbonyldiimidazole.
converting of **27** into phthlascidin by reacting with tributyltin hydride and dichloro palladium-bis(triphenylphosphine) or basic media, followed by reaction with acetyl chloride.

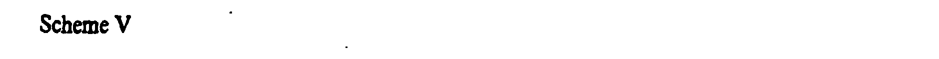

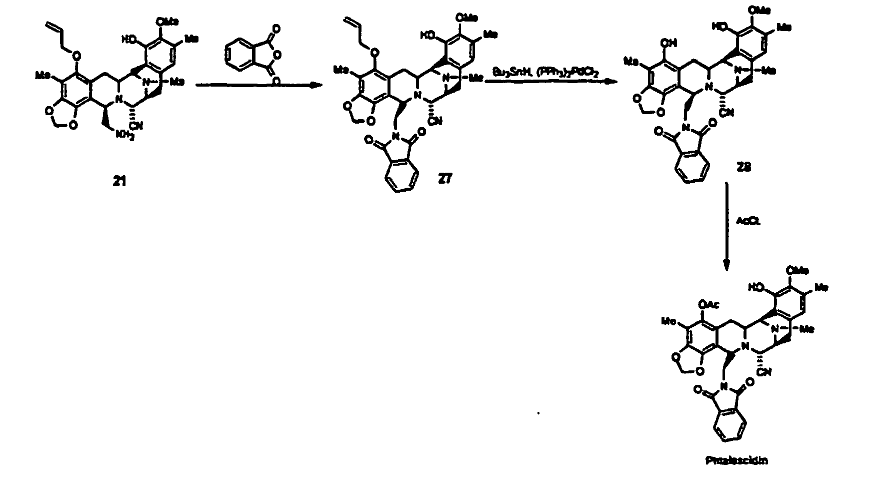

As shown in scheme VI, the process for the synthetic formation of phthlascidin starting from Intermediate **17** comprises the sequential steps of:
acetylation of the hydroxyl group of compound of formula **17** with acetyl chloride and pyridine to give the acetylated intermediate compound of formula **42;**
removal of the *tert*-butoxycarbonyl and the methyloxymethyl protecting groups of **42** to afford the compound of Formula **43** by reacting with a solution of HCl in dioxane. Also this reaction is achieved by mixing **42** with a solution of trifluoroacetic acid in dichloromethane;
formation of the thiourea compound of Formula **44** by reacting **43** with phenylisothiocyanate;
converting compound of Formula **44** into the amine compound of Formula **45** by reacting with a solution of hydrogen chloride in dioxane;
transforming of **45** into phthlascidin by reaction with phthalic anhydride in dichloromethane and carbonyldiimidazole.

Other analogs can be made for example from **43** or **45** by a similar manner.

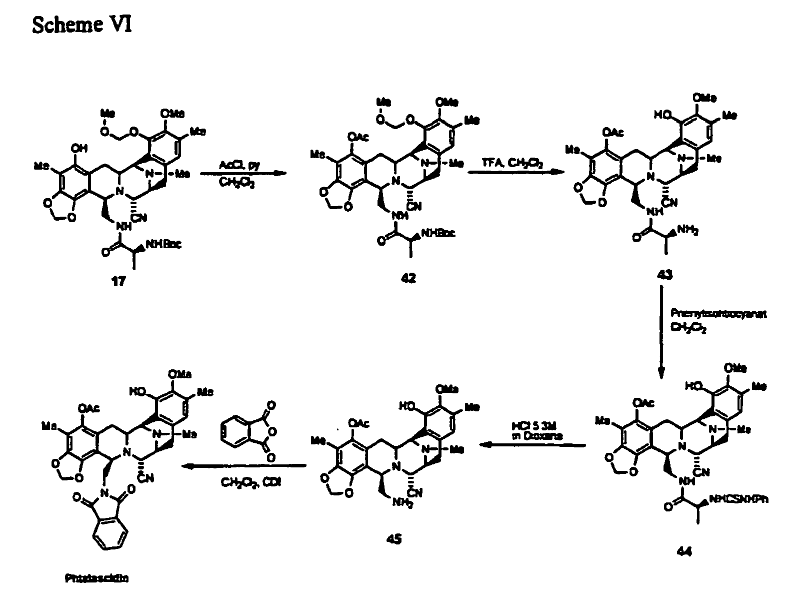

The conversion of the 21-cyano compound to Intermediate 11 or a related intermediate of formula (XXI) usually involves the following steps:
a) conversion if necessary of a quinone system for the ring *E* into the phenol system
b) formation of the -OProt¹ group at the 18-position, in ring *E;*
c) formation of the -CH₂-OProt² group at the 1-position, in ring *B;* and
d) conversion if necessary of a quinone system for the ring *A* into the phenol system;
e) conversion of the phenol system for the ring *A* into the methylenedioxyphenol ring.

Step (b), formation of the -OProt¹ group at the 18-position in ring *E*, is a typical protection reaction for a phenol group, and no special comments need to be made. Appropriate conditions are chosen depending on the nature of the protecting group. The other steps are similar to the other reactions.

Step (b), formation of the -CH₂-OProt² group at the 1-position in ring *B*, is normally carried out by forming a group -CH₂NH₂ at the 1-position and then converting the amine function to a hydroxy function and protecting. Thus, where the starting material has a group R¹ which is -CH₂NH-CO-CR^{25a}R^{25b}R^{25c} then it is matter of removing the N-acyl group. Where the starting material has a group R¹ which is -CH₂-O-CO-R then no change may be needed for an ecteinascidin product where the substituent R¹ is the same. For other products, it is matter of removing the O-acyl group. Various procedures are available for such de-acylations. In one variation, the deacylation and conversion to a hydroxy function are performed in one step. Thereafter, the hydroxy group can be acylated or otherwise converted to give the appropriate R¹ group.

U.S. Patent N° 5,721,362 describe synthetic methods to make ET-743 through a long multistep synthesis. One of the Intermediates of this synthesis is Intermediate 11. Using cyanosafracin B as starting material it is possible to reach Intermediate 11 providing a much shorter way to make such Intermediate and therefor improving the method to make ET-743

Cyanosafracin B can be converted into Intermediate **25** by the methods described above. From Intermediate **25** is possible to reach Intermediate 11 using the following steps, see scheme VII.
formation of the protected hydroxy compound of Formula **26** by reacting **25** with *tert-*butyldiphenylsilyl chloride in the presence of a base;
final cleavage of the allyl group with tributyltin hydride and dichloropalladium-bis (triphenylphosphine) in **26** that leads to the formation of the intermediate **11.**

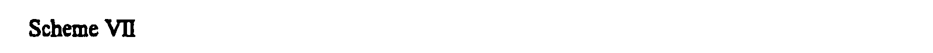

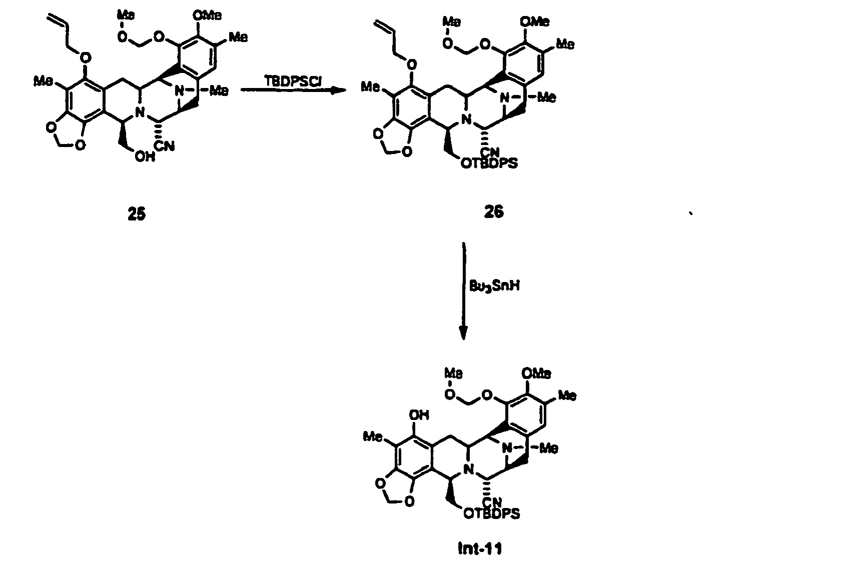

One embodiment of the synthetic process of the present invention to transform safracin B into intermediate **11** is a modification and extension of Scheme VIII and comprises the sequential steps of:
stereospecifically converting the compound Safracin B to the compound of Formula **2** by selective replacement of OH by CN by reacting with KCN in acid media;
forming the thiourea compound of Formula **3** by reacting compound of Formula **2** with phenyl isothiocyanate;
converting the thiourea compound of Formula **3** into the acetamide of Formula **5** by an hydrolysis in acid media followed by addition of acetic anhydride; The intermediate amine compound of Formula **4** can be isolated by quenching the hydrolysis in acid media with sodium bicarbonate, but this intermediate is highly unstable, and is transformed quickly into a five member cyclic imine, named compound **6**;
forming the protected compound of Formula **7** by reacting with bromomethylmethyl ether and diisopropylethylamine in dichloromethane;
selectively de-methylating the methoxy group of the quinone system of compound of Formula **7** into the compound of Formula **8** by reacting with methanolic solution of sodium hydroxide;
transforming the compound of Formula **8** into methylenedioxy-compound of Formula **9** by the preferred following sequence: (1) quinone group of compound **8** is reduced with 10% Pd/C under hydrogen atmosphere; (2) the hydroquinone intermediate is converted into the methylene-dioxy compound of Formula **9** by reacting with bromochloromethane and cesium carbonate under hydrogen atmosphere; (3) compound of Formula **9** is transformed into compound of Formula **10** by protecting the free hydroxyl group as a OCH₂R group, by reacting with BrCH₂R and cesium carbonate, where R can be aryl. CH=CH₂, OR' etc.; converting the acetamide group of compound of Formula **10** into the corresponding hydroxyl group of Formula **11** by reaction with nitrogen tetroxide in a mixture of acetic acid and acetic acetate followed by treatment with sodium hydroxide; alternatively can be used sodium nitrite in a mixture of acetic anhydride acetic acid, followed by treatment with sodium hydroxide; alternatively the acetamide group of compound of Formula **10** can be converted into the primary amine group by reacting with hydrazine or with Boc₂O. DMAP followed by hydrazine; such primary amine can be converted into the corresponding hydroxyl group (compound of Formula **11**) by an oxidative conversion of the primary amine into the corresponding aldehyde with 4-formyl-1-methylpyridinium benzenesulphonate or other pyridinium ion, followed by DBU or other base treatment and further hydrolization, and followed by the reduction of the aldehyde to the corresponding hydroxyl group with lithium aluminium hydride or other reducing agent;
forming the protected compound of Formula **26** by reacting with t-butyldiphenylsilyl chloride and dimethylaminopyridine in dichloromethane;
transforming the silylated compound of Formula **26** into the intermediate **11** by deprotection of the OCH₂R protecting group, by reacting under reductive conditions or acid conditions. Typical procedures are with palladium black under hydrogen atmosphere, or aqueous TFA, or tributyltin hydride and dichlorobis (triphenylphosphine palladium).

In yet another preferred modification, the cyano compound of Formula **2** can be transformed into Intermediate **11** using an extension of the scheme II, involving the further steps of.
formation of the protected hydroxy compound of Formula **26** by reacting **25** with *tert-*butyldiphenylsilyl chloride in the presence of a base;
final cleavage of the allyl group with tributyltin hydride and dichloropalladium-bis (triphenylphosphine) in **26** that leads to the formation of the intermediate **11**.

Thus, it is possible to transform cyanosafracin B into a number of intermediates and derivatives with potential antitumor therapeutic activity. These intermediates can be made starting from already described compounds, or using alternative routes.

Intermediates described herein comprise compound **47,** and a numbers of amide derivatives made using compounds **45** or **43.**

In Scheme VIII is described formation of compound **47** using the following sequence:
forming the thiourea compound of Formula **3** by reacting compound of Formula **2** with phenyl isothiocyanate;
converting the thiourea compound of Formula **3** into the acetamide of Formula **5** by an hydrolysis in acid media followed by addition of acetic anhydride; The intermediate amine compound of Formula **4** can be isolated by quenching the hydrolysis in acid media with sodium bicarbonate, but this intermediate is highly unstable, and is transformed quickly into a five member cyclic imine, named compound **6**;
forming the protected compound of Formula **7** by reacting with bromomethylmethyl ether and diisopropylethylamine in dichloromethane;
selectively de-methylating the methoxy group of the quinone system of compound of Formula **7** into the compound of Formula **8** by reacting with methanolic solution of sodium hydroxide;
transforming the compound of Formula **8** into methylenedioxy-compound of Formula **10** by the preferred following sequence: (1) quinone group of compound **8** is reduced with 10% Pd/C under hydrogen atmosphere; (2) the hydroquinone intermediate is converted into the methylene-dioxy compound of Formula **9** by reacting with bromochloromethane and cesium carbonate under hydrogen atmosphere; (3) compound of Formula **9** is transformed into compound of Formula **10** by protecting the free hydroxyl group as a allyloxy group, by reacting with allyl-bromide and cesium carbonate;
transforming the compound of formula **9** into acetyl-derivative **46** by reaction with acetyl chloride in pyridine;
transforming compound of formula **46** into de-protected compound **47** by reaction with hydrochloric acid in dioxane.

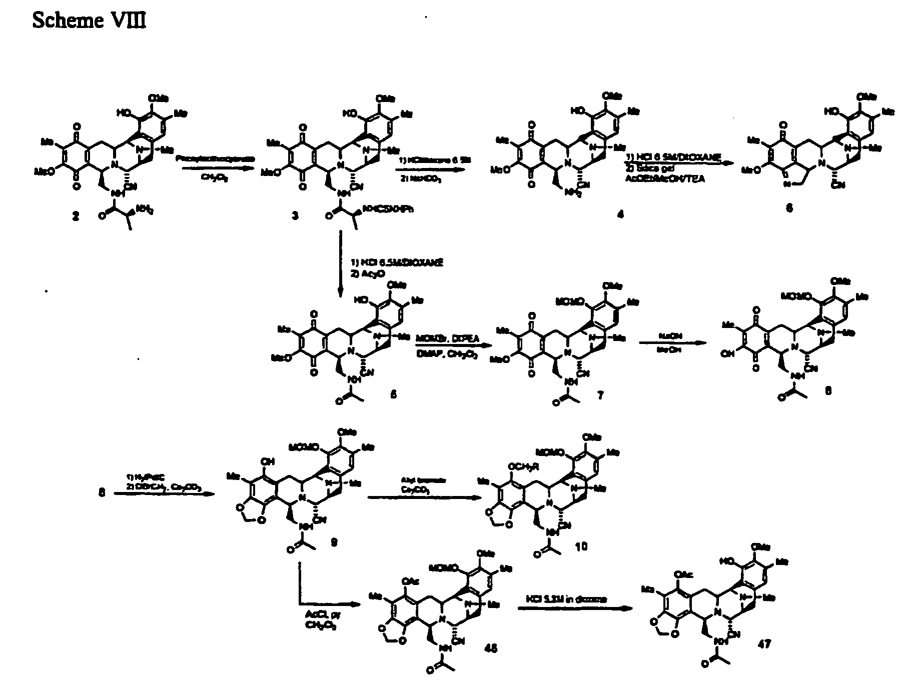

Other useful amide intermediate derivatives are made starting from already described intermediate **45** using the next scheme: 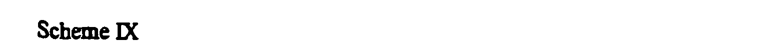 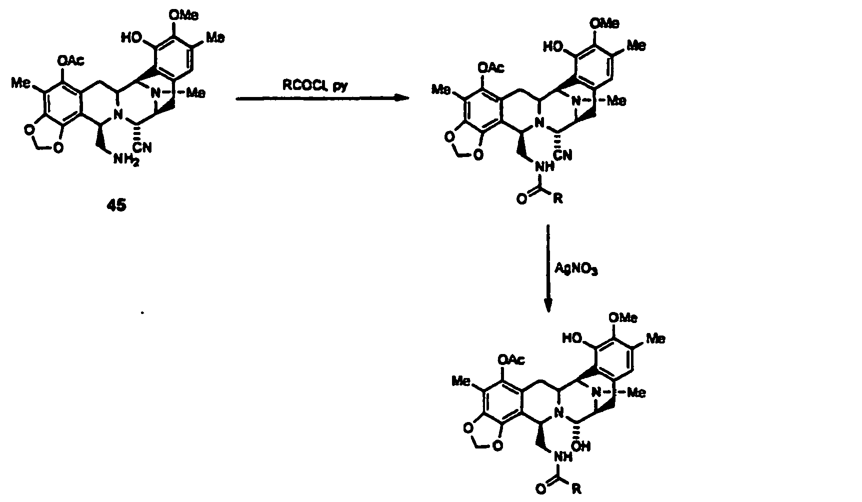 The second step is optional. This process is an important part of the invention, particularly where the group R is a group R^{a} as previously defined. Furthermore, the Scheme VIII can be readily broadened to enable preparation of compounds of formula (XXIII), by inclusion in the starting material of a different group at the 5-position, either a group directly intended for the product or a group which can be removed or otherwise modified to give the desired group.

From compound **45** can be made a group of analogs through the following sequence:
acylation in the amino group of compound of Formula **45** by a wide range of acyl derivatives to provide the corresponding amides, where preferred acyl groups are acetyl, cinnamoyl chloride, p-trifluorocinnamoyl chloride, isovaleryl chloride phenylisothiocyanate or aminoacids, or the other examples previously given of groups R^{a}CO-.
transforming the CN group into an OH group by reaction with silver nitrate in a mixture AcN/H₂O.

Other useful amide intermediate derivatives are made starting from already described intermediate **43** using the next scheme:

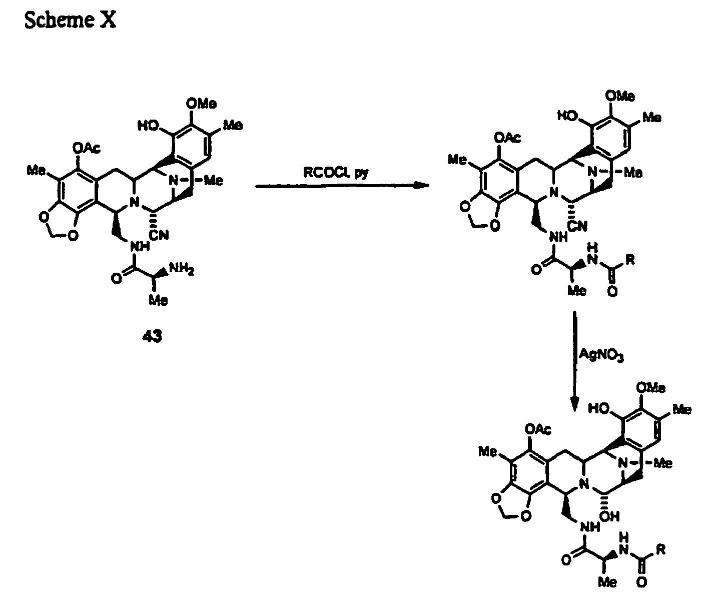

From Compound **43** can be obtained another group of interesting derivatives using the following sequence:
(a) acylation in the amino group of compound of Formula **43** by a wide range of acyl derivatives to provide the corresponding amides, where preferred acyl groups are acetyl, cinnamoyl chloride, p-trifluorocinnamoyl chloride, isovaleryl chloride or aminoacids, or the other examples previously given of groups R^{a}CO-.
(b) transforming the CN group into an OH group by reaction with silver nitrate in a mixture AcN/H₂O

Reflecting the active compounds, an important process in accordance with this invention is as follows: 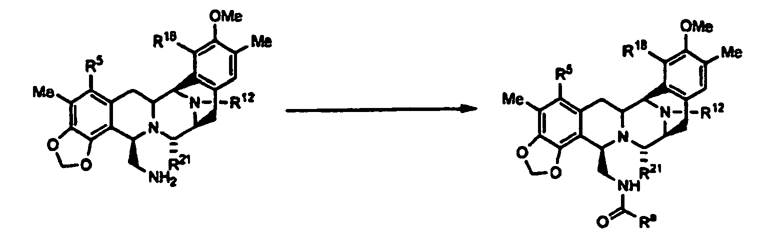 where R⁵ for the end product is as defined for the compound (XXII) and may be different in the starting material and converted thereto as part of the process,
R¹⁸ is a hydroxy group in the end product but may be a protected hydroxy group in the starting material and converted thereto as part of the process,
R¹² for the end product may be the same as in the starting material or may be converted thereto as part of the process,
R²¹ for the end product is as defined and if a hydroxy group may be formed from a cyano group as part of the process,
R^{a} is as defined, and may be further acylated as part of the process to give an end product with an acylated R^{a} group as discussed.

R⁵ is preferably oxyacetyl or other small oxyacyl group in the starting material and is not changed in the reaction. R¹⁸ is preferably a hydroxy group in the starting material and is not changed in the reaction. R¹² is preferably -NCH₃- in the starting material and is not changed in the reaction. R²¹ the end product is as defined and if a hydroxy group may be formed from a cyano group as part of the process. R^{a} is in the final product is preferably as defined in relation to the compound of formula (XXIII).

Another important method of this invention includes the reaction:

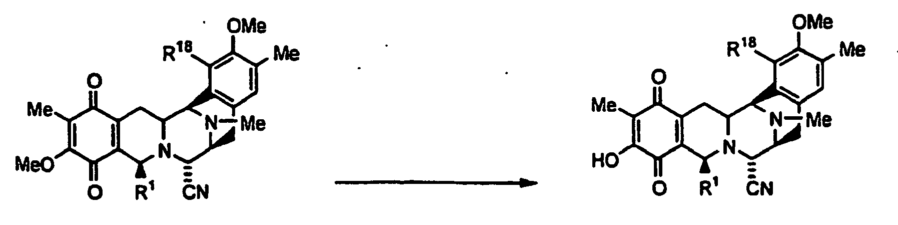

Another important method of this invention includes the reaction:

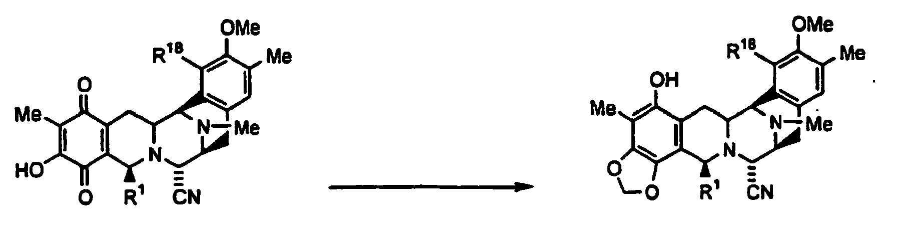

Another important method of this invention includes the reaction includes the reaction where a group R¹ is aminomethylene is converted to a hydroxymethylene group.

Another important method of this invention includes the reaction for preparing a 21-cyano compound of formula (XVI) which comprises reacting a compound of formula (XV): 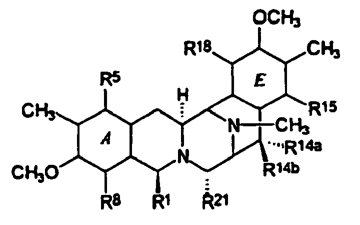 where R^{1,} R⁵, R⁸, R^{14a}, R^{14b}, R¹⁵ and R¹⁸ are as defined and R²¹ is a hydroxy group, with a source of cyanide ion, to give the desired 21-cyano compound.

In addition, processes using other nucleophile-containing compounds, to produce similar compounds of formula (XVI) wherein the 21-position is protected by another nucleophilic group, a 21-Nuc group, are also envisaged. For example, a 21-Nuc compound of formula (XVI) with an alkylamino substituent at the 21-position can be produced by reacting the compound of formula (XV) wherein R²¹ is a hydroxy group with a suitable alkylamine. A 21-Nuc compound of formula (XVI) with an alkylthio substituent at the 21-position can also be produced by reacting the compound of formula (XV) wherein R²¹ is a hydroxy group with a suitable alkanethiol. Alternatively, a 21-Nuc compound of formula (XVI) with an α-carbonylalkyl substituent at the 21-position can be produced by reacting the compound of formula (XV) wherein R²¹ is a hydroxy group with a suitable carbonyl compound, typically in the presence of a base. Other synthetic routes are available for other 21-Nuc compounds.

Another important reaction of this invention involves treatment of a 21-cyano product of this invention to form a 21-hydroxy compound. Such compounds have interesting *in vivo* properties.

For the avoidance of doubt, the stereochemistries indicated in this patent specification are based on our understanding of the correct stereochemistry of the natural products. To the extent that an error is discovered in the assigned stereochemistry, then the appropriate correction needs to be made in the formulae given throughout in this patent specification. Furthermore, to the extent that the syntheses are capable of modification, this invention extends to stereoisomers.

**CYTOTOXIC ACTIVITIY**

| **Compound** | **IC₅₀ (µM)** | | | | | |
|---|---|---|---|---|---|---|
| | **P-388** | **A-549** | **HT-29** | **MEL-28** | **CV-1** | **DU-145** |
| 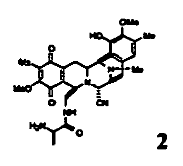 | 0.009 | 0.018 | 0.018 | 0.018 | 0.023 | |
| 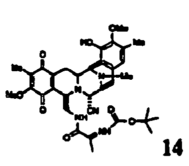 | 0.15 | >0.15 | 0.15 | >0.15 | | |
| 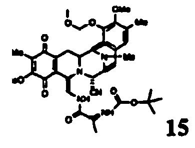 | 1.44 | 1.44 | 1.44 | 1.44 | | |
| 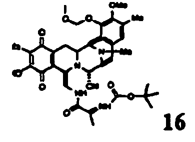 | >1.5 | >1.5 | >1.5 | >1.5 | | |
| 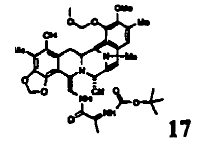 | 1.4 | 1.4 | 1.4 | 1.4 | | |
| 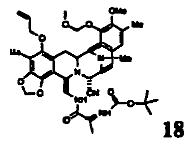 | 0.01 | 0.01 | 0.01 | 0.01 | | |
| 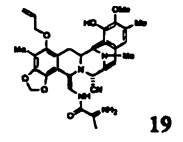 | 0.08 | 0.16 | 0.01 | 0.16 | | |
| 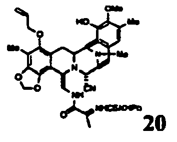 | 0.01 | 0.01 | 0.01 | 0.01 | | |
| 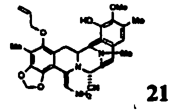 | 0.019 | 0.019 | 0.019 | 0.019 | | |
| 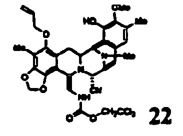 | 0.014 | 0.014 | 0.014 | 0.014 | 0.014 | 0.014 |
| 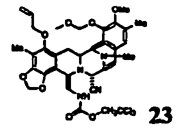 | 0.13 | 0.13 | 0.13 | 0.13 | 0.13 | 0.13 |
| 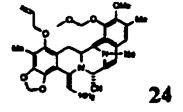 | 0.18 | 1.8 | 1.8 | 1.8 | 1.8 | 1.8 |
| 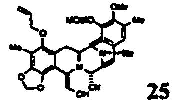 | 0.2 | 0.2 | 0.2 | 0.2 | | 0.2 |
| 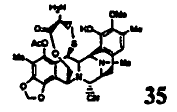 | 0.008 | 0.008 | 0.008 | 0.008 | | |
| 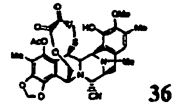 | 0.01 | 0.01 | 0.01 | 0.01 | | |
| 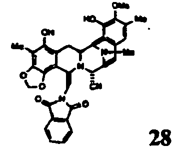 | 0.001 | 0.001 | 0.001 | 0.001 | 0.001 | 0.001 |
| 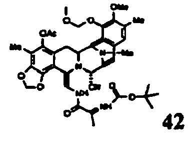 | 0.13 | 0.13 | 0.13 | 0.13 | | 0.13 |
| 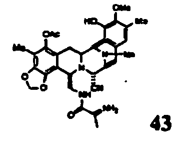 | 0.008 | 0.016 | 0.008 | 0.008 | | 0.016 |
| 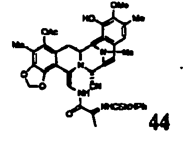 | 0.001 | 0.001 | 0.001 | 0.001 | | 0.001 |
| 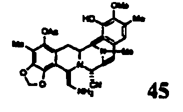 | 0.01 | 0.01 | 0.01 | 0.01 | | 0.01 |
| 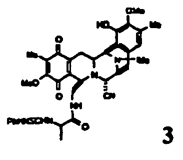 | 0.015 | 0.015 | 0.015 | 0.015 | 0.018 | |
| 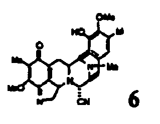 | 2.171 | 2.171 | 2.171 | 2.171 | 2.171 | |
| 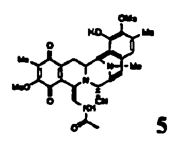 | 0.005 | 0.005 | 0.005 | 0.005 | | |
| 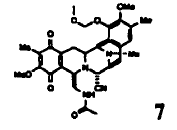 | 0.22 | 0.22 | 0.22 | 0.22 | 0.22 | |
| 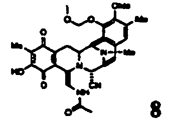 | >9 | >18.1 | >18.1 | >18.1 | >18.1 | |
| 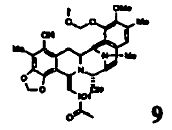 | >1.77 | >1.77 | >1.77 | >1.77 | | >1.77 |
| 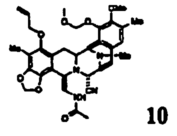 | >1.65 | >1.65 | >1.65 | >1.65 | | >1.65 |
| 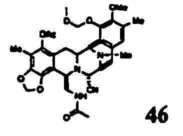 | 0.016 | 0.016 | 0.016 | 0.016 | | 0.016 |
| 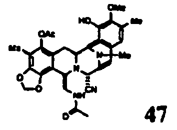 | 0.001 | 0.001 | 0.001 | 0.001 | | 0.001 |
| 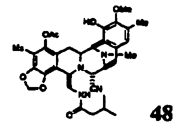 | 0.0008 | 0.001 | 0.0008 | 0.0008 | | 0.001 |
| 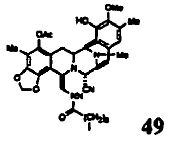 | 0.007 | 0.007 | 0.007 | 0.007 | | 0.007 |
| 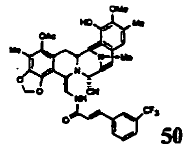 | 0.0001 | 0.0001 | 0.0001 | 0.0001 | | 0.0001 |
| 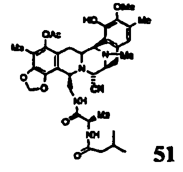 | 0.0001 | 0.0001 | 0.0001 | 0.0001 | | 0.0001 |
| 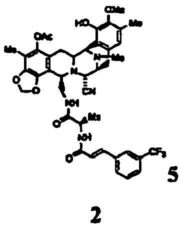 | 0.001 | 0.001 | 0.001 | 0.001 | | 0.001 |
| 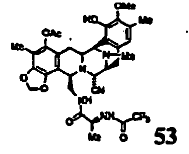 | 0.0001 | 0.0001 | 0.0001 | 0.0001 | | 0.0001 |
| 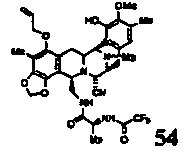 | 0.001 | 0.001 | 0.001 | 0.001 | | 0.001 |
| 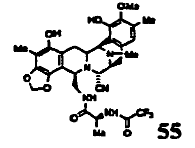 | 0.01 | 0.01 | 0.01 | 0.01 | | 0.01 |
| 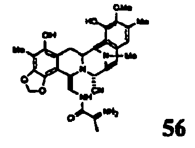 | 0.18 | 0.9 | 0.18 | 0.8 | | 0.9 |
| 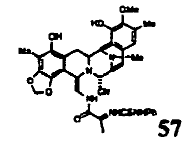 | 0.14 | 0.14 | 0.14 | 0.14 | | 0.14 |
| 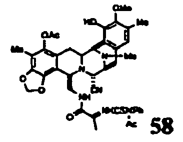 | 0.001 | 0.001 | 0.001 | 0.001 | | 0.001 |
| 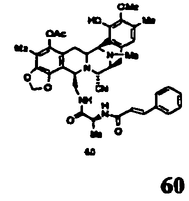 | 0.001 | 0.001 | 0.0001 | 0.001 | | 0.0005 |
| 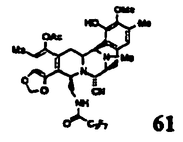 | 0.001 | 0.001 | 0.001 | 0.001 | | 0.001 |
| 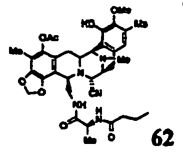 | 0.001 | 0.001 | | 0.0005 | | 0.001 |
| 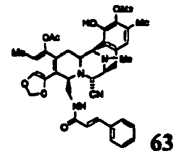 | 0.0001 | 0.0001 | 0.0001 | 0.0001 | | 0.0001 |
| 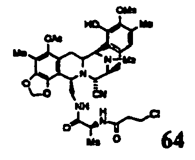 | 0.001 | 0.001 | | 0.001 | | 0.001 |
| 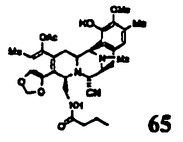 | 0.0001 | 0.0001 | 0.0001 | 0.0001 | | 0.0001 |
| 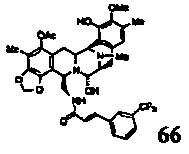 | 0.0001 | 0.0001 | 0.0001 | 0.0001 | | 0.0001 |
| 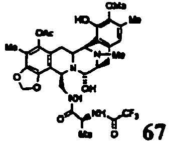 | 0.0001 | 0.0001 | 0.0001 | 0.0001 | | 0.0001 |
| 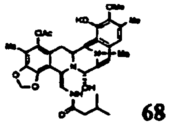 | 0.0008 | 0.001 | 0.0008 | 0.0008 | | 0.001 |
| 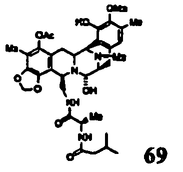 | 0.001 | 0.001 | 0.001 | 0.001 | | 0.001 |
| 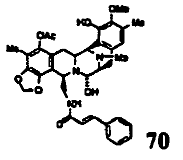 | 0.0001 | 0.0001 | 0.0001 | 0.0001 | | 0.0001 |
| 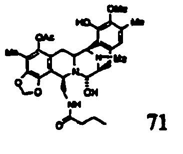 | 0.0008 | 0.0008 | 0.0001 | 0.0008 | | 0.0001 |
| 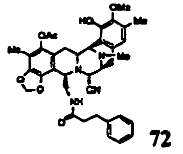 | 0.0001 | 0.0001 | 0.0001 | 0.0001 | | 0.0001 |
| 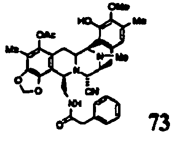 | 0.0001 | 0.0001 | 0.0001 | 0.0001 | | 0.0001 |
| 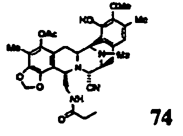 | 0.0001 | 0.0001 | 0.0001 | 0.0001 | | 0.0001 |
| 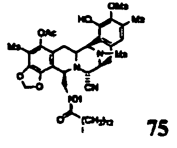 | 0.1 | 0.1 | 0.1 | 0.1 | | 0.1 |
| 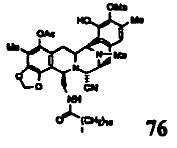 | 0.1 | 0.1 | 0.1 | 0.1 | | 0.1 |
| 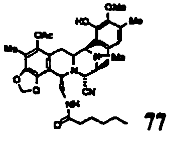 | 0.0001 | 0.0001 | 0.0001 | 0.0001 | 0.0001 | 0.0001 |
| 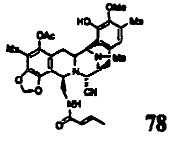 | 0.0001 | 0.0008 | 0.0001 | 0.0001 | | 0.0008 |
| 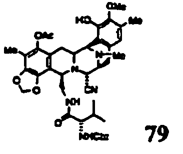 | 0.001 | 0.001 | 0.001 | 0.001 | | 0.001 |
| 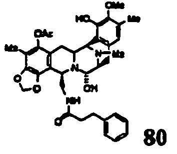 | 0.0001 | 0.0001 | 0.0001 | 0.0001 | | 0.0001 |
| 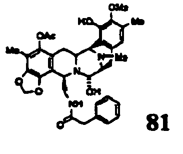 | 0.0007 | 0.0007 | 0.0007 | 0.0007 | | 0.0007 |
| 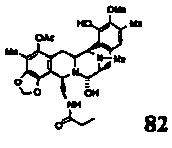 | 0.0001 | 0.0001 | 0.0001 | 0.0001 | | 0.0001 |
| 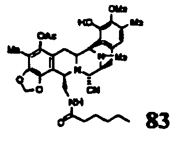 | 0.0001 | 0.0001 | 0.0001 | 0.0001 | 0.0001 | 0.0001 |
| 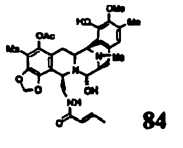 | 0.0001 | 0.0008 | 0.0001 | 0.0001 | | 0.0008 |
| 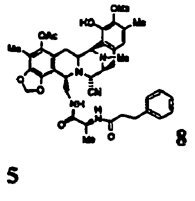 | 0.0006 | 0.001 | 0.0006 | 0.001 | | 0.0006 |
| 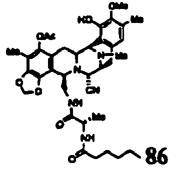 | 0.001 | 0.001 | 0.001 | 0.001 | 0.001 | 0.001 |
| 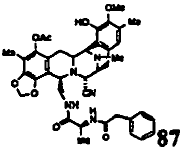 | 0.0001 | 0.0001 | 0.0001 | 0.0001 | | 0.0001 |
| 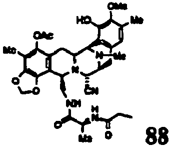 | 0.0007 | 0.0007 | 0.0007 | 0.0007 | | 0.0007 |
| 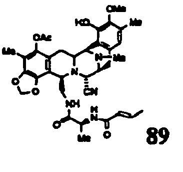 | 0.001 | 0.007 | 0.001 | 0.001 | | 0.007 |
| 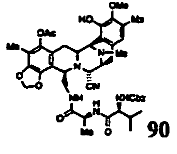 | 0.01 | 0.01 | 0.01 | 0.01 | | 0.01 |
| 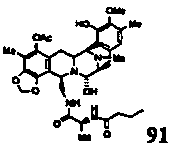 | 0.001 | 0.001 | 0.001 | 0.001 | | 0.001 |
| 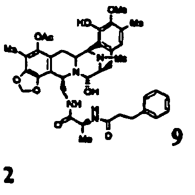 | 0.0001 | 0.0001 | 0.0001 | 0.0001 | | 0.0001 |
| 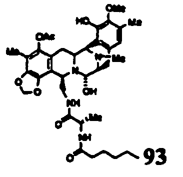 | 0.001 | 0.001 | 0.001 | 0.001 | 0.001 | 0.001 |
| 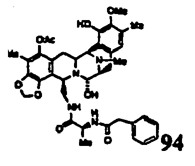 | 0.0007 | 0.0007 | 0.0007 | 0.0007 | | 0.0007 |
| 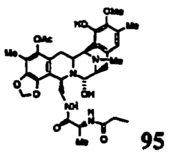 | 0.0001 | 0.0001 | 0.0001 | 0.0001 | | 0.0001 |
| 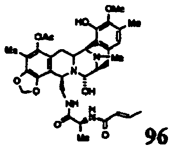 | 0.001 | 0.007 | 0.001 | 0.001 | | 0.007 |
| 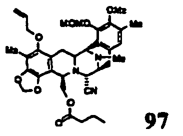 | >1 | >1 | >1 | >1 | | >1 |
| 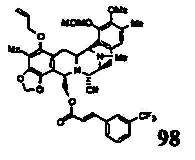 | >1 | >1 | >1 | >1 | | >1 |
| 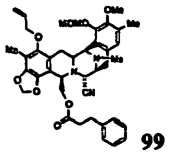 | 0.7 | 0.7 | 0.7 | 0.7 | 0.7 | 0.7 |
| 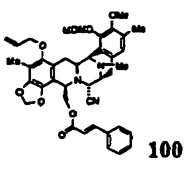 | 0.1 | 0.1 | 0.1 | 0.1 | | 0.1 |
| 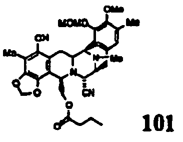 | 0.1 | 0.1 | 0.1 | 0.1 | | 0.1 |
| 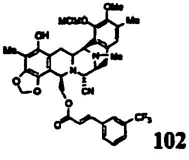 | 0.1 | 0.1 | 0.1 | 0.1 | | 0.1 |
| 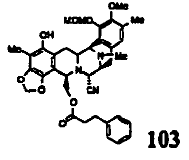 | 0.1 | 0.1 | 0.1 | 0.1 | | 0.1 |
| 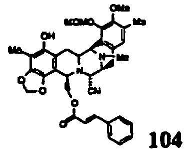 | 0.1 | 0.1 | 0.1 | 0.1 | | 0.1 |
| 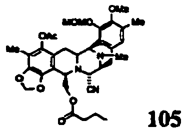 | 0.1 | 0.1 | 0.1 | 0.1 | | 0.1 |
| 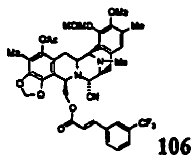 | 0.6 | 0.6 | 0.6 | 0.6 | | 0.6 |
| 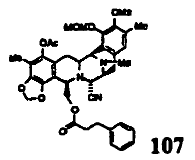 | 0.1 | 0.1 | 0.1 | 0.1 | | 0.1 |
| 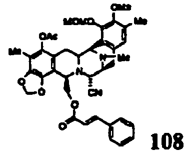 | 0.01 | 0.07 | 0.07 | 0.07 | | 0.07 |
| 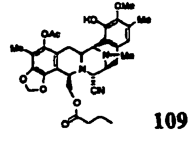 | 0.0001 | 0.0008 | 0.0008 | 0.0008 | | 0.0008 |
| 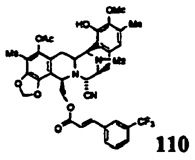 | 0.001 | 0.001 | 0.001 | 0.001 | | 0.001 |
| 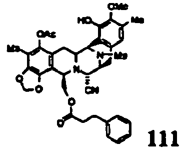 | 0.0001 | 0.0001 | 0.0001 | 0.0001 | | 0.0001 |
| 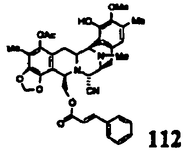 | 0.0007 | 0.0007 | 0.0007 | 0.0007 | | 0.0007 |
| 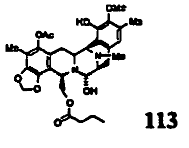 | 0.0001 | 0.0001 | 0.0001 | 0.0001 | | 0.0001 |
| 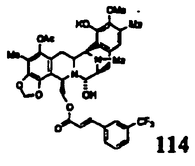 | 0.0001 | 0.0001 | 0.0001 | 0.0001 | | 0.0001 |
| 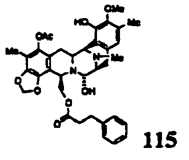 | 0.0001 | 0.0001 | 0.0001 | 0.0001 | | 0.0001 |
| 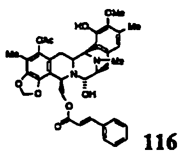 | 0.0001 | 0.0007 | 0.0007 | 0.0007 | | 0.0007 |
| 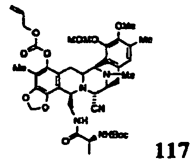 | 0.06 | 0.06 | 0.06 | 0.06 | | 0.06 |
| 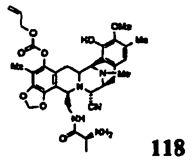 | 0.001 | 0.001 | 0.001 | 0.001 | | 0.001 |
| 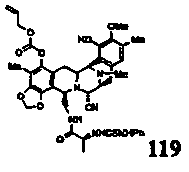 | 0.001 | 0.001 | 0.001 | 0.001 | | 0.001 |
| 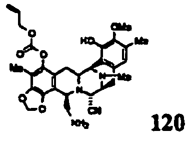 | 0.06 | 0.06 | 0.06 | 0.06 | | 0.06 |
| 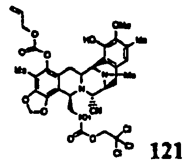 | 0.006 | 0.006 | 0.006 | 0.006 | | 0.006 |
| 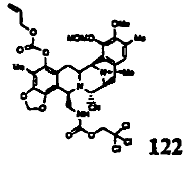 | 0.1 | 0.1 | 0.1 | 0.1 | | 0.1 |
| 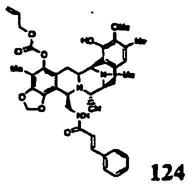 | 0.0001 | 0.0001 | 0.0001 | 0.0001 | | 0.0001 |
| 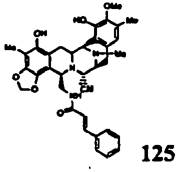 | 0.001 | 0.001 | 0.001 | 0.001 | | 0.001 |
| 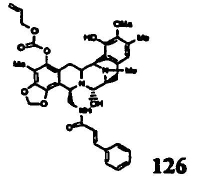 | 0.0001 | 0.0001 | 0.0001 | 0.0001 | | 0.0001 |
| 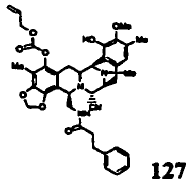 | 0.0001 | 0.0001 | 0.0001 | 0.0001 | | 0.0001 |
| 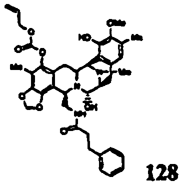 | 0.0001 | 0.0001 | 0.0001 | 0.0001 | | 0.0001 |
| 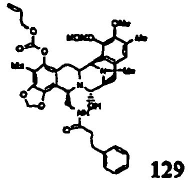 | 0.1 | 0.1 | 0.1 | 0.1 | | 0.1 |
| 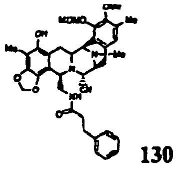 | 0.1 | 0.1 | 0.1 | 0.1 | | 0.1 |
| 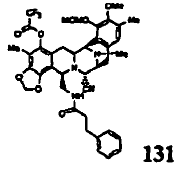 | 0.5 | 0.5 | 0.5 | 0.5 | | 0.5 |
| 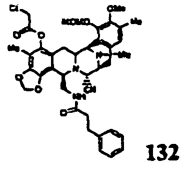 | 0.1 | 0.1 | 0.1 | 0.1 | | 0.1 |
| 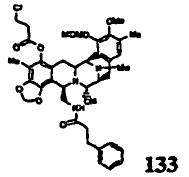 | 0.05 | 0.05 | 0.05 | 0.05 | | 0.05 |
| 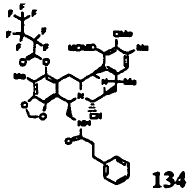 | 0.5 | 0.5 | 0.5 | 0.5 | 0.5 | 0.5 |
| 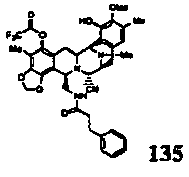 | 0.01 | 0.01 | 0.01 | 0.01 | | 0.01 |
| 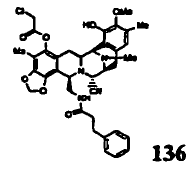 | 0.001 | 0.001 | 0.001 | 0.001 | | 0.001 |
| 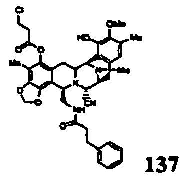 | 0.01 | 0.01 | 0.01 | 0.01 | | 0.01 |
| 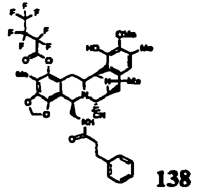 | 0.006 | 0.006 | 0.006 | 0.006 | | 0.006 |
| 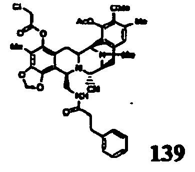 | 0.01 | 0.01 | 0.01 | 0.01 | | 0.01 |
| 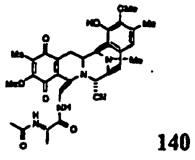 | 0.08 | 0.08 | 0.08 | 0.08 | | 0.08 |
| 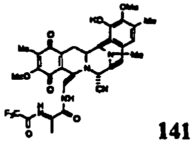 | 0.01 | 0.01 | 0.01 | 0.01 | 0.01 | 0.01 |
| 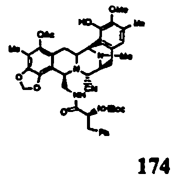 | | 0.0013 | 0.0013 | | | |
| 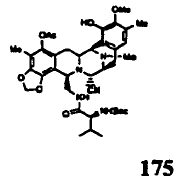 | | 0.007 | 0.007 | | | |
| 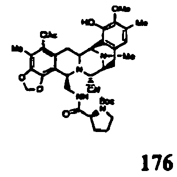 | | 0.014 | 0.014 | | | |
| 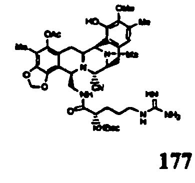 | | >1 | >1 | | | |
| 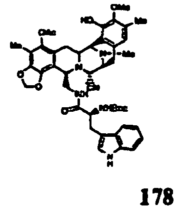 | | 0.00012 | 0.00012 | | | |
| 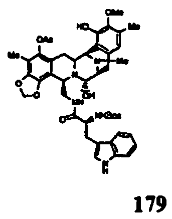 | | 0.012 | 0.012 | | | |
| 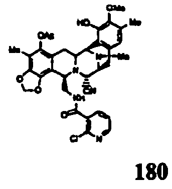 | | 0.00015 | 0.00015 | | | |
| 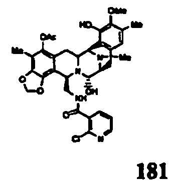 | | 0.00015 | 0.00015 | | | |
| 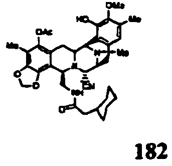 | | 0.0015 | 0.0015 | | | |
| 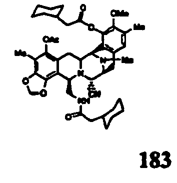 | | 0.013 | 0.013 | | | |
| 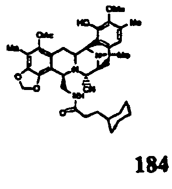 | | 0.0015 | 0.0015 | | | |
| 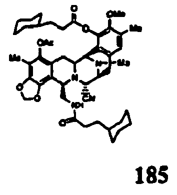 | | 0.12 | 0.12 | | | |
| 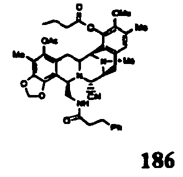 | | 0.0014 | 0.0014 | | | |
| 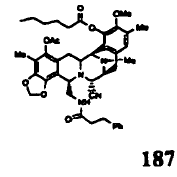 | | 0.013 | 0.013 | | | |
| 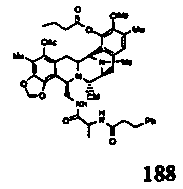 | | 0.012 | 0.012 | | | |
| 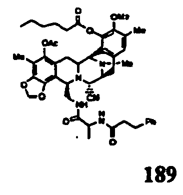 | | 0.06 | 0.06 | | | |
| 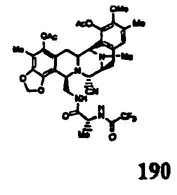 | | 0.013 | 0.013 | | | |
| 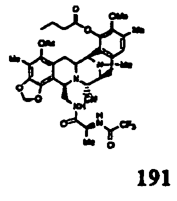 | | 0.13 | 0.13 | | | |
| 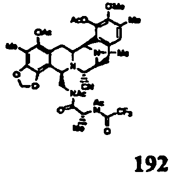 | | 0.12 | 0.12 | | | |
| 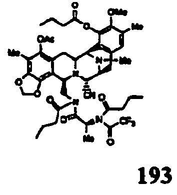 | | 0.11 | 0.11 | | | |
| 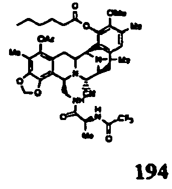 | | 0.012 | 0.012 | | | |
| 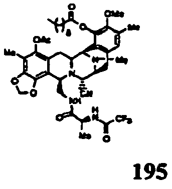 | | 0.012 | 0.012 | | | |
| 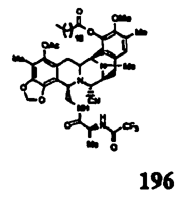 | | 0.1 | 0.1 | | | |
| 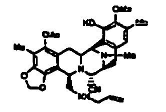 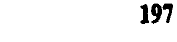 | | 0.0018 | 0.0018 | | | |
| 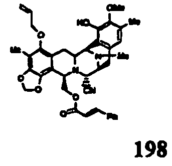 | | 0.0015 | 0.0015 | | | |
| 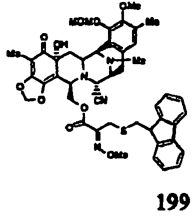 | | >1 | >1 | | | |
| 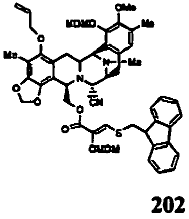 | | 0.056 | 0.056 | | | |

**CYTOTOXIC ACTIVITY (M)**

| **SOLID TUMORS** | **LINE** | 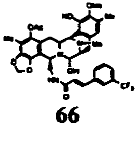 | 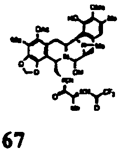 | 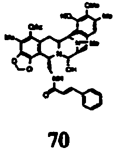 | 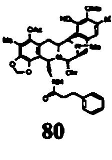 |
|---|---|---|---|---|---|
| Bladder | 5637 | 6.02E-10 | 3.42E-10 | 1.91E-10 | 2.04E.11 |
| Breast | MX-1 | 1.65E-06 | NA | 2.38E-09 | NA |
| Colon | HT-29 | 7.84E-10 | 1.97E-08 | 2.12E-09 | 8.44E-12 |
| Gastric | Hs746t | 7.90E-12 | 2.18E-09 | 7.10E-11 | 2.21E-09 |
| Liver | SK-HEP-1 | 1.79E-12 | 6.01E.11 | 3.15E-09 | 9.91E-11 |
| NSCL | A549 | 3.25E-09 | 7.68E-06 | NA | NA |
| Ovary | SK-OV-3 | 4.39E- 11 | 1.02E-07 | 8.74E-09 | NA |
| Pancreas | PANC-1 | 7.22E-11 | 4.17E-09 | 1.29E-10 | 1.19E-10 |
| Pharnynx | FADU | 5.41E-11 | 1.58E-09 | 3.71E-10 | 5.98E-09 |
| Prostate | PC3 | 6.65E-09 | 2.15E-09 | 4.70E-09 | 1.52E-10 |
| Prostate | DU-145 | 5.73E-10 | 1.83E-07 | 2.22E-09 | NA |
| Prostate | LNCAP | 5.45E-10 | 2.17E-10 | 3.94E-11 | |
| Renal | 786-O | 6.58E-12 | 1.59E-09 | 1.72E-09 | 1.03E-10 |
| SCL | NCI-H187 | 7.14E-14 | 9.57E-10 | 7.78E-14 | |
| Retinoblastoma | Y-79 | 7.14E-14 | 7.36E-10 | 8.85E-11 | |
| Melanoma | Mel-28 | 2.60E-10 | 3.17E-09 | 2.18E-09 | 1.23E-10 |
| Fibrosarcoma | SW-694 | 9.91E-10 | NA | 1.39E-06 | NA |
| Chondrosarcoma | CHSA | 3.24E-10 | 6.77E-09 | 1.39E-09 | 2.30E-10 |
| Osteosarcoma | OSA-FH | 1.94E-09 | 1.39E-09 | 1.09E-09 | 1.11E-10 |

| **SOLID TUMORS** | **LINE** | 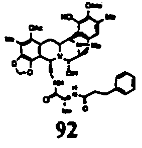 | 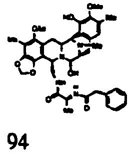 | 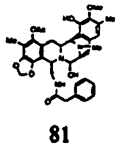 |
|---|---|---|---|---|
| Bladder | 5637 | 1.65E-10 | 7.85E-10 | 3.18-09 |
| Breast | MX-1 | NA | 2.85E-06 | NA |
| Colon | HT-29 | 7.43E-10 | 1.2E-10 | NA |
| Gastric | Hs746t | 9.35E-10 | 6.25E-09 | 1.37E-07 |
| Liver | SK-HEP-1 | 1.40E-09 | 9.03E-10 | 9.50E-09 |
| NSCL | A549 | NA | NA | NA |
| Ovary | SK-OV-3 | NA | NA | |
| Pancreas | PANC-1 | 8.93E-10 | 2.58E-9 | 1.03E-08 |
| Pharnynx | FADU | 8.41E-10 | 3.77E-08 | 1. 1 4E-09 |
| Prostate | PC3 | 8.13E-10 | | 9.34E-09 |
| Prostate | DU-145 | NA | NA | NA |
| Prostate | LNCAP | | NA | |
| Renal | 786-O | 7.88E-10 | 2.90E-09 | 1.00E-08 |
| SCL | NCI-H187 | | 2.07E-12 | |
| Retinoblastoma | Y-79 | | 1.31E-11 | 7.78E-09 |
| Melanoma | Mel-28 | 1.08E-09 | 1.13E-09 | 4.48E-09 |
| Fibrosarcoma | SW-694 | NA | | |
| Chondrosarcoma | CHSA | 1.08E-09 | 2.25E-09 | 1.09E-08 |
| Osteosarcoma | OSA-FH | 8.84E-10 | 1.35-08 | 9.50E-09 |

| **LEUKEMIAS & LYMPHOMAS** | **LINE** | 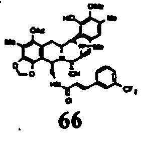 | 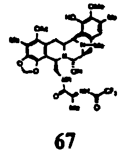 | 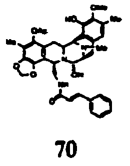 | 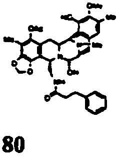 |
|---|---|---|---|---|---|
| ALL Promyelocytic leukemia | HL60 | | | | 9.38E-09 |
| ALL Acute lymphoblastic | Molt 3 | 6.13E-10 | 2.8E-09 | 5.66E-10 | 1.55E-14 |
| CML Chronic myelogenous | K562 | | | | 2.33E-07 |
| Leukemia Hairy B-cell | Mo-B | | | | |
| Lymphoma T-cell | H9 | | | | 1.99E-11 |
| Lymphoma Cutaneus T cell | Hut 78 | 5.50E-11 | 2.57E-10 | 4.62E-9 | 6.21E-11 |
| Lymphoma undifferentiated | MC116 | 2.15E-10 | 2.65E-10 | 3.8E-09 | NA |
| Lymphoma Burkitts B celll | RAMOS | | | | 7.77E-13 |
| Lymphoma | U-937 | 1.77E-10 | 5.27E-11 | 3.28E-11 | 3.06E-11 |
| Histiocytic | | | | | |

| **LEUKEMIAS & LYMPHOMAS** | **LINE** | 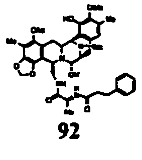 | 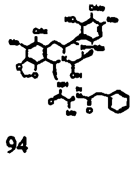 | 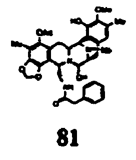 |
|---|---|---|---|---|
| ALL Promyelocytic leukemia | HL60 | 5.92E-09 | 123E-10 | 3.97E-10 |
| ALL Acute lymphoblastic | Molt 3 | 7.53E-12 | 8.85E-10 | 2.54E-09 |
| CML Chronic myelogenous | K562 | 1.09E-08 | 4.45E-08 | |
| Leukemia Hairy B-cell | Mo-B | | | |
| Lymphoma T-cell | H9 | 4.48E-09 | 1.14E-08 | |
| Lymphoma Cutaneus T cell | Hut 78 | 9.9E-10 | 1.06E-08 | 7.46E-09 |
| Lymphoma undifferentiated | MC116 | NA | 1.41E-09 | 1.13E-08 |
| Lymphoma Burkitts B celll | RAMOS | 5.26-11 | 8.85E-10 | 7.15E-09 |
| Lymphoma Histiocytic | U-937 | 5.15E-10 | | |

| **SOLID TUMORS** | **LINE** | 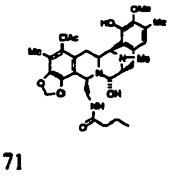 | 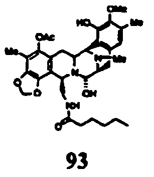 |
|---|---|---|---|
| Bladder | 5637 | 2.81E-09 | 2.84E-10 |
| Breast | MX-1 | 2.50E-06 | NA |
| Colon | HT-29 | NA | 8.97E-09 |
| Gastric | Hs746t | 2.97E-08 | 9.19E-09 |
| Liver | SK-HEP-1 | 5.07E-09 | 1.08E-09 |
| NSCL | A549 | NA | 9.41E-09 |
| Ovary | SK-OV-3 | 2.21E-07 | NA |
| Pancreas | PANC-1 | 2.90E-09 | 1.00E-09 |
| Pharnynx | FADU | 7.94E-09 | 1.39E-08 |
| Prostate | PC3 | 1.46-08 | 9.32E-10 |
| Prostate | DU-145 | NA | NA |
| Prostate | LNCAP | 5.39E-09 | |
| Renal | 786-0 | 6.55E-09 | 1.72E-09 |
| SCL | NCI-HI87 | 3.98E- 11 | |
| Retinoblastoma | Y-79 | 3.14E-09 | |
| Melanoma | Mel-28 | 3.05E-08 | 1.15E-09 |
| Fibrosarcoma | SW-694 | NA | NA |
| Chondrosarcoma | CHSA | 1.73E-08 | 2.10E-09 |
| Osteosarcoma | OSA-FH | 8.56E-08 | 1.30E-09 |

| **SOLID TUMORS** | **LINE** | 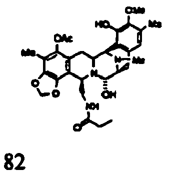 | 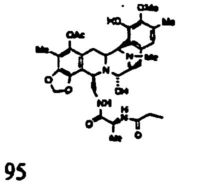 |
|---|---|---|---|
| Bladder | 5637 | 9.91E-10 | 1.17E-09 |
| Breast | MX-1 | NA | 1.92E-09 |
| Colon | HT-29 | NA | NA |
| Gastric | Hs746t | 1.36E-09 | 8.15E-09 |
| Liver | SK-BEP-1 | 1.17E-09 | 6.21 E-09 |
| NSCL | A549 | NA | NA |
| Ovary | SK-OV-3 | 2.90E-08 | NA |
| Pancreas | PANC-1 | 1.37E-09 | 8.61E-09 |
| Pharnynx | FADU | 3.05E-08 | 4.38E-08 |
| Prostate | PC3 | | |
| Prostate | DU-145 | NA | NA |
| Prostate | LNCAP | 2.38E-08 | 1.77E-08 |
| Renal | 786-0 | 2.27E-09 | 1.54E-08 |
| SCL | NCI-H187 | 2.41E-11 | 9.89E-11 |
| Retinoblastoma | Y-79 | 3.08E-10 | 7.45E-10 |
| Melanoma | Mel-28 | 2.85E-09 | 1.42E-08 |
| Fibrosarcoma | SW-694 | | |
| Chondrosarcoma | CHSA | 1.63E-09 | 2.91E-08 |
| Osteosarcoma | OSA-FH | 4.37E-09 | 1.15E-08 |

| **LEUKEMIAS & LYMPHOMAS** | **LINE** | 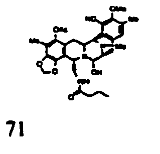 | 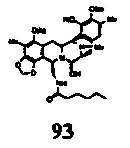 |
|---|---|---|---|
| ALL Promyelocytic leukemia | HL60 | | 1.50E-08 |
| ALL Acute lymphoblastic | Molt 3 | 1.62E-09 | 3.87E-09 |
| CML Chronic | K562 | | 6.89E-08 |
| myelogenous | | | |
| Lymphoma T-cell | H9 | | 1.08E-08 |
| Lymphoma Cutaneus T cell | Hut 78 | 7.33E-09 | 1.97E-09 |
| Lymphoma undifferentiated | MC 116 | 1.62E-08 | 3.81 E-09 |
| Lymphoma Burkitts B cellI | RAMOS | | 1.1E-09 1. 1E-09 |
| Lymphoma Histiocytic | U-937 | 1.92E-09 | 1.08E-09 |

| **LEUKEMIAS & LYMPHOMAS** | **LINE** | 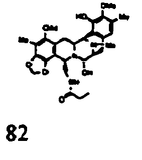 | 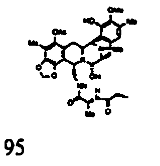 |
|---|---|---|---|
| ALL Promyelocytic leukemia | HL60 | 4.93E-10 | 7.36E-09 |
| ALL Acute lymphoblastic | Molt 3 | 9.86E-10 | 9.86E-10 |
| CML Chronic myelogenous | K562 | 1.87E-08 | 1.18E-08 |
| Lymphoma T-cell | H9 | 1.20E-08 | 2.43-08 |
| Lymphoma Cutaneus T cell | Hut 78 | | |
| Lymphoma undifferentiated | MC116 | 1.04E-09 | 1.49E-09 |
| Lymphoma Burkitts B celll | RAMOS | | 5.01E-09 |
| Lymphoma Histiocytic | U-937 | | |

| **SOLID TUMORS** | **LINE** | 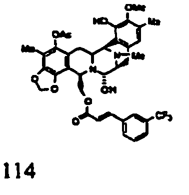 | 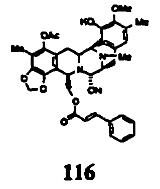 |
|---|---|---|---|
| Bladder | 5637 | 1.14E-08 | 1.71E-08 |
| Breast | MX-1 | 2.81E-08 | 7.25E-13 |
| Colon | HT-29 | 4.08E-07 | 2.96E-07 |
| Gastric | Hs746t | 3.57E-08 | 1.24E-09 |
| Liver | SK-HEP-1 | 1.63-08 | 1.94E-09 |
| NSCL | A549 | 2.81E-06 | 1.56-05 |
| Ovary | SK-OV-3 | 7.03E-06 | 7.78E-08 |
| Pancreas | PANC-1 | 1.03E-08 | 9.47E-09 |
| Pharnynx | FADU | 4.59E-07 | 2.46E-08 |
| Prostate | PC3 | 7.88E-08 | |
| Prostate | DU-145 | 7.03E-08 | 1.56E-06 |
| Prostate | LNCAP | 5.98E-07 | 6.83E-08 |
| Renal | 786-O | 1.46E-08 | 526E-12 |
| SCL | NCI-H187 | 8.02E-10 | 7.78E-14 |
| Retinoblastoma | Y-79 | 8.85E- 10 | 7.78E-14 |
| Melanoma | Mel-28 | 1.76E-08 | 5.89E-08 |
| Fibrosarcoma | SW-694 | 3.38E-06 | 6.69E-06 |
| Chondrosarcoma | CHSA | 2.53E-08 | 4.49E-08 |
| Osteosarcoma | OSA-FH | 6.34E-08 | 526E-07 |

| **SOLID TUMORS** | **LINE** | 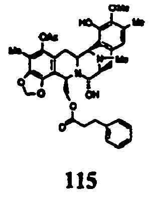 | 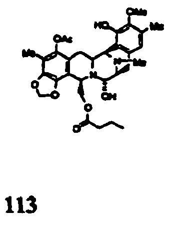 |
|---|---|---|---|
| Bladder | 5637 | 7.88E-10 | 3.02E-08 |
| Breast | MX-1 | NA | 4.75E-08 |
| Colon | HT-29 | 8.99E-09 | 1.34E-08 |
| Gastric | Hs746t | 2.95E-08 | 7.05E-07 |
| Liver | SK-HEP-1 | 1.29E-09 | 6.12E-08 |
| NSCL | A549 | 8.22E-06 | 8.49E-09 |
| Ovary | SK-OV-3 | | 3.55E-08 |
| Pancreas | PANC-1 | 5.68E-10 | 1.28E-08 |
| Pharnynx | FADU | 5.40E-11 | 2.47E-08 |
| Prostate | PC3 | 7.71E-10 | 6.18E-10 |
| Prostate | DU-145 | NA | 1. 17E-08 |
| Prostate | LNCAP | | 3.29E-07 |
| Renal | 786-O | 9.23E.10 | 1.13E-08 |
| SCL | NCI-H187 | | 2.33E-10 |
| Retinoblastoma | Y-79 | 1.03E-08 | 2.64E-09 |
| Melanoma | Mel-28 | 2.23E-08 | 1.25E-08 |
| Fibrosarcoma | SW-694 | 8.53E-06 | NA |
| Chondrosarcoma | CHSA | 1.55E-05 | 2.95E-08 |
| Osteosarcoma | OSA-FH | 1.29E-09 | 5.01E-08 |

| **LEUKEMIAS& LYMPHOMAS** | **LINE** | 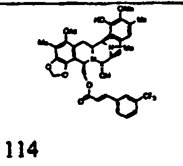 | 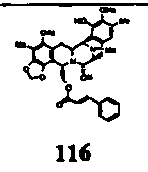 |
|---|---|---|---|
| ALL Promyelocytic leukemia | HL60 | | 1.34E-08 |
| ALL Acute lymphoblastic | Molt 3 | 1.44E-08 | 2.48E-09 |
| CML Chronic myelogenous | K562 | 1.56E-07 | 6.13E-08 |
| Lymphoma T-cell | H9 | 1.56E-07 | 1.91E-08 |
| Lymphoma Cutaneus T cell | Hut 78 | 6.47E-08 | 7.31E.09 |
| Lymphoma undifferentiated | MC116 | 1.69E-08 | 6.38E-09 |
| Lymphoma Burkitts B celll | RAMOS | 8.86E-09 | 7.15E-10 |
| Lymphoma Histiocytic | U-937 | 7.6E-08 | |

| **LEUKEMIAS & LYMPHOMAS** | **LINE** | 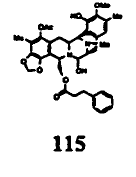 | 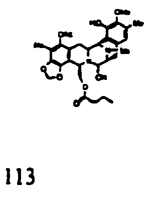 |
|---|---|---|---|
| ALL Promyelocytic leukemia | HL60 | 3.1E-09 | |
| ALL Acute lymphoblastic | Molt 3 | 8.69E-11 | 4.63E-08 |
| CML Chronic myelogenous | K562 | | 2.11E-08 |
| Lymphoma T-cell | H9 | 2.17E-08 | 6.76E-08 |
| Lymphoma Cutaneus T cell | Hut 78 | 4.81 E-08 | 2.06E-08 |
| Lymphoma undifferentiated | MC 116 | 5.27E-11 | 1.51 E-08 |
| Lymphoma Burkitts B celll | RAMOS | 1.86E-09 | 9.09E-09 |
| Lymphoma Histiocytic | U-937 | | 1.03E-08 |

### EXAMPLES OF THE INVENTION

The present invention is illustrated by the following examples.

### Example 1

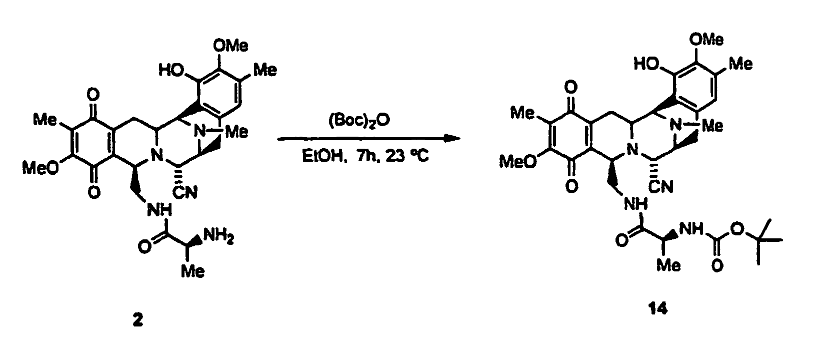

To a solution of **2** (21.53 g, 39.17 ml) in ethanol (200 ml), *tert-*butoxycarbonyl anhydride (7.7 g, 35.25 ml) was added and the mixture was stirred for 7 h at 23 °C. Then, the reaction was concentrated *in vacuo* and the residue was purified by flash column chromatography (SiO₂ hexane:ethyl acetate 6:4) to give **14** (20.6 g, 81 %) as a yellow solid.
Rf: 0.52 (ethyl acetate:CHCl₃ 5:2).
¹H NMR (300 MHz, CDCl₃): δ 6.49 (s, 1H), 6. 32 (bs, 1H), 5.26 (bs. 1H), 4.60 (bs, 1H), 4.14 (d, *J*= 2.4 Hz, 1H), 4.05 (d, *J*= 2.4 Hz, 1H), 3.94 (s. 3H), 3.81 (d, *J*= 4.8 Hz. 1H), 3.7 (s, 3H), 3.34 (br d, *J*= 7.2 Hz, 1H), 3.18-3.00 (m. 5H), 2.44 (d, *J*= 18.3 Hz, 1H), 2.29 (s, 3H), 2.24 (s, 3H), 1.82 (s, 3H), 1.80-1.65 (m, 1H). 1.48 (s, 9H), 0.86 (d. *J*= 5.7 Hz, 3H)
¹³C NMR (75 MHz, CDCl₃): δ 185.5, 180.8, 172.7. 155.9, 154.5. 147.3. 143.3. 141.5, 135.3, 130.4, 129.2, 127.5, 120.2, 117.4, 116.9, 80.2, 60.7, 60.3, 58.5, 55.9, 55.8, 54.9, 54.4, 50.0 41.6, 40.3, 28.0, 25.3, 24.0, 18.1, 15.6, 8.5.
ESI-MS m/z: Calcd. for C₃₄H₄₃N₅O₈: 649.7. Found (M+H)⁺: 650.3.

### Example 2

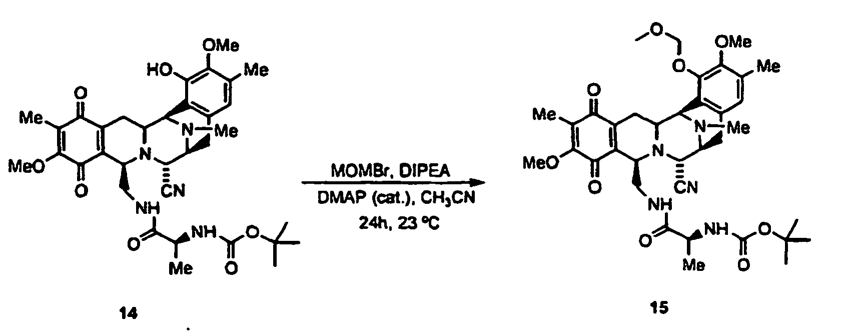

To a stirred solution of **14** (20.6 g, 31.75 ml) in CH₃CN (159 ml), diisopropylethylamine (82.96 ml, 476.2 ml), methoxymethylene bromide (25.9 ml, 317.5 ml) and dimethylaminopyridine (155 mg, 1.27 ml) were added at 0 °C. The mixture was stirred at 23 °C for 24h. The reaction was quenched at 0 °C with aqueous 0.1N HCl (750 ml) (pH = 5), and extracted with CH₂Cl₂ (2 x 400 ml). The organic phase was dried (sodium sulphate) and concentrated *in vacuo.* The residue was purified by flash column chromatography (SiO₂, gradient hexane:ethyl acetate 4:1 to hexane:ethyl acetate 3:2) to give **15** (17.6 g, 83 %) as a yellow solid.
Rf: 0.38 (hexane:ethyl acetate 3:7).
¹H NMR (300 MHz, CDCl₃): δ 6.73 (s, 1H), 5.35 (bs. 1H), 5.13 (s, 2H), 4.50 (bs. 1H), 4.25 (d, *J*= 2.7 Hz, 1H), 4.03 (d, *J=* 2.7 Hz, 1H), 3.97 (s, 3H), 3.84 (bs, 1H), 3.82-3.65 (m. 1H), 3.69 (s, 3H), 3.56 (s, 3H), 3.39-3.37 (m, 1H), 3.20-3.00 (m, 5H), 2.46 (d, *J*= 18 Hz, 1H), 2.33 (s, 3H), 2.23 (s, 3H), 1.85 (s, 3H), 1.73-1.63 (m, 1H), 1.29 (s, 9H). 0.93 (d, *J*= 5.1 Hz, 3H)
¹³C NMR (75 MHz, CDCl₃): δ 185.4, 180.9, 172.4, 155.9, 154.5, 149.0, 148.4, 141.6, 135.1, 131.0, 129.9, 127.6, 124.4, 123.7, 117.3, 99.1, 79.3, 60.7, 59.7, 58.4, 57.5, 56.2, 55.9, 55.0, 54.2, 50.0, 41.5, 39.9, 28.0, 25.2, 24.0. 18.1, 15.6, 8.5.
ESI-MS m/z: Calcd. for C₃₆H₄₇N₅O₉: 693.8. Found (M+H)⁺: 694.3.

### Example 3

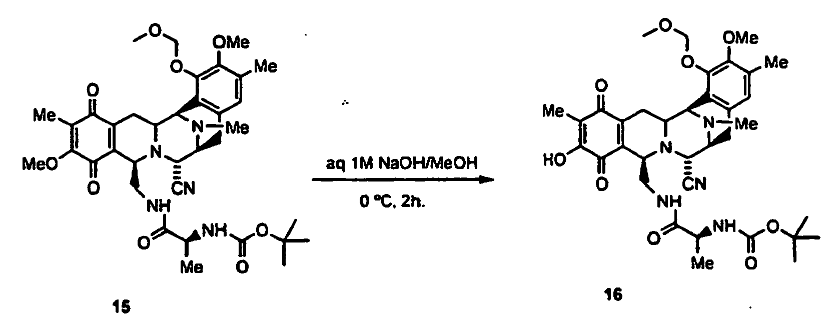

To a flask containing **15** (8 g, 1.5 ml) in methanol (1.6 l) an aqueous solution of 1M sodium hydroxide (3.2 l) was added at 0 °C. The reaction was stirred for 2h at this temperature and then, quenched with 6M HCl to pH = 5. The mixture was extracted with ethyl acetate (3x11) and the combined organic layers were dried over sodium sulphate and concentrated *in vacuo.* The residue was purified by flash column chromatography (SiO₂, gradient CHCl₃ to CHCl₃:ethyl acetate 2:1) to afford **16** (5.3 mg, 68 %).
Rf: 0.48 (CH₃CN:H₂O 7:3, RP-C18)
¹H NMR (300 MHz, CDCl₃): δ 6.73 (s, 1H), 5.43 (bs, 1H), 5.16 (s, 2H), 4.54 (bs, 1H), 4.26 (d, *J*= 1.8 Hz, 1H), 4.04 (d, *J=* 2.7 Hz 1H), 3.84 (bs, 1H), 3.80-3.64 (m, 1H), 3.58 (s, 3H), 3.41-3.39 (m, 1H), 3.22-3.06 (m, 5H), 2.49 (d, *J*= 18.6 Hz 1H), 2.35 (s, 3H), 2.30-2.25 (m, 1H), 2.24 (s, 3H), 1.87 (s, 3H), 1.45-1.33 (m, 1H), 1.19 (s, 9H), 1.00 (br d. *J*= 6.6 Hz 3H)
¹³C NMR (75 MHz, CDCl₃):δ 184.9.180.9.172.6,154.7.151.3,149.1.148.6.144.7.132.9. 131.3, 129.8, 124.5, 123.7, 117.3, 116.8, 99.1, 79.4, 59.8, 58.6, 57.7, 56.2, 55.6, 54.9, 54.5, 50.1, 41.6, 40.1, 28.0, 25.3, 24.4, 18.1, 15.7, 8.0.
ESI-MS m/z: Calcd. for C₃₅H₄₅N₅O₉: 679.7. Found (M+H)⁺ : 680.3.

### Example 4

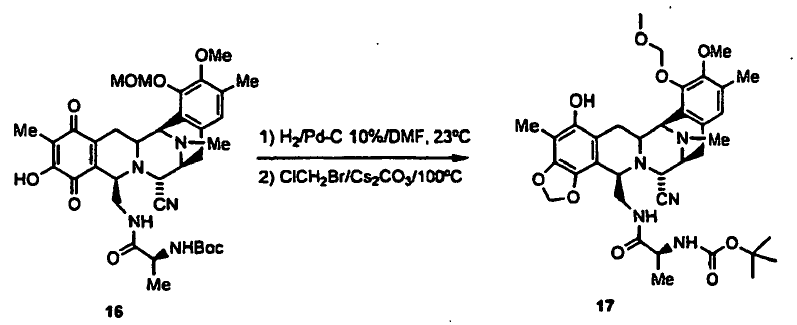

To a degassed solution of compound **16** (1.8 g, 2.64 ml) in DMF (221 ml) 10 % Pd/C (360 mg) was added and stirred under H₂ (atmospheric pressure) for 45 min. The reaction was filtered through celite under argon, to a flask containing anhydrous Cs₂CO₃ (2.58 g, 7.92 ml). Then, bromochloromethane (3.40 ml 52.8 ml), was added and the tube was sealed and stirred at 100 °C for 2h. The reaction was cooled, filtered through a pad of celite and washed with CH₂Cl₂. The organic layer was concentrated and dried (sodium sulphate) to afford **17** as a brown oil that was used in the next step with no further purification.
Rf: 0.36 (hexane:ethyl acetate 1:5, SiO₂).
¹H NMR (300 MHz. CDCl₃): δ 6.68 (s, 1H), 6.05 (bs, 1H), 5.90 (s, 1H), 5.79 (s, 1H), 5.40 (bs, 1H), 5.31-5.24 (m, 2H), 4.67 (d, *J*= 8.1 Hz, 1H), 4.19 (d, *J=* 2.7 Hz, 1H), 4.07 (bs, 1H), 4.01 (bs, 1H), 3.70 (s, 3H), 3.67 (s, 3H), 3.64-2.96 (m, 5H), 2.65 (d, *J*=18.3 Hz, 1H), 2.33 (s, 3H), 2.21 (s, 3H), 2.04 (s, 3H), 2.01-1.95 (m, 1H), 1.28 (s, 9H), 0.87 (d, *J*= 6.3 Hz, 3H)
¹³C NMR (75 MHz, CDCl₃): δ 172.1, 162.6, 154.9, 149.1, 145.7, 135.9, 130.8, 130.7, 125.1, 123.1, 117.8, 100.8, 99.8, 76.6, 59.8, 59.2, 57.7, 57.0, 56.7, 55.8, 55.2, 49.5, 41.6, 40.1, 36.5. 31.9, 31.6, 29.7, 28.2, 26.3, 25.0, 22.6, 18.2, 15.8, 14.1, 8.8.
ESI-MS m/z: Calcd. for C₃₆H₄₇N₅O₉: 693.34. Found (M+H)⁺: 694.3.

### Example 5

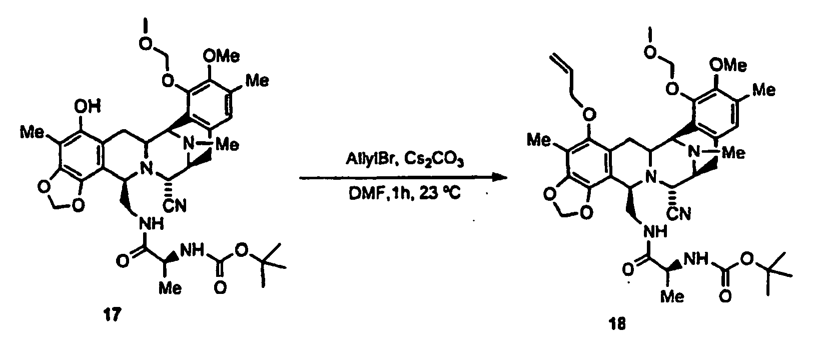

To a flask containing a solution of **17** (1.83 g, 2.65 ml) in DMF (13 ml), Cs₂CO₃ (2.6 g, 7.97 ml), and allyl bromide (1.15 ml, 13.28 ml) were added at 0° C. The resulting mixture was stirred at 23°C for 1h. The reaction was filtered through a pad of celite and washed with CH₂Cl₂. The organic layer was dried and concentrated (sodium sulphate). The residue was purified by flash column chromatography (SiO₂, CHCl₃:ethyl acetate 1:4) to afford **18** (1.08 mg, 56 %) as a white solid.
Rf: 0.36 (CHCl₃:ethyl acetate 1:3).
¹H NMR (300 MHz, CDCl₃): δ 6.70 (s, 1H), 6.27-6.02 (m, 1H), 5.94 (s, 1H), 5.83 (s, 1H), 5.37 (dd, *J₁=* 1.01 Hz, *J*₂= 16.8 Hz, 1H), 5.40 (bs,1H), 5.25 (dd, *J₁=* 1.0 Hz, *J₂=* 10.5 Hz, 1H), 5.10 (s, 2H), 4.91 (bs, 1H), 4.25-4.22 (m, 1H), 4.21 (d, *J*= 2.4 Hz, 1H), 4.14-4.10 (m, 1H), 4.08 (d, *J*=2.4 Hz, 1H), 4.00 (bs, 1H), 3.70 (s, 3H), 3.59 (s, 3H), 3.56-3.35 (m, 2H), 3.26-3.20 (m, 2H), 3.05-2.96 (dd, *J*₁*=* 8.1 Hz, *J₂=*18 Hz, 1H), 2.63 (d, *J*=18 Hz, 1H), 2.30 (s, 3H), 2.21 (s, 3H), 2.09 (s, 3H), 1.91-1.80 (m, 1H), 1.24 (s, 9H), 0.94 (d, *J*= 6.6 Hz, 3H)
¹³C NMR (75 MHz, CDCl₃): δ 172.0, 154.8, 148.8, 148.6, 148.4, 144.4, 138.8, 133.7, 130.9, 130.3, 125.1, 124.0, 120.9, 117.8, 117.4, 112.8, 112.6, 101.1, 99.2, 73.9, 59.7, 59.3, 57.7, 56.9, 56.8, 56.2, 55.2, 40.1, 34.6, 31.5, 28.1, 26.4, 25.1, 22.6, 18.5, 15.7, 14.0, 9.2.
ESI-MS m/z: Calcd. for C₃₉H₅₁N₅O₉: 733.4. Found (M+H)⁺: 734.4.

### Example 6

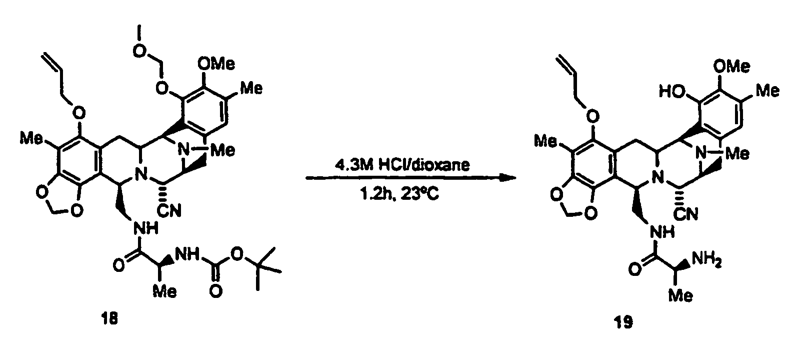

To a solution of **18** (0.1 g, 0.137 ml) in dioxane (2 ml), 4.2M HCl/dioxane (1.46 ml) was added and the mixture was stirred for 1.2h at 23 °C. The reaction was quenched at 0 °C with sat. Aqueous sodium bicarbonate (60 ml) and extracted with ethyl acetate (2x70 ml). The organic layers were dried (sodium sulphate) and concentrated *in vacuo* to afford **19** (267 mg, 95%) as a white solid that was used in subsequent reactions with no further purification.
Rf: 0.17 (ethyl acetate:methanol 10:1, SiO₂)
¹H NMR (300 MHz, CDCl₃): δ 6.49 (s, 1H), 6.12-6.00 (m, 1H), 5.94 (s, 1H), 5.86 (s, 1H), 5.34 (dd, *J*= 1.0 Hz, *J*= 17.4 Hz, 1H), 5.25 (dd, *J*= 1.0 Hz, *J*= 10.2 Hz, 1H), 4.18-3.76 (m, 5H), 3.74 (s, 3H), 3.71-3.59 (m, 1H, 3.36-3.20 (m, 4H), 3.01-2.90 (m, 1H), 2.60 (d, *J*= 18.0 Hz, 1H), 2.29 (s, 3H), 2.24 (s, 3H), 2.11 (s, 3H), 1.97-1.86 (m, 1H), 0.93 (d, *J*= 8.7 Hz, 3H)
¹³CNMR (75 MHz, CDCl₃): δ 175.5, 148.4, 146.7, 144.4, 142.4, 138.9, 133.7, 131.3, 128.3, 120.8, 117.9, 117.4, 113.8, 112.4, 101.1, 74.2, 60.5, 59.1, 56.5, 56.1, 56.3, 56.0, 50.5, 41.6, 39.5, 29.5, 26.4, 24.9, 21.1, 15.5, 9.33.
ESI-MS m/z: Calcd. for C₃₂H₃₉N₅O₆: 589. Found (M+H)⁺: 590.

### Example 7

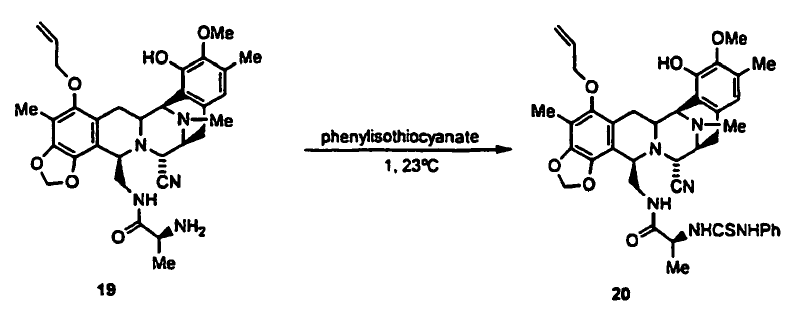

To a solution of **19** (250 mg, 0.42 ml) in CH₂Cl₂ (1.5 ml), phenyl isothiocyanate (0.3 ml, 2.51 ml) was added and the mixture was stirred at 23° C for 1h. The reaction was concentrated *in vacuo* and the residue was purified by flash column chromatography (SiO₂, gradient Hexane to 5:1 hexane:ethyl acetate) to afford **20** (270 mg, 87 %) as a white solid.
Rf: 0.56 (CHCl₃:ethyl acetate 1:4).
¹H NMR (300 MHz, CDCl₃): δ 8.00 (bs, 1H), 7.45-6.97 (m, 4H), 6.10 (s, 1H), 6.08-6.00 (m 1H), 5.92 (s, 1H), 5.89 (s, 1H), 5.82 (s, 1H), 5.40 (dd, *J=* 1.5 Hz, *J*= 17.1 Hz, 1H), 3.38 (bs, 1H), 5.23 (dd, *J*= 1.5 Hz, *J*= 10.5 Hz,1H), 4.42-4.36 (m,1H), 4.19-4.03 (m, 5H), 3.71 (s, 3H), 3.68-3.17 (m, 4H), 2.90 (dd. *J*=7.8 *Hz, J=* 18.3 Hz, 1H), 2.57 (d, *J*= 18.3 Hz, 1H), 2.25 (s, 3H), 2.12 (s, 3H), 2.10 (s, 3H), 1.90 (dd, *J*= 12.3 Hz, *J=* 16.5 Hz, 1H), 0.81 (d, *J=* 6.9 Hz, 3H).
¹³C NMR (75 MHz, CDCl₃): δ 178.4, 171.6, 148.6, 146.8, 144.3, 142.7, 138.7, 136.2, 133.6, 130.7, 129.8, 126.6, 124.2, 124.1, 120.9, 120.5, 117.7, 117.4, 116.7, 112.6, 112.5, 101.0, 74.0, 60.6, 59.0, 57.0, 56.2, 56.1, 55.0, 53.3, 41.4, 39.7, 26.3, 24.8, 18.3, 15.5, 9.2.
ESI-MS m/z: Calcd: for C₃₉H₄₄N₆O₆S: 724.8 Found (M+H)⁺: 725.3.

### Example 8

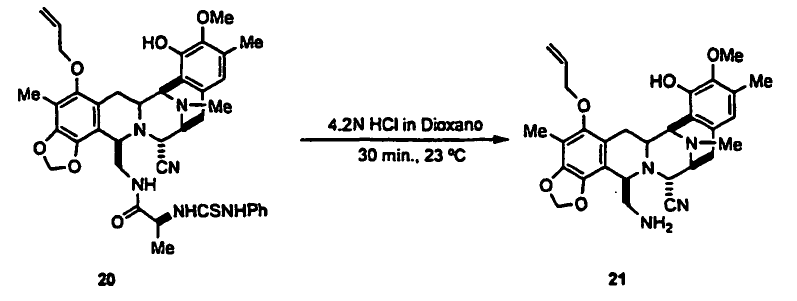

To a solution of **20** (270 mg, 0.37 ml) in dioxane (1 ml), 4.2N HCl/dioxane (3.5 ml) was added and the reaction was stirred at 23 °C for 30 min. Then, ethyl acetate (20 ml) and H₂O (20 ml) were added and the organic layer was decanted. The aqueous phase was basified with saturated aqueous sodium bicarbonate (60 ml) (pH = 8) at 0 °C and then, extracted with CH₂Cl₂ (2 x 50 ml). The combined organic extracts were dried (sodium sulphate), and concentrated *in vacuo.* The residue was purified by flash column chromatography (SiO₂, ethyl acetate:methanol 5:1) to afford compound **21** (158 mg, 82%) as a white solid.
Rf: 0.3 (ethyl acetate:methanol 1:1).
¹H NMR (300 MHz, CDCl₃): δ 6.45 (s, 1H), 6.12-6.03 (m, 1H), 5.91 (s, 1H), 5.85 (s, 1H), 5.38 (dd*, J₁*= 1.2 Hz, *J₂=* 17.1 Hz, 1H), 5.24 (dd, *J₁ =* 1.2 Hz, *J*₂= 10.5 Hz, 1H), 4.23-4.09 (m, 4H), 3.98 (d, *J*= 2.1 Hz, 1H), 3.90 (bs, 1H), 3.72 (s, 3H), 3.36-3.02 (m, 5H), 2.72-2.71 (m, 2H), 2.48 (d, *J*= 18.0 Hz, 1H), 2.33 (s, 3H), 2.22 (s, 3H), 2.11 (s, 3H), 1.85 (dd, *J*₁= 11.7 Hz, *J₂=* 15.6 Hz, 1H)).
¹³C NMR (75 MHz, CDCl₃): δ 148.4, 146.7, 144.4, 142.8, 138.8, 133.8, 130.5,128.8,121.5, 120.8, 118.0, 117.5, 116.9, 113.6, 112.2, 101.1, 74.3, 60.7, 59.9, 58.8, 56.6, 56.5, 55.3,44.2, 41.8, 29.7, 26.5, 25.7, 15.7, 9.4.
ESI-MS m/z: Calcd. for C₂₉H₃₄N₄O₅: 518.3. Found (M+H)⁺: 519.2.

### Example 9

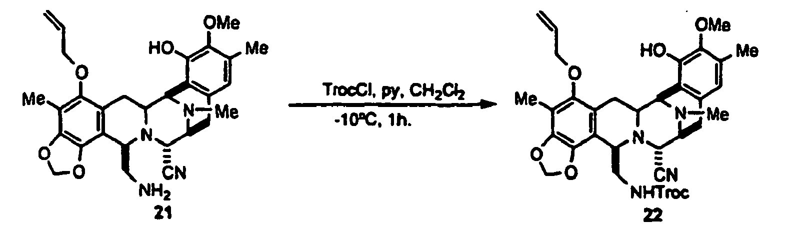

To a solution of **21** (0.64 g, 1.22 ml) in CH₂Cl₂ (6.13 ml), pyridine (0.104 ml, 1.28 ml) and 2,2,2-trichloroethyl chloroformate (0.177 ml, 1.28 ml) were added at -10 °C. The mixture was stirred at this temperature for 1h and then, the reaction was quenched by addition of 0.1N HCl (10 ml) and extracted with CH₂Cl₂ (2 x 10 ml). The organic layer was dried over sodium sulphate and concentrated *in vacuo.* The residue was purified by flash column chromatography SiO₂, (hexane:ethyl acetate 1:2) to afford **22** (0.84 g, 98%) as a white foam solid.
Rf: 0.57 (ethyl acetate:methanol 5:1).
¹H NMR (300 MHz, CDCl₃): δ 6.50 (s, 1H), 6.10-6.00 (m, 1H), 6.94 (d, *J=* 1.5 Hz, 1H), 5.87 (d, *J*= 1.5 Hz, 1H), 5.73 (bs, 1H), 5.37 (dq, *J₁*= 1.5 Hz, *J₂*= 17.1 Hz, 1H), 5.26 (dq, *J₁*= 1.8 Hz, *J*₂= 10.2 Hz, 1H), 4.60 (d, *J=* 12 Hz, 1H), 4.22-4.10 (m, 4H), 4.19 (d, *J*= 12 Hz, 1H), 4.02 (m, 2H), 3.75 (s, 3H), 3.37-3.18 (m, 5H), 3.04 (dd, *J₁*= 8.1 Hz, *J₂*= 18 Hz, 1H), 2.63 (d, *J=* 18 Hz, 1H), 2.31 (s, 3H), 2.26 (s, 3H), 2.11 (s, 3H), 1.85 (dd, *J₁*= 12.3 Hz, *J₂=* 15.9 Hz, 1H).
¹³C NMR (75 MHz, CDCl₃) δ 154.3, 148.5, 146.7, 144.5, 142.8, 139.0, 133.8, 130.7, 128.7, 121.3, 120.8, 117.8, 117.7,116.8, 112.7, 101.2, 77.2, 74.3, 60.7, 59.9, 57.0, 56.4, 55.3, 43.3, 41.7, 31.6, 26.4, 25.3, 22.6, 15.9, 14.1, 9.4.
ESI-MS m/z: Calcd. for C₃₂H₃₅Cl₃N₄O₇: 694.17. Found (M+H)⁺: 695.2.

### Example 10

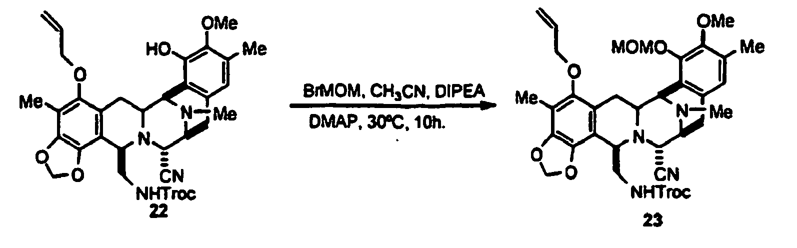

To a solution of **22** (0.32 g, 0.46 ml) in CH₃CN (2.33 ml), diisopropylethylamine (1.62 ml, 9.34 ml), bromomethyl methyl ether (0.57 ml, 7.0 ml) and dimethylaminopyridine (6 mg, 0.046 ml) were added at 0 °C. The mixture was heated at 30 °C for 10h. Then, the reaction was diluted with dichloromethane (30 ml) and poured in an aqueous solution of HCl at pH = 5 (10 ml). The organic layer was dried over sodium sulphate and the solvent was eliminated under reduced pressure to give a residue which was purified by flash column chromatography (SiO₂, hexane:ethyl acetate 2:1) to afford **23** (0.304 g, 88%) as a white foam solid.
Rf: 0.62 (hexane:ethyl acetate 1:3).
¹H NMR (300 MHz, CDCl₃): 6 6.73 (s, 1H), 6.10 (m, 1H), 5.94 (d, *J*= 1.5 Hz, 1H), 5.88 (d, *J=* 1.5 Hz, 1H), 5.39 (dq, *J₁=* 1.5 Hz, *J₂=* 17.1 Hz, 1H), 5.26 (dq, *J₁=* 1.8 Hz, *J*₂= 10.2 Hz, 1H), 5.12 (s, 2H), 4.61 (d, *J*= 12 Hz, 1H), 4.55 (t, *J=* 6.6 Hz, 1H), 4.25 (d, *J=* 12 Hz, 1H), 4.22-4.11 (m, 4H), 4.03 (m, 2H), 3.72 (s, 3H), 3.58 (s, 3H), 3.38-3.21 (m. 5H), 3.05 (dd, *J₁*= 8.1 Hz, *J₂=* 18 Hz, 1H), 2.65 (d, *J*= 18 Hz, 1H), 2.32 (s, 3H), 2.23 (s, 3H), 2.12 (s, 3H), 1.79 (dd, *J₁=* 12.3 Hz, *J₂=* 15.9 Hz, 1H);
¹³C NMR (75 MHz, CDCl₃) δ 154.3, 148.6, 148.4, 144.5, 139.0, 133.6, 130.6, 130.1, 125.07, 124.7, 124.0, 121.1, 117.7, 112.6, 101.2, 99.2, 77.2, 74.4, 74.1, 59.8, 59.8, 57.7, 57.0, 56.8, 56.68, 553, 43.2, 41.5, 26.4, 25.2, 15.9, 9.3.
ESI-MS m/z: Calcd. for C₃₄H₃₉Cl₃N₄O₈: 738.20. Found (M+H)⁺: 739.0.

### Example 11

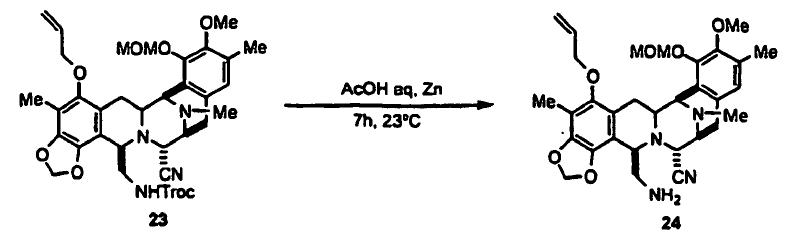

To a suspension of **23** (0.304 g, 0.41 ml) in 90% aqueous acetic acid (4 ml), powder zinc (0.2 g, 6.17 ml) was added and the reaction was stirred for 7 hour at 23 °C. The mixture was filtered through a pad of celite which was washed with CH₂Cl₂- The organic layer was washed with an aqueous sat. solution of sodium bicarbonate (pH = 9) (15 ml) and dried over sodium sulphate. The solvent was eliminated under reduced pressure to give **24** (0.191 g, 83%) as a white solid.
Rf: 0.3 (ethyl acetate:methanol 5:1).
¹H NMR (300 MHz, CDCl₃): δ 6.68 (s, 1H), 6.09 (m, 1H), 5.90 (d, *J*= 1.5 Hz, 1H), 5.83 (d, *J=* 1.5 Hz, 1H), 5.39 (dq, *J₁=* 1.5 Hz, *J₂=* 17.1 Hz, 1H), 5.25 (dq, *J₁=* 1.5 Hz, *J₂=* 10.2 Hz, 1H), 5.10 (s, 2H), 4.22-4.09 (m, 3H), 3.98 (d, *J*= 2.4 Hz, 1H), 3.89 (m, 1H), 3.69 (s, 3H), 3.57 (s, 3H), 3.37-3.17 (m, 3H), 3.07 (dd, *J₁=* 8.1 Hz, *J₂=* 18 Hz, 1H), 2.71 (m, 2H), 2.48 (d, *J*= 18 Hz, 1H), 2.33 (s, 3H), 2.19 (s, 3H), 2.17 (s, 3H), 1.80 (dd, *J₁*= 12.3 Hz,*J₂=* 15.9 Hz,
¹³C NMR (75 MHz, CDCl₃): δ 148.5, 148.2, 144.3,138.7, 133.7,130.7, 129.9, 125.0, 123.9, 121.3, 117.9, 117.5, 113.6, 112.0, 101.0, 99.2, 74.0, 59.8, 59.7, 58.8, 57.6, 57.0, 56.2, 55.2, 44.2, 41.5, 31.5, 26.4, 25.6, 22.5, 16.7, 14.0, 9.2.
ESI-MS m/z: Calcd. for C₃₁H₃₈N₄O₆ : 562.66. Found (M+H)⁺ : 563.1.

### Example 12

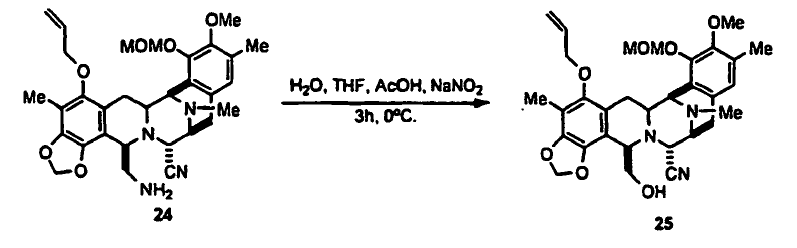

To a solution of **24** (20 mg, 0.035 ml), in H₂O (0.7 ml) and THF (0.7 ml). NaNO₂ (12 mg, 0.17 ml) and 90% aqueous AcOH (0.06 ml) were added at 0 °C and the mixture was stirred at 0 °C for 3h. After dilution with CH₂Cl₂ (5 ml), the organic layer was washed with water (1ml), dried over sodium sulphate and concentrated *in vacuo.* The residue was purified by flash column chromatography (SiO₂, hexane:ethyl acetate 2:1) to afford **25** (9.8 mg, 50%) as a white solid.
Rf: 0.34 (hexane:ethyl acetate 1:1).
¹H NMR (300 MHz, CDCl₃): δ 6.71 (s, 1H). 6.11 (m, 1H), 5.92 (d, *J*= 1.5 Hz 1H). 5.87 (d, *J=* 1.5 Hz, 1H), 5.42 (dq, *J₁=* 1.5 Hz, *J*₂= 17.1 Hz, 1H), 5.28 (dq, *J₁=* 1.5 Hz *J₂=* 10.2 Hz, 1H), 5.12 (s, 2H), 4.26-4.09 (m, 3H), 4.05 (d, *J*= 2.4 Hz, 1H), 3.97 (t, *J*= 3.0 Hz 1H), 3.70 (s, 3H), 3.67-3.32 (m, 4H), 3.58 (s, 3H), 3.24 (dd, J₁= 2.7 Hz, *J₂* = 15.9 Hz 1H). 3.12 (dd, *J₁=* 8.1 Hz*, J₂*= 18.0 Hz, 1H), 2.51 (d, *J*= 18 Hz, 1H), 2.36 (s, 3H), 2.21 (s, 3H), 2.12 (s, 3H), 1.83 (dd, *J₁*= 12.3 Hz, *J₂*= 15.9 Hz,1H)
¹³C NMR (75 MHz, CDCl₃) δ 148.7, 148.4, 138.9, 133.7, 131.1, 129.4, 125.1, 123.9, 120.7, 117.6, 117.5, 113.2, 112.3, 101.1, 99.2, 74.0, 63.2, 59.8, 59.7, 57.9, 57.7, 57.0, 56.5, 55.2, 41.6, 29.6, 26.1, 25.6, 22.6, 15.7, 9.2.
ESI-MS m/z: Calcd. for C₃₁H₃₇N₃O₇: 563.64. Found (M+H)⁺: 564.1.

### Example 13

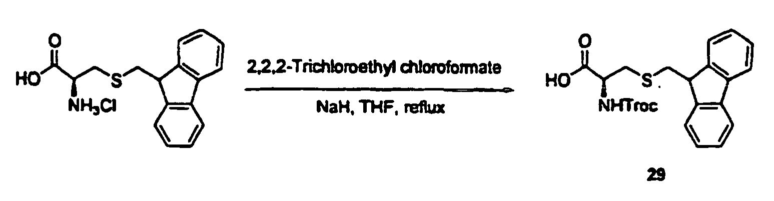

The starting material (2.0 g, 5.90 ml) was added to a suspension of sodium hydride (354 mg, 8.86 ml) in THF (40 ml) at 23 °C, following the suspension was treated with allyl chloroformate (1.135 ml, 8.25 ml) at 23 °C and then refluxed for 3 hours. The suspension was cooled, filtered off, the solid washed with ethyl acetate (100 ml), and the filtrate was concentrated. The oil crude was ground with hexane (100 ml) and kept at 4°C overnight. After, the solvent was decanted and the light yellow slurry was treated with CH₂Cl₂ (20 ml), and precipitated with hexane (100 ml). After 10 minutes, the solvent was decanted again. The operation was repeated until appearing a white solid. The white solid was filtered off and dried to afford compound **29** (1.80 g, 65%) as a white solid.
¹H-NMR (300 MHz, CDCl₃): δ 7.74 (d, *J*= 7.5 Hz, 2H), 7.62 (d, *J*= 6.9 Hz, 2H), 7.33 (t, *J*= 7.5 Hz, 2H), 7.30 (t, *J*= 6.3 Hz, 2H), 5.71 (d, *J*= 7.8 Hz, 1H), 4.73 (d, *J*= 7.8 Hz, 2H), 4.59 (m, 1H), 4.11 (t, *J*= 6.0 Hz, 1H), 3.17 (dd, *J=* 6.0 Hz, *J*= 2.7 Hz, 2H), 3.20 (dd, *J=* 5.4 Hz, *J*= 2.1 Hz, 2H).
¹³C-NMR (75 MHz, CDCl₃): δ 173.6, 152.7, 144.0, 139.7, 137.8, 126.0, 125.6, 123.4, 118.3, 73.4, 52.4, 45.5, 35.8, 33.7.
ESI-MS m/z: Calcd.. for C₂₀H₁₈Cl₃NO₄S: 474.8. Found (M+Na)⁺: 497.8

### Example 14

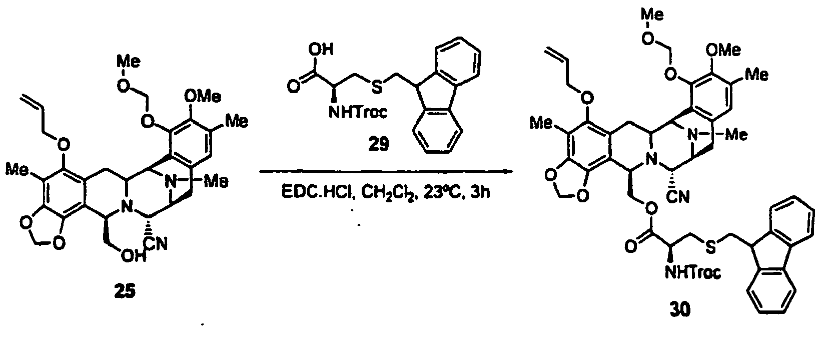

A mixture of compound **25** (585 mg, 1.03 ml) and compound **29** (1.47 mg, 3.11 ml) were azeotroped with anhydrous toluene (3 x 10 ml). To a solution of **25** and **29** in anhydrous CH₂Cl₂ (40 ml) was added DMAP (633 mg, 5.18 ml) and EDC·HCl (994 mg, 5.18 ml) at 23 °C. The reaction mixture was stirred at 23 °C for 3 hours. The mixture was partitioned with saturated aqueous solution of sodium bicarbonate (50 ml ) and the layers were separated. The aqueous layer was washed with CH₂Cl₂ (50 ml). The combined organic layers were dried over sodium sulphate, filtered and concentrated. The crude was purified by flash column chromatography (ethyl acetate/hexane 1:3) to obtain **30** (1.00 g, 95%) as a pale cream yellow solid.
¹H-NMR (300 MHz, CDCl₃): δ 7.72 (m, 2H), 7.52 (m, 2H), 7.38 (m, 2H), 7.28 (m, 2H), 6.65 (s,1H), 6.03 (m, 1H), 5.92 (d, *J*= 1.5 Hz, 1H), 5.79 (d, *J*= 1.5 Hz,1H), 5.39 (m, 1H), 5.29 (dq, *J*= 10.3 Hz, *J*= 1.5 Hz, 1H), 5.10 (s, 2H), 4.73 (d, *J*= 11.9 Hz, H), 4.66 (d, *J*= 11.9 Hz, 1H), 4.53 (m, 1H), 4.36-3.96 (m, 9H), 3.89 (t, *J*= 6.4 Hz, 1H), 3.71 (s, 3H), 3.55 (s, 3H), 3.33 (m, 1H), 3.20 (m, 2H), 2.94 (m, 3H), 2.59 (m, 1H), 2.29 (s, 3H), 2.23 (s, 3H), 2.02 (s, 3H), 1.83 (dd, *J*= 16.0 Hz, *J*= 11.9 Hz, 1H).
¹³C-NMR (75 MHz, CDCl₃): δ 169.7, 154.0, 148.8, 148.4, 145.7, 144.5, 140.9. 139.0, 133.7, 130.9, 130.6, 127.6, 127.0, 124.8, 124.6, 124.1, 120.8, 119.9, 118.2, 117.7, 117.3, 112.7, 112.1, 101.3, 99.2, 74.7, 73.9, 64.4, 59.8, 57.7, 57.0, 56.8, 55.4, 53.3, 46.7, 41.4, 36.5, 34.7, 31.5, 26.4, 24.9, 22.6, 15.7, 14.0, 9.1.
ESI-MS m/z: Calcd., for C₅₁H₅₃Cl₃N₄O₁₀S: 1020.4. Found (M+H)⁺: 1021.2

### Example 15

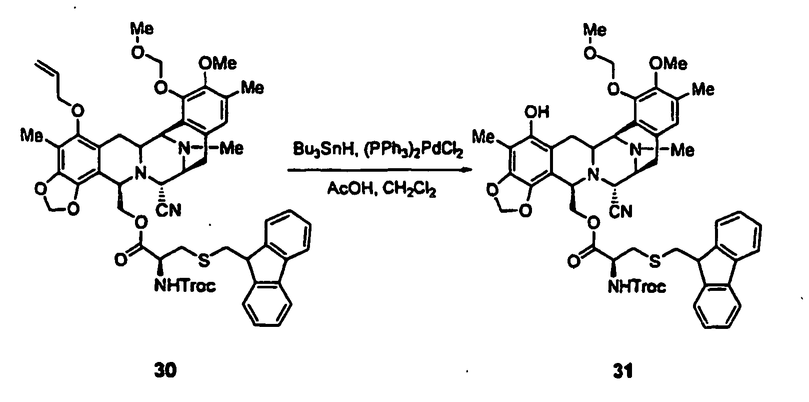

To a solution of **30** (845 mg, 0.82 ml), acetic acid (500 mg, 8.28 ml) and (PPh₃)₂PdCl₂ (29 mg, 0.04 ml) in anhydrous CH₂Cl₂ 20 ml at 23 °C was added, dropwise, Bu₃SnH (650 mg, 2.23 ml). The reaction mixture was stirred at this temperature for 15 min., bubbling was. The crude was quenched with water (50ml) and extracted with CH₂Cl₂ (3 x 50 ml). The organic layers were dried over sodium sulphate, filtered and concentrated. The crude was purified by flash column chromatography (ethyl acetate/hexane in gradient from 1:5 to 1:3) to obtain compound **31** (730 mg, 90%) as a pale cream yellow solid.
¹H-NMR (300 MHz, CDCl₃): δ 7.72 (m, 2H), 7.56 (m, 2H), 7.37 (m, 2H), 7.30 (m. 2H), 6.65 (s, 1H), 5.89 (s, 1H), 5.77 (s, 1H), 5.74 (s, 1H), 5.36 (d, *J*= 5.9 Hz, 1H), 5.32 (d, *J*= 5.9 Hz 1H), 5.20 (d, *J=* 9.0, 1H), 4.75 (d, *J*= 12.0 Hz, 1H), 4.73 (m, 1H), 4.48 (d. *J=* 11.9 Hz, 1H), 4.08 (m, 4H), 3.89 (m, 1H), 3.86, (t, *J*= 6.2 Hz, 1H), 3.70 (s, 3H), 3.69 (s, 3H), 3.38 (m, 1H), 3.25 (m, 1H), 3.02-2.89 (m, 4H), 2.67 (s, 1H), 2.61 (s, 1H), 2.51 (dd, *J*= 14.3 Hz, *J*= 4.5 Hz, 1H), 2.29 (s, 3H), 2.23 (s, 3H), 1.95 (s, 3H), 1.83 (m, 1H).
¹³C-NMR (75 MHz, CDCl₃): δ 168.2, 152.5, 148.1, 146.2, 144.4, 144.3, 143.3, 139.6, 134.6, 129.7, 129.6, 126.2, 125.6, 123.4, 123.3, 121.6, 118.5, 116.3, 110.7, 110.2, 105.1, 99.4, 98.5, 75.2, 73.3, 61.7, 58.4, 57.9, 56.3, 56.1, 55.1, 54.7, 53.9, 51.9, 45.2, 40.1, 35.6, 33.3, 24.8, 23.3., 14.5, 7.3.
ESI-MS m/z: Calcd.. for C₄₈H₄₉Cl₃N₄O₁₀S: 980.3. Found (M+H)⁺: 981.2

### Example 16

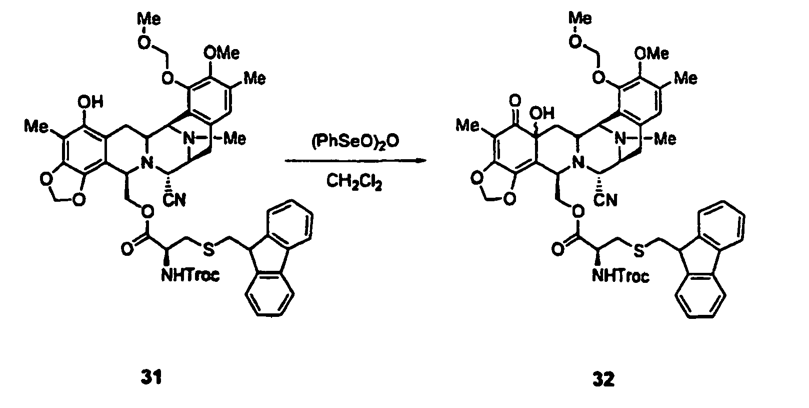

To a solution of **31** (310 mg, 0.32 ml), in anhydrous CH₂Cl₂ (15 ml) at -10°C was added a solution of benzeneseleninic anhydride 70 % (165 mg, 0.32 ml), in anhydrous CH₂Cl₂ (7 ml), *via* cannula, keeping the temperature at -10 °C. The reaction mixture was stirred at -10 °C for 5 min. A saturated solution of sodium bicarbonate (30 ml) was added at this temperature. The aqueous layer was washed with more CH₂Cl₂ (40 ml). The organic layers were dried over sodium sulphate, filtered and concentrated. The crude was purified by flash column chromatography (ethyl acetate/hexane in gradient from 1:5 to 1:1) to obtain 32 (287 mg, 91%, HPLC: 91.3%) as a pale cream yellow solid and as a mixture of two isomers (65:35) which were used in the next step.
¹H-NMR (300 MHz, CDCl₃): δ (Mixture of isomers) 7.76 (m, 4H), 7.65 (m, 4H). 7.39 (m, 4H), 7.29 (m, 4H), 6.62 (s, 1H), 6.55 (s, 1H), 5.79-5.63 (m, 6H), 5.09 (s, 1H). 5.02 (d, *J*= 6.0 Hz, 1H), 4.99 (d, *J=* 6.0 Hz, 1H), 4.80-4.63 (m, 6H), 4.60 (m, 1H), 4.50 (m, 1H). 4.38 (d, *J=* 12.8 Hz, *J=* 7.5 Hz, 1H), 4.27 (dd, *J=* 12.8 Hz, *J=* 7.5 Hz, 1H), 4.16-3.90 (m, 10H), 3.84 (s, 3H), 3.62 (s, 3H), 3.50 (s, 3H), 3.49 (s, 3H), 3.33-2.83 (m, 14H), 2.45-2.18 (m, 2H), 2.21 (s, 6H), 2.17 (s, 6H),1.77 (s, 6H), 1.67 (m, 2H).
C-NMR (75 MHz, CDCl₃): δ (Mixture of isomers) 168.6, 168.4, 158.6, 154.8, 152.8, 152.5, 147.3, 147.2, 146.8, 144.1, 144.0, 140.8, 139.7, 137.1, 129.8, 129.3. 128.4, 128.7, 126.5, 125.5, 123.7, 123.6, 123.5, 123.4, 122.2, 121.3, 118.3, 115.8, 115.5, 110.2, 106.9, 103.5, 103.2, 100.1, 99.6, 97.9, 97.7, 93.8, 73.4, 70.9, 69.2, 64.9, 62.5, 59.3, 58.9, 58.4, 56.7, 56.3, 56.2, 55.4, 55.2, 55.1, 54.9, 54.7, 54.3, 54.1, 53.8, 52.8, 45.5, 40.5, 40.0, 39.8, 35.8, 35.5, 33.9, 33.7, 30.1, 28.8, 24.2, 24.1, 21.2, 14.5, 14.4, 12.7, 6.0, 5.7.
ESI-MS m/z: Calcd. for C₄₈H₄₉Cl₃N₄O₁₁S: 996.3. Found (M+H)⁺: 997.2

### Example 17

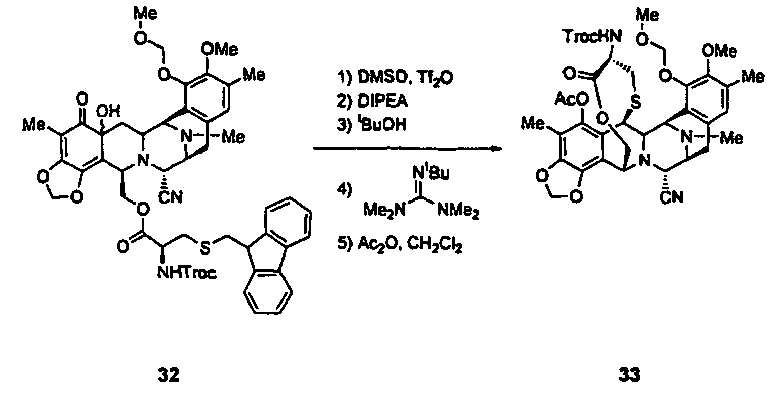

The reaction flask was flamed twice, purged vacuum/Argon several times and kept under Argon atmosphere for the reaction. To a solution of DMSO (39.1 ml, 0.55 ml, 5 equivalents.) in anhydrous CH₂Cl₂ (4.5 ml) was dropwise added triflic anhydride (37.3 ml, 0.22 ml, 2 equivalents.) at -78 °C . The reaction mixture was stirred at -78 °C for 20 minutes, then a solution of **32** (110 mg, 0.11 ml, HPLC: 91.3%) in anhydrous CH₂Cl₂ (1 ml, for the main addition and 0.5 ml for wash) at -78 °C was added, *via* cannula. During the addition the temperature was kept at -78 °C in both flasks and the colour changed from yellow to brown. The reaction mixture was stirred at -40 °C for 35 minutes. During this period of time the solution was turned from yellow to dark green. After this time, ⁱPr₂NEt (153 ml, 0.88 ml, 8 equivalents.) was dropwise added and the reaction mixture was kept at 0 °C for 45 minutes, the colour of the solution turned to brown during this time. Then t-butanol (41.6 ml, 0.44 ml, 4 equivalents.) and 2-^{t}Butyl-1,1,3,3-tetramethylguanidine (132.8 ml, 0.77 ml, 7 equivalents.) were dropwise added and the reaction mixture was stirred at 23 °C for 40 minutes. After this time, acetic anhydride (104.3 ml, 1.10 ml, 10 equivalents.) was dropwise added and the reaction mixture was kept at 23 °C for 1 hour more. Then the reaction mixture was diluted with CH₂Cl₂ (20ml) and washed with aqueous saturated solution of NH₄Cl (50ml), sodium bicarbonate (50ml), and sodium chloride (50ml). The combined organic layers were dried over sodium sulphate, filtered and concentrated. The residue was purified by flash column chromatography (eluent: ethyl acetate/hexane gradient from 1:3 to 1:2) to afford compound **33** (54 mg, 58%) as a pale yellow solid.
¹H-NMR (300 MHz, CDCl₃): δ 6.85 (s, 1H), 6.09 (s, 1H), 5.99 (s, 1H), 5.20 (d, *J=* 5.8 Hz, 1H), 5.14 (d, *J=* 5.3 Hz, 1H). 5.03 (m, 1H), 4.82 (d, *J=* 12.2, 1H), 4.63 (d, *J*= 12.0 Hz,1H), 4.52 (m, 1H), 4.35-4.17 (m, 4H), 3.76 (s, 3H), 3.56 (s, 3H), 3.45 (m, 2H), 2.91 (m, 2H), 2.32 (s, 3H), 2.28 (s, 3H), 2.21 (s, 3H), 2.12 (m, 2H), 2.03 (s. 3H).
¹³C-NMR (75 MHz, CDCl₃): δ 168.5,167.2,152.7. 148.1, 147.1, 144.5, 139.6. 139.1. 130.5. 129.0,123.7,123.5,123.3,118.8, 116.5,112.1, 100.6. 97.8, 73.3, 60.5, 59.4,59.2, 58.3. 57.6,57.4,56.1,53.3.53.1,40.6.40.0.31.0.22.2.18.9, 14.4.8.1.
ESI-MS m/z: Calcd.. for C₃₆H₃₉Cl₃N₄O₁₁S: 842.1. Found (M+H)⁺: 843.1

### Example 18

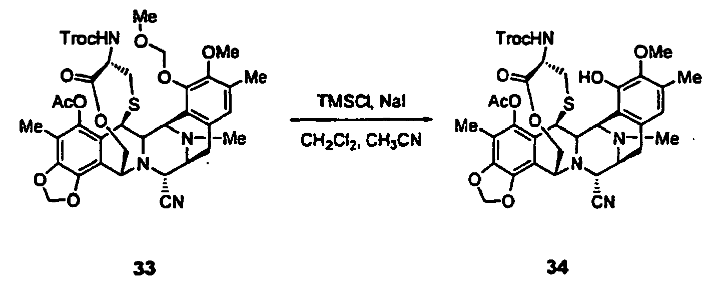

To a solution of **33** (12 mg, 0.014 ml)in dry dichloromethane (1,2 ml) and HPLC grade acetonitrile (1.2 ml) was added at 23 °C sodium iodide (21 mg, 0.14 ml) and freshly distilled (over calcium hydride at atmospheric pressure) trimethylsilyl chloride (15.4 mg, 0.14ml). The reaction mixture turned to orange colour. After 15 min the solution was diluted with dichloromethane (10 ml) and was washed with a freshly aqueous saturated solution of Na₂S₂O₄ (3 x 10 ml). The organic layer was dried over sodium sulphate, filtered and concentrated. It was obtained compound **34** (13 mg, quantitative) as pale yellow solid which was used without further purification.
¹H-NMR (300 MHz, CDCl₃): δ 6.85 (s,1H), 6.09 (s,1H), 5.99 (s, 1H), 5.27 (d, *J*= 5.8 Hz, 1H), 5.14 (d, *J=* 53 Hz,1H), 5.03 (d, *J*= 11.9 Hz, 1H), 4.82 (d, *J*= 12.2, 1H), 4.63 (d, *J*= 13.0 Hz, 1H), 4.52 (m, 1H), 4.34 (m, 1H), 4.27 (bs, 1H), 4.18 (m, 2H), 3.76 (s, 3H), 3.56 (s. 3H), 3.44 (m, 1H), 3.42 (m, 1H), 2.91 (m, 2H), 2.32 (s, 3H), 2.28 (s, 3H), 2.21 (s, 3H), 2.03 (s, 3H).
ESI-MS m/z: Calcd. for C₃₄H₃₅N₄O₁₀S: 798.1. Found (M+H)⁺: 799.1

### Example 19

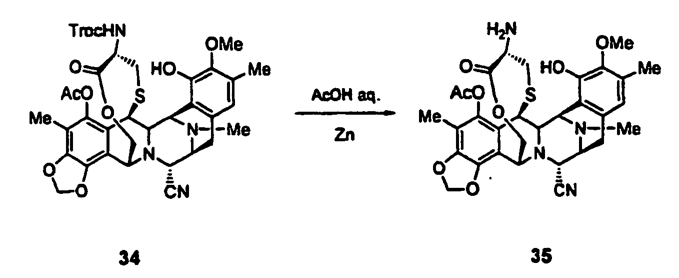

To a solution of **34** (13 mg, 0.016 ml) in a mixture of acetic acid/H₂O (90:10.1 ml) was added powder Zinc (5.3 mg, 0.081 ml) at 23 °C. The reaction mixture was heated at 70 °C for 6 h. After this time, was cooled to 23 °C, diluted with CH₂Cl₂ (20 ml) and washed with aqueous saturated solution of sodium bicarbonate (15 ml) and aqueous solution of Et₃N (15 ml). The organic layer was dried over sodium sulphate, filtered and concentrated. The residue was purified by flash column chromatography with Silica-NH₂ (eluent: ethyl acetate/hexane gradient from 0:100 to 50:50) to afford compound **35** (6.8 mg, 77% for two steps) as a pale yellow solid.
¹H-NMR (300 MHz, CDCl₃): δ 6.51 (s,1H), 6.03 (dd, *J*= 1.3 Hz, *J*= 26.5 Hz, 2H), 5.75 (bs, 1H), 5.02 (d, *J*= 11.6 Hz, 1H), 4.52 (m, 1H), 4.25 (m, 2H), 4.18 (d, *J*= 2.5 Hz, 1H), 4.12 (dd, *J*= 1.9 Hz, *J*= 11.5 Hz, 1H), 3.77 (s, 3H), 3.40 (m, 2H), 3.26 (t, *J*= 6.4 Hz, 1H), 2. 88 (m, 2H), 2.30-2.10 (m, 2H), 2.30 (s, 3H), 2.28 (s, 3H), 2.18 (s, 3H), 2.02 (s, 3H).
¹³C-NMR (75 MHz, CDCl₃): δ 174.1, 168.4, 147.8, 145.4, 142.9, 140.8, 140.1, 131.7, 130.2, 129.1, 128.3, 120.4, 118.3, 117.9, 113.8, 111.7, 101.7, 61.2, 59.8, 59.2, 58.9, 54.4, 53.8, 54.4, 41.3, 41.5, 34.1, 23.6, 20.3, 15.5, 9.4.
ESI-MS m/z: Calcd. for C₃₁H₃₄N₄O₈S: 622.7. Found (M+H)⁺: 623.2.

### Example 20

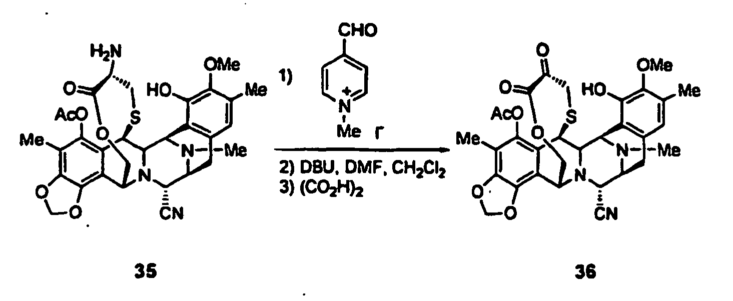

A solution of *N*-methyl pyridine-4-carboxaldehyde iodide (378 mg, 1.5 mmol) in anhydrous DMF (5.8 mL) was treated with anhydrous toluene (2 x 10 mL) to eliminate the amount of water by azeotropic removal of the toluene. A solution of **35** (134 mg, 0.21 mmol), previously treated with anhydrous toluene (2 x 10 mL), in anhydrous CH₂Cl₂ (distilled over CaH₂, 7.2 mL) was added, *via* cannula, at 23 °C to this orange solution. The reaction mixture was stirred at 23 °C for 4 hours. After this time DBU (32.2 µL, 0.21 mmol) was dropwise added at 23 °C and it was stirred for 15 minutes at 23 °C. A freshly aqueous saturated solution of oxalic acid (5.8 mL) was added to the reaction mixture and was stirred for 30 minutes at 23 °C. Then the reaction mixture was cooled to 0 °C and NaHCO₃ was portionwise added followed by addittion of aqueous saturated solution of NaHCO₃. The mixture was extracted with Et₂O. K₂CO₃ was added to the aqueous layer and it was extrated with Et₂O. The combined organic layers were dried over MgSO₄ and the solvent was removed under reduced pressure. The crude was purified by flash column chromatography (AcOEt/hexane from 1/3 to 1/1) to afford compound **36** (77 mg, 57%) as pale yellow solid.
¹H-NMR (300 MHz, CDCl₃): δ 6.48 (s, 1H), 6.11 (d,*J=* 1.3 Hz, 1H), 6.02 (d,*J=* 1.3 Hz, 1H), 5.70 (bs, 1H), 5.09 (d, *J=* 11.3 Hz, 1H), 4.66 (bs, 1H), 4.39 (m, 1H), 4.27 (d, *J*= 5.6 Hz, 1H), 4.21 (d, *J=* 10.5 Hz, 1H), 4.16 (d, *J=* 2.6 Hz,1H), 3.76 (s,3 H), 3.54 (d, *J=* 5.1 Hz, 1H), 3.42 (d, *J=* 8.5 Hz, 1H), 2.88-2.54 (m, 3H), 2.32 (s, 3H), 2.24 (s, 3H), 2.14 (s, 3H), 2.04 (s, 3H). ¹³C-NMR (75 MHz, CDCl₃): δ 186.7, 168.5, 160.5, 147.1, 146.4, 142.9, 141.6, 140.7, 130.4, 129.8, 121.7 (2C), 120.0, 117.8, 117.1, 113.5, 102.2, 61.7, 61.4, 60.3, 59.8, 58.9, 54.6, 41.6, 36.9, 29.7, 24.1, 20.3, 15.8, 14.1, 9.6.
ESI-MS m/z: Calcd.. for C₃₁H₃₁N₃O₉S: 621.7. Found (M+H)⁺: 622.2.

### Example 21

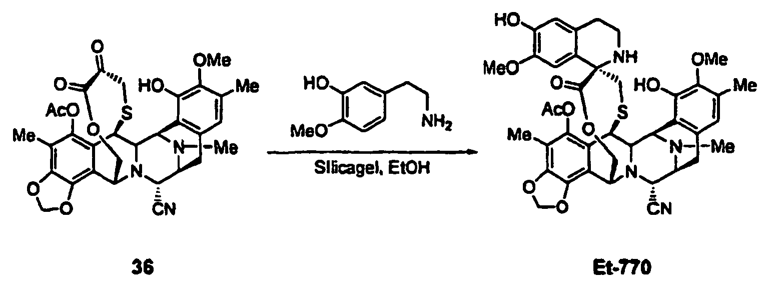

To a solution of **36** (49mg, 0.08 ml) and 2-[3-hydroxy-4-methoxyphenyl]ethylamine (46.2 mg, 0.27 ml) in ethanol (2.5 ml) was added silica gel (105 mg) at 23 °C. The reaction mixture was stirred at 23 °C for 14 h. It was diluted with hexane and poured into a column of chromatography (ethyl acetate/hexane from 1/3 to 1/1) to afford **Et-770** (55 mg. 90%) as a pale yellow solid.
¹H-NMR (300 MHz, CDCl₃): δ 6.60 (s, 1H), 6.47 (s, 1H), 6.45 (s, 1H), 6.05 (s, 1H), 5.98 (s, 1H), 5.02 (d, *J=*11.4 Hz, 1H), 4.57 (bs, 1H), 4.32 (bs, 1H), 4.28 (d, *J*= 5.3 Hz, 1H), 4.18 (d, *J=* 2.5 Hz, 1H), 4.12 (dd, *J=* 2.1 Hz, *J*= 11.5 Hz, 1H), 3.78 (s, 3 H), 3.62 (s, 3H), 3.50 (d, *J*= 5.0 Hz, 1H), 3.42 (m, 1H), 3.10 (ddd, *J= 4.0* Hz, *J*= 10.0 Hz, *J*= 11.0 Hz, 1H), 2.94 (m, 2H), 2.79 (m, 1H), 2.61 (m, 1H), 2.47 (m, 1H), 2.35 (m, 1H), 2.32 (s, 3H), 2.27 (s, 3H), 2.20 (s, 3H), 2.09 (m, 1H), 2.04 (s, 3H).
ESI-MS m/z: Calcd.. for C₄₀H₄₂N₄O₁₀S: 770.7. Found (M+H)⁺: 771.2

### Example 22

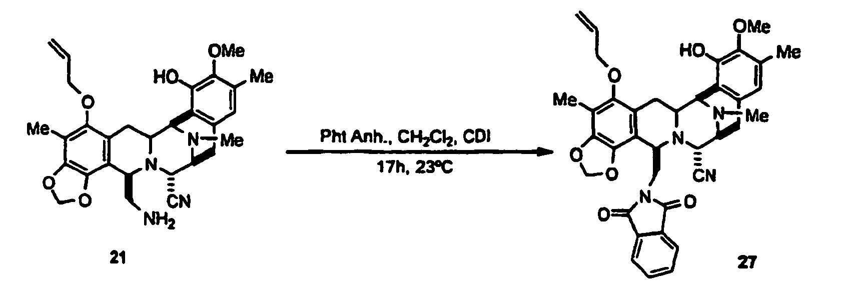

To a solution of **21** (22 mg, 0.042 ml) in CH₂Cl₂ (0.8 ml) was added phthalic anhydride (6.44 mg, 0.042 ml) and the reaction mixture was stirred for 2h at 23 °C. Then, carbonyldiimidazole (1mg, 0.006 ml) was added and the mixture was stirred at 23 °C for 7h. Then, carbonyldiimidazole (5.86mg, 0.035 ml) was added and the reaction was stirred at 23 °C for an additional 17h. The solution was diluted with CH₂Cl₂ (15 ml) and washed with 0.1 N HCl (15 ml). The organic layer was dried over sodium sulphate, filtered, and the solvent was eliminated under reduced pressure. The residue was purified by flash column chromatography (SiO₂ hexane:ethyl acetate 2:1) to afford **27** (26.4 mg, 96%) as a white solid.
Rf: 0.58 (ethyl acetate).
¹H NMR (300 MHz, CDCl₃): 7.73-7.64 (m, 4H), 6.40 (s, 1H), 6.12-6.01 (m, 1H), 5.63 (s, 1H), 5.58 (d, *J=* 1.5 Hz, 1H), 5.37 (dd, *J₁=* 1.8 Hz, *J₂=* 17.4 Hz), 5.23 (dd, *J₁=* 1.8 Hz, *J₂=* 10.5 Hz, 1H), 5.12 (d, *J=* 1.5 Hz, 1H), 4.22-4.15 (m, 3H), 4.08 (d, *J=* 1.8 Hz, 1H), 3.68 (s, 3H), 3.59-3.55 (m 2H), 3.35 (d, *J=* 8.1 Hz, 1H), 3.27-3.16 (m, 2H), 3.05 (dd, *J₁=* 8.1 Hz, *J₂=* 18.3 Hz, 1H), 2.64 (d, *J*= 18.0Hz, 1H), 2.30 (s, 3H), 2.24 (s, 3H), 2.09 (s, 3H), 1.80 (dd, *J₁*= 11.4 Hz, *J*₂= 15 Hz, 1H);
¹³C NMR (75 MHz, CDCl₃): δ 167.7, 148.9, 146.4, 144.2, 142.6, 139.5, 134.0, 133.5, 132.0, 131.0, 128.3, 123.0, 121.3, 120.9, 118.1, 117.5, 116.8, 113.6, 112.4, 100.8, 74.5, 60.6, 60.5, 57.7, 56.6, 55.6, 55.5, 42.3, 41.7, 26.6, 25.5, 15.9, 9.46.
ESI-MS m/z: Calcd. for C₃₇H₃N₄O₇: 648.79. Found (M+H)⁺: 649.3.

### Example 23

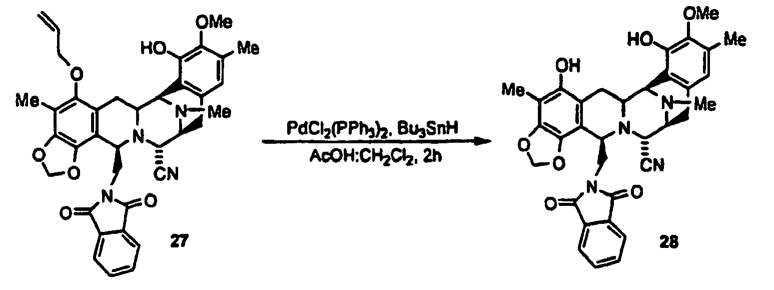

To a solution of **27** (26 mg, 0.041 ml) in CH₂Cl₂ (11 ml), acetic acid (11 ml), (PPh₃)₂PdCl₂ (2.36 mg) and Bu₃SnH (28 ml, 0.10 ml) were added at 23 °C. After stirring at that temperature for 2h the reaction was poured into a pad of flash column (SiO₂, gradient Hex to hexane:ethyl acetate 2:1) to afford **28** (24.7 mg, 99 %) as a white solid.
Rf: 0.33 (hexane:ethyl acetate 2:1).
¹H NMR (300 MHz, CDCl₃): δ 7.75-7.70 (m, 2H), 7.69-7.65 (m, 2H), 6.39 (s, 1H), 5.82 (bs, 1H), 5.50 (d, *J*= 1.5 Hz, 1H), 5.0 (d, *J=* 1.5 Hz, 1H), 4.45 (bs, 1H), 4.23-4.19 (m, 2H). 4.10-4.09 (m, 1H), 3.73 (s, 3H), 3.60-3.48 (m, 2H), 3.36-3.33 (m, 1H), 326-3.20 (m, 1H), 3.14-3.08 (m, 1H), 3.98 (d, *J*= 14.4 Hz, 1H), 2.61 (d, *J*= 18.3 Hz, 1H), 2.30 (s, 3H). 2.23 (s, 3H), 2.06 (s, 3H), 1.85 (dd, *J₁=* 12 Hz, *J₂=* 15.3 Hz);
¹³C NMR (75 MHz, CDCl₃): δ 167.8, 146.4, 145.1, 143.9, 142.7, 137.1, 133.5, 131.9, 130.8, 128.4, 122.9, 120.8, 118.0, 116.8, 114.0, 113.4, 106.4, 100.4, 60.6, 60.5, 57.8, 56.6, 55.5, 55.2, 42.6, 41.5, 25.6, 25.5, 15.8, 8.9.
ESI-MS m/z: Calcd. for C₃₄H₃₂N₄O₇: 608.6. Found (M+H)⁺: 609.2.

### Example 24

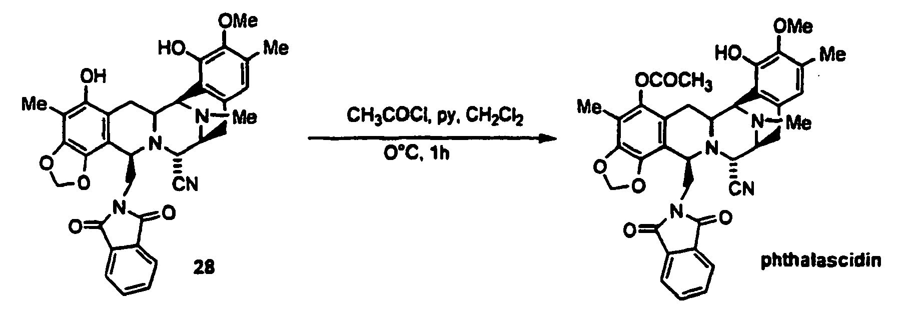

To a solution of **28** (357 mg, 0.058 ml) in CH₂Cl₂ (3 ml), acetyl chloride (41.58 ml, 0.58 ml) and pyridine (47.3 ml, 0.58 ml) were added at 0 °C. The reaction mixture was stirred for 1h and then, the solution was diluted with CH₂Cl₂ (15 ml) and washed with 0.1 N HCl (15 ml). The organic layer was dried over sodium sulphate, filtered, and the solvent was eliminated under reduced pressure. The residue was purified by flash column chromatography (RP-18, CH₃CN:H₂O 60:40) to afford phthalascidin (354 mg, 94%) as a white solid.
Rf: 0.37 (CH₃CN:H₂O 7:3, RP-18).
¹H NMR (300 MHz, CDCl₃): δ 7.72-7.68 (m, 2H), 7.67-7.63 (m, 2H), 6.38 (s, 1H), 5.69 (d, *J*= 1.2 Hz, 1H), 5.64 (d, *J*= 1.2Hz, 1H), 5.30 (bs, 1H), 4.25-4.21 (m, 2H), 4.02 (d, *J=* 2.1 Hz, 1H), 3.64-3.62 (m, 5H), 3.33 (d, *J*= 8.4 Hz, 1H), 3.21-3.16 (m, 1H), 3.02 (dd, *J₁=* 8.1 Hz, *J₂=* 18 Hz, 1H), 2.76 (dd, *J₁=* 1.8 Hz, *J₂=* 15.6 Hz, 1H), 2.63 (d, *J*= 17.7 Hz, 1H), 2.29 (s, 3H), 2.28 (s,3H), 2.21 (s, 3H), 2.0 (s, 3H), 1.73 (dd, *J₁=* 12.0 Hz, *J₂=* 15.3 Hz,1H))
¹³C NMR (75 MHz, CDCl₃)): δ 168.5, 167.6, 146.2, 144.2, 142.5, 141.0, 140.5, 133.4, 131.8, 130.7, 128.2, 120.9, 120.8, 117.9, 116.4, 113.6, 101.1, 60.4, 60.0, 57.0, 56.3, 55.6, 55.4, 41.6, 41.5, 26.5, 25.2, 20.2, 15.7, 9.4.
ESI-MS m/z: Calcd. for C₃₆H₃₄N₄O₈: 650. Found (M+H)⁺: 651.2.

### Example 25

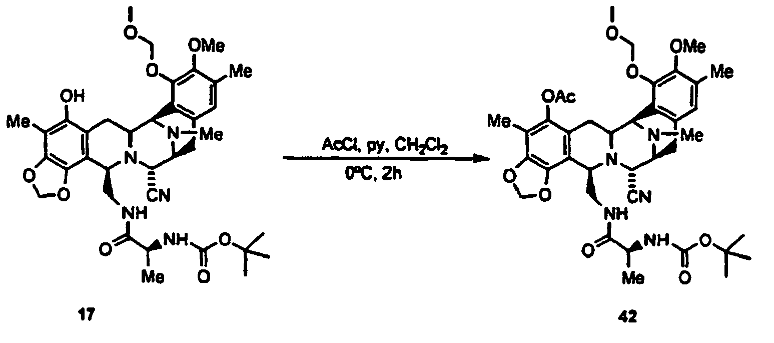

To a solution of **17** (300 mg, 0.432 ml) in CH₂Cl₂ (2 ml), acetyl chloride (30.7 ml, 0.432 ml) and pyridine (34.9 ml, 0.432 ml) were added at 0 °C. The reaction mixture was stirred for 2h at that temperature and then, the solution was diluted with CH₂Cl₂ (15 ml) and washed with 0.1 N HCl (15 ml). The organic layer was dried over sodium sulphate, filtered, and the solvent was eliminated under reduced pressure to afford **42** (318 mg, 100%) as a white solid that was used in subsequent reactions with no further purification.
Rf: 0.5 (ethyl acetate:methanol 5:1).
¹H NMR (300 MHz, CDCl₃). δ 6.66 (s, 1H), 5.93 (d, *J=* 1.2 Hz, 1H), 5.83 (d,*J*= 1.2 Hz, 1H), 5.42 (t, *J*= 6.6 Hz, 1H), 5.07 (d, *J=* 5.7 Hz, 1H), 4.98 (d, *J=* 5.7 Hz, 1H), 4.16 (d, *J*= 1.8 Hz,1H), 4.11 (d, *J*= 2.7 Hz, 1H), 3.98 (bs, 1H), 3.73-3.61 (m, 2H), 3.64 (s, 3H), 3.52-3.48 (m, 1H), 3.50 (s, 3H), 3.33 (d, *J*= 9.6 Hz, 1H), 3.17-3.14 (m, 1H), 2.97-2.87 (m, 1H), 2.75-2.70 (d, *J=* 16.8 Hz, 1H), 2.26 (s, 6H), 2.16 (s, 3H), 1.96 (s, 3H), 1.70 (dd, *J*₁= 11.7 Hz, *J₂*= 15.6 Hz, 1H), 1.33 (s, 9H), 0.59 (d, *J=* 6.0 Hz, 3H).
¹³C NMR (75 MHz, CDCl₃)): δ 172.0, 168.3, 162.3, 148.2, 144.4, 140.4, 140.2, 130.9, 130.5, 125.3, 123.4, 120.8, 117.6, 112.7, 111.7, 101.4, 99.1, 79.2, 59.5, 58.8, 57.5, 57.4, 56.4, 55.5, 55.0, 41.3, 39.0, 28.2, 26.4, 24.6, 19.9, 18.4, 15.4, 9.1.
ESI-MS m/z: Calcd. for C₃₈H₄₉N₅O₁₀: 735.82. Found (M+H)⁺: 736.3.

### Example 26

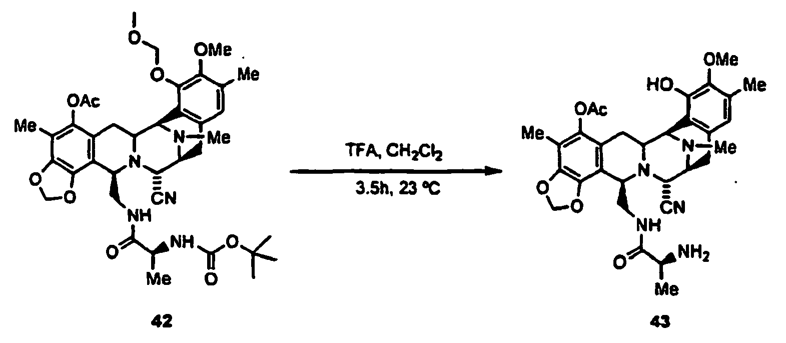

To a solution of **42** (318 mg, 0.432 ml) in CH₂Cl₂ (2.16 ml), trifluoroacetic acid (1.33 ml, 17.30 ml) was added and the reaction mixture was stirred for 3.5h at 23 °C. The reaction was quenched at 0 °C with saturated aqueous sodium bicarbonate (60 ml) and extracted with CH₂Cl₂ (2 x 70 ml). The combined organic layers were dried (sodium sulphate) and concentrated *in vacuo.* The residue was purified by flash column chromatography (SiO₂, ethyl acetate:methanol 20:1) to afford **43** (154 mg, 60%) as a white solid.
Rf: 0.22 (ethyl acetate:methanol 5:1).
¹H NM (300 MHz, CDCl₃). δ 6.47 (s, 1H), 6.22 (bs, 1H), 5.95 (d, *J*= 1.2 Hz, 1H), 5.88 (d, *J=* 1.2 Hz, 1H), 4.08-4.06 (m, 2H), 4.01 (bs, 1H), 3.69 (s, 3H), 3.49 (d, *J=* 3.6 Hz, 1H), 3.33 (d, *J* = 8.1 Hz, 1H), 3.26-3.22 (m, 1H), 2.95 (dd, *J₁*= 8.1 Hz, *J₂* = 18 Hz, 1H), 2.80-2.76 (m, 2H), 2.58 (d, *J*=18Hz, 1H), 2.29 (s, 3H), 2.27 (s, 3H), 2.21 (s, 3H), 1.96 (s, 3H), 1.77 (dd, *J₁=* 12.3 Hz, *J₂=* 15.6 Hz, 1H), 0.90 (d, *J=*6.9 Hz, 3H).
¹³C NMR (75 MHz, CDCl₃)):δ 174.8, 169.0, 146.8, 144.4, 142.8, 140.5, 140.2, 131.1, 128.8, 120.8, 120.5, 117.1, 112.9, 111.6, 101.5, 60.3, 59.0, 56.5, 56.3, 55.6, 55.1, 50.2, 41.6, 39.5, 26.8, 26.3, 24.9, 20.2, 15.4, 9.2.
ESI-MS m/z: Calcd. for C₃₁H₃₇N₅O₇: 591.65. Found (M+H)⁺: 592.3.

### Example 27

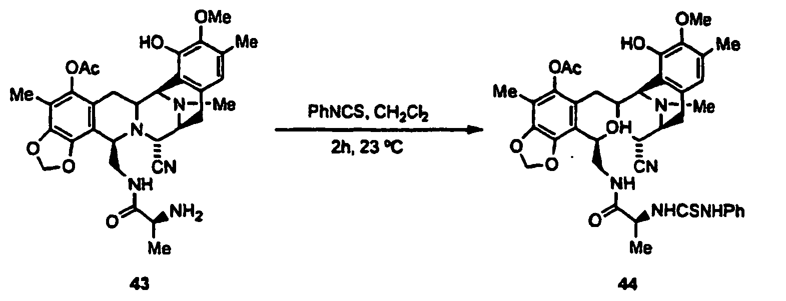

To a solution of **43** (154 mg, 0.26 ml) in CH₂Cl₂ (1.3 ml), phenyl isothiocyanate (186 ml, 1.56 ml) was added and the mixture was stirred at 23° C for 2h. The reaction was concentrated *in vacuo* and the residue was purified by flash column chromatography (SiO₂, gradient Hexane to hexane:ethyl acetate 1:1) to afford **44** (120 mg, 63 %) as a white solid.
Rf: 0.41 (ethyl acetate:methanol 5:1).
¹HNMR(300 MHz, CDCl₃). δ 8.17 (s, 1H), 7.49-7.44 (m, 3H), 7.31-7.24 (m. 3H). 7.05 (d, *J=* 6.9 Hz, 1H), 5.98 (d,*J*=1.2 Hz, 1H), 5.87 (d,*J*= 1.2 Hz, 1H), 5.52 (bs,1H), 4.54 (t,*J*= 6.6 Hz, 1H), 4.15 (d,*J*= 2.1 Hz, 1H), 4.03 (d, *J=* 2.7 Hz, 2H), 3.80 (bs, 1H), 3.66 (s, 3H), 3.40 (bs, 1H), 3.32 (d, *J*= 7.8 Hz, 1H), 3.16 (d, *J=* 11.7 Hz, 1H), 2.82-2.61 (m, 3H), 2.29 (s, 3H), 2.20 (s, 3H), 2.01 (s, 3H), 1.99 (s, 3H), 1.80 (dd, *J₁ =* 12.0 Hz, *J*₂= 15.9 Hz, 1H), 0.62 (d, *J*= 6.0 Hz, 3R).
¹³C NMR (75 MHz, CDCl₃) δ 178.5, 71.9, 168.7, 146.7, 144.5, 142.6, 140.6, 140.3, 136.3, 131.0, 129.9, 128.9, 126.7, 124.4, 120.9, 120.6, 117.7, 116.6, 112.7, 111.9, 101.4, 60.4, 58.7, 57.5, 56.1, 55.7, 55.1, 533, 41.4, 38.8, 26.3, 24.4, 20.2, 18.1,15.3, 9.2.
ESI-MS m/z: Calcd. for C₃₈H₄₂N₆O₇S: 726.3. Found (M+H)⁺: 727.3.

### Example 28

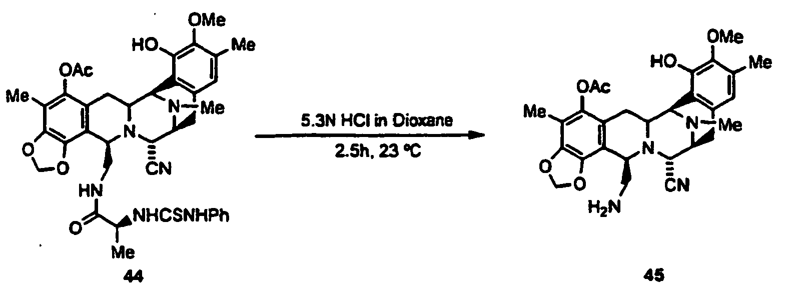

To a solution of **44** (120 mg, 0.165 ml) in dioxane (0.9 ml), 5.3N HCl/dioxane (1.8 ml) was added and the reaction was stirred at 23 °C for 2.5h. Then, CH₂Cl₂ (10 ml) and H₂O (5 ml) were added to this reaction and the organic layer was decanted. The aqueous phase was basified with saturated aq sodium bicarbonate (20 ml) (pH = 8) at 0 °C and then, extracted with CH₂Cl₂ (2x15 ml). The combined organic extracts were dried (sodium sulphate), and concentrated *in vacuo* to afford **45** (75 mg, 87%) as a white solid that was used in subsequent reactions with no further purification.
Rf: 0.23 (ethyl acetate:methanol 5:1).
¹H NMR (300 MHz, CDCl₃): δ 6.43 (s, 1H), 5.94 (d, *J=* 1.2 Hz, 1H), 5.87 (d, *J*= 1.2Hz, 1H), 4.10 (d, *J=* 2.1 Hz, 1H), 3.98 (d, *J*= 2.4 Hz, 1H), 3.91 (bs, 1H), 3.69 (s, 3H), 3.34-3.25 (m, 2H), 3.05 (dd, *J₁=* 1.8 Hz, *J₂=* 8.1 Hz, 1H), 2.80-2.73 (m, 3H), 2.46 (d, *J=* 18 Hz, 1H), 2.30 (s, 3H), 2.28 (s,3H), 2.20 (s, 3H), 1.98 (s, 3H), 1.79 (dd, *J₁=* 12.6 Hz, *J*₂*=* 16.2 Hz, 1H);
¹³C NMR (75 MHz, CDCl₃)): δ 168.7, 146.7, 144.4, 142.9, 140.4, 130.4, 128.9, 121.1, 120.8, 117.8, 116.8, 113.6, 111.5, 101.4, 67.6, 60.5, 59.8, 58.4, 56.6, 55.8, 55.3, 43.6, 41.8, 31.3, 25.6, 20.2, 15.6, 9.2.
ESI-MS m/z: Calcd. for C₂₈H₃₂N₄O₆: 520.58. Found (M+H)⁺: 521.3.

### Example 29

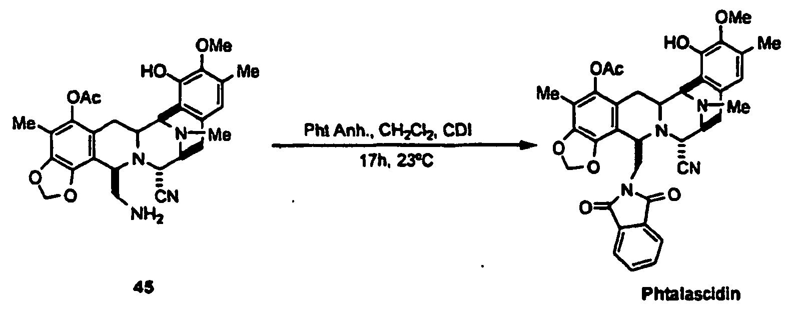

To a solution of **45** (10 mg, 0.02 ml) in CH₂Cl₂ (0.4 ml) was added phthalic anhydride (2.84 mg, 0.02 ml) and the reaction mixture was stirred for 2 h at 23 °C. Then, carbonyldiimidazole (0.5 mg, 0.003 ml) was added and the mixture was stirred at 23 °C for 7h. Then, carbonyldiimidazole (2.61 mg, 0.016 ml) was added and the reaction was stirred at 23 °C for an additional 17h. The solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (RP-18, CH₃CN:H₂O 60:40) to afford phthalascidin (11.7 mg, 93%) as a white solid.
Rf: 0.37 (CH₃CN:H₂O 7:3, RP-18).
¹H NMR (300 MHz, CDCl₃): δ 7.72-7.68 (m, 2 h), 7.67-7.63 (m, 2 h), 6.38 (s, 1H), 5.69 (d, *J*= 1.2 Hz, 1H), 5.64 (d, *J=* 1.2 Hz, 1H), 5.30 (bs, 1H), 4.25-4.21 (m, 2 h), 4.02 (d, *J*= 2.1 Hz, 1H), 3.64-3.62 (m, 5H), 3.33 (d, *J*= 8.4 Hz, 1H), 321-3.16 (m, 1H), 3.02 (dd, *J₁=* 8.1 Hz, *J₂=* 18 Hz, 1H), 2.76 (dd, *J₁*= 1.8 Hz, *J*₂= 15.6 Hz, 1H), 2.63 (d, *J*= 17.7 Hz, 1H), 2.29 (s,3H), 2.28 (s,3H), 2.21 (s, 3H), 2.0 (s, 3H), 1.73 (dd, *J₁*=12.0 Hz, *J₂=* 15.3 Hz, 1H));
¹³C NMR (75 MHz, CDCl₃)):δ 168.5, 167.6, 146.2, 144.2, 142.5, 141.0, 140.5, 133.4, 131.8, 130.7, 128.2, 120.9, 120.8, 117.9, 116.4, 113.6, 101.1, 60.4, 60.0, 57.0, 56.3, 55.6, 55.4, 41.6, 41.5, 26.5, 25.2, 20.2, 15.7, 9.4.
ESI-MS m/z: Calcd. for C₃₆H₃₄N₄O₈: 650. Found (M+H)⁺: 651.2.

### Example 30

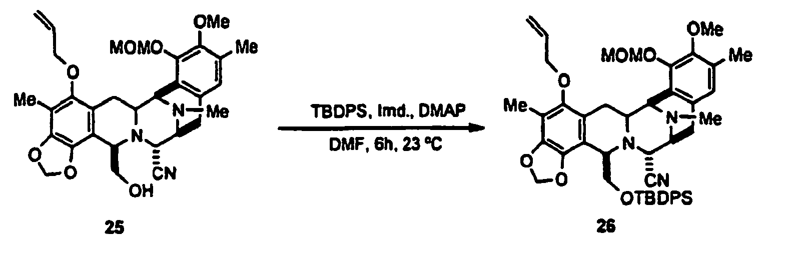

To a solution of **25** (18 mg, 0.032 ml) in DMF (0.05 ml), cat. DMAP (0.5 mg, 0.004 ml), imidazole (5 mg, 0.08 ml) and *tert*-Butyldiphenylsilyl chloride (12.5 ml, 0.048 ml) were added at 0 °C and the reaction mixture was stirred for 6h at 23 °C. Water (10 ml) was added at 0 °C and the aqueous phase was extracted with hexane:ethyl acetate 1:10 (2 x 10 ml). The organic layer was dried (sodium sulphate), filtered, and the solvent was removed under reduced pressure. The crude was purified by flash column chromatography (SiO₂, hexane:ethyl acetate 3:1) to afford **26** (27 mg, 88 %) as a white solid.
Rf: 029 (hexane:ethyl acetate 3:1).
¹H NMR (300 MHz, CDCl₃) δ 7.61-7.58 (m, 2 h), 7.42-7.28 (m, 8H), 6.71 (s, 1H), 6.19-6.02 (m, 1H), 5.78 (d, *J=* 1.2 Hz, 1H), 5.64 (d, *J=* 1.2 Hz, 1H), 5.40 (dd, *J₁ =* 1.2 Hz. *J₂=* 17.1 Hz, 1H), 5.27 (dd, *J₁ =* 1.2 Hz, *J₂ =* 10.2 Hz, 1H), 5.13 (s, 2 h), 4.45 (d, *J*= 2.4 Hz, 1H), 4.24 (d, *J*= 2.1 Hz, 1H), 4.17-4.06 (m, 3H), 3.75 (s, 3H), 3.64 (dd, *J₁=* 2.4 Hz, *J₂*= 9.9 Hz, 1H), 3.59 (s, 3H), 3.42-3.21 (m, 4H), 3.10 (dd, *J₁=* 8.1 Hz, *J₂*= 17.7 Hz, 1H), 2.70 (d, *J=* 17.7 Hz, 1H), 2.33 (s, 3H), 2,26 (s, 3H), 2.11 (s, 3H), 2.08-1.89 (m, 1H), 0.87 (s, 9H);
¹³C NMR (75 MHz., CDCl₃): δ 148.5, 148.3, 148.1, 144.0, 139.0, 135.6, 135.4, 133.8, 133.1, 132.6, 130.5, 130.3, 129.6, 129.4, 127.5, 127.4, 125.1, 124.3, 121.6, 118.5, 117.5, 112.9, 111.7, 100.8, 99.2, 74.0, 67.7, 61.5, 59.6, 59.0, 57.7, 57.1, 55.4, 41.6, 29.6, 26.6, 25.5, 18.8, 15.8, 9.2.
ESI-MS m/z: Calcd. for C₄₇H₅₅N₃O₇Si: 801.3. Found (M+H)⁺: 802.3.

### Example 31

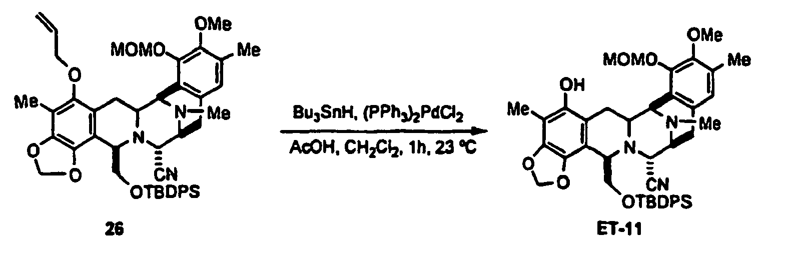

To a solution of **26** (7 mg, 0.0087 ml) in CH₂Cl₂ (0.15 ml), acetic acid (2.5 ml, 0.044 ml), (PPh₃)₂PdCl₂ (0.5 mg, 6.96 x 10⁻⁴ ml) and Bu₃SnH (3.5 ml, 0.013 ml) were added at 23 °C. The reaction mixture was stirred at that temperature for 1h. The solution was diluted with a mixture of hexane:ethyl acetate 5:1 (0.5 ml) and poured into a pad of flash column (SiO₂, gradient 5:1 to 1:1 hexane:ethyl acetate) affording **ET-11** (5 mg, 75 %) as a white solid.
Rf: 0.36 (hexane:ethyl acetate 1:5, silica).
¹H NMR (300 MHz, CDCl₃): δ 7.56 (m, 2 h), 7.41-7.25 (m, 8H), 6.67 (s, 1H), 5.72 (d, *J*= 1.0 Hz, 1H), 5.58 (d, *J=* 1.0 Hz, 1H), 5.51 (s, 1H), 5.38 (d, *J=* 5.75 Hz, 1H), 5.16 (d, *J=* 5.7 Hz, 1H), 4.57 (d, *J*= 2.9 Hz, 1H), 4.21 (m, 1H), 4.09 (m, 1H), 3.72 (s, 3H), 3.71 (s, 3H), 3.68 (dd, *J₁=* 2.1 Hz, *J₂=* 10.4 Hz, 1H), 3.38-3.26 (m, 3H), 3.11 (dd, *J₁*= 2.5 Hz, *J₂*= 15.7 Hz, 1H), 3.01 (dd, *J*₁= 8.9 Hz*, J₂=* 17.9 Hz, 1H), 2.70 (d, *J*= 17.9 Hz,1H), 2.31 (s, 3H), 2.25 (s, 3H), 2.06 (s, 3H), 1.89 (dd, *J₁*=12.1 Hz, *J₂.* 15.7 Hz, 1H), 0.9 (s, 9H).);
¹³C NMR (75 MHz, CDCl₃): δ 149.0, 147.4, 145.3, 144.3, 136.3, 135.7, 135.4, 133.2, 130.9, 130.5, 129.6, 129.5, 127.5, 125.0, 118.6, 112.5, 112.1, 105.7, 100.5, 99.8, 68.5, 61.5, 59.7, 58.8, 57.7, 56.9, 56.5, 55.4, 41.7, 26.6, 26.2, 25.5, 18.9, 15.8, 14.2, 8.7.
ESI-MS m/z: Calcd. for C₄₄H₅₁N₃O₇Si: 761. Found (M+H)⁺: 762.

### Example 32

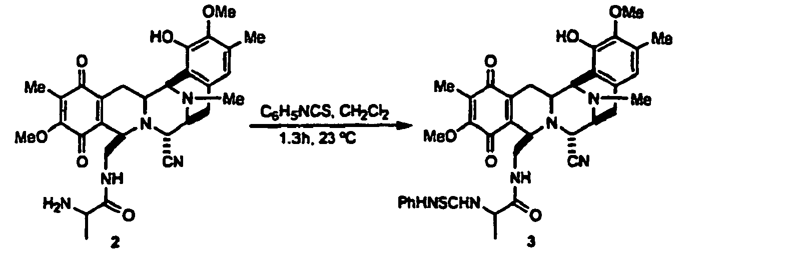

A solution of 2 (3.0 g, 5.46 ml) and phenyl isothiocyanate (3.92mL. 32.76 ml) in CH₂Cl₂ (27 ml) was stirred at 23° C for 1.5h. The reaction mixture was partitioned between CH₂Cl₂ (10 ml) and H₂O (5 ml). The organic layer was dried over sodium sulphate, filtered and concentrated. The residue was purified by flash column chromatography (SiO₂, gradient Hex to 2:3 hexane:ethyl acetate) to give **3** (3.29 g, 88%) as a yellow solid.
Rf: 0.27 (ACN:H₂O 3:2, RP-C18);
¹H NMR (300 MHz, CDCl₃): δ 7.77 (bs, 1H), 7.42-7.11 (m, 5H), 6.65 (d, 1H), 6.29 (s, 1H). 5.6-5.5 (m, 1H), 4.19-4.14 (m, 2 h), 4.08 (d, 1H), 3.92 (s, 3H), 3.87-3.65 (m, 6H), 3.77 (s, 3H), 3.37-2.98 (m, 8H), 2.50 (d, 1H), 2.31 (s, 3H), 2.20 (s, 3H), 1.96 (d, 1H), 1.87 (s, 3H), 1.81-1.75 (m, 1H), 0.96 (d, 3H);
¹³C NMR (75 MHz,
CDCl₃):δ 185.7, 180.9, 178.9, 172.0, 155.7, 147.1, 143.2, 142.4, 136.0, 135.1, 130.5, 129.9, 129.3, 128.5, 126.9, 124.4, 120.2, 117.4, 116.3, 77.1, 60.9, 58.6, 56.2, 55.8, 55.0, 54.6, 53.5, 41.7, 40.3, 25.1, 24.5, 18.4, 15.8, 8.7
ESI-MS m/z: Calcd. for C₃₆H₄₀N₆O₆S: 684.8. Found (M+H)⁺: 685.2.

### Example 33

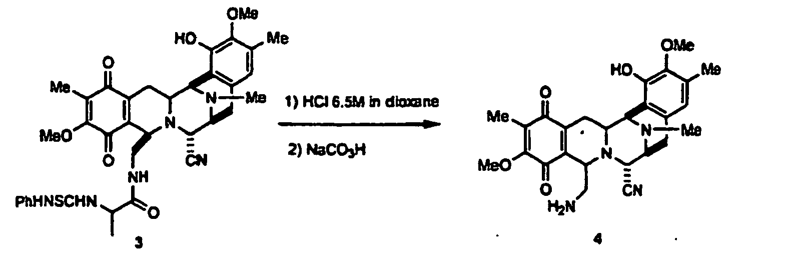

A solution of **3** (0.143 g, 0.208 ml) in 6.5 M HCl/dioxane (150 ml) was stirred at 23 °C for 6h. Then, toluene (3 ml) was added to this reaction and the organic layer was decanted. The residue was partitioned between saturated aqueous sodium bicarbonate (3 ml) and CHCl₃ (3x3 ml) The organic layers were dried and concentrated to afford title compound as a mixture of **4** and **6 (4:6** 90:10) which slowly cyclizes to **6** on standing.
Rf: 0.4 (ethyl acetate:methanol5:1. silica);
¹H NMR (300 MHz, CDCl₃): δ 6.45 (s, 1H), 4.16 (m, 1H), 4.02 (d, 1H), 3.96 (s. 3H). 3.79 (m, 2 h), 3.75 (s, 3H), 3.35 (m, 1H), 3.20-3.00 (m, 3H), 2.87 (d, 1H), 2.75 (d. 1H), 2.43 (d, 1H), 2.34 (s, 3H), 2.30 (s, 3H), 1.93 (s, 3H), 1.72-1.5 (m, 3H);
ESI-MS m/z: Calcd. for C₂₆H₃₀N₄O₅ : 478.5. Found (M+H)⁺ : 479.2

### Example 34

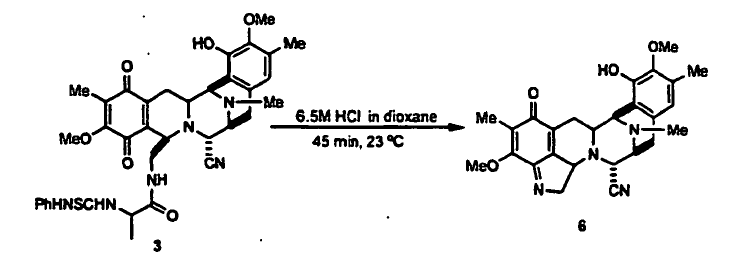

A solution of 3 (0.143 g, 0.208 ml) in 6.5M HCl/dioxane (150 ml) was stirred at 23 °C for 1h. Evaporation of the solvent gave a residue which was purified by flash column chromatography (ethyl acetate/methanol/triethylamine 100:25:0.1) to give 6 (80 mg, 83%) as a yellow solid.
Rf: 026 (ACN:H₂O 3:2, RP-C18);
¹H NMR (500 MHz, CDCl₃): δ 6.46 (s, 1H), 5.9 (bs, 1H) 4.67 (dd, *J* = 18.3 Hz, *J*= 7.8 Hz, 1H), 4.24 (d, 1H), 4.16 (s, 3H), 3.93 (d, *J*=2.7 Hz, 1H), 3.8 (m, 2 h). 3.77 (s. 3H). 3.45 (m, 2 h), 3.08 (dd, *J* =17.9 Hz, *J*=3.6 Hz, 1H), 2.78 (m, 1H), 2.55 (d, 1H), 2.3 (m, 1H), 2.3 (s. 3H). 2. 28 (s, 3H), 1.90 (s, 3H);
¹³C NMR (75 MHz, CDCl₃) : δ 186.2, 162.1, 154.9, 146.9, 145.3, 143.0, 130.1, 129.4, 128,1. 125.0, 121.4, 116.4, 116.2, 66.6, 60.7, 60.7, 60.1, 59.6, 58.8, 55.6, 54.9, 41.9, 25.3, 24.7, 15.7,8.9.
ESI-MS m/z: Calcd. for C₂₆H₂₈N₄O₄ 460.5. Found (M+H)⁺: 461.1

### Example 35

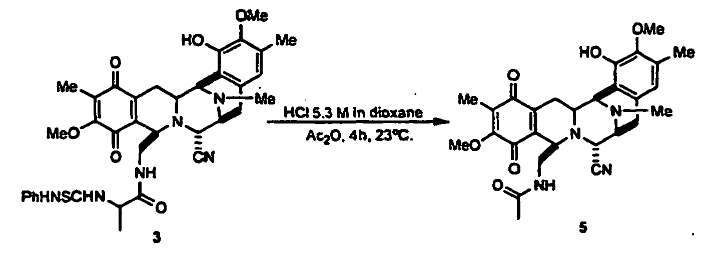

To a solution of **3** (2.38 g, 3.47 ml) in dioxane (5 ml) 5.3M HCl in dioxane (34 ml) was added and the reaction was stirred at 23 °C for 45 minutes. Then Ac₂O (51 ml, 539.5 ml) was added and the mixture was stirred for 4h. The reaction was cooled at 0 °C and partitioned between aqueous saturated Na₂CO₃ (300 ml) and ethyl acetate (300 ml) at this temperature. The organic phase was dried over sodium sulphate, filtered and concentrated. The residue was purified by flash column chromatography (SiO₂, gradient CH₂Cl₂ to CH₂Cl₂:ethyl acetate 1:2) to give **5** (1.75 g, 97%) as a yellow solid.
Rf: 0.53 (ACN:H₂O 3:2, RP-C18);
¹H NMR (300 MHz, CDCl₃): δ 6.51 (s, 1H), 5.98 (bs, 1H), 4.84 (dd, 1H), 4.17 (d, 1H), 4.00 (d, 1H), 3.99 (s, 3H), 3.85 (bs, 1H), 3.81 (m, 1H), 3.74 (s, 3H), 3.70 (d, 1H), 3.23 (m, 1H), 3.11 (dd, 1H), 3.09 (m, 1H), 2.93 (m, 2 h), 2.44 (d, 1H), 3.67 (s, 3H), 2.25 (s, 3H), 1.70 (s, 3H), 1.60-1.50 (m, 2 h), 1.29 (s, 3H);
¹³C NMR (75 MHz, CDCl₃): δ 185.9, 180.8, 169.9, 160.2, 156.2, 147.0, 143.1, 140.4, 136.1. 130.6, 129.6, 127.9, 120.4, 117.2, 61.0, 60.7, 58.6, 56.1, 55.7, 55.1, 54.3, 41.8, 41.1, 25.7. 23.9, 22.2, 15.7, 8.7.
ESI-MS m/z: Calcd. for C₂₈H₃₂N₄O₆: 520.6. Found (M+H)⁺: 521.1

### Example 36

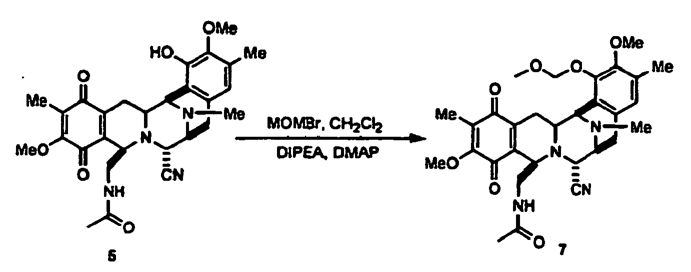

To a solution of **5** (1.75 g, 3.36 ml) in CH₂Cl₂ (17 ml) diisopropylethylamine (11.71 ml, 67.23 ml), DMAP (20 mg, 0.17 ml) and bromomethyl methyl ether (4.11 ml, 50.42 ml) were added at 0 °C. After 6 h at 23 °C the reaction was partitioned between CH₂Cl₂ (50 ml) and aqueous saturated sodium bicarbonate (25 ml). The organic layer was dried over sodium sulphate and the solvent was eliminated under reduced pressure. The crude was purified by flash column chromatography (RP-18, CH₃CN/H₂O 1/1) to give **7** (1.32 g, 70%) as a yellow solid.
Rf: 0.34 (ACN:H₂O 2:3, RP-C 18);
¹H NMR (300 MHz, CDCl₃): δ 6.74 (s, 1H), 5.14 (s, 2 h), 4.82 (m, 1H), 4.22 (d, 1H), 4.00 (s, 3H), 4.0 (m, 1H), 3.83 (m, 2 h), 3.7 (s, 3H), 3.58 (s, 3H), 3.4 (m, 1H), 3.2-2.95 (m, 6H), 2.43 (d, 1H), 2.37 (5,3H).222 (s, 3H). 1.89 (s, 3H), 5-1.4 (m, 2 h), 1.31 (s, 3H);
¹³C NMR (75 MHz. CDCl₃): δ 185.9, 190.7, 169.6, 156.2, 148.9,148.5,140.3, 136.2, 131.3, 130.1, 127.7, 124.6, 123.7, 117.3, 99.5, 99.2, 60.9, 59.7, 58.8, 57.7, 56.4, 55.7, 55.0, 54.2, 51.0, 41.6, 41.0, 40.5, 25.5, 23.9, 22.3, 19.3, 15.6, 14.6, 8.6.
ESI-MS m/z: Calcd. for C₃₀H₃₆N₄O₇: 564.6. Found (M+H)⁺: 565.3

### Example 37

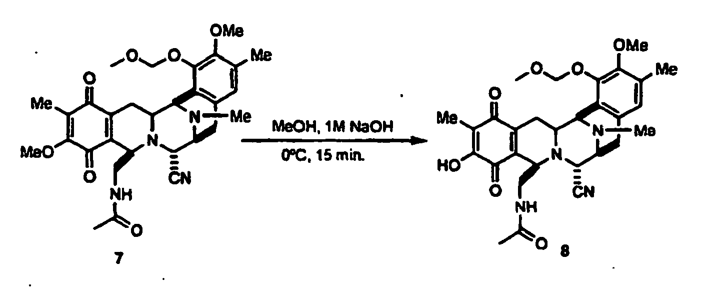

To a solution of 7 (0.37 g, 0.65 ml) in methanol (74 ml) at 0 °C was added 1M sodium hydroxide (130 ml). The reaction was stirred for 15 minutes and then, quenched at 0 °C with 6M HCl to pH = 5. The mixture was extracted with ethyl acetate (3 x 50 ml) and the combined organic layers were dried over sodium sulphate and concentrated *in vacuo.* The residue was purified by flash column chromatography (RP-C18 CH₃CN:H₂O 1/:1) to afford **8** (232 mg, 65%) as a yellow oil.
Rf: 0.5(ACN:H₂O 3:2, RP-C18);
¹H NMR (300 MHz, CDCl₃): δ 6.75 (s, 1H), 5.15 (s, 2 h), 4.86 (m, 1H), 4.26 (d, 1H),), 4.01 (d, 1H), 3.88-3.81 (m, 2 h), 3.70 (s, 3H), 3.58 (s, 3H), 3.39 (m, 1H), 3.27-3.21 (m, 1H), 3.18-3.08 (m, 2 h), 3.03-2.97 (m, 1H) 2.47 (d, 1H), 2.37 (s, 3H), 2. 22 (s, 3H), 1.90 (s, 3H), 1.57-1.46 (m, 2 h), 1.33 (s, 3H);
¹³C NMR (75 MHz, CDCl₃): δ 185.3, 180.6, 175.9, 170.1, 151.5, 148.9, 148.6, 143.3, 133.7, 131.5, 129.9, 124.7, 123.5, 117.1, 117.0, 99.2, 59.8, 58.7, 57.8, 56.3, 55.3, 54.9, 54.3, 41.5, 40.7, 29.6, 25.5, 24.4, 22.2, 20.7, 15.7, 8.0.
ESI-MS m/z: Calcd. for C₂₉H₃₄N₄O₇: 550.6. Found (M+H)⁺: 551.2

### Example 38

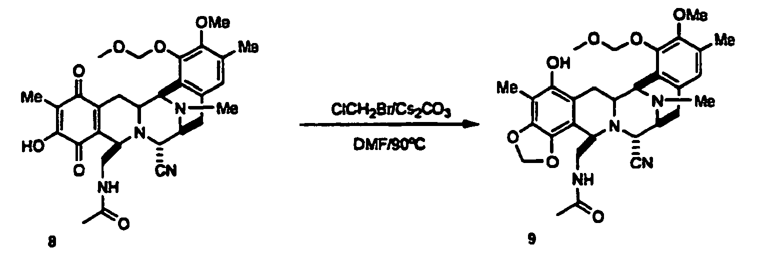

To a degassed solution of compound **8** (240mg, 0.435 ml) in DMF (30 ml) 10 % Pd/C (48 mg) was added and the reaction was stirred under H₂ (atmospheric pressure.) for 1h. The reaction was filtered through a pad of celite under Argon to a Schlenk tube, as a colourless solution, containing anhydrous Cs₂CO₃ (240 mg, 0.739 ml). Then, bromochloromethane (0.566 ml, 8.71 ml) was added. The tube was sealed and stirred at 90 °C for 3h. The reaction was cooled and filtrated through celite and washed with CH₂Cl₂. The organic layer was concentrated and dried (sodium sulphate) to afford **9** as a brown oil that was used in the next step with no further purification.
Rf: 0.36 (SiO₂, hexane:ethyl acetate 1:5)
¹H NMR (300 MHz, CDCl₃): δ 6.71 (s, 3H), 5.89 (d, 1H), 5.81 (d, 1H), 5.63 (bs, 1H), 5.33 (d, 1H), 5.17 (d, 1H), 4.97 (m, 1H), 4.20 (d, 1H), 4.09 (m, 1H), 3.99 (m, 1H), 3.68 (m, 1H), 3.65 (s, 6H), 3.59-3.47 (m, 4H), 3.37-3.27 (m, 2 h), 3.14- 2.97 (m, 2 h), 2.62 (d, 1H), 2.32 (s, 3H), 2.20 (s, 3H), 2.08 (s, 3H), 1.72 (m, 1H), 1.36 (s, 3H);
¹³C NMR (75 MHz, CDCl₃): δ 169.8, 149.1, 147.4, 145.5, 136.2, 130.9, 130.8. 125.0, 122.9, 117.7, 112.6, 111.8, 106.4, 100.8, 99.8, 59.8, 58.9, 57.7, 56.6, 56.4, 55.5, 55.2, 41.6, 40.1, 29.6, 25.9, 25.0, 22.6, 15.6, 8.8.
ESI-MS m/z: Calcd. for C₃₀H₃₆SiN₄O₇: 564.6. Found (M+H)⁺: 565.3.

### Example 39

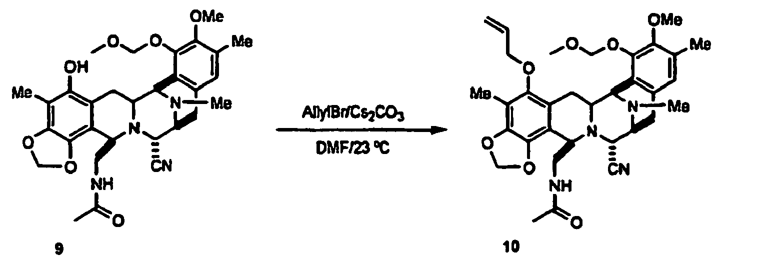

To a flask containing **9** (245 mg, 0.435 ml) in DMF, (4 ml), cesium carbonate (425 mg, 1.30 ml) and allyl bromide (376 ml, 4.35 ml) were added at 0 °C and the mixture was stirred at 23 °C for 1h. The reaction was filtered though a pad of celite and partitioned between CH₂Cl₂ (25 ml) and H₂O (10 ml). The organic phase was dried (sodium sulphate) and concentrated at reduced pressure to afford a residue that was purified by flash column chromatography (SiO₂, CHCl₃:ethyl acetate 1:2) to give **10** as a yellow oil. (113 mg, 43 %).
Rf: 0.36 (hexane:ethyl acetate 1:5)
¹H NMR (300 MHz, CDCl₃): δ 6.74 (s, 1H), 6.3-6.0 (m, 1H), 5.94 (d, 1H), 5.87 (d, 1H), 5.43-5.36 (m, 2 h), 5.22 (s, 2 h), 5.00 (m, 1H), 4.22 (m, 1H), 4.17-4.01 (m. 1H), 3.98 (m, 2 h), 3.71-3.67 (m, 1H), 3.69 (s, 3H), 3.62-3.51 (m, 3H), 3.58 (s, 3H), 3.39-3.37 (m, 1H), 3.31-3.26 (m, 3H), 3.09 (dd, 1H), 2.56 (d, 1H), 2.36 (s, 3H), 2.21 (s, 3H), 2.11 (s, 3H), 2.24-2.10 (m, 1H), 1.82-1.73 (m, 1H), 1.24 (bs, 3H)
¹³C NMR (75 MHz, CDCl₃): δ 169.4, 148.8, 148.3, 139.1, 133.7, 130.9, 130.3, 125.2, 120.2, 117.7, 113.1, 112.6, 101.3, 99.3, 74.1, 59.7, 59.3, 57.8, 57.0, 56.1, 56.1, 55.2, 41.6, 41.0, 40.9, 29.7, 26.3, 22.5, 15.6, 9.3
ESI-MS m/z: Calcd. for C₃₃H₄₀N₄O₇: 604.7. Found (M+H)⁺: 605.3.

### Example 40

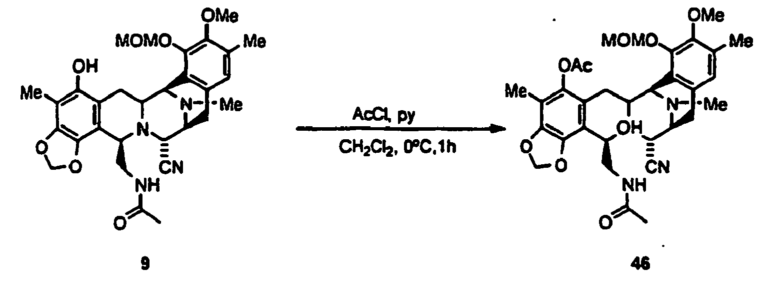

To a solution of **9** (22 mg, 0.039 ml) in CH₂Cl₂ (0.2 ml), acetyl chloride (2.79 ml, 0.039 ml) and pyridine (3.2 ml, 0.039 ml) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure to afford **46** (22 mg, 93%) as a white solid.
Rf: 0.4 (hexane:ethyl acetate 1:5).
¹H NMR (300 MHz, CDCl₃). δ 6.74 (s, 1H), 5.97 (d, *J=* 0.9 Hz, 1H), 5.91 (d, *J*= 0.9 Hz, 1H), 5.12 (d, *J*= 5.7 Hz, 2 h), 5.04 (d, *J*= 5.7 Hz, 1H) 4.90 (t, *J*= 6 Hz, 1H), 4.17 (d, *J*= 2.7 Hz, 1H), 4.05 (d, *J=* 2.7 Hz, 1H), 4.01 (bs, 1H), 3.71 (s, 3H), 3.57 (s, 3H), 3.50-3.44 (m, 2 h), 3.38-3.36 (m, 1H), 3.30-3.26 (m, 1H), 3.00 (dd, *J₁=* 7.8 Hz, *J₂=* 18.0 Hz, 1H), 2.79 (d, *J*= 12.9 Hz, 1H), 2.60 (d, *J*=18.0 Hz, 1H), 2.35 (s, 3H), 2.32 (s, 3H), 2.21 (s, 3H), 2.00 (s, 3H), 1.68 (dd, *J*₁=11.7 Hz, *J₂=* 15.6 Hz, 1H).
ESI-MS m/z: Calcd. for C_{32 h38}N₄O₈: 606.67. Found (M+H)⁺: 607.3.

### Example 41

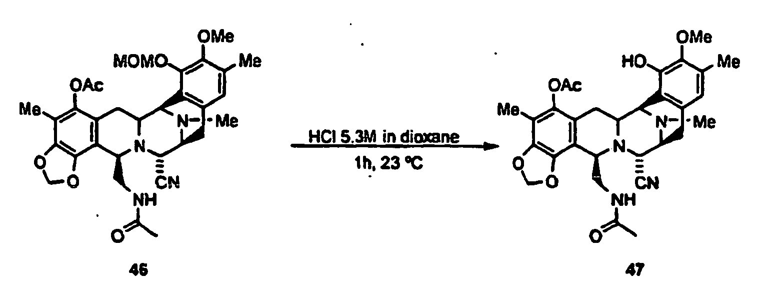

To a solution of **46** (8 mg, 0.013 ml) in dioxane (0.1 ml), 5.3N HCl/dioxane (0.5 ml) was added and the reaction was stirred at 23 °C for 1h. Then, the solution was diluted with CH₂Cl₂ (5 ml) and washed with 0.1 N HCl (3 ml). The organic layer was dried over sodium sulphate, filtered, and the solvent was eliminated under reduced pressure to afford **47** (5 mg. 70%) as a white solid.
Rf: 0.4 (hexane:ethyl acetate 1:5).
¹H NMR (300 MHz, CDCl₃). δ 6.51 (s, 1H), 5.97 (d, *J* = 1.2 Hz, 1H), 5.91 (d. *J* = 1.2 Hz, 1H), 4.97 (bs, 1H), 4.11 (bs, 1H), 4.04-4.02 (m, 2 h), 3.75 (s, 3H),), 3.65 (d, *J*= 2.1 Hz, 2 h), 3.56-3.30 (m, 2 h), 3.04 (dd, *J₁*= 7.5 Hz, *J₂*=18 Hz, 1H), 2.80 (d, *J*= 14.4 Hz, 1H), 2.59 (d, *J*= 18.3 Hz, 1H), 2.33 (s, 3H), 2.24 (s, 3H), 2.00 (s, 3H), 1.76 (dd, *J₁*= 12.0 Hz, *J₂*= 15.9 Hz, 1H), 1.33 (s, 3H), 1.25 (s, 3H).
ESI-MS m/z: Calcd. for C₃₀H₃₄N₄O₇: 562.61. Found (M+H)⁺: 563.3.

### Example 42

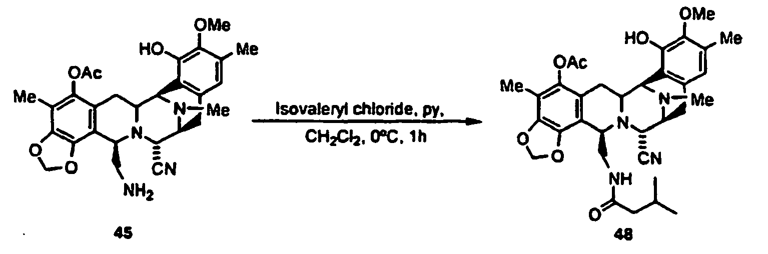

To a solution of **45** (10 mg, 0.0192 ml) in CH₂Cl₂ (0.3 ml), isovaleryl chloride (2.34 ml, 0.0192 ml) and pyridine (1.55 ml, 0.0192 ml) were added at 0 °C. The reaction mixture was stirred for 1h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 1:2) to afford **48** (11 mg, 95%) as a white solid.
Rf: 0.12 (Hex: ethyl acetate 1:2).
¹H NMR (300 MHz, CDCl₃): δ 6.50 (s, 1H), 5.98 (d, *J*=1.5Hz, 1H), 5.91 (d, *J*= 1.5 Hz, 1H), 5.75 (s, 1H), 5.02 (t, *J=* 5.4 Hz, 1H), 4.10 (d, *J=* 1.5 Hz, 1H), 4.06 (d, *J=* 2.7 Hz, 1H), 4.02 (d, *J=* 2.7 Hz, 1H), 3.77 (s, 3H), 3.76-3.71 (m, 1H), 3.86-328 (m, 3H), 3.04 (dd, *J₁*= 8.1 Hz, *J₂* = 18.3Hz, 1H), 2.78 (d, *J*=15.9 Hz, 1H), 2.55 (d, *J=*18 Hz, 1H), 2.32 (s, 6H), 2.26 (s, 3H), 1.98 (s, 3H), 1.84-1.68 (m, 2 h), 1.36 (d, *J*= 7.2 Hz, 2 h), 0.69 (d, *J=* 6.6 Hz, 3H), 0.62 (d, *J*=6.6 Hz, 3H).
ESI-MS m/z: Calcd. for C₃₃H₄₀N₄O₇: 604.69. Found (M+H)⁺: 605.3.

### Example 43

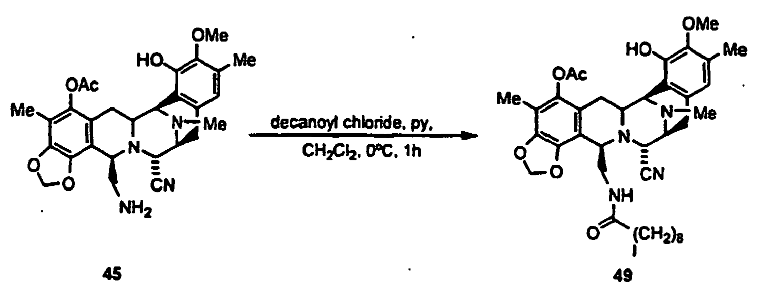

To a solution of 45 (10 mg, 0.0192 ml) in CH₂Cl₂ (0.3 ml), isovaleryl chloride (3.98 ml, 0.0192 ml) and pyridine (1.55 ml, 0.0192 ml) were added at 0°C. The reaction mixture was stirred for 1h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 1:2) to afford **49** (12.4 mg, 96%) as a white solid.
Rf: 0.7 (ethyl acetate:methanol10:1).
¹H NMR (300 MHz, CDCl₃): δ 6.50 (s, 1H), 5.98 (d, *J*=1.5Hz, 1H), 5.91 (d, *J*= 1.5 Hz, 1H), 5.73 (s, 1H), 5.08 (t, *J=* 5.4 Hz, 1H), 4.10 (d, *J=* 1.5 Hz, 1H), 4.05 (m., 1H), 4.01 (m, 1H), 3.76 (s, 3H), 3.65-3.61 (m, 1H), 3.40-3.27 (m, 3H), 3.03 (dd, *J₁=* 8.1 Hz, *J₂=*18.6 Hz, 1H), 2.78 (d, *J*=13.2 Hz, 1H), 2.57 (d, *J*=18.3 Hz, 1H), 2.32 (s, 3H), 2.31 (s, 3H), 2.25 (s, 3H), 1.99 (s, 3H), 1.79 (dd, *J₁*=12.0 Hz, *J₂=* 16.5 Hz, 1H), 1.73-1.42 (m, 4H), 1.33-1.18 (m, 10H), 1.03 (m, 2 h), 0.87 (t, *J=* 6.6 Hz, 3H).
ESI-MS m/z: Calcd. for C₃₈H₅₀N₄O₇: 674.83. Found (M+H)⁺: 675.5.

### Example 44

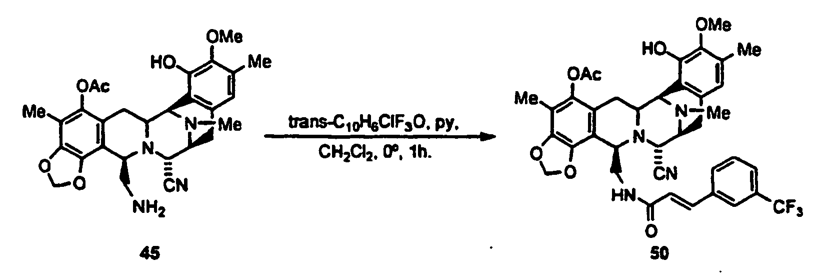

To a solution of **45** (14.5 mg, 0.0278 ml) in CH₂Cl₂ (0.3 ml), trans-3-trifluoromethyl cinnamoyl chloride (4.76 ml, 0.0278 ml) and pyridine (2.25 ml, 0.0278 ml) were added at 0 °C. The reaction mixture was stirred for 1h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 1:1) to afford **50** (18.7 mg, 94%) as a white solid.
Rf: 0.64 (ethyl acetate:methanol5:1).
¹H NMR (300 MHz, CH₃OD). δ 7.74-7.55 (m, 4H), 7.23 (d, *J*= 16.0 Hz, 1H), 6.34 (s, 1H), 6.12 (d, *J=* 16.0 Hz, 1H), 6.07 (d, *J*= 0.9 Hz, 1H), 5.96 (d, *J=* 0.9 Hz, 1H), 4.39 (d, *J=* 2.4 Hz, 1H), 4.07-4.05 (m, 1 H), 3.81 (bs, 1H), 3.46-3.51 (m, 3H), 3.42 (s, 3H), 3.09 (br d, *J*= 12.0 Hz, 1H), 2.94-2.85 (m, 2 h), 2.74 (d, *J=*18.3 Hz, 1H), 2.38 (s, 3H), 2.23 (s, 3H), 2.02 (s, 3H), 1.80 (s, 3H), 1.84-1.75 (m, 1H).
¹³C NMR (75 MHz, CDCl₃)): δ 168.7, 165.3, 146.5, 144.7, 142.6, 140.6, 138.0, 135.9, 131.0, 130.9, 129.1, 128.6, 125.8, 125.7, 124.5, 124.4, 122.7, 121.2, 117.8, 116.5, 113.0, 112.0, 101.7, 60.4, 59.1, 56.5, 56.4, 55.6, 55.3, 41.8, 40.3, 26.6, 25.1, 20.3, 15.4, 9.3.
ESI-MS m/z: Calcd. for C₃₈H₃₇F₃N₄O₇: 718.72. Found (M+H)⁺: 719.3.

### Example 45

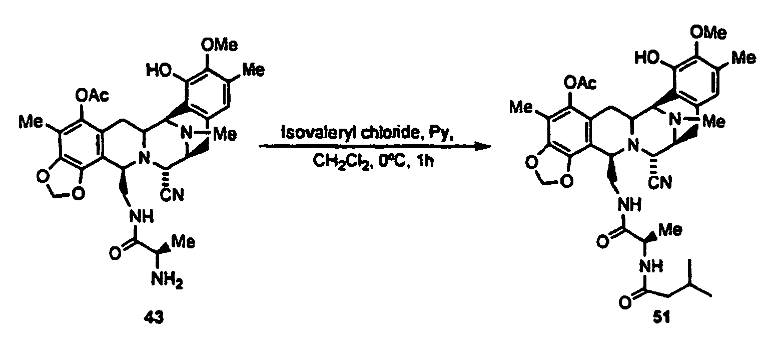

To a solution of **43** (33 mg, 0.0557 ml) in CH₂Cl₂ (0.4 ml), isovaleryl chloride (6.79 ml, 0.0557 ml) and pyridine (4.5 ml, 0.0557 ml) were added at 0 °C. The reaction mixture was stirred for 1h and then, the solution was diluted with CH₂Cl₂ (5ml) and washed with 0.1 N HCl (3 ml). The organic layer was dried over sodium sulphate, filtered, and the solvent was eliminated under reduced pressure. The residue was purified by flash column chromatography (SiO₂ Hex: ethyl acetate 1:2) to afford **51** (34 mg, 91%) as a white solid.
Rf: 0.09 (Hex: ethyl acetate 1:2).
¹H NMR (300 MHz, CDCl₃): δ 6.46 (s, 1H), 6.10 (bs, 1H), 5.99 (d, *J=* 0.9Hz, 1H), 5.90 (d, *J*= 0.9 Hz, 1H), 5.30 (t, *J=* 6.0 Hz. 1H), 4.10-4.05 (m, 3H),3.81 (bs, 1H), 3.74 (s, 3H), 3.54 (bs, 1H), 3.38-3.36 (m, 1H), 3.29-3.21 (m, 1H), 3.00 (dd, *J₁ =* 8.0 Hz, *J₂* = 18.0 Hz, 1H), 2.25 (s, 3H), 2.20 (s, 3H), 2.00 (s, 3H), 1.95-1.90 (m, 3H), 0.87 (d, *J=*6.6 Hz, 6H), 0.76 (d, *J*=6.0 Hz, 3H).
ESI-MS m/z: Calcd. for C₃₆H₄₅N₅O₈: 675.77. Found (M+H)⁺: 676.3.

### Example 46

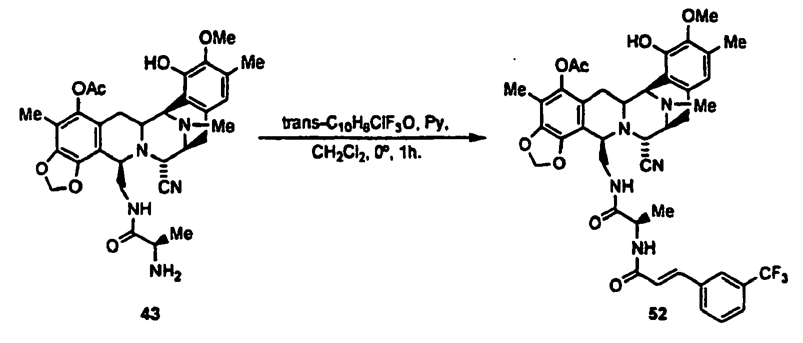

To a solution of **43** (33 mg, 0.0557 ml) in CH₂Cl₂ (0.4 ml), trans-3-trifluoromethyl cinnamoyl chloride (9.52 ml, 0.0557 ml) and pyridine (4.5 ml, 0.0557 ml) were added at 0 °C. The reaction mixture was stirred for 1h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 1:2) to afford **52** (40 mg, 92%) as a white solid.
Rf: 0.21 (hexane:ethyl acetate 1:2).
¹H NMR (300 MHz, CD₃OD). δ 7.74-7.47 (m, 4H), 6.49 (s, 1H), 6.40 (d, *J*= 15.6 Hz, 1H), 6.00 (d, *J*= 1.5 Hz, 1H), 5.90 (d, *J*= 1.5 Hz, 1H), 5.47 (t, *J*= 6 Hz, 1H), 4.12-4.09 (m, 3H), 3.93 (bs, 1H), 3.71 (s, 3H), 3.59-3.58 (m, 1H), 3.38 (d, *J=7.8* Hz, 1H), 3.29 (d, *J*=12.0 Hz, 1H), 3.00 (dd*, J₁=* 8.1 Hz*, J₂=* 18.3 Hz,1H), 2.79-2.78 (m, 1 H), 2.65 (d, *J*=18.3 Hz, 1H) 2.29 (s, 6H), 2.28 (s, 3H), 2.22 (s, 3H), 1.84-1.80 (m, 1H), 0.85-0.84 (m, 3H).
¹³C NMR (75 MHz, CDCl₃) δ 171.9, 168.8, 164.4, 146.9, 144.6, 143.0, 140.5, 140.5, 139.3, 135.7, 131.1, 131.0, 129.4, 129.1, 126.0, 124.1, 124.0, 122.4, 121.1, 120.7,120.6, 117.7, 116.9, 112.8, 112.0, 101.6, 60.6, 59.3, 57.1, 56.3, 55.9, 55.2, 49.0, 41.7, 49.9, 26.5, 25.1, 20.2, 18.4, 15.7, 9.3
ESI-MS m/z: Calcd. for C₄₁H₄₂F₃N₅O₈: 789.8. Found (M+H)⁺: 790.3.

### Example 47

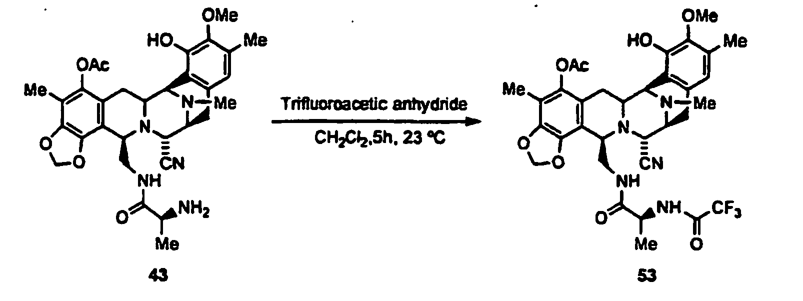

To a solution of **43** (10 mg, 0.0169 ml) in CH₂Cl₂ (0.2 ml) trifluoroacetic anhydride (2.38µl, 0.0169 ml) was added at 23 °C. The reaction mixture was stirred for 5h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 3:2) to afford **53** (10.7 mg, 93%) as a white solid.
Rf: 0.57 (ethyl acetate:methanol5:1).
¹H NMR (300 MHz, CDCl₃) δ 6.45 (s, 1H), 6.00 (d, *J=* 1.2 Hz, 1H), 5.90 (d, *J*=1.2 Hz, 1H), 5.87 (bs, 1H), 5.32 (bs, 1H), 4.12(d, *J*= 2.1 Hz, 1H), 4.08 (d, *J*= 1.8 Hz, 1H), 3.78-3.56 (m, 3H), 3.72 (s, 3H), 3.40 (d, *J*= 8.1 Hz, 1H), 3.25 (d,*J*= 9.3 Hz, 1H), 3.00 (dd, *J₁*= 8.4 Hz, *J₂=* 18.0 Hz, 1H), 2.77 (dd, *J₁=* 2.1 Hz,*J*₂= 15.9 Hz, 1H), 2.68 (d, *J* = 18.6 Hz,1H), 2.30 (s, 3H), 2.28 (s, 3H), 2.22 (s, 3H), 2.00 (s, 3H), 1.75 (dd*, J₁*=11.4 H_{z} *J₂=* 15.9 Hz, 1H), 0.69 (d, *J*= 6.3 Hz, 3H).
¹³C NMR (75 MHz, CDCl₃) δ 170.1, 168.6, 156.0, 147.0, 144.6, 143.0, 140.6, 140.4, 131.0, 129.4, 120.9, 120.7, 117.6, 116.8, 112.4, 112.1, 101.6, 60.5, 59.0, 57.1, 56.3, 55.6, 55.2, 48.7, 41.6, 39.4, 26.5, 24.9, 20.2, 17.8, 15.4, 9.2.
ESI-MS m/z: Calcd. for C₃₃H₃₆F₃N₅O₈: 687.63. Found (M+H)⁺: 688.66.

### Example 48

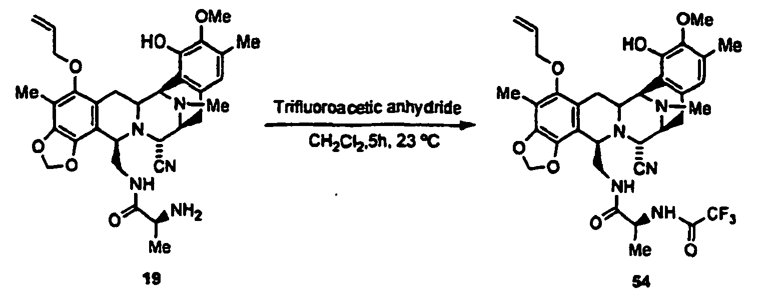

To a solution of **19** (11 mg, 0.0169 ml) in CH₂Cl₂ (0.2 ml) trifluoroacetic anhydride (2.38 ml, 0.0169 ml) was added at 23 °C. The reaction mixture was stirred for 5h and then, the solution was diluted with CH₂Cl₂ (5 ml) and washed with 0.1 N HCl (3 ml). The organic layer was dried over sodium sulphate, filtered, and the solvent was eliminated under reduced pressure. The residue was purified by flash column chromatography (SiO₂, Hex: ethyl acetate 3:2) to afford **54** (10.7 mg, 93%) as a white solid.
Rf: 0.6 (ethyl acetate:methanol5:1).
¹H NMR (300 MHz, CDCl₃) δ 7.33 (d, *J*= 6.3 Hz, 1H), 6.45 (s, 1H), 6.04 (m,1H), 5.95 (d, *J*= 1.5 Hz, 1H), 5.84 (d, *J=* 1.5 Hz, 1H), 5.32 (m, 2 h), 5.21 (m, 1H), 4.11 (m, 4H), 3.73 (s, 3H), 3.64 (m, 2 h), 3.51 (m, 1H), 3.37 (d, *J*= 7.8 Hz, 1H), 3.22 (m, 2 h), 3.03 (dd, 1H, *J₁=* 8.1 Hz, *J₂=* 18.3 Hz, 1H), 2.60 (d, *J*= 18.3 Hz, 1H), 2.29 (s, 3H), 2.24 (s, 3H), 2.08 (s, 3H), 1.86 (dd, *J*₁= 12 Hz, *J₂=* 16.2 Hz, 1H), 0.82 (d, *J=* 7.2 Hz, 3H).
¹³CNMR (75 MHz, CDCl₃) δ 170.0, 156.0, 148.4, 147.1, 144.3, 143.0, 138.7, 133.8, 130.5, 129.4, 120.6, 120.4, 117.6, 117.5, 117.0, 113.5, 112.5, 112.4, 101.1, 74.1, 66.8, 60.4, 59.3, 56.9, 56.6, 56.3, 55.4, 48.7, 41.6, 40.1, 26.2, 25.0, 17.6, 15.4, 9. 1.
ESI-MS m/z: Calcd. for C₃₅H₃₉F₃N₅O₇: 685.69. Found (M+H)⁺: 686.3.

### Example 49

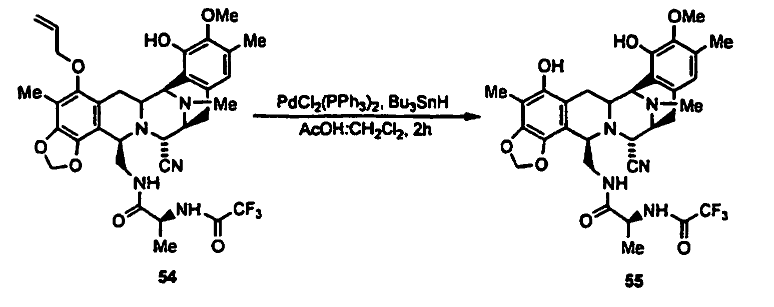

To a solution of **54** (100 mg, 0.415 ml) in CH₂Cl₂ (4 ml), acetic acid (40 ml), (PPh₃)₂PdCl₂ (8.4 mg, 0.012 ml) and Bu₃SnH (157 ml, 0.56 ml) were added at 23 °C. After stirring at that temperature for 2 h the reaction was poured into a pad of flash column (SiO₂, gradient Hex to hexane:ethyl acetate 2:1) to afford **55** (90 mg, 96%) as a white solid.
Rf: 0.6 (hexane:ethyl acetate 1:2).
¹H NMR (300 MHz, CDCl₃) δ 7.55 (d, *J*= 7.2 Hz, 1H), 6.45 (s, 1H), 5.90 (d, *J*= 1.2 Hz, 1H), 5.82 (d, *J*= 1.2 Hz, 1H), 5.37 (t, *J*= 6.0 Hz,1H), 4.15 (d, *J*= 2.1 Hz, 1H), 4.04 (d, *J*= 1.8 Hz, 1H), 3.70 (s, 3H), 3.66-3.53 (m, 2 h), 3.37-3.31 (m, 2 h), 3.19-3.15 (d, *J*= 11.7 Hz, 1H), 3.08-3.00 (m, 2 h), 2.56 (d, *J*=18.3 Hz, 1H), 2.30 (s, 3H), 2.24 (s, 3H), 2.04 (s, 3H), 1.91 (dd, *J*₁= 12.0 Hz, *J₂=* 15.6 Hz, 1H), 0.84 (d, *J=* 6.9 Hz, 3H).
¹³C NMR (75 MHz, CDCl₃) δ 170.1, 156.3, 147.3, 144.9, 144.4, 143.3, 136.7, 130.7, 129.3, 120.6, 117.6, 117.4, 114.4, 112.1, 107.7, 101.0, 85.8, 60.5, 59.3, 56.5, 56.4, 56.2, 55.2, 48.9, 41.6, 40.9, 25.7, 25.3, 18.0, 15.6, 8.7.
ESI-MS m/z: Calcd. for C_{32 h35}F₃N₅O₇: 645.63. Found (M+H)⁺: 646.2.

### Example 50

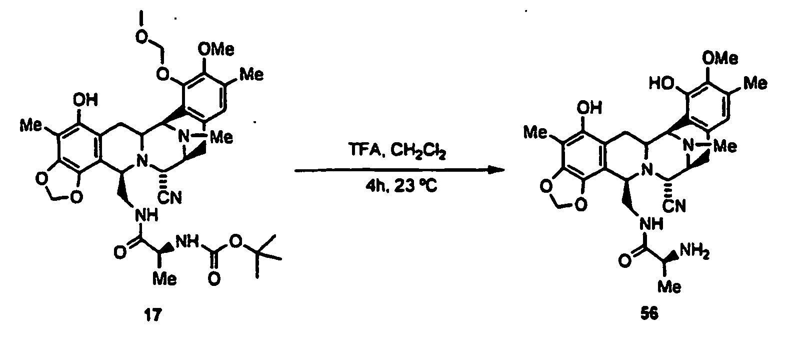

To a solution of **17** (200 mg, 0.288 ml) in CH₂Cl₂ (1.44 ml), trifluoroacetic acid (888 ml, 11.53 ml) was added and the reaction mixture was stirred for 4h at 23 °C. The reaction was quenched at 0 °C with saturated aqueous sodium bicarbonate (60 ml) and extracted with ethyl acetate (2 x 70 ml). The combined organic layers were dried (sodium sulphate) and concentrated *in vacuo* to afford **56** (147 mg, 93%) as a white solid that was used in subsequent reactions with no further purification.
Rf: 0.19 (ethyl acetate:methanol5:1).
¹H NMR (300 MHz, CD₃OD). 8 6.48 (s, 1H), 5.88, d, *J=* 0.9 Hz, 1H), 5.81 (d, *J=* 0.9 Hz 1H), 4.35 (d, *J* = 2.4 Hz, 1H),4.15 (d, *J=* 1.8 Hz, 1H), 3.99-3.98 (m, 1H), 3.70(s, 3H), 3.52-2.96 (m, 7H), 2.68 (d, *J=* 18.3 Hz, 1H), 2.24 (s, 3H), 2.23 (s, 3H), 2.06 (s, 3H), 1.85 (dd, *J₁*= 11.7 Hz, *J₂=* 15.6 Hz, 1H), 0.91 (d, *J=* 6.6 Hz, 3H).
¹³C NMR (75 MHz, CD₃OD): δ 173.2.149.1.145.6,144.9.138.0.132.2.130.6.121.4. 119.6, 117.4,114.3, 109.2, 102.5, 82.3, 60.4, 58.4, 58.3, 57.8, 56.6, 50.1, 42.3, 41.6, 27.8, 26.2, 19.5, 15.5, 9.8.
ESI-MS m/z: Calcd. for C₂₉H₃₅N₅O₆: 549.62. Found (M+H)⁺: 550.3.

### Example 51

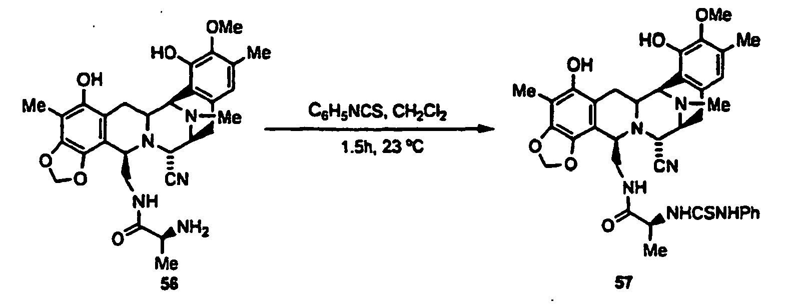

To a solution of **56** (10 mg, 0.018 ml) in CH₂Cl₂ (0.4 ml), phenyl isothiocyanate (13 ml, 0.109 ml) was added and the reaction was stirred at 23° C for 1.5h. The mixture was concentrated *in vacuo* and the residue was purified by flash column chromatography (SiO₂, gradient Hexane to 1:1 hexane:ethyl acetate) to afford **57** (8 mg, 65%) as a white solid.
Rf: 0.57 (ethyl acetate:methanol10:1).
¹H NMR (300 MHz, CDCl₃): δ 7.88 (bs, 1H), 7.41-7.36 (m, 2h), 7.27-7.22 (m, 1H), 7.02-7.00 (d, *J*= 7.8 Hz, 2 h), 6.71 (d, *J*= 7.2 Hz, 1H), 6.31 (s, 1H), 6.17 (bs, 1H), 5.93 (d, *J*=1.2 Hz,1H), 5.83 (d,*J*=: 12 Hz, 1H), 5.55 (bs, 1H), 5.20-5.17 (m, 1H), 4.16 (d, *J=* 1.8 Hz, 1H), 4.05 (bs, 1H), 4.02 (d, *J=* 2.4 Hz, 1H), 3.79 (s, 3H), 3.75-3.71 (m, 1H), 3.35 (d, *J*= 7.8 Hz, 1H), 3.28-3.19 (m, 2 h), 3.12-2.97 (m, 2 h), 2.50 (d, *J*=18.3 Hz, 1H), 2.32 (s, 3H), 2.21 (s, 3H), 2.15-2.09 (dd, *J₁*=11.4 Hz, *J*₂=15.9 Hz, 1H), 1.95 (s, 3H), 0.88 (d, *J*=6.9 Hz, 3H).
¹³C NMR (75 MHz, CDCl₃): δ 178.5, 171.7, 147.2, 145.0, 144.3, 143.3, 137.0, 135.7, 130.6, 130.4, 129.6, 127.5, 124.3, 120.6, 117.7, 117.2, 115.3, 112.1, 108.3, 100.9, 60.9, 59.5, 56.7, 56.5, 56.2, 55.2, 54.1, 41.7, 41.1, 26.3, 25.4, 18.5, 15.8, 9.0.
ESI-MS m/z: Calcd. for C₃₆H₄₀N₆O₆S: 684.81. Found (M+H)⁺: 685.3.

### Example 52

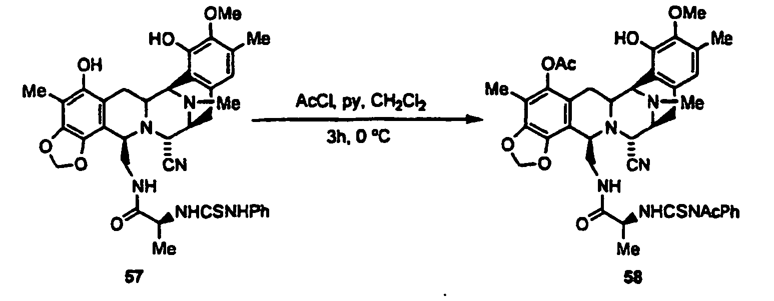

To a solution of **57** (45 mg, 0.065 ml) in CH₂Cl₂ (0.5 ml), acetyl chloride (4.67 ml, 0.065 ml) and pyridine (5.3 ml, 0.065 ml) were added at 0 °C. The reaction mixture was stirred for 3h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (RP-18, CH₃CN : H₂O 40:60) to afford **58** (14 mg, 28%) as a white solid.
Rf: 0.34 (CH₃CN: H₂O 7:15).
¹H NMR (300 MHz, CDCl₃). δ 11.90 (d, *J*= 6.6 Hz, 1H), 7.45-7.40 (m, 3H). 7.18-7.15 (m, 2 h), 6.58 (s, 1H), 6.00 (d, *J* = 1.2 Hz, 1H), 5.89 (d, *J* = 1.2 Hz, 1H), 5.70 (s. 1H). 5.37 (t, *J*= 4.8 Hz, 1H), 4.48 (m, 1H), 4.23 (bs, 1H), 4.07 (bs, 2 h), 3.85-3.75 (m, 1H), 3.70 (s, 3H), 3.46-3.41 (m, 2 h), 3.24-3.20 (m, 1H), 3.00-2.95 (m, 1H), 2.87-2.75 (m, 1H), 2.31 (s, 3H), 228 (s, 3H), 2.24 (s, 3H), 2.00 (s, 3H), 1.85 (dd, *J₁*= 11.4 Hz, *J₂ =*15.6 Hz, 1H), 1.66 (s, 3H), 0.82 (d, *J=* 6.0 Hz, 3H).
¹³C NMR(75MHz, CDCl₃)):δ 182.6, 174.3, 171.0, 146.6, 144.6, 142.7, 142.3, 140.7, 140.2, 131.3, 129.8, 129.3, 128.9, 128.8, 121.5, 120.4, 117.3, 116.6, 112.8, 112.0, 111.3, 101.5, 60.5, 59.0, 57.6, 56.2, 55.9, 55.3, 55.1, 41.6, 39.4, 27.8, 26.5, 24.8, 20.2, 17.1, 15.5, 9.3.
ESI-MS m/z: Calcd. for C₄₄H₄₄N₆O₈S: 768.88. Found (M+H)⁺: 769.2.

### Example 53

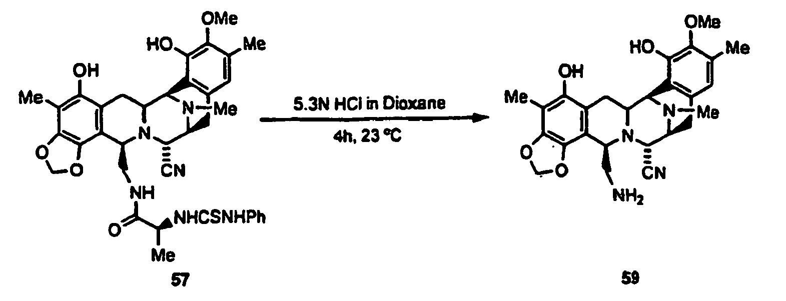

A solution of **57** (130 mg, 0.189 ml) in dioxane (1 ml), 5.3N HCl/dioxane (1.87 ml) was added and the reaction was stirred at 23 °C for 4h. Then, CH₂Cl₂ (15 ml) and H₂O (10 ml) were added to this reaction and the organic layer was decanted. The aqueous phase was basified with saturated aq sodium bicarbonate (60 ml) (pH = 8) at 0 °C and then, extracted with ethyl acetate (2x50 ml). The combined organic extracts were dried (sodium sulphate), and concentrated *in vacuo* to afford **59** (63 mg, 70%) as a white solid.
Rf: 0.15 (ethyl acetate:methanol5:1).
¹H NMR (300 MHz, CDCl₃). δ 6.67 (s, 1H), 5.99 (d,*J*= 0.9 Hz. 1H), 5.91(d. *J*= 1.2 Hz, 1H), 5.10 (bs, 1H), 4.32 (d, *J*= 7.2 Hz,1H), 4.25 (dd, *J₁=* 3.6 Hz, *J₂*= 9.3 Hz, 1H), 3.7 (s, 3H), 3.71-3.64 (m, 2 h), 3.50 (dd, *J₁=* 2.4 Hz, *J₂=* 15.9 Hz, 1 H), 3.42-3.37 (m, 2 h), 3.16 (dd, *J₁*=3.6 Hz, *J₂*= 12.9 Hz, 1H), 2.57 (dd, *J₁*= 9.3 Hz, *J₂*= 12.9 Hz, 1H), 2.27 (s, 3H), 2.11 (s, 3H), 1.91 (dd, *J₁=* 12.0 Hz, *J₂=* 15.9 Hz, 1H).
ESI-MS m/z: Calcd. for C₂₆H₃₀N₄O₅: 478.5. Found (M+H)⁺: 479.3.

### Example 54

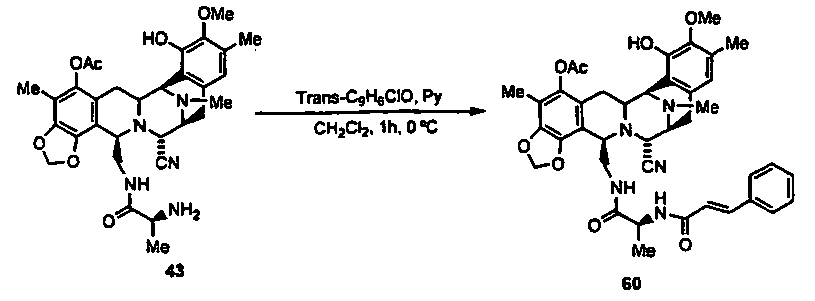

A solution of **43** (20 mg, 0.0338 mmol) in CH₂Cl₂ (0.3 ml), cinnamoyl chloride (5.63 mg , 0.0338 mmol) and pyridine (2.73 ml, 0.0338 mmol) were added at 0°C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **60** (22 mg, 90%) as a white solid.
Rf: 0.56 (EtOAc:MeOH 5:1).
¹H NMR (300 MHz, CDCl₃). 7.51 (s, 1H), 7.50-7.47 (m, 2H), 7.36-7.35 (m, 2H), 6.43 (s 1H), 6.36 (brd, *J*= 15.9 Hz, 2H), 6.01 (d, *J*= 1.5 Hz, 1H), 5.90 (brd, *J*= 1.5 Hz, 2H), 5.42 (t, *J*= 6.0 Hz 1H), 4.12-4.07 (m, 3H), 3.96-3.95 (m, 1H), 3.73 (bs, 3H), 3.58 (bs, 2H), 3.39 (d, *J*= 8.7 Hz, 1H), 3.25 (d, *J=* 11.7 Hz, 1H), 3.0 (dd, *J₁=* 7.5 Hz, *J₂=* 17.7 Hz, 1H), 2.78 (d, *J*= 15.9 Hz, 1H), 2.67 (d, *J*= 16.5 Hz, 1H), 2.29 (s, 6H), 2.23 (s, 3H), 1.99 (s, 3H), 1.82 (dd, *J₁*= 11.4 Hz, *J₂*= 15.6 Hz, 1H), 0.83 (d, *J*= 6.0 Hz, 3H).
¹³CNMR (75 MHz, CDCl₃)): δ. 172.0,165.0,146.9,144.6,143.1,141.0,140.5,134.8, 131.0, 129.7, 129.1, 128.8, 127.8, 125.5, 123.8, 123.0, 121.1, 120.5, 117.7, 116.9, 112.8, 112.0, 101.9, 60.6, 59.2, 57.1, 56.4, 55.9, 55.3, 48.8, 41.7, 40.0, 26.5, 25.1, 20.3, 18.5, 15.7, 9.3.
ESI-MS m/z: Calcd. for C₄₀H₄₃N₅O₈: 721.8. Found (M+H)⁺: 722.3.

### Example 55

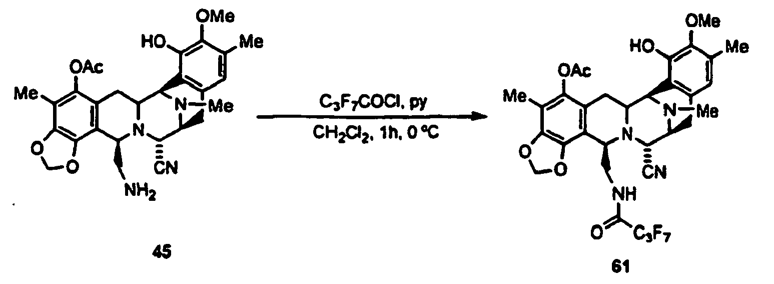

A solution of **45** (19 mg, 0.0364 mmol) in CH₂Cl₂ (0.3 ml), heptafluorobutyryl chloride ( 5.44 ml, 0.0364 mmol) and pyridine (2.95 ml, 0.0364 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **61** (11.7 mg, 45%) as a white solid.
Rf: 0.76 (EtOAc:MeOH 5:1).
¹H NMR (300 MHz, CDCl₃) δ 6.46 (s, 1H), 6.12 (bs, 1H), 5.98 (d, *J*= 1.2 Hz, 1H), 5.93 (d, *J*= 1.2 Hz, 1H), 5.72 (bs, 1H), 4.13-4.11 (m, 2H), 4.0 (d,*J*= 2.4 Hz, 1H), 3.98-3.96 (m, 1H), 3.73 (s, 3H), 3.39 (d, *J=* 7.5 Hz, 1H), 3.39-3.28 (m, 2H), 3.09 (dd, *J₁=* 8.1 Hz, *J*₂= 18.0 Hz, 1H), 2.80 (d, *J*= 16.2 Hz, 1H), 2.46 (d, *J*= 18.3 Hz, 1H), 2.32 (s, 6H), 2.21 (s, 3H), 1.99 (s, 3H), 1.80 (dd, *J₁=* 12.0 Hz, *J*₂*=* 16.2 Hz, 1H).
ESI-MS m/z: Calcd. for C₃₂H₃₁F₇N₄O₇: 716.6. Found (M+H)⁺: 717.2.

### Example 56

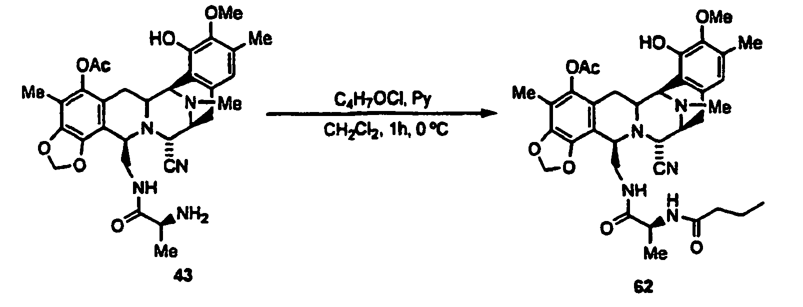

A solution of **43** (24 mg, 0.04 mmol) in CH₂Cl₂ (0.3 ml), butyryl chloride (4.15 ml, 0.04 mmol) and pyridine (3.28 ml, 0.04 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **62** (24 mg, 90%) as a white solid.
Rf: 0.35 (EtOAc:MeOH 5:1).
¹H NMR (300 MHz, CDCl₃) δ 6.47 (s, 1H), 6.10 (d, *J*= 6.5 Hz, 1H), 6.0 (d. *J*= 1.5 Hz, 1H), 5.91 (d, *J*=1.5 Hz, 1H), 5.86 (bs, 1H), 5.31 (d, *J*= 6.9 Hz, 1H), 4.11-4.06 (m. 3H), 3.85-3.81 (m, 1H), 3.75 (s, 3H), 3.59-3.53 (m, 2H), 3.38 (d, *J*= 7.5 Hz, 1H), 3.27-3.22 (m, 1H), 3.0 (dd, *J₁=* 7.8 Hz, *J₂*= 17.4 Hz, 1H), 2.79 (d, *J*= 15.3 Hz, 1H), 2.63 (d, *J*= 17.7 Hz, 1H), 2.31 (s, 3H), 2.0 (s, 3H), 1.80 (dd, J*₁*= 12.0 Hz, *J₂*= 15.9 Hz, 1H), 1.58 (q, *J=* 7.2 Hz, 2H), 0.89 (t, *J*= 7.2 Hz, 3H), 0.76 (d, *J*= 6.6 Hz, 3H).
ESI-MS m/z: Calcd. for C₃₅H₄₃N₅O₈: 661.64. Found (M+H)⁺: 662.3

### Example 57

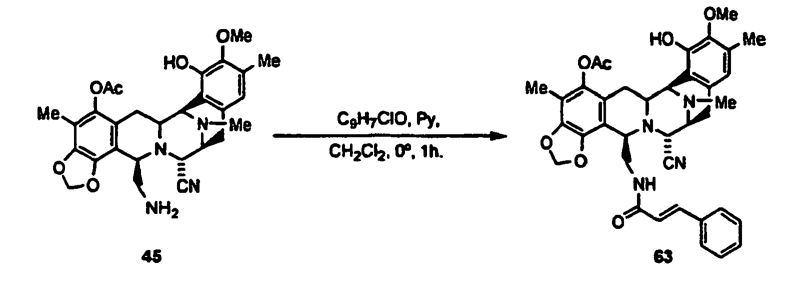

A solution of **43** (19 mg, 0.0364 mmol) in CH₂Cl₂ (0.3 ml), cinnamoyl chloride (6.06 mg, 0.0364 mmol) and pyridine (2.95 ml, 0.0364 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **63** (20.1 mg, 85%) as a white solid.
Rf: 0.65 (EtOAc:MeOH 5:1).
¹H NMR (300 MHz, CDCl₃) δ 7.39-7.29 (m, 5H), 6.42, (s, 1H), 6.01 (d, *J*= 1.5 Hz, 1H), 5.92 (d,*J*= 1.5 Hz, 1H), 5.73 (bs, 1H), 5.24 (t,*J*= 6.8 Hz, 1H), 4.12-4.08 (m, 3H), 3.66-3.64 (m, 2H), 3.58 (bs, 3H), 3.36 (d, *J=* 8.7 Hz, 1H), 3.29 (d, *J*= 12.0 Hz, 1H), 2.98 (dd, *J₁=* 8.1 Hz, *J₂=* 18 Hz, 1H), 2.33 (s, 6H), 2.29 (s, 3H), 2.01 (s, 3H), 1.84 (dd, *J₁=* 12.0 Hz, *J₂=* 15.9 Hz,1H).
ESI-MS m/z: Calcd. for C₃₇H₃₈N₄O₇: 650.72. Found (M+H)⁺: 651.2.

### Example 58

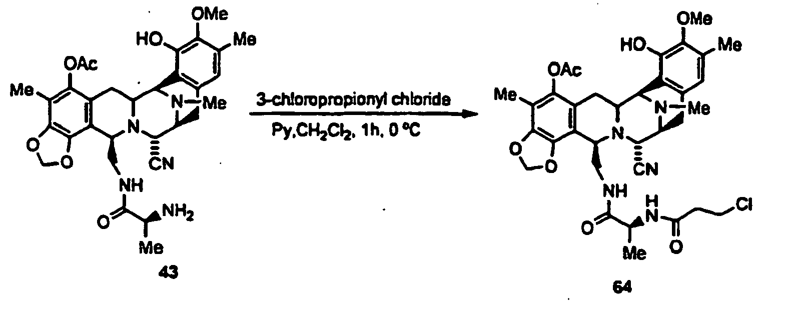

A solution of **43** (20 mg, 0.0338 mmol) in CH₂Cl₂ (0.3 ml), 3-chloropropionyl chloride (3.22 ml , 0.0338 mmol) and pyridine (2.73 ml, 0.0338 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **64** (20.5 mg, 89%) as a white solid.
Rf: 0.32 (EtOAc:Hexane 5:1).
¹H NMR (300 MHz, CDCl₃) 6.48 (s, 3H), 6.28 (m, 1H), 5.99 (d, *J=* 1.2 Hz, 1H). 5.91 (d,*J*= 1.2 Hz, 1H), 5.86 (bs, 1H), 5.31 (m, 1H), 4.08-4.07 (m, 3H), 3.75 (s, 3H), 3.72-3.53 (m, 5H), 3.39 (d,*J*= 8.1 Hz, 1H), 3.24 (d,*J*= 12.0 Hz, 1H), 3.00 (dd, *J₁*= 8.1 Hz, *J*₂= 18.0 Hz, 1H), 2.79 (d, *J*= 13.5 Hz, 1H), 2.50 (t, *J*= 6.3 Hz, 2H), 2.32 (s, 3H), 2.28 (s, 3H), 2.25 (s, 3H), 2.0 (s, 3H), 1.79 (dd, *J₁=* 12.3 Hz, *J₂=* 14.8 Hz, 1H), 0.81 (d, *J=* 6.3 Hz, 3H).

### Example 59

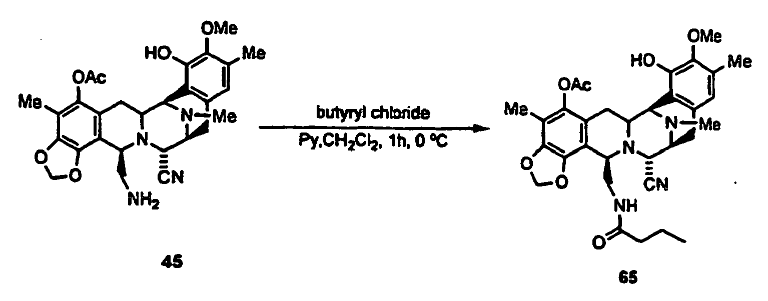

A solution of **43** (19 mg, 0.0364 mmol) in CH₂Cl₂ (0.3 ml), butyryl chloride (3.78 ml , 0.0364 mmol) and pyridine (2.95 ml, 0.0364 mmol) were added at 0 °C. The reaction mixture was stirred for 1h and then, the solution was diluted with CH₂Cl₂ (10 ml) and washed with 0.1 N HCl (5 ml). The organic layer was dried over sodium sulphate, filtered, and the solvent was eliminated under reduced pressure. The residue was purified by flash column chromatography (SiO₂, EtOAc:MeOH 20:1) to afford **64** (19 mg, 87%) as a white solid.
Rf: 0.60 (EtOAc:MeOH 5:1).
¹H NMR (300 MHz, CDCl₃) 6.50 (s, 1H), 5.98 (d, *J*= 1.5 Hz,1 H), 5.91 (d. *J*= 1.5 Hz, 1H), 5.75 (s,1H), 5.01 (t, *J*= 6.4 Hz, 1H), 4.10 -4.09 (m, 1H), 4.06 (d, *J*= 2.1 Hz, 1H). 4.03-4.02 (m, 1H), 3.76 (s, 3H), 3.67-3.60 (m, 1H), 3.42-3.35 (m, 2H), 3.29 (d, *J*= 12.0 Hz. 1H), 3.02 (dd, *J₁ =* 7.8 Hz, *J₂=* 17.7 Hz, 1H), 2.79 (d, *J*= 14.1 Hz, 1H), 2.56 (d, *J*= 18.3 Hz, 1H), 2.32 (s, 3H), 2.31 (s, 3H), 2.25 (s, 3H), 1.78 (dd, *J₁*= 12.0 Hz, *J₂*= 15.9 Hz, 1H), 1.63 (s, 3H), 1.53-1.46 (m, 2H), 1.28-1.16 (m, 2H), 0.68 (t, *J*= 7.2 Hz, 3H).
ESI-MS m/z: Calcd. for C₃₂H₃₈N₄O₇: 590.67. Found (M+H)⁺: 591.2.

### Example 60

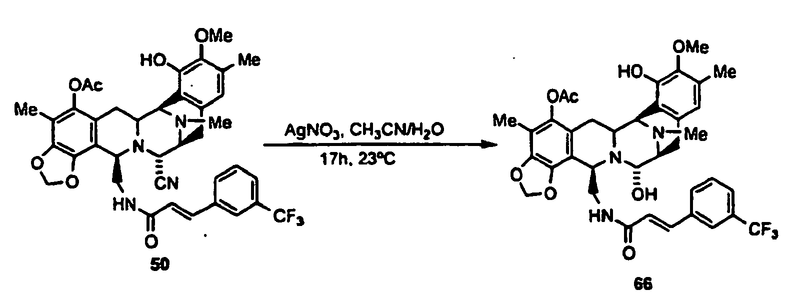

To a solution of **50** (31.7 mg, 0.044 mmol) in CH₃CN/H₂O (1.5 ml/0.5 ml), AgNO₃ (225 mg, 1.32 mmol) was added and the reaction was stirred at 23°C for 17 h. Then brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo.* The residue was purified by flash column chromatography (SiO₂, EtOAc:MeOH 5:1) to afford **66** (16 mg, 51%) as a white solid.
Rf: 0.26 (EtOAc:MeOH 5:1).
¹H NMR (300 MHz, CDCl₃) δ 7.66-7.42 (m, 4H), 7.20 (bs. 1H), 6.44 (s, 1H), 5.97 (b, *J*= 1.2 Hz, 1H), 5.90 (d,*J*= 1.2 Hz, 1H), 5.76 (bs, 1H), 5.28 (bs, 1H), 4.54 (bs, 1H), 4.43 (bs, 1H), 4.00 (bs, 1H), 3.68-3.57 (m, 4H), 3.47 (d, *J*= 3.3 Hz, 1H), 3.40 (d, *J*= 11.7 Hz, 1H), 3.17 (d. *J*= 6.9 Hz, 1H), 2.92 (dd, *J₁=* 8.1 *Hz, J₂=* 17.7 Hz, 1H), 2.74 (d, *J*= 17.1 Hz, 1H), 2.48 (d, *J*= 18.6 Hz, 1H), 2.32 (s, 6H), 2.28 (s, 3H), 1.99 (s, 3H), 1.76 (dd, *J₁*= 12.0 Hz. *J₁=* 16.2 Hz. 1H).
ESI-MS m/z: Calcd. for C₃₇H₃₈F₃N₃O₈: 709. Found (M⁺-17): 692.3.

### Example 61

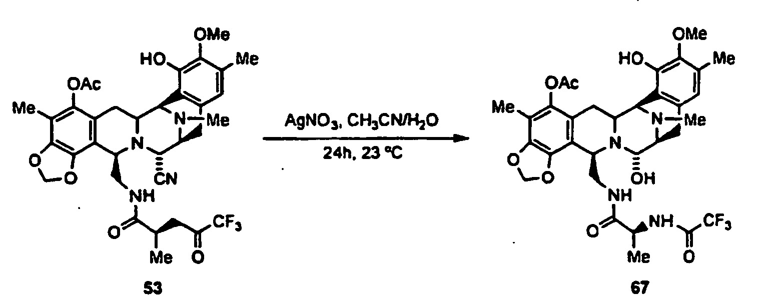

To a solution of **53** (57 mg, 0.0828 mmol) in CH₃CN/H₂O (1.5 ml/0.5 ml), AgNO₃ (650 mg, 3.81 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo.* The residue was purified by flash column chromatography (SiO₂, EtOAc:MeOH 5:1) to afford **67** (28 mg, 50%) as a white solid.
Rf: 0.28 (EtOAc:MeOH 10:1).
¹H NMR (300 MHz, CDCl₃) d
6.47 (s, 1H), 5.97 (s, 1H), 5.88 (s, 1H), 5.35 (bs, 1H), 4.51 (bs, 1H), 4.41 (bs, 1H), 4.12-4.05 (m, 1H), 4.00 (d, *J=* 2.7 Hz, 1H), 3.77 (s, 3H), 3.64 (bs, 1H), 3.46 (d, *J=* 3.3 Hz, I H), 3.34 (d, *J=* 11.4 Hz, 1H), 3.18 (d, *J=* 7.5 Hz, 1H), 2.95 (dd, *J₁=* 8.4 Hz, *J₂*=18.3 Hz 1H), 2.70 (d. *J*= 15.6 Hz, 1H), 2.48 (d, *J*= 17.7 Hz, 1H), 2.28 (s, 3H), 2.27 (s, 3H), 2.26 (s. 3H). 1.98 (s, 3H), 1.68 (dd, *J₁=*12 Hz, *J₂=15.6* Hz, 1H), 0.86 (d, *J*= 6.3 Hz, 3H).
ESI-MS m/z: Calcd. for C₃₂H₃₇F₃N₄O₉: 678.66. Found (M⁺-17): 661.2.

### Example 62

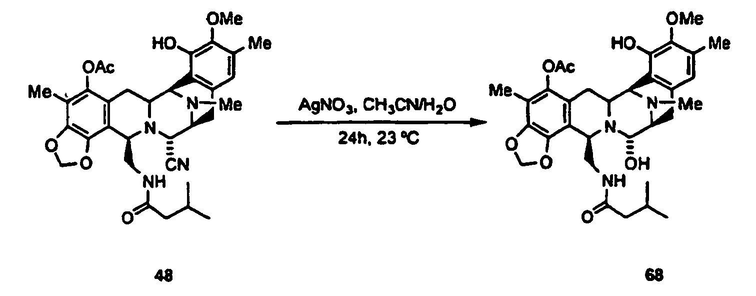

To a solution of **48** (32 mg, 0.0529 mmol) in CH₃CN/H₂O (1.5 ml/0.5 ml), AgNO₃ (270 mg, 1.58 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo.* The residue was purified by flash column chromatography (SiO₂, EtOAc:MeOH 5:1) to afford **68** (18 mg, 56%) as a white solid.
Rf: 0.40 (EtOAc:MeOH 5:1)
¹H NMR (300 MHz, CDCl₃) d 6.50 (s, 1H), 5.95 (d, *J=* 1.2 Hz, 1H), 5.88 (d,*J*= 1.2 Hz, 1H), 5.23 (d, *J*= 6.9 Hz, 1H), 4.45 (d, *J*= 3.3 Hz, 1H), 4.38 (s, 1H), 4.01 (d, *J*= 2.4 Hz, 1H), 3.78 (m, 1H), 3.77 (s, 3H), 3.41-3.37 (m, 1H), 3.17-3.15 (m, 1H), 2.96 (dd, *J₁*= 7.8 Hz, *J₂=* 18.0 Hz, 1H), 2.70 (d, *J*= 15.3 Hz, 1H), 2.40 (d, *J*= 18.0 Hz, 1H), 2.30 (s, 6H), 2.27 (s, 3H), 1.76-1.65 (m, 1H), 1.35-1.25 (m, 2H), 0.89-0.82 (m, 1H), 0.69 (d, *J=* 6.6 Hz, 3H),0.58 (d, *J=* 6.6 Hz, 3H)

### Example 63

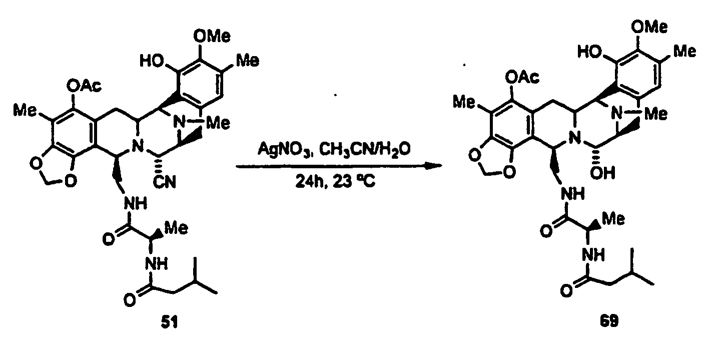

To a solution of **51** (27 mg, 0.04 mmol) in CH₃CN/H₂O (1.5 ml/0.5 ml). AgNO₃ (204 mg, 1.19 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo.* The residue was purified by flash column chromatography (SiO₂, EtOAc:MeOH 5:1) to afford **69** (10 mg, 38%) as a white solid.
Rf: 0.38 (EtOAc:MeOH 5:1).
¹H NMR (300 MHz, CDCl₃) d 6.48 s, 1H), 6.16 (bs, 1H), 5.98 (d, *J*= 1.5 Hz, 1H), 5.89 (d, *J*= 1.5 Hz, 1H), 5.33 (t, *J*= 6.0 Hz, 1H), 4.50 (m, 1H), 4.40 (m, 1H), 4.11-4.09 (m, 1H), 4.00 (d, *J*=2.6Hz, 1H), 3.78 (s, 3H), 3.41-3.32 (m, 3H), 3.18 (d, *J*= 8.4 Hz, 1H), 2.94 (dd, *J₁*= 8.4 Hz, *J₂=* 18.3 Hz, 1H), 2.70 (d,*J*= 14.4 Hz, 1H), 4.45 (d,*J*= 18.3 Hz, 1H), 2.31 (s, 3H), 2.28 (s, 3H), 2.27 (s, 3H), 2.04 (s, 3H), 2.00-1.86 (m, 3H), 1.73 (m, 1H), 0.87 (d, *J*= 6.3 Hz, 6H).

### Example 64

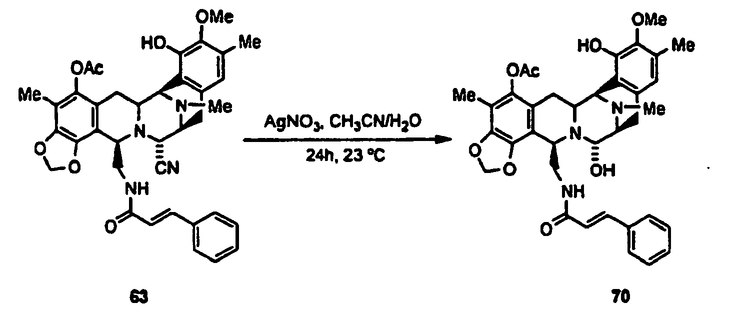

To a solution of **63** (15 mg, 0.023 mmol) in CH₃CN/H₂O (1.5 ml/0.5 ml), AgNO₃ (118 mg, 0.691 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo.* The residue was purified by flash column chromatography (SiO₂, EtOAc:MeOH 5:1) to afford **70** (20.1 mg, 85%) as a white solid.
Rf: 0.43 (EtOAc:MeOH 5:1).
¹H NMR (300 MHz, CDCl₃) d 7.38-7.28 (m, 5H), 6.48 (s, 1H), 5.98 (d, *J*=1.5 Hz, 1H), 5.91 (d, *J*=1.5 Hz,1H), 5.75 (bs,1H), 5.38 (brd, 1H), 5.30 (bs,1H), 4.53 (m, 1H), 4.42 (m, 1H), 4.02 (d, *J*=2.7 Hz, 1H), 3.78-3.65 (m, 5H), 3.46-3.40 (m, 2H), 3.17 (d, *J*=7.8 Hz, 1H), 2.94 (dd, *J*₁=7.8 Hz, *J₂=*17*.*7 Hz, 1H), 2.73 (d, *J*=16.8 Hz, I H), 2.45 (d, *J=*18.0 Hz, 1H), 2.31 (s, 6H), 2.28 (s, 3H), 1.97 (s, 3H), 1.77 (dd, *J₁*=12.0 Hz, *J₂=*15.3 Hz, 1H).

### Example 65

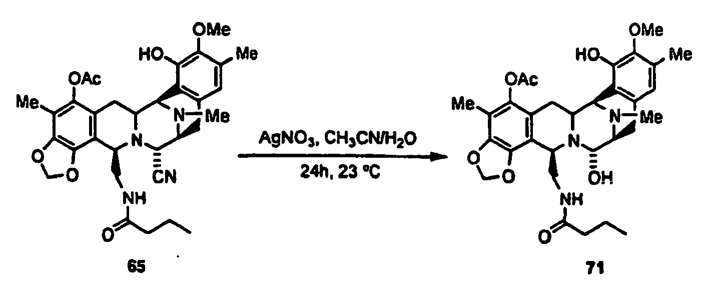

To a solution of **65** (25 mg, 0.042 mmol) in CH₃CN/H₂O (1.5 ml/0.5 ml), AgNO₃ (215.56 mg, 1.269 mmol) was added and the reaction was stirred at 23°C for 24 h. Then, brine (10 ml) and Aq sat NaHCO₃ (10 ml) were added at 0°C and the mixture was stirred for 15 min, filtered through a pad of celite and washed with CH₂Cl₂ (20 ml). The solution was decanted and the organic layer was dried and concentrated *in vacuo.* The residue was purified by flash column chromatography (SiO₂, EtOAc:MeOH 5:2) to afford **71** (16mg, 65%) as a white solid.
Rf: 0.0.5 (EtOAc:MeOH 5:2).
¹H NMR (300 MHz, CDCl₃) d 6.50 (s, 1H), 5.95 (d, *J=*1.5 Hz, 1H), 5.78 (s, 1H), 5.19 (bs, 1H), 4.45 (d, *J*=3.3 Hz, 1H), 4.37 (bs, 1H), 4.11 (brd, *J*=4.8 Hz, 1H), 4.01 (d, *J*=2.1 Hz, 1H), 3.76 (s, 1H), 3.71-3.69 (m, 1H), 3.49-3.35 (m, 1H), 3.24 (d, *J*=13.5 Hz, 1H), 3.15 (d, *J=*9.3 Hz, 1H), 2.95 (dd, *J*₁=8.1 Hz, *J₂=*17.7 Hz, 1H), 2.70 (d, *J*=15.6 Hz, 1H), 2.40 (d, *J*=18.0 Hz, 1H), 2.31 (s, 3H), 2.29 (s, 3H), 2.26 (s, 3H), 1.96 (s, 3H), 1.75-1.66 (m, 1H), 1.52-1.17 (m, 2H), 0.66 (t,*J*=7.2 Hz, 3H).

### Example 66

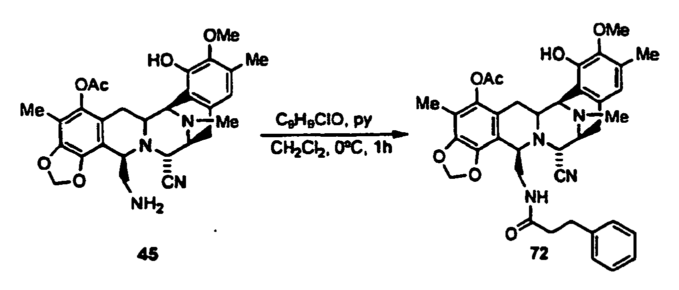

To a solution of 45 (35 mg, 0.0672 mmol) in CH₂Cl₂ (0.3 mL), hydrocinnamoyl chloride (11.58 µl, 0.0672 mmol) and pyridine (5.43 µL, 0.0672 mmol) were added at 0 °C. The reaction mixture was stirred for 1.5 h and then, the solution was diluted with CH₂Cl₂ (10 mL) and washed with 0.1 N HCl (5 mL). The organic layer was dried over Na₂SO₄, filtered, and the solvent was eliminated under reduced pressure. The residue was purified by flash column chromatography (SiO₂, gradient Hex: ethyl acetate 2:1 to ethyl acetate) to afford **72** (30 mg, 68%) as a white solid.
Rf: 0.51 (ethyl acetate:MeOH 10:1).
¹H NMR (300 MHz, CDCl₃) δ 7.23-7.12 (m, 3H), 7.05-7.00 (m, 2H). 5.97 (d. *J*= 1.2 Hz 1H), 5.91 (d, *J=* 1.2 Hz,1H), 5.73 (s, 1H), 5.04 (brt, 1H), 4.08 (d, *J*= 2.4 Hz, 1H), 4.02 (bs. 1H), 4.00 (d, *J*= 2.4 Hz, 1H), 3.58 (dd, *J₁=* 4.5 Hz, *J₂=* 13.8 Hz, 1H), 3.47 (bs,3H). 3.33 (d. *J*= 7.5 Hz, 1H), 3.29 (dt*, J₁=* 2.7 Hz, *J₂=* 11.7 Hz, 1H), 3.00 (dd, *J₁=* 7.8 Hz, *J₂*= 18.3 Hz, 1H), 2.79 (d, *J*=14.1 Hz, 1H), 2.58-2.50 (m, 3H), 2.32 (s, 3H), 2.29 (s. 3H), 2.03 (s, 3H), 2.01 (s, 3H), 1.94-1.76 (m, 4H).
ESI-MS m/z: Calcd. for C₃₇H₄₀N₄O₇: 652.7. Found (M+Na)⁺: 675.3.

### Example 67

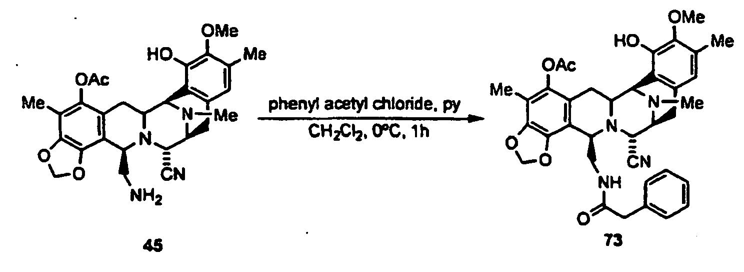

To a solution of **45** (45 mg, 0.0576 mmol) in CH₂Cl₂ (0.3 mL), phenyl acetyl chloride ( 7.61 µl, 0.0576 mmol) and pyridine (4.6 µL, 0.0576 mmol) were added at 0 °C. The reaction mixture was stirred for 1h and then, the solution was diluted with CH₂Cl₂ (10 mL) and washed with 0.1 N HCl (5 mL). The organic layer was dried over Na₂SO₄. filtered, and the solvent was eliminated under reduced pressure. The residue was purified by flash column chromatography (SiO₂, gradient Hex:ethyl acetate 3:1 to Hex:ethyl acetate 1:1) to afford **73** (25.8 mg, 70 %) as a white solid.
Rf: 0.5 (Hex:ethyl acetate:MeOH 5:10:2).
¹H NMR (300 MHz, CDCl₃) δ 7.18-7.17 (m, 3H), 6.85 (bs, 2H), 6.54 (s, 1H), 5.89 (d, *J=* 1.5Hz, 1H), 5.83 (d, *J*= 1.5 Hz, 1H), 5.76 (s, 1H), 5.08 (bs,1 1H), 4.12 (d, *J*= 2.1 Hz, 1H), 4.09 (d, *J=* 2.1 Hz, 1H), 3.98 (bs, 1H), 3.73 (s, 3H), 3.51-3.46 (m, 2H), 335 (d, *J* = 8.4 Hz, 1H), 3.25 (dt, *J*₁= 2.7 Hz, *J₂=* 12.0 Hz, 1H), 3.03 (d, *J*= 8.7 Hz, 1H), 3.02-2.94 (m, 2H), 2.75 (d, *J*=16.8 Hz, 1H), 2.63 (d, *J=*18.0 Hz, 1H), 2.35 (s, 3H), 2.30 (s, 3H), 2.22 (s. 3H9, 1.98 (s, 3H), 1.80 (dd*, J₁=*12.0 Hz, *J₂=*16.2 Hz, 1H).
ESI-MS m/z: Calcd. for C₃₆H₃₈N₄O₇: 638.7. Found (M+1)⁺: 639.2.

### Example 68

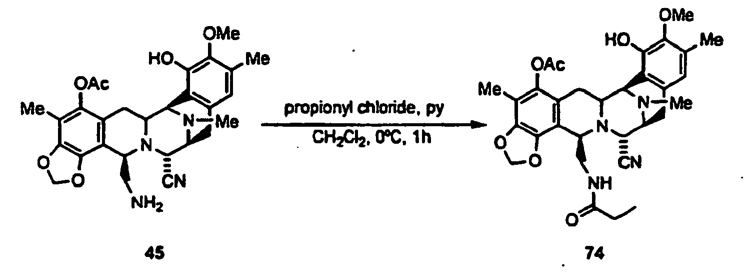

To a solution of **45** (30 mg, 0.0576 mmol) in CH₂Cl₂ (0.3 mL), propyonyl chloride ( 5 µL, 0.0576 mmol) and pyridine (4.6 µL, 0.0576 mmol) were added at 0 °C. The reaction mixture was stirred for 1h and then, the solution was diluted with CH₂Cl₂ (10 mL) and washed with 0.1 N HCl (5 mL). The organic layer was dried over Na₂SO₄, filtered, and the solvent was eliminated under reduced pressure. The residue was purified by flash column chromatography (SiO₂, gradient Hex:ethyl acetate 5:1 to Hex:ethyl acetate 1:1 to ethyl acetate) to afford **74** (23 mg, 70 %) as a white solid.
Rf : 0.59 ((Hex:ethyl acetate:MeOH 5:10:2).
¹H NMR (300 MHz, CDCl₃) δ 6.50 (s, 1H), 5.97 (d, *J*= 1.2 Hz, 1H), 5.91 (d, *J*= 1.2 Hz, 1H), 5.76 (s, 1H), 5.00 (t, 1H), 4.09 (d, *J=* 1.2 Hz, 1H), 4.04 (bs, 2H), 3.74 (s, 3H), 3.62 (dd, *J₁=* 6.6 Hz, *J₂=* 13.2 Hz, 1H), 3.43 (bs, 1H), 3.37 (d, *J=* 8.4 Hz, 1H), 3.29 (d, *J*= 12.0 Hz, 1H), 3.02 (dd, *J₁*= 8.1 Hz, *J₂*= 18.3 Hz, 1H), 2.80 (d,*J*= 14.4 Hz, 1H), 2.55 (d, *J*= 18.0 Hz, 1H), 2.31 (s, 3H), 2.24 (s, 3H), 2.00 (s, 3H), 1.78 (dd, *J₁*= *12.0* Hz, *J₂=* 15.6 Hz, 1H), 1.64-1.50 (m, 2H), 0.70 (t, *J*= 7.8 Hz, 3H).
ESI-MS m/z: Calcd. for C₃₁H₃₆N₄O₇: 576.6. Found (M+1)⁺: 577.2.

### Example 69

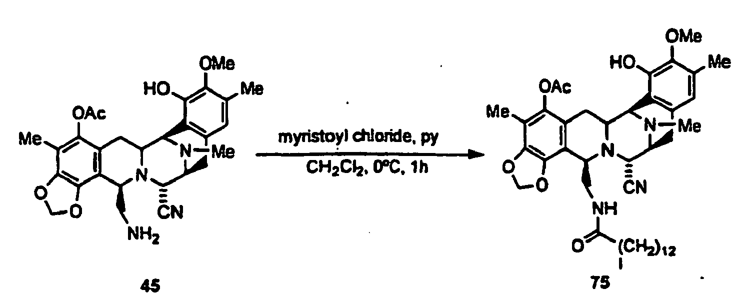

To a solution of 45 (15 mg, 0.0288 mmol) in CH₂Cl₂ (0.25 mL), myristoyl chloride ( 7.83 µL, 0.0288 mmol) and pyridine (2.3 µL, 0.0288 mmol) were added at 0°C. The reaction mixture was stirred for h and then, the solution was diluted with CH₂Cl₂ (10 mL) and washed with 0.1 N HCl (5 mL). The organic layer was dried over Na₂SO₄, filtered, and the solvent was eliminated under reduced pressure. The residue was purified by flash column chromatography (SiO₂, gradient Hex:ethyl acetate 6:1 to Hex:ethyl acetate 1:1) to afford **75** (15 mg, 71 %) as a white solid.
Rf: 0.65 (Hex:ethy acetate:MeOH 10:10:1).
¹H NMR (300 MHz, CDCl₃) δ 6.49 (s, 1H), 5.97 (d, *J=* 1.2 Hz, 1H), 5.91 (d, *J=* 1.2 Hz, 1H), 5.72 (s, 1H), 4.99 (t, 1H), 4.09 (d, *J=* 1.5 Hz, 1H), 4.05 (d, *J=* 1.5 Hz, 1H), 4.02 (bs, 1H), 3.76 (s, 3H), 3.61-3.59 (m, 1H), 3.39 (bs, 1H), 3.35 (d, *J=* 7.8 Hz, 1H), 3.29 (d, *J=* 12.3 Hz, 1H), 3.04 (dd, *J₁=* 8.1 Hz, *J₂=* 18.3 Hz, 1H), 2.78 (d, *J*= 15.6 Hz, 1H), 2.55 (d, *J=* 18.3 Hz, 1H), 2.32 (s, 6H), 2.25 (s, 3H), 1.99 (s, 3H), 1.78 (dd, *J*₁= 12.3 Hz, *J₂=* 15.0 Hz, 1H), 1.25-1.24 (m, 12H), 0.87 (d, *J*= 6.0 Hz, 3H).
ESI-MS m/z: Calcd. for C₄₂H₅₈N₄O₇: 730.9. Found (M+1)⁺: 731.4.

### Example 70

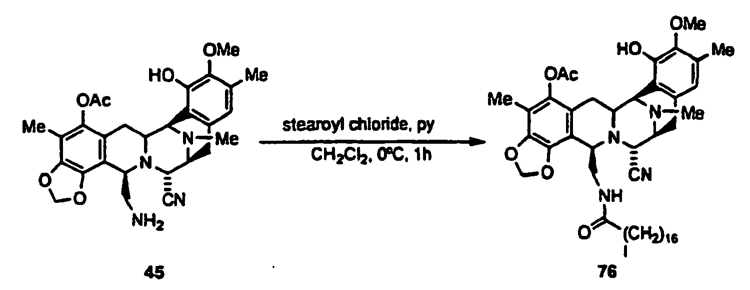

To a solution of **45** (15 mg, 0.0288 mmol) in CH₂Cl₂ (0.25 mL), stearoyl chloride ( 9.7 µL, 0.0288 mmol) and pyridine (2.3 µL, 0.0288 mmol) were added at 0 °C. The reaction mixture was stirred for 1h and then, the solution was diluted with CH₂Cl₂ (10 mL) and washed with 0.1 N HCl (5 mL). The organic layer was dried over Na₂SO₄, filtered, and the solvent was eliminated under reduced pressure. The residue was purified by flash column chromatography (SiO₂, gradient Hex:ethyl acetate 3:1 to Hex:ethyl acetate 1:1) to afford **76** (16 mg, 70 %) as a white solid.
Rf: 0.46 (Hex:ethyl acetate:MeOH 10:10:1).
¹H NMR NMR (300 MHz, CDCl₃) δ 6.49 (s, 1H), 5.98 (d, *J=* 1.5 Hz, 1H), 5.91 (d, *J=* 1.5 Hz, 1H), 5.73 (s, 1H), 4.99 (t, *J*= 5.7 Hz, 1H), 4.09 (d, *J*= 1.8 Hz, 1H), 4.05 (d, *J*= 2.4 Hz, 1H), 4.01 (bs, 1H), 3.76 (s, 3H), 3.61-3.59 (m, 1H), 3.38 (bs, 1H), 3.36 (d, *J*=7.2 Hz, 1H), 3.28 (d, *J*= 12.0 Hz, 1H), 3.03 (dd, *J₁=* 7.8 Hz, *J₂=*18.3 Hz, 1H), 2.78 (d, *J=*15.9 Hz, 1H), 2.57 (d, *J=*18.3 Hz, 1H), 2.32 (s, 3H), 2.31 (s, 3H), 2.24 (s, 3H), 1.99 (s, 3H), 1.77 (dd, *J₁*= 11.7 Hz, *J₂=* 15.6 Hz, 1H), 1.25-1.24 (m, 16H), 0.87 (d, *J=* 6.3 Hz, 3H).
ESI-MS m/z: Calcd. for C₄₆H₆₆N₄O₇: 786.4. Found (M+22)⁺: 809.5.

### Example 71

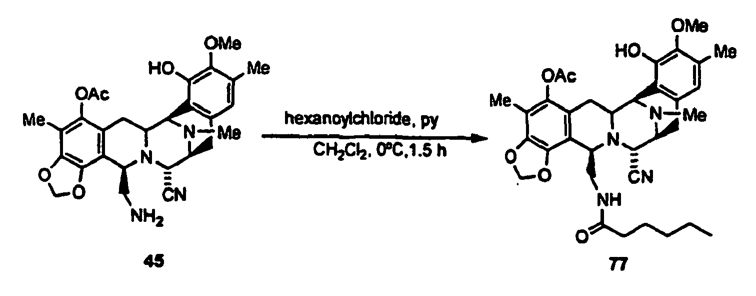

To a solution of 45 (31 mg, 0.0595 mmol) in CH₂Cl₂ (0.3 mL), hexanoyl chloride ( 8.32 µL, 0.0595 mmol) and pyridine (4.8 µL, 0.0595 mmol) were added at 0 °C. The reaction mixture was stirred for 1.5 h and then, the solution was diluted with CH₂Cl₂ (10 mL) and washed with 0.1 N HCl (5 mL). The organic layer was dried over Na₂SO₄, filtered, and the solvent was eliminated under reduced pressure. The residue was purified by flash column chromatography (SiO₂, gradient Hex:ethyl acetate 3:2 to ethyl acetate) to afford **77** (26 mg, 70 %) as a white solid.
Rf: 0.65 (ethyl acetate MeOH 10:1).
¹H NMR (300 MHz, CDCl₃) δ 6.50 (s, 1H), 5.98 (d, *J*= 1.5 Hz, 1H), 5.91 (d, *J=* 1.5 Hz, 1H), 5.74 (s, 1H), 5.00 (t,*J=* 5.4 Hz, 1H), 4.09 (d,*J*= 2.7 Hz, 1H), 4.05 (d, *J=* 2.4 Hz, 1H), 4.01 (bs, 1H), 3.76 (s, 3H), 3.61-3.58 (m, 1H), 3.02 (dd, *J₁=* 8.1 Hz, *J₂*= 18.3 Hz, 1H), 2.78 (d, *J*= 14.4 Hz, 1H), 2.56 (d, *J*= 18.3 Hz, 1H), 2.31 (s, 6H), 2.25 (s, 3H), 2.00 (s, 3H), 1.78 (dd, *J₁*= 12.0 Hz, *J₂*= 15.9 Hz, 1H), 1.53-1.40 (m, 2H), 1.29-1.12 (m, 4H), 1.07-0.97 (m, 2H), 0.81 (t, *J*= 7.5 Hz, 3H).
ESI-MS m/z: Calcd. for C₃₄H₄₂N₄O₇: 618.7. Found (M+1)⁺; 619.3.

### Example 72

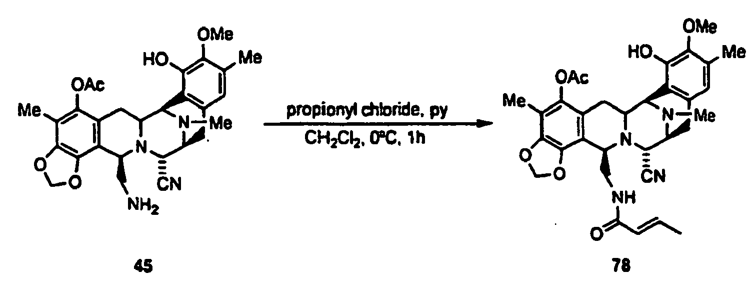

To a solution of **45** (20 mg, 0.0384 mmol) in CH₂Cl₂ (0.3 mL), trans-crotonyl chloride (3.68 µL, 0.0384 mmol) and pyridine (3.1 µL, 0.0384 mmol) were added at 0 °C. The reaction mixture was stirred for 1h and then, the solution was diluted with CH₂Cl₂ (10 mL) and washed with 0.1 N HCl (5 mL). The organic layer was dried over Na₂SO₄, filtered, and the solvent was eliminated under reduced pressure. The residue was purified by flash column chromatography (SiO₂, gradient Hex:ethyl acetate 4:1 to ethyl acetate) to afford **78** (16 mg, 71 %) as a white solid.
Rf: 0.55 (ethyl acetate:MeOH 5:1).
¹H NMR (300 MHz, CDCl₃) δ 6.50-6.40 (m, 1H), 6.46 (s, 1H), 5.97 (d,*J*= 1.5 Hz, 1H), 5.91 *(d,J=* 1.5 Hz, 1H), 5.77 (s, 1H), 5.08 (bst, 1H), 4.10 (d, *J=* 1.5 Hz, 1H), 4.05 (m, 2H), 3.78 (s, 3H), 3.67 (bs, 1H), 3.42-3.29 (m, 3H), 3.04 (dd, *J₁=* 8.1 Hz, *J*₂ = 18.3 Hz ,1H), 2.78 (d, *J* = 15.3 Hz, 1H), 2.53 (d,*J*= 18.3 Hz, 1H), 2.32 (s, 3H), 2.26 (s, 3H), 1.98 (s, 3H), 1.79 (dd, *J*₁= 12.0 Hz, *J*₂ = 15.6 Hz, 1H), 1.70 (dd, *J₁* = 1.2 Hz, *J*₂ = 6.6 Hz, 3H).
ESI-MS m/z : Calcd. for C₃₂H₃₆N₄O₇: 588.6. Found (M+1)⁺: 589.3.

### Example 73

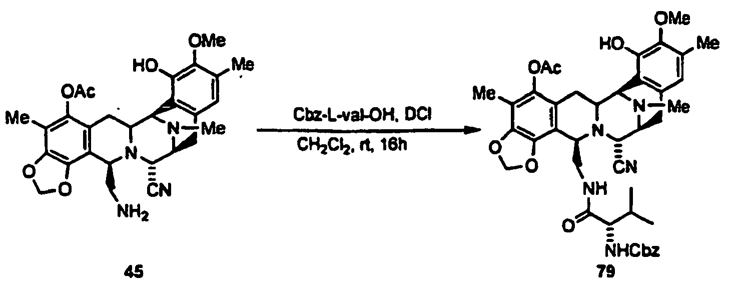

To a solution of **45** (50 mg, 0.096 mmol) in CH₂Cl₂ (0.5 mL), Cbz-L-Val-OH (24.12 mg, 0.096 mmol) and carbonyl diimidazole (18.7 mg, 0.115 mmol) were added at 0 °C. The reaction mixture was stirred for 16 h at room temperature and then, the solution was diluted with CH₂Cl₂ (15 mL) and washed with 0.1 N HCl (10 mL). The organic layer was dried over Na₂SO₄, filtered, and the solvent was eliminated under reduced pressure. The residue was purified by flash column chromatography (SiO₂, Hex:EtOAc 4:1) to afford **79** (25 mg, 34 %) as a white solid.
Rf: 0.7 (EtOAc:MeOH 5:1).
¹H NMR (300 MHz, CDCl₃) δ 7.33-7.28 (m, 5H), 6.45 (s, 1H), 5.96 (s, 1H), 5.90 (bs, 1H), 5.82 (s, 1H), 5.53 (bs, 1H), 5.09 (bs, 1H), 5.05 (d, *J*= 3.3 Hz, 2H), 4.16 (bs, 1H), 4.09 (d, *J*= 2.4 Hz, 1H), 4.02 (bs, 1H), 3.75 (s, 3H), 3.74 (m, 1H), 3.37-3.35 (m, 2H), 3.26-3.21 (m, 3H), 3.00 (dd, *J₁=* 8.1 Hz, *J₂=* 18.3 Hz, 1H), 2.77 (d, *J*= 15.6 Hz, 1H), 2.55 (d, *J*= 18.0 Hz, 1H), 2.30 (s, 3H), 2.27 (s, 3H), 2.25 (s, 3H), 1.98 (s, 3H), 1.70-1.66 (m, 1H), 0.65 (d, *J*= 6.6 Hz, 3H).
ESI-MS m/z: Calcd. for C₄₁H₄₇N₅O₉: 753.8. Found (M+1)⁺: 754.2.

### Example 74

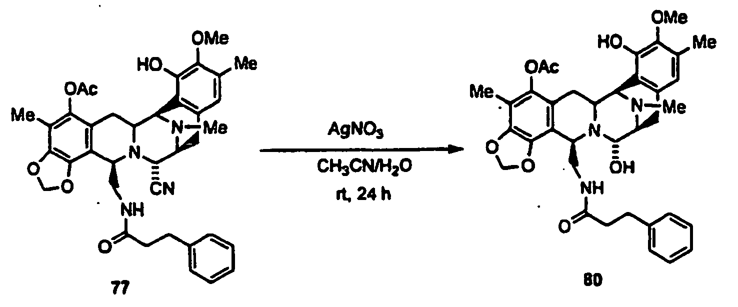

To a solution of **72** (18 mg, 0.0275 mmol) in CH₃CN/H₂O (1.5 mL/0.5 mL). AgNO₃ (140.5 mg, 0.827 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (20 mL). The solution was extracted and the organic layer was dried over Na₂SO₄ filtered and concentrated *in vacuo.* The residue was purified by flash column chromatography (SiO₂, EtOAc:MeOH 10:1) to afford **80** (13 mg, 74 %) as a white solid.
Rf: 0.37 (EtOAc:MeOH 5:1).
¹HNMR (300 MHz, CDCl₃) δ 7.23-7.11 (m, 3H), 7.06-7.01 (m, 2H), 6.43 (s, 1H), 5.95 (d, *J*= 1.2 Hz,1H), 5.88 (d, *J*= 1.2 Hz, 1H), 5.71 (bs,1H), 5.19 (bs,1H), 4.45 (d, *J*= 3.0 Hz, 1H), 4.37 (bs, 1H), 4.02-3.96 (m, 1H), 3.75-3.68 (m, 2H), 3.48 (s, 3H), 3.41-3.36 (m, 2H), 3.28-3.24 (m, 1H), 3.15 (d, *J* = 7.5 Hz, 1H), 3.01-2.88 (m, 2H), 2.70 (d, *J* = 15.9 Hz, 1H), 2.57-2.51 (m, 2H), 2.31 (s, 3H), 2.27 (s, 3H), 2.00 (s, 6H), 1.77-1.68 (m, 1H).
ESI-MS m/z: Calcd. for C₃₆H₄₁N₃O₈: 643.3. Found (M-17)⁺: 626.2.

### Example 75

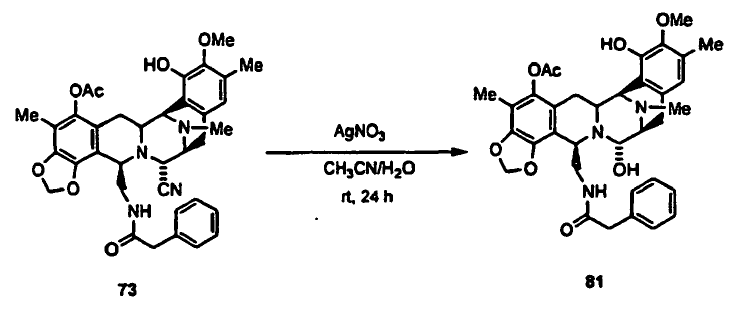

To a solution of **73** (23 mg, 0.036 mmol) in CH₃CN/H₂O (1.5 mL/1 mL), AgNO₃ (183 mg, 1.08 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (20 mL). The solution was extracted and the organic layer was dried over Na₂SO₄, filtered and concentrated *in vacuo.* The residue was purified by flash column chromatography (SiO₂ gradient EtOAc:MeOH 5:1 to MeOH) to afford **81** (9.3 mg, 41 %) as a white solid.
Rf: 0.3 (EtOAc:MeOH 5:1).
¹HNMR (300 MHz, CDCl₃) δ 7.17-7.13 (m, 3H), 6.85 (m, 2H), 6.54 (s, 1H), 5.90 (d, *J*= 1.5 Hz, 1H), 5.84 (d, *J* = 1.5 Hz, 1H), 5.22 (m, 1H), 4.43 (bs, 1H), 4.39 (d, *J=* 2.4 Hz, H), 4.00 (d, *J=* 2.4 Hz, 1H), 3.71 (s, 3H), 3.64-3.29 (m, 2H), 3.16 (d, *J=* 8.7 Hz, 1H), 2.98-2.88 (m, 3H), 2.67 (d, *J=* 14.8 Hz, 1H), 2.45 (d, *J*= 18.3 Hz, 1H), 2.33 (s, 3H), 2.28 (s, 3H), 2.22 (s, 3H), 1.97 (s, 3H), 1.68 (dd, *J₁=* 12.8 Hz, J₂= 14.7 Hz,1H).
ESI-MS m/z: Calcd. for C₃₅H₃₉N₃O₈: 629.7. Found (M⁺- OH): 612.3.

### Example 76

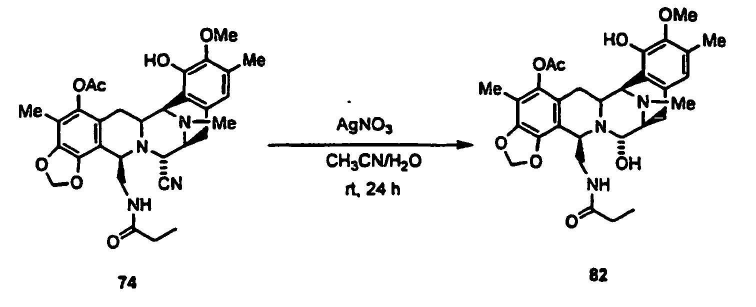

To a solution of **74** (20 mg, 0.0346 mmol) in CH₃CN/H₂O (1.5 mL/1 mL), AgNO₃ (176.6 mg, 1.04 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (20 mL). The solution was extracted and the organic layer was dried over Na₂SO₄, filtered and concentrated *in vacuo.* The residue was purified by flash column chromatography (SiO₂, EtOAc:MeOH 1:1) to afford **82** (12.9 mg, 66 %) as a white solid.
Rf: 0.3 (EtOAc:MeOH 5:1).
¹H NMR (300 MHz, CDCl₃) δ 6.50 (s,1H), 5.95 (d, *J*= 1.2 Hz, 1 H), 5.89 (d, *J*=1.2 Hz, 1H), 5.19 (d, 1H), 4.46 (d, *J*= 3.0 Hz, 1H), 4.38 (d, *J*= 1.8 Hz, 1H), 4.00 (d, *J*= 2.1 Hz, 1H), 3.74 (s, 3H), 3.70-3.66 (m, 1H), 3.38 (dt, *J₁*= 2.7 Hz, *J₂=* 13.2 Hz, 1H), 3.25 (d, *J*= 13.8 Hz, 1H), 3.16 (d, *J*= 7.5 Hz, 1H), 2.96 (dd, *J₁* = 7.2 Hz, *J₂ =* 17.7 Hz, 1H), 2.71 (d, *J*= 15.6 Hz, 1H), 2.40 (d, *J*=18.0 Hz, 1H), 2.30 (s, 3H), 2.29 (s, 3H), 2.24 (s, 3H), 1.97 (s, 3H), 1.71 (dd, *J*₁= 11.7 Hz, *J*₂= 15.3 Hz, 1H), 1.60-1.48 (m, 2H), 0.67 (t, *J*= 7.5 Hz, 3H).
ESI-MS m/z: Calcd. for C₃₀H₃₇N₃O₈: 567.6. Found (M-17)⁺: 550.2.

### Example 77

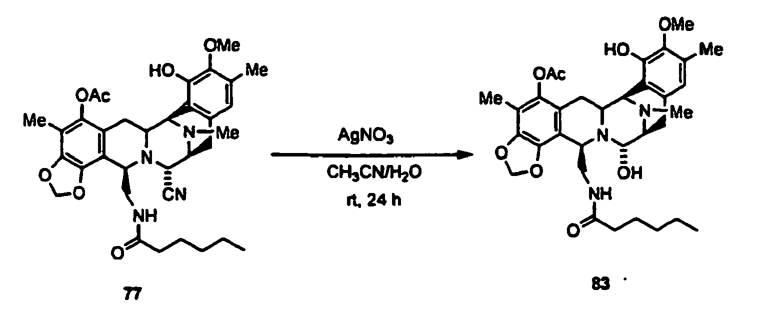

To a solution of **77** (14 mg, 0.0226 mmol) in CH₃CN/H₂O (1.5 mL/1 mL), AgNO₃ (115.3 mg, 0.68 mmol) was added and the reaction was stirred at 23 °C for 24 h. Then, brine (10 mL) and Aq sat NaHCO₃ (10 mL) were added at 0°C and the mixture was stirred for 15 min, filtered through a pad of celite and washed with CH₂Cl₂ (15 mL). The solution was extracted and the organic layer was dried over Na₂SO₄. filtered and concentrated *in vacuo.* The residue was purified by flash column chromatography (SiO₂ EtOAc:MeOH 5:1) to afford **83** (9 mg, 65 %) as a white solid.
Rf: 0.25 (EtOAc:MeOH 5:1).
¹H NMR (300 MHz, CDCl₃) δ 6.50 (s, 1H), 5.96 (d, *J=* 1.5 Hz, 1H), 5.89 (d, *J=* 1.5 Hz, 1H), 5.73 (bs, 1H), 4.44 (d, *J=* 3.6 Hz, 1H), 4.37 (s, 1H), 4.01 (d, *J=* 2.4 Hz, 1H), 3.77 (s, 3H), 3.73-3.64 (m, 1H), 3.39 (dt,*J₁*= 3.0 Hz, *J₂*= 9.3 Hz, 1H), 3.22 (d, *J=* 14.5 Hz, 1H), 3.16 (d, *J=* 7.5 Hz,1H), 2.95 (dd, *J₁=* 8.1 Hz, *J₂=* 17.4 Hz, 1H), 2.70 (d, *J=* 14.5 Hz, 1H), 2.41 (d,*J=* 18.3 Hz, 1H), 2.30 (s, 3H), 2.29 (s, 3H), 2.25 (s, 3H), 1.96 (s, 3H), 1.71 (dd,*J₁=* 12.0 Hz,*J₂=* 15.6 Hz, 1H), 1.48-1.46 (m, 2H), 1.24-1.10 (m, 4H), 1.00-0.95 (m, 2H), 0.80 (t, *J*= 7.2 Hz, 3H).
ESI-MS m/z: Calcd. for C₃₃H₄₃N₃O₈: 609.7. Found (M-17)⁺: 592.3.

### Example 78

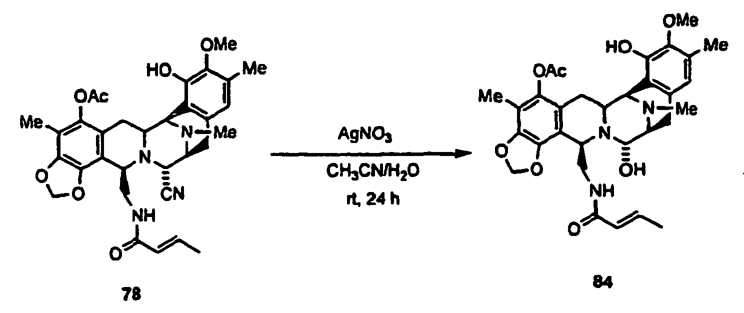

To a solution of 78 (15 mg, 0.025 mmol) in CH₃CN/H₂O (1.5 mL/1 mL), AgNO₃ (130 mg, 0.764 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂, gradient EtOAc to EtOAc:MeOH 1:1) to afford **84** (10 mg, 71 %) as a white solid.
Rf: 0.19 (EtOAc:MeOH 5:1).
¹H NMR (300 MHz, CDCl₃) δ 6.49 (s, 1H), 6.47-6.37 (m, 1H), 5.94 (d, *J*=1.5 Hz, 1H), 5.88 (d, *J=*1.5 Hz, 1H), 5.77 (bs, 1H), 5.26 (d, *J=*5.7 Hz, 1H), 4.93 (d, *J=*14.7 Hz, 1H), 4.48 (d, *J=* 11.1 Hz,1H), 4.38 (d, *J=* 2.7 Hz, 1H), 4.02 ((d, *J=* 2.1 Hz, 1H), 3.79 (s, 3H). 3.76-3.72 (m, 1H), 3.42 (dt, *J₁=* 2.7 Hz, *J*₂=12.0 Hz, 1H), 3.28 (d, *J=* 13.2 Hz, 1H), 3.15 (d, *J=* 6.6 Hz, 1H), 2.96 (dd, *J₁=* 8.7 Hz, *J₂=*18.0 Hz, 1H), 2.70 (d, *J=* 15.0 Hz, 1H), 2.38 (d, *J*= 18.0 Hz, 1H), 2.30 (s, 3H), 2.28 (s, 3H), 1.95 (s, 3H), 1.72 (dd, *J*₁= 12.3 Hz, *J₂=* 17.4 Hz, 1H), 1.98 (dd, *J₁=*1.5 Hz, *J₂*= 6.9 Hz, 3H).
ESI-MS m/z: Calcd. for C₃₂H₃₇N₃O₈: 579.6. Found (M-17)⁺: 562.3.

### Example 79

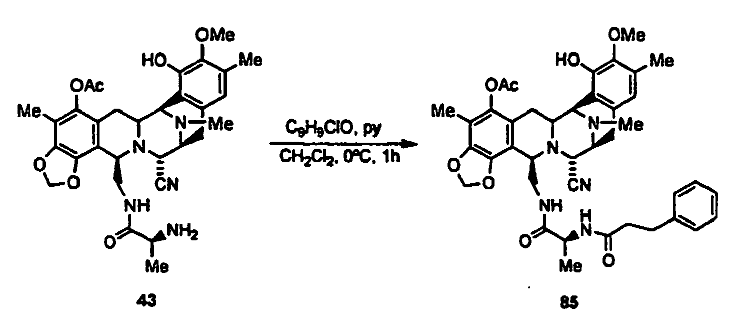

To a solution of 43 (25 mg, 0.422 mmol) in CH₂Cl₂ (0.3 mL), hydrocinnamoyl chloride (6.27 µL, 0.422 mmol) and pyridine (3.41 µL, 0.422 mmol) were added at 0°C. The reaction mixture was stirred for 1h and then, the solution was diluted with CH₂Cl₂ (10 mL) and washed with 0.1 N HCl (5 mL). The organic layer was dried over Na₂SO₄, filtered, and the solvent was eliminated under reduced pressure. The residue was purified by flash column chromatography (SiO₂, gradient Hex: EtOAc 4:1 to EtOAc) to afford **85** (30 mg, 68 %) as a white solid.
Rf: 0.54 (EtOAcMeOH 10:1).
¹H NMR (300 MHz, CDCl₃) δ 7.28-7.14 (m, 5H), 6.45 (s, 1H), 6.07 (brd, 1H), 5.99 (d, *J*= 1.2 Hz, 1H), 5.90 (d, *J* = 1.2 Hz, 1H), 5.8 (s, 1H), 5.31 (brt, 1H), 4.09-4.06 (m, 3 H), 3.80-3.75 (m, 1H), 3.73 (s, 3H), 3.57-3.51 (m, 2H), 3.38 (d, *J* = 7.5 Hz, 1H), 3.24 (m, 1H), 3.00 (dd, *J₁ =* 8.4 HZ, *J₂=* 18.0 Hz, 1H), 2.89-2.85 (m, 2H), 2.79 (d, *J*=16.5 Hz, 1H), 2.61 (d,*J*= 18.0 Hz, 1H), 2.31 (s, 3H), 2.28 (s, 3H), 2.22 (s, 3H), 2.00 (s, 3H), 1.79 (dd, *J₁*= 12.3 Hz, *J₂=* 16.2 Hz, 1H), 0.72 (d, *J*= 6.6 Hz, 3H).
ESI-MS m/z: Calcd. for C₄₀H₄₅N₅O₈: 723.8. Found (M+23)⁺: 746.3.

### Example 80

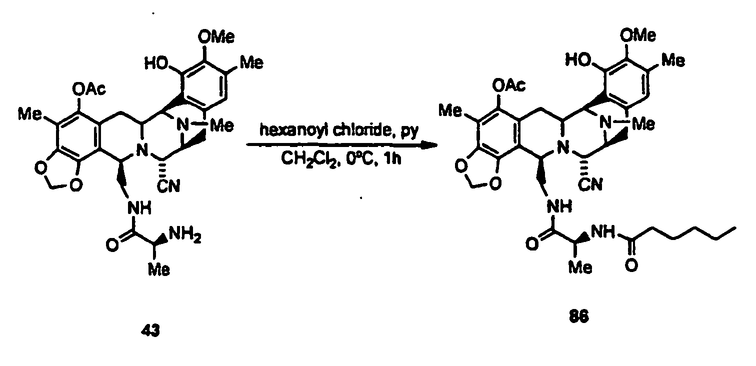

To a solution of **43** (20 mg, 0.0338 mmol) in CH₂Cl₂ (0.25 mL), hexanoyl chloride (4.72 µL, 0.0338 mmol) and pyridine (2.73 µL, 0.0338 mmol) were added at 0 °C. The reaction mixture was stirred for 1h and then, the solution was diluted with CH₂Cl₂ (10 mL) and washed with 0.1 N HCI (5 mL). The organic layer was dried over Na₂SO₄, filtered, and the solvent was eliminated under reduced pressure. The residue was purified by flash column chromatography (SiO₂, gradient Hex:EtOAc 1:1 to EtOAc) to afford **86** (10 mg, 43 %) as a white solid.
Rf: 0.74 (EtOAc:MeOH 10:1).
¹H NMR (300 MHz, CDCl₃) δ 6.47 (s, 1H), 6.12 (brd, 1H), 6.00 (d, *J*= 1.2 Hz, I H), 5.91 (d, *J*=1.2 Hz, 1H), 5.30 (m, 1H), 4.09-3.99 (m, 3H), 3.84-3.82 (m, 1H), 3.75 (s, 3H), 3.57-3.55 (m, 2H), 3.39 (d, *J*= 6.9 Hz,1H), 3.24 (d, *J*= 12.0 Hz, 1H), 3.04 (dd, *J₁=* 9.0 Hz, *J₂*= 18.3 Hz, 1H), 2.77 (d, *J*= 115.3Hz, 1H), 2.63 (d, *J*=18.0 Hz, 1H), 2.32 (s, 3H), 2.28 (s, 3H), 2.25 (s, 3H), 2.00 (s, 3H), 1.80 (dd, *J₁=* 11.7 Hz, *J₂=* 15.6 Hz, 1H), 1.55-1.50 (m, 2H), 1.30-1.22 (m, 6H), 0.87 (t, *J*= 6.9 Hz, 3H), 0.75 (d, *J*= 6.6 Hz, 3H).
ESI-MS m/z: Calcd. for C₃₇H₄₇N₅O₈: 689.8. Found (M+1)⁺: 6903.

### Example 81

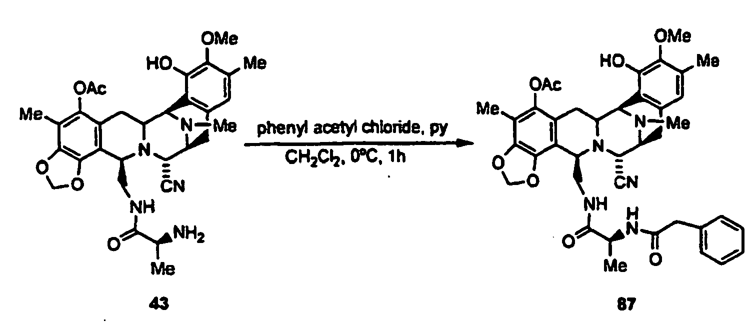

To a solution of **43** (33 mg, 0.0557 mmol) in CH₂Cl₂ (0.4 mL), phenyl acetyl chloride (7.36 µL, 0.0557 mmol) and pyridine (4.5 µL, 0.0557 mmol) were added at 0 °C. The reaction mixture was stirred for 1h and then, the solution was diluted with CH₂Cl₂ (10 mL) and washed with 0.1 N HCl (5 mL). The organic layer was dried over Na₂SO₄, filtered, and the solvent was eliminated under reduced pressure. The residue was purified by flash column chromatography (SiO₂, gradient Hex:EtOAc 2:1) to afford **87** (13 mg, 32 %) as a white solid.
Rf : 0.63 (Hex : EtOAc : MeOH 5:10:2).
¹H NMR (300 MHz, CDCl₃) δ 7.37-7.20 (m, 5H), 6.26 (s, 1H), 6.14 (d, *J*= 6.6 Hz, 1H), 5.98 (d, *J* = 1.2 Hz, 1H), 5.83 (s, 1H), 5.27 (t, *J*= 6.2 Hz, 1H), 4.11 (d, *J=* 2.1 Hz, 1H), 4.07 (d, *J*= 3.0 Hz, 1H), 4.04 (s, 1H), 3.86-3.81 (m, 1H), 3.70 (s, 3 H), 3.54-3.53 (m, 2H), 3.44 (bs, 2H), 3.36 (d, *J=* 8.1 Hz, 1H), 3.22 (dt, *J₁*= 2.7 Hz, *J₂=* 12.0 Hz, 1H), 2.93 (dd, *J₁ =* 7.2 Hz, *J*₂= 18.3 Hz, 1H), 2.77 (d, *J*= 14.4 Hz, 1H), 2.59 (d, *J*= 18.0 Hz, H), 2.31 (s, 3H), 2.26 (s, 3H), 2.17 (s, 3H), 2.01 (s, 3H), 1.78 (dd, *J₁ =* 10.8 Hz, *J₂=* 15.6 Hz,1H), 0.65 (d, *J=* 6.3 Hz, 1H).
ESI-MS m/z: Calcd. for C₃₉H₄₃N₅O₈: 709.8. Found (M+1)⁺: 710.3.

### Example 82

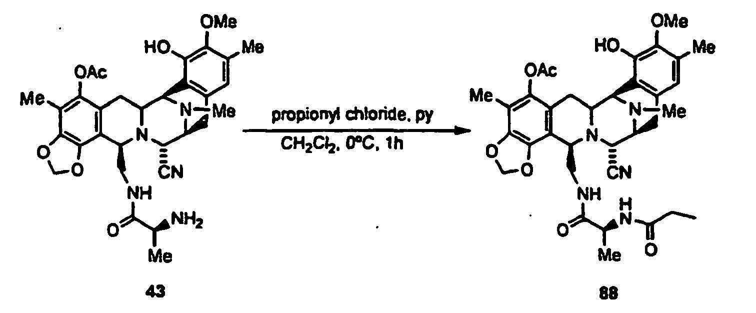

To a solution of 43 (30 mg, 0.05 mmol) in CH₂Cl₂ (0.3 mL), propionyl chloride (4.40 µL, 0.05 mmol) and pyridine (4.04 µL, 0.05 mmol) were added at 0 °C. The reaction mixture was stirred for 1h and then, the solution was diluted with CH₂Cl₂ (15 mL) and washed with 0.1 N HCl (10 mL). The organic layer was dried over Na₂SO₄. filtered, and the solvent was eliminated under reduced pressure. The residue was purified by flash column chromatography (SiO₂, gradient Hex:EtOAc 1:1 to EtOAc) to afford **88** (18 mg, 56 %) as a white solid.
Rf: 0.49 (Hex:EtOAc:MeOH 1:10:2).
¹H NMR (300 MHz, CDCl₃) δ 6.46 (s, 1H), 6.16 (brd, 1H), 5.99 (d, *J=* 1.2 Hz, 1H), 5.95 (s, 1H), 5.90 (d, *J*= 12 Hz, 1H), 5.34 brt, 1H), 4.12-4.06 (m, 3H), 3.84 (bs, 1H), 3.74 (s, 3H), 3.63 (dd, *J₁=* 6.3 Hz, *J₂=*12.9 Hz, 1H), 3.50-3.48 (m,1 H), 339 (d, *J*= 8.1 Hz, 1H), 3.23 (d, *J*= 11.7 Hz, 1H), 3.00 (dd, *J₁*=8.4 Hz, *J₂=*18.3 Hz, 1H), 2.78 (d, *J=* 15.6 Hz, 1H), 2.63 (d, *J*= 18.3 Hz, 1H), 2.31 (s, 3H), 2.27 (s, 3H), 1.87-1.80 (m, 1H), 1.06 (t, *J*= 7.5Hz, 3H), 0.74 (d, *J*= 6.9 Hz, 3H).
ESI-MS m/z: Calcd. for C₃₄H₄₁N₅O₈: 647.7. Found (M+1)⁺: 648.2.

### Example 83

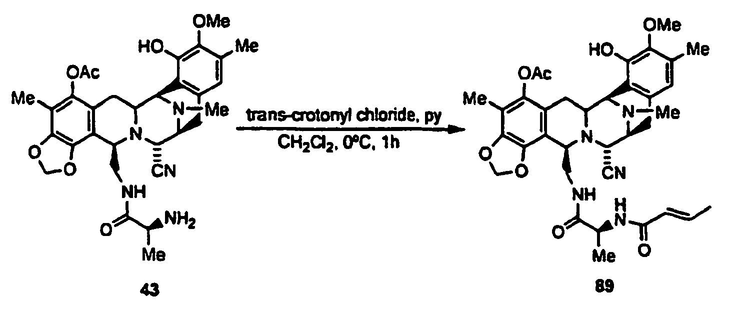

To a solution of 43 (20 mg, 0.0338 mmol) in CH₂Cl₂ (0.3 mL), propionyl chloride (3.238 µL, 0.0338 mmol) and pyridine (2.73 µL, 0.0338 mmol) were added at 0 °C. The reaction mixture was stirred for 1h and then, the solution was diluted with CH₂Cₗ₂ (10 mL) and washed with 0.1 N HCl (5 mL). The organic layer was dried over Na₂SO₄, filtered, and the solvent was eliminated under reduced pressure. The residue was purified by flash column chromatography (SiO₂ gradient Hex:EtOAc 3:1 to AcOEt) to afford **89** (11.5 mg, 52 %) as a white solid.
Rf: 0.57 (EtOAc:MeOH 10:1).
¹H NMR (300 MHz, CDCl₃) δ 6.82-6.70 (m, 1H), 6.46 (s, 1H), 6.11 (d, 1H), 6.00 (d, *J=* 1.5 Hz,1H), 5.89 (d, *J=* 1.5 Hz, 1H), 5.85 (s, 1H), 5.77 (dd, *J*₁= 1.5 Hz, *J₂=* 15.3 Hz, 1H), 5.37 (bst, 1H), 4.13-4.06 (m, 3H), 3.19 (m, 1H), 3.73 (s, 3H), 3.55 (m, 2H), 3.38 (d, *J=* 1.5 Hz, 1H), 3.23 (d, *J=* 11.4 Hz, 1H), 3.00 (dd, *J₁=* 8.4 Hz, *J₂=* 18.3 Hz, 1H), 2.78 (d, *J=* 15.0 Hz, 1H), 2.65 (d, *J=* 18.0 Hz. 1H), 2.31 (s, 3H), 2.28 (s, 3H), 2.22 (s, 3H), 2.00 (s, 3H), 1.85-1.82 (m, 4H), 0.77 (d, *J=* 6.3 Hz, 3H).
ESI-MS m/z: Calcd. for C₃₅H₄₁N₅O₈: 659.7. Found (M+1)⁺: 660.3.

### Example 84

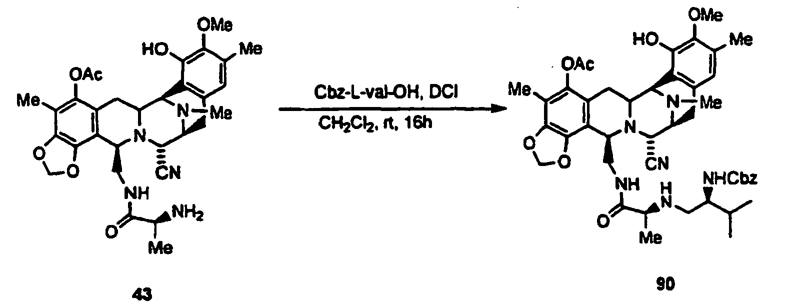

To a solution of **43** (15 mg, 0.0253 mmol) in CH₂Cl₂ (0.3 mL), Cbz-L-Val-OH (6.39 mg, 0.0253 mmol) and carbonyl diimidazole (4.86 mg, 0.03 mmol) were added at 0 °C. The reaction mixture was stirred for 16 h at room temperature and then, the solution was diluted with CH₂Cl₂ (15 mL) and washed with 0.1 N HCl (10 mL). The organic layer was dried over Na₂SO₄, filtered, and the solvent was eliminated under reduced pressure. The residue was purified by flash column chromatography (SiO₂, gradient Hex:EtOAc 1:1 to EtOAc) to afford **90** (6.7 mg, 32 %) as a white solid.
Rf: 0.79 (EtOAc:MeOH 5:1).
¹H NMR (300 MHz, CDCl₃) δ 7.35 (bs, 5H), 6.46 (s, 1H), 6.28 (d, *J*= 6.0 Hz, 1H), 5.98 (d, *J=* 1.2 Hz,1H), 5.89 (d, *J*= 1.2 Hz, 1H), 5.77 (s, 1H), 5.44 (bs,1H), 5.30 (bs,1H), 5.08 (s, 2H), 4.09-4.06 (m, 3H), 3.94-3.89 (m, 1H), 3.70-3.66 (m, 5H), 3.38 (d, *J=* 11.7 Hz, 1H), 3.01 96 (dd, *J₁=* 7.8 Hz, *J₂*= 18.3 Hz, 1H), 2.79 (d, *J*= 14.1 Hz, 1H), 2.63 (d, *J*= 18.0 Hz, 1H), 2.30 (s, 3H), 2.28 (s, 3H), 2.20 (s, 3H), 1.99 (s, 3H9,1.97-1.81 (m, 2H), 0.83 (d, *J*= 6.6 Hz, 3H), 0.80 (d, *J*= 6.6 Hz, 3H), 0.75 (d, *J*= 6.9 Hz, 3H).
ESI-MS m/z: Calcd. for C₄₄H₅₂N₆O₁₀: 824.9. Found (M +1)⁺: 825.4.

### Example 85

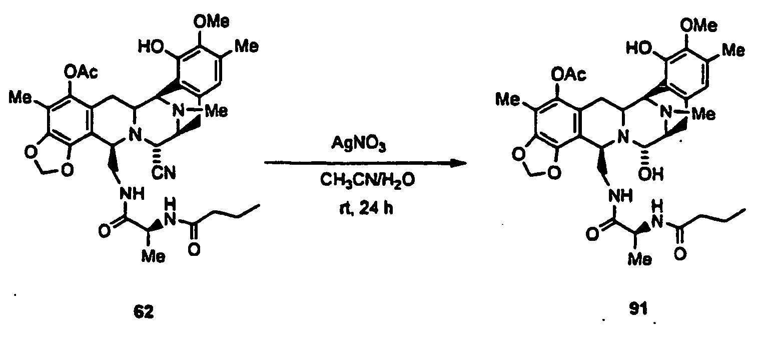

To a solution of 62 (20 mg, 0.030 mmol) in CH₃CN/H₂O (1.5 mL/1 mL), AgNO₃ (154 mg, 0.90 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂, gradient EtOAc to EtOAc:MeOH 3:1) to afford **91** (13 mg, 66 %) as a white solid.
Rf: 0.18 (EtOAc:MeOH 10:1).
¹H NMR (300 MHz, CDCl₃) δ 6.49 (s, 1H), 6.16 (d, 1H), 5.98 (d, *J =* 1.5 Hz, 1H), 5.89 (d*, J* = 1.5 Hz, 1H), 5.32 (bs, 1H), 4.41 (bs, 1H), 4.00 (bs, 1H), 3.79 (s, 3H), 3.70-3.65 (m, 2H), 3.37-3.32 (m,2H), 3.19-3.17 (m, 1H), 2.94 (dd, *J₁=* 9.0 Hz, *J₂=* 15.0 Hz, 1H), 2.74 (d, *J*= 15.9 Hz, 1H), 2.46 (d, *J=* 17.1 Hz, 1H), 2.31 (s, 3H), 2.28 (s, 3H), 2.27 (s, 3H), 2.04-2.01 (m, 2H), 1.98 (s, 3H), 1.64-1.62 (m, 1H), 1.54-1.52 (m, 2H), 0.89-0.84 (m, 6H).
ESI-MS m/z: Calcd. for C₃₄H₄₄N₄O₉: 652.7. Found (M-17)⁺: 635.3.

### Example 86

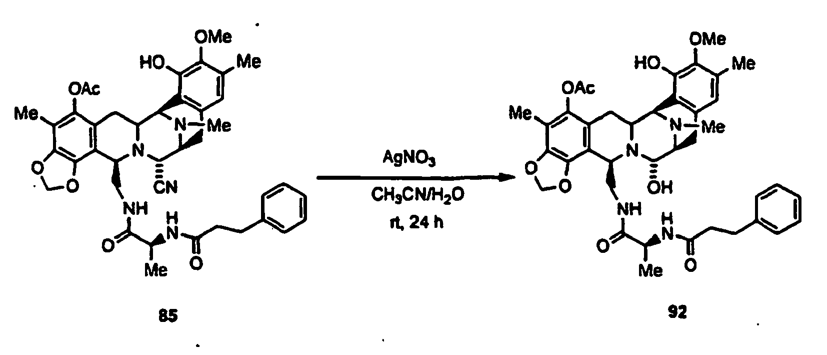

To a solution of **85** (10 mg, 0.0138 mmol) in CH₃CN/H₂O (1.5 mL/1 mL), AgNO₃ (70.4 mg, 0.414 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂, gradient EtOAc to EtOAc:MeOH 4:1) to afford **92** (7 mg, 71 %) as a white solid.
Rf: 0.20 (EtOAc:MeOH 5:1).
¹H NMR (300 MHz, CDCl₃) δ 7.25-7.13 (m, 5H), 6.47 (s, 1H), 6.13 (brd, 1H), 5.97 (d, *J*= 1.2 Hz, 1H), 5.88 (d, *J=* 1.2 Hz, 1H), 5.34 (brt,1H), 4.50 (bs, 1H), 4.40 (bs, 1H), 4.00 (bs, 1H), 3.76 (s, 3H), 3.70-3.65 (m, 3H), 3.34 (d, *J=* 11.7 Hz, 1H), 3.17 (d, *J=* 5.1 Hz, 1H), 2.98-2.83 (m, 3H), 2.72 (d, *J=* 14.4 Hz, 1H), 2.44 (d, *J=* 19.2 Hz, 1H), 2.30 (s, 3H), 2.27 (s, 6H), 1.97 (s, 3H), 1.72 (m, 1H), 0.82 (d, *J=* 6.6 Hz, 3H).
ESI-MS m/z: Calcd. for C₃₉H₄₆N₄O₉: 714.8. Found (M-17)⁺: 697.3.

### Example 87

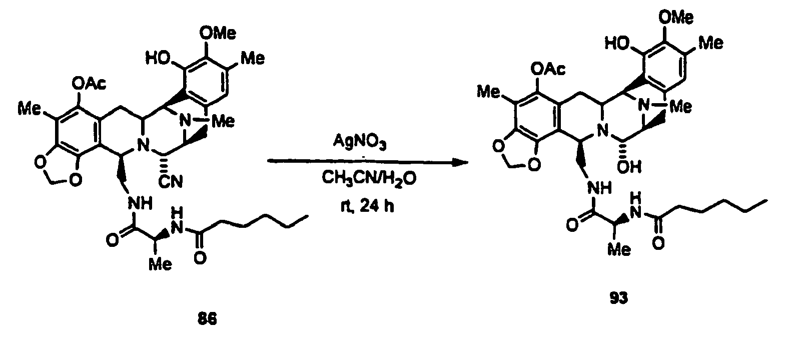

To a solution of 86 (6 mg, 0.0087 mmol) in CH₃CN/H₂O (1.5 mL/1 mL). AgNO₃ (44 mg, 0.26 mmol) was added and the reaction was stirred at 23 °C for 24 h. Then, brine (10 mL) and Aq sat NaHCO₃ (10 mL) were added at 0 °C and the mixture was stirred for 15 min. filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂ gradient EtOAc to EtOAc:MeOH 5:1) to afford **93** (5 mg, 85 %) as a white solid.
Rf: 0.018 (EtOAc:MeOH 5:1).
¹H NMR (300 MHz, CDCl₃) δ 6.48 (s, 1H), 6.17 (d, 1H), 5.98 (d, *J=* 1.5 Hz, 1H), 5.89 (d, *J*= 1.5 Hz, 1H), 5.33 (bs, 1H), 4.51 (d, 1H), 4.40 (d, 1H), 4.00 (d, 1H), 3.78 (s, 3H), 3.76-3.65 (m, 2H), 3.36-3.32 (m, 2H), 3.18 (d, *J*= 6.9 Hz, 1H), 2.98-2.89 (m, 1H), 2.71 (d, *J*= 15.0 Hz, 1H), 2.45 (d,*J*= 17.7 Hz, 1H), 2.31 (s, 3H), 2.27 (s, 3H), 2.26 (s, 3H), 1.98 (s, 3H), 1.68-1.50 (m, 3H), 1.29-1.19 (m, 6H), 0.88-0.84 (m, 6H).
ESI-MS m/z: Calcd. for C₃₆H₄₈N₄O₉: 680.7. Found (M-17)⁺: 663.3.

### Example 88

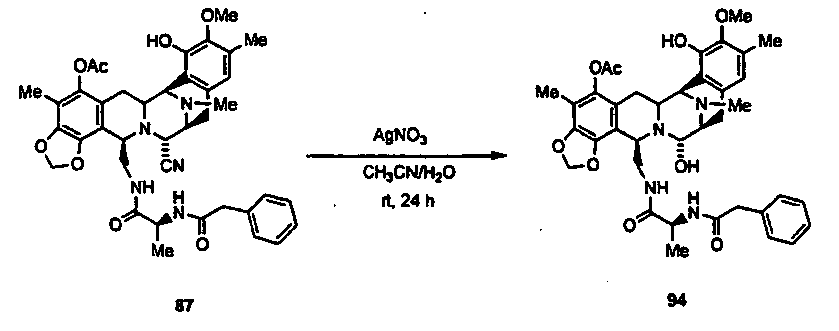

To a solution of **87** (12 mg, 0.0169 mmol) in CH₃CN/H₂O (1.5 mL/1 mL), AgNO₃ (86 mg, 0.507 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂ gradient EtOAc to EtOAc:MeOH 5:1) to afford **94** (8.8 mg, 74 %) as a white solid.
Rf: 0.28 (EtOAc:MeOH 5:1).
H NMR (300 MHz, CDCl₃) δ 7.34-7.18 (m, 5H), 6.37 (s, 1H), 6.20 (d, 1H), 5.96 (d, *J*= 1.5 Hz, 1H), 5.88 (d, *J*= 1.5 Hz, 1H), 5.30 (t, 1H), 4.50 (bs, 1H), 4.39 (d, *J*= 1.8 Hz, 1H), 3.99 (d, *J*= 2.1 Hz, 1H), 3.73 (s, 3H), 3.69-3.60 (m, 3H), 3.37-3.30 (m, 3H), 3.17 (d, *J*= 18.1 Hz, 1H), 2.89 (dd, *J₁=* 7.5 Hz, *J₂* 18.3 Hz, 1H), 2.31 (s, 3H), 2.25 (s, 3H), 2.21 (s, 3H), 1.99 (s, 3H), 1.71 (dd*, J₁=* 11.7 Hz, *J₂*=15.0 Hz, 1H), 0.77 (d,*J=* 6.6 Hz, 1H).
ESI-MS m/z: Calcd. for C₃₈H₄₄N₄O₉: 700.7. Found (M-17)⁺: 683.2.

### Example 89

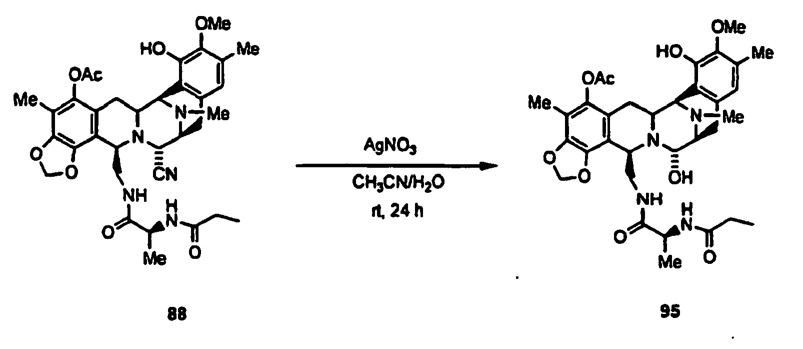

To a solution of 88 (14 mg, 0.0216 mmol) in CH₃CN/H₂O (1.5 mL/1 mL), AgNO₃ (110 mg, 0.648 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂, gradient EtOAc to EtOAc:MeOH 5:1) to afford **95** (9.7 mg, 70 %) as a white solid.
Rf: 0.16 (EtOAc:MeOH 5:1).
¹H NMR (300 MHz, CDCl₃) δ 6.48 (s, 1H), 6.10 (d, 1H), 5.97 (d, *J=* 1.2 Hz. 1H), 5.89 (d, *J*= 1.2 Hz, 1H), 5.36 (bs, 1H), 4.51 (bs, 1H), 4.40 (d, *J*= 2.1 Hz, 1H), 4.00 (d, *J*= 2.1 Hz, 1H), 3.78 (s, 3H), 3.76-3.62 (m, 3H), 3.33 (d, *J*= 11.7Hz, 1H), 3.18 (d, *J=* 8.4 Hz,1H), 2.94 (dd, *J₁*= 8.4 Hz, *J₂*= 16.5 Hz, 1H), 2.72 (d, *J=* 15.0 Hz, 1H), 2.45 (d, *J=* 18.3 Hz, 1H), 2.31 (s, 3H), 2.27 (s, 3H), 2.22 (s, 3H), 1.97 (s, 3H), 1.86 (m, 2H), 1.73 (dd, *J₁=* 12.0 Hz, *J₂=* 15.0 Hz, 1H), 1.05 (t, *J=* 7.8 Hz, 3H), 0.83 (d, *J=* 6.9 Hz, 3H).
ESI-MS m/z: Calcd. for C₃₃H₄₂N₄O₉: 638.7. Found (M-17)⁺: 6212.

### Example 90

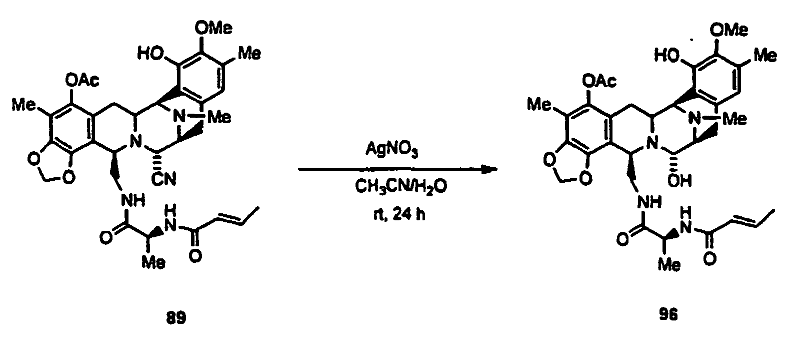

To a solution of **89** (10mg, 0.015 mmol) in CH₃CN/H₂O (1.5 mL/1 mL). AgNO₃ (77.2 mg, 0.454 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂ gradient EtOAc to EtOAc:MeOH 1:1) to afford **96** (9 mg, 92 %) as a white solid.
Rf: 0.016 (EtOAc:MeOH 5:1).
¹H NMR (300 MHz, CDCl₃) δ 6.76-6.69 (m, 1H), 6.47 (s, 1H), 6.18 (brd, 1H), 5.97 (d, *J=* 1.5 Hz, 1H), 5.88 (d,*J*= 1.5 Hz, 1H), 5.71 (dd, *J₁=* 1.5 Hz, *J₂=* 16.2 Hz,3 H), 5.32 (bs, 1H), 4.50 (m, 1H), 4.41 (m, 1H), 3.99 (m, 1H), 3.78 (m, 4H), 3.64-3.58 (m, 2H), 3.34 (d, *J*= 11.1 Hz, 1H), 3.17 (d, *J=* 8.6 Hz, 1H), 2.95 (dd, J₁= 7.5 Hz, *J₂=* 17.4 Hz, 1H), 2.70 (d, *J=* 16.2 Hz, 1H), 2.48 (d, *J=* 17.7 Hz, 1H), 2.31 (s, 3H), 2.27 (s, 3H), 2.17 (s, 6H), 1.97 (s, 3H), 1.82-1.74 (m, 4H), 0.88 (t, *J=* 5.2 Hz, 3H).
ESI-MS m/z: Calcd. for C₃₄H₄₂N₄O₉: 650.7. Found (M-17)⁺: 633.3.

### Example 91

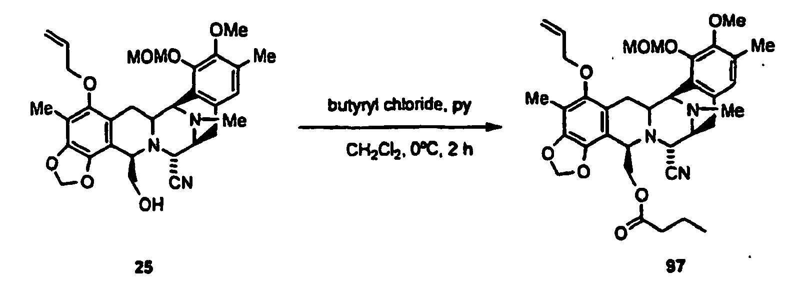

To a solution of 25 (100 mg, 0.177 mmol) in CH₂Cl₂ (0.5 mL), butyryl chloride (24 µL, 0.23 mmol) and pyridine (17 µL, 0.212 mmol) were added at 0 °C. The reaction mixture was stirred for 2h at room temperature and then, the solution was diluted with CH₂Cl₂ (30 mL) and washed with 0.1 N HCl (20 mL). The organic layer was dried over Na₂SO₄, filtered, and the solvent was eliminated under reduced pressure. The residue was purified by flash column chromatography (SiO₂, Hex:EtOAc 3:1) to afford **97** (99 mg, 88 %) as a colorless oil.
Rf: 0.64 (Hex:EtOAc 1:1).
¹H NMR (300 MHz, CDCl₃) δ 6.66 (s, 1H), 6.16-6.05 (m,1H), 5.93 (d, *J*= 1.2 Hz, 1H), 5.87 (d, *J*= 1.2 Hz, 1H), 5.40 (dd, *J₁=* 1.2 Hz, *J₂=* 17.1 Hz, 1H), 5.26 (dd, *J₁*= 1.2 Hz, *J₂=* 10.2 Hz, 1H), 5.13-5.08 (m, 2H), 4.44 (dd, *J₁=* 3.6 Hz, *J₂*= 11.1 Hz, 1H), 4.21-4.07 (m, 5H), 3.74 (m, 1H), 3.72 (s, 1H), 3.57 (s, 3H), 3.35 (d, *J*= 10.5 Hz, 1H), 3.26-3.21 (m, 2H), 3.98 (dd, *J₁=* 8.7 Hz, *J₂=* 18.0 Hz, 1H), 2.54 (d, *J*= 18.0 Hz), 2.30 (s, 3H), 2.21 (s, 3H), 2.13 (s, 3H), 1.92-1.65 (m, 3H), 1.42-1.34 (m, 2H), 0.80 (t, *J* = 7.5 Hz, 3H).
ESI-MS m/z: Calcd. for C₃₅H₄₃N₃O₉ : 633.7. Found (M+1)⁺: 634.3.

### Example 92

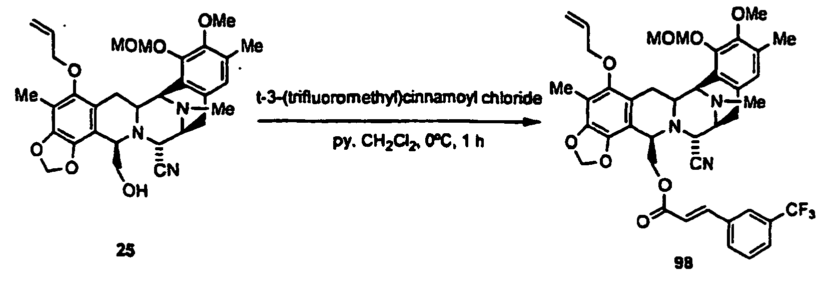

To a solution of **25** (100 mg, 0.177 mmol) in CH₂Cl₂ (0.4 mL), trans-3-(trifluoromethyl)cinnamoyl chloride (35 µL, 0.23 mmol) and pyridine (17 µL, 0.212 mmol) were added at 0 °C. The reaction mixture was stirred for 1 h at room temperature and then, the solution was diluted with CH₂Cl₂ (30 mL) and washed with 0.1 N HCl (20 mL). The organic layer was dried over Na₂SO₄, filtered, and the solvent was eliminated under reduced pressure. The residue was purified by flash column chromatography (SiO₂, gradient Hex:EtOAc 6:1 to Hex:EtOAc 1:1) to afford 98 (122 mg, 90 %) as a white solid. Rf: 0.478 (Hex:EtOAc 1:1).
¹H NMR (300 MHz, CDCl₃) δ 7.64-7.48 (m, 4H), 7.37 (d, *J*= 15.6 Hz, 1H), 6.62 (s, 1H), 6.16-6.07 (m, 1H), 6.12 (d, *J=* 15.6 Hz, 1H), 5.94 (d, *J=* 1.2 Hz, 1H), 5.89 (d, *J*= 1.2 Hz, 1H), 5.41 (dd, *J₁=* 1.8 Hz, *J₂=* 17.1 Hz, 1H), 5.28 (dd, *J₁*= 1.8 Hz, *J₂* =12.0 Hz, 1H), 5.04 (q, *J* = 6.0 Hz, 1H), 4.60 (dd, *J₁ =* 3.3 Hz, *J₂=* 11.1 Hz, 1H), 4.22-4.15 (m, 5H), 3.90 (dd, *J₁*= 4.2 Hz, *J₂=* 11.1 Hz, 1H), 3.55 (s, 3H), 3.38 (s, 3H), 3.35-3.34 (m, 1H), 3.27-3.25 (m, 1H), 3.22 (bs, 1H), 2.98 (dd, *J₁*= 7.8 Hz, *J₂=* 18.0 Hz, 1H), 2.61 (d, *J*= 17.7 Hz, 1H), 2.29 (s, 3H), 2.16 (s, 3H), 2.00 (s, 3H), 1.80 (dd, *J₁* =11.7 Hz, *J₂=* 15.6 Hz, 1H).
ESI-MS m/z: Calcd. for C₄₁H₄₂F₃N₃O₈: 761.7. Found (M+1)⁺: 762.3.

### Example 93

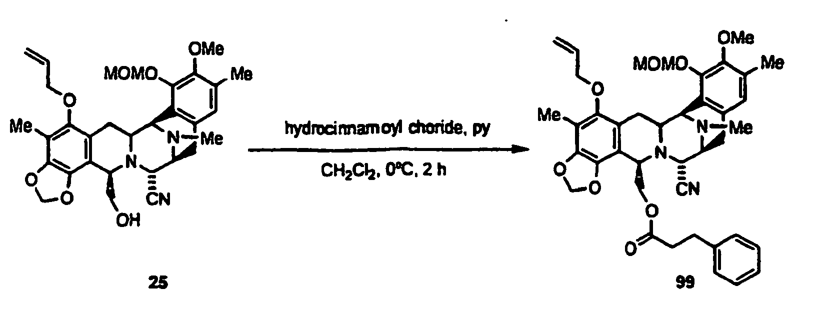

To a solution of 25 (68 mg, 0.12 mmol) in CH₂Cl₂ (0.4 mL), hydrocynnamoyl chloride (20 µL, 1.12 mmol) and pyridine (10 µL, 1.01 mmol) were added at 0 °C. The reaction mixture was stirred for 2h at room temperature and then, the solution was diluted with CH₂Cl₂ (30 mL) and washed with 0.1 N HCl (20 mL). The organic layer was dried over Na₂SO₄, filtered, and the solvent was eliminated under reduced pressure. The residue was purified by flash column chromatography (SiO₂ gradient Hex:EtOAc 5:1 to Hex:EtOAc 2:1) to afford **99** (41 mg, 49 %) as a white solid. Rf: 0.47 (Hex:EtOAc 1:1).
¹H NMR (300 MHz, CDCl₃) δ 7.29-7.18 (m, 3H), 7.04-7.02 (m, 2H), 6.66 (s, 1H), 6.16-6.07 (m, 1H), 5.93 (d,*J=* 1.2 Hz, 1H), 5.87 (d, *J*= 1.2 Hz, 1H), 5.40 (dd, *J₁=* 1.7 Hz, *J₂=* 17.4 Hz, 1H), 5.26 (dd, *J₁=* 1.7 Hz, *J₂=* 10.2 Hz, 1H), 5.09 (dd, *J₁=* 6.0 Hz, *J₂=* 8.7 Hz, 2H), 4.43 (dd, *J₁=* 3.3 Hz, *J₂=* 11.1 Hz, 1H), 4.20-4.14 (m, 3H), 4.06 (t, *J=* 3.7 Hz, 1 H), 4.02 (d, *J=* 2.4 Hz, 1H), 3.72 (dd, *J₁=* 4.5 Hz, *J₂*=11.1 Hz, 1H), 3.56 (s, 3H), 3.55 (s, 3H), 3.32 (brd, *J*= 8.7 Hz, 1H), 3.26 (dd, *J₁*= 1.9 Hz, *J₂=* 8.1 Hz, 1H), 3.23-3.20 (m, 1H), 3.01 (brd, *J=* 8.1 Hz, 1H), 3.23-3.20 (m, 1H), 3.26 (dd, *J₁=* 1.9 Hz, *J₂=* 8.1 Hz, 1H), 2.95 (d, *J=* 1.8 Hz, 1H), 2.71-2.64 (m, 3H), 2.53 (d, *J*= 17.7 Hz, 1H), 2.26 (s, 3H), 2.14 (s, 6H), 1.83 (dd, *J₁*= 12.3 Hz, *J₂=* 15.9 Hz, 1H).
ESI-MS m/z: Calcd. for C₄₀H₄₅F₃N₃O₈: 695.3. Found (M+1)⁺: 696.3.

### Example 94

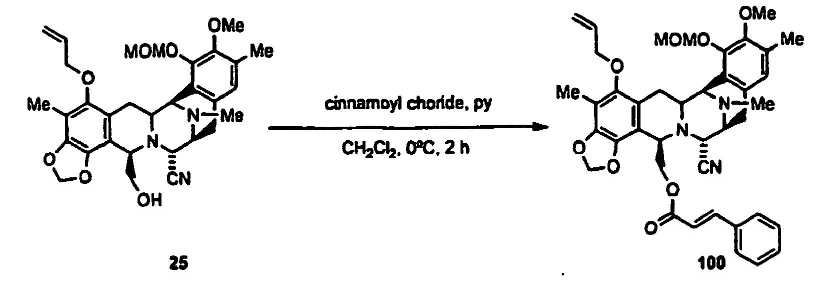

To a solution of 25 (100 mg, 0.177 mmol) in CH₂Cl₂ (0.4 mL), cynnamoyl chloride (35 mg, 0.21 mmol) and pyridine (17 µL, 0.21 mmol) were added at 0 °C. The reaction mixture was stirred for 2h at room temperature and then, the solution was diluted with CH₂Cl₂ (30 mL) and washed with 0.1 N HCl (20 mL). The organic layer was dried over Na₂SO₄, filtered, and the solvent was eliminated under reduced pressure. The residue was purified by flash column chromatography (SiO₂, Hex:EtOAc 6:1) to afford **100** (94 mg, 76 %) as a white solid. Rf: 0.49 (Hex:EtOAc 1:1).
¹H NMR (300 MHz, CDCl₃) δ 7.42-7.33 (m, 6H), 6.62 (s, 1H), 6.16-6.05 (m, 1H), 6.10 (d, *J*= 15.9Hz, 1H), 5.94 (d, *J*= 1.2 Hz, 1H), 5.88 (d, *J*= 1.2 Hz,1H), 5.43 (dd, *J₁=* 3.0 Hz, *J₂*= 17.1 Hz, 1H), 5.27 (dd, *J₁=* 3.0 Hz, *J₂*=12.0 Hz, 1H), 5.04 (q, *J*= 6.0 Hz, 1H). 4.55 (dd, *J₁*= 3.9 Hz, *J₂*=11.1 Hz,1H), 4.22-4.15 (m, 5H), 3.87 (dd, *J₁=* 4.5 Hz, *J₂=* 11.1 Hz,1H), 3.55 (s, 3H), 3.39 (s, 3H), 3.36-3.33 (m, 1H), 3.26-3.22 (m, 2H), 2.98 (dd, *J₁=* 8.1 Hz, *J₂*= 17.7 Hz, 1H), 2.63 (d, *J*=17.7 Hz, 1H), 2.29 (s, 3H), 2.03 (s, 3H), 1.82 (dd, *J₁*= 11.7 Hz, *J₂*=15.3Hz, 1H).
ESI-MS m/z: Calcd. for C₄₀H₄₃N₃O₈: 693.3. Found (M+1)⁺: 694.3.

### Example 95

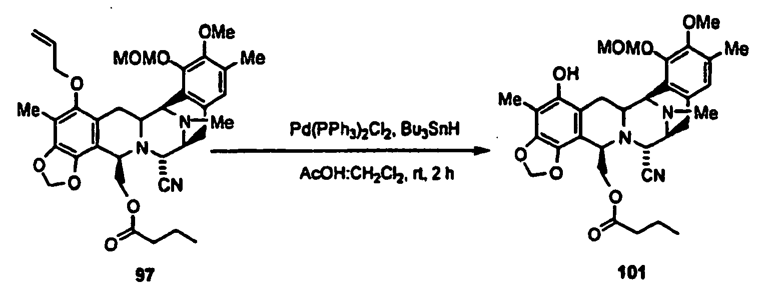

To a solution of 97 (40 mg, 0.063 mmol) in CH₂Cl₂ (0.7 mL), acetic acid (17.8 µL), Pd(PPh₃)₂Cl₂ (3.64 mg, 0.0052 mmol) and Bu₃SnH (67.9 µL, 0.252 mmol ) were added at 23 °C. The reaction mixture was stirred for 2h at that temperature and then, the solution was poured into a pad of flash column (SiO₂, gradient Hex:EtOAc 5:1 to Hex:EtOAc 3:1) to afford **101** (30 mg, 80 %) as a white solid. Rf: 0.4 (Hex:EtOAc 1:1).
¹H NMR (300 MHz, CDCl₃) δ 6.65 (s, 1H), 5.90 (d, *J*=1.5 Hz,1H), 5.82 (d, *J*=1.5 Hz, 1H), 5.54 (s, 1H), 5.33 (d, *J=* 6.0 Hz, 1H), 5.13 (d, *J=* 6.0 Hz, 1H), 4.54 (dd, *J₁=* 3.6 Hz, *J₂=* 11.4 Hz, 1H), 4.18 (d, *J*= 2.1 Hz, 1H), 4.13 (d, *J*= 2.4 Hz, 1H), 4.07 (t, *J*=3.3 Hz, 1H), 3.75 (dd, *J₁=* 3.9 Hz, *J₂=* 11.1 Hz, 1H), 3.70 (s, 3H), 3.35 (d, *J*= 8.4 Hz,1 H), 3.24 (dd, *J₁=* 2.7 Hz, *J₂=*8.7 Hz,1H), 3.10 (dd, *J₁=* 2.4 Hz, *J₂=* 15.0 Hz, 1H), 3.01 (d, *J=* 8.1 Hz,1H), 2.95 (d, *J=* 7.8 Hz, 1H), 2.58 (d, *J=* 18.3 Hz, 1H), 2.29 (s, 3H), 2.21 (s, 3H), 2.10 (s, 3H), 1.89-1.66 (m, 3H), 1.36-1.25 (m, 2H), 0.77 (t, *J*= 7.5 Hz, 3H).
ESI-MS m/z: Calcd. for C₃₂H₃₉N₃O₈: 593.6. Found (M+1)⁺: 594.8

### Example 96

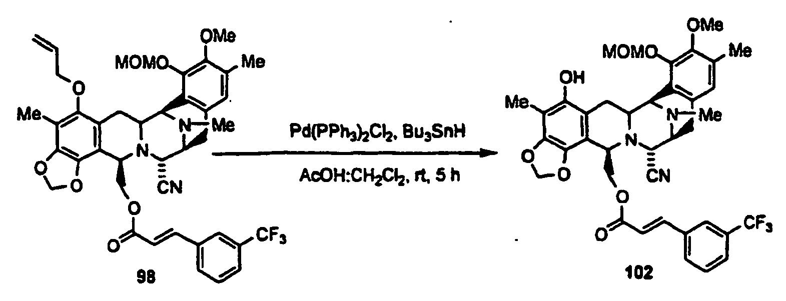

To a solution of **98** (37 mg, 0.0485 mmol) in CH₂Cl₂ (0.7 mL), acetic acid (20 µL). Pd(PPh₃)₂Cl₂ (4 mg, 0.0057 mmol) and Bu₃SnH (53 µL, 0.194 mmol) were added at 23 °C. The reaction mixture was stirred for 5h at that temperature and then, the solution was poured into a pad of flash column (SiO₂, gradient Hex:EtOAc 6:1 to Hex:EtOAc 2:1) to afford **102** (25 mg, 71 %) as a white solid. Rf: 0.38 (Hex:EtOAc 1:1).
¹H NMR (300 MHz, CDCl₃) δ 7.63-7.60 (M, 2H), 7.50-7.49 (M, 2H), 7.24 (d, *J*= 15.9 Hz, 1H), 6.59 (s, 1H), 5.98 (d, *J*= 15.9 Hz, 1H), 5.92 (d, *J*= 1.5 Hz, 1H), 5.84 (d, *J*= 1.5 Hz, 1H), 5.66 (s, 1H), 5.20 (d, *J*= 6.0 Hz, 1H), 4.87 (d, *J*= 6.0 Hz,1H), 4.71 (dd, *J₁=* 2.7 Hz, *J₂=* 10.8 Hz, 1H), 4.16-4.15 (m, 3H), 3.93 (dd, *J₁=* 3.3 Hz, *J₂=* 11.1 Hz, 1H), 3.66 (s, 3H), 3.36 (brd, *J*= 10.2 Hz, 1H), 3.26 (brd, *J*= 11.7 Hz, 1H), 3.10 (brd, *J=* 15.0 Hz, 1H), 2.96 (dd, *J₁=* 7.8 Hz, *J*₂= 17.7 Hz, 1H), 2.62 (d, *J*= 17.7 Hz, 1H), 2.27 (s, 3H), 2.14 (s, 3H), 1.97 (s, 3H), 1.79 (dd, *J₁=* 12.0 Hz, *J₂=* 15.8 Hz, 1H).
ESI-MS m/z: Calcd. for C₃₈H₃₈F₃NO₈: 721.7. Found (M+1)⁺: 722.2.

### Example 97

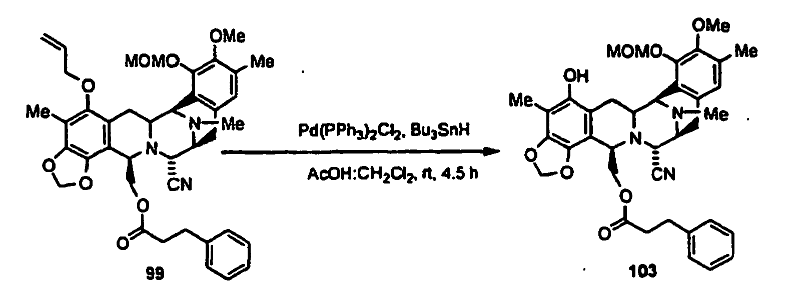

To a solution of 99 (41 mg, 0.059 mmol) in CH₂Cl₂ (1 mL), acetic acid (25 µL), Pd(PPh₃)₂Cl₂ (5 mg, 0.0071 mmol) and Bu₃SnH (63 µL, 0.235 mmol ) were added at 23 °C. The reaction mixture was stirred for 4.5 h at that temperature and then, the solution was poured into a pad of flash column (SiO₂, gradient Hex:EtOAc 6:1 to Hex:EtOAc 1:1) to afford **103** (34.2 mg, 89 %) as a white solid. Rf: 0.49 (Hex:EtOAc 1:1).
¹H NMR (300 MHz, CDCl₃) δ 7.24-7.15 (m, 3H), 7.03-7.01 (m, 2H), 6.65 (s, 1H). 5.89 (bs, 1H), 5.82 (bs, 1H), 5.49 (s, 1H), 5.31 (d, *J =* 6.0 Hz, 1H), 5.12 (d, *J* = 6.0 Hz. 1H), 4.53 (dd, *J₁ =* 3.3 Hz, *J₂=* 11.1 Hz, 1H), 4.18 (d, *J=* 2.7 Hz, 1H), 4.07 (m, 2H), 3.75 (dd, *J₁=* 3.9 Hz, *J₂ =* 11.1 Hz, 1H), 3.69 (s, 3H), 3.62 (s, 3H), 3.32 (d,*J=* 7.8 Hz, 1H), 3.25 (d, *J =* 10.8 Hz, 1H), 3.12 (d, *J=* 14.7Hz, 1H), 3.00 (d,*J=* 7.8 Hz, 1H), 2.94 (d, *J=* 8.1 Hz, 1H), 2.66-2.60 (m, 3H), 2.57 (d, *J=* 18.0 Hz, 1H), 2.28 (s. 3H), 2.14 (s, 3H), 2.10 (bs, 3H), 1.83-1.74 (m, 1H).
ESI-MS m/z: Calcd. for C₃₇H₄₁N₃O₈: 655.7. Found (M+1)⁺: 656.3.

### Example 98

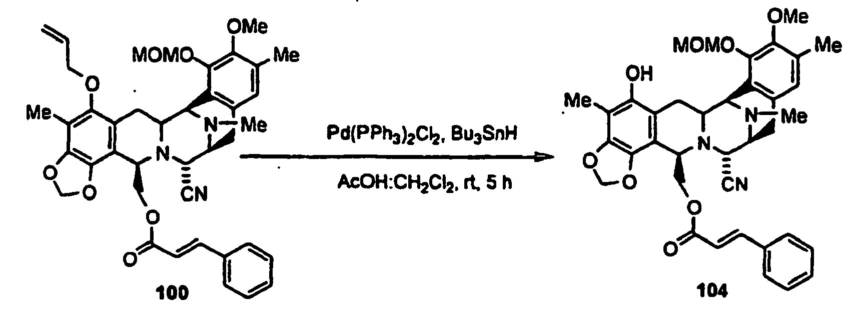

To a solution of 100 (40 mg, 0.0576 mmol) in CH₂Cl₂ (1 mL), acetic acid (25 µL). Pd(PPh₃)₂Cl₂ (4.8 mg, 0.007 mmol) and Bu₃SnH (62 µL, 0.23 mmol) were added at 23 °C. The reaction mixture was stirred for 5 h at that temperature and then, the solution was poured into a pad of flash column (SiO₂, gradient Hex:EtOAc 4:1 to Hex:EtOAc 1:1) to afford **104** (30 mg, 82 %) as a white solid. Rf: 0.41 (Hex:EtOAc 1:1).
¹H NMR (300 MHz, CDCl₃) δ 7.36 (s, 5H), 7.30 (d,*J*=16.2 Hz, 1H), 6.59 (s, 1H), 5.99 (d, *J*= 16.2 Hz, 1H), 5.91 (d, *J*= 1.2 Hz, 1H), 5.84 (d, *J*= 1.2 Hz, 1H), 5.60 (s. 1H). 5.20 (d,*J*= 5.6 Hz, 1H), 4.94 (d, *J=* 5.6 Hz, 1H), 4.63 (dd, *J₁=* 3.3 Hz,*J₂=* 11.4 Hz, 1H), 4.18-4.15 (m, 3H), 3.91 (dd, *J₁=* 3.9 Hz, *J₂=* 11.1 Hz, 1H), 3.66 (s, 3H), 3.49 (s, 3H), 3.35 (brd, *J=* 15.0 Hz 1H), 3.26 (brd, *J=* 11.4 Hz, 1H), 3.10 (brd, *J*= 15.0 Hz, 1H), 2.96 (dd, *J₁*= 8.4 Hz, *J₂=* 18.0 Hz*,* 1H), 2.63 (d, *J*= 18.0 Hz, 1H), 2.27 (s, 3H), 2.13 (s, 3H), 2.00 (s, 3H), 1.80 (dd,*J₁=* 12.0Hz, *J₂*= 14.4 Hz, 1H).
ESI-MS m/z: Calcd. for C₃₇H₃₉N₃O₈: 653.7. Found (M+ 23)⁺: 676.2.

### Example 99

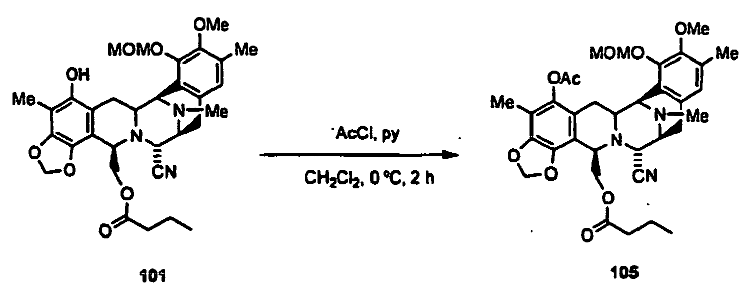

To a solution of **101** (24 mg, 0.041 mmol) in CH₂Cl₂ (0.4 mL), acetyl chloride (3 µL, 0.041 mmol), and pyridine (3.3 µL, 0.041 mmol ) were added at 0 °C. The reaction mixture was stirred for 2 h and then, the solution was diluted with CH₂Cl₂ (15 mL) and washed with 0.1 N HCl (5 mL). The organic layer was dried over sodium sulphate, filtered, and the solvent was eliminated under reduced pressure. The residue was purified by flash column chromatography (SiO₂, gradient Hex:EtOAc 5:1 to Hex:EtOAc 1:1) to afford **105** (23 mg. 88 %) as a white solid. Rf: 0.40 (Hex:EtOAc 1:1).
¹HNMR (300 MHz, CDCl₃) δ 6.66 (s, 1H), 5.97 (d, *J*=1.2 Hz, 1H), 5.91 (d *J*= 1.2 Hz, 1H), 4.58 (d, *J*= 3.0 Hz,1H), 4.54 (d, *J*= 3.0 Hz, 1H), 4.07 (t, *J*= 3.3 Hz, 1H), 3.77 (dd, J*₁*= 3.9 Hz, *J₂=* 11.4 Hz, 1H), 3.73 (s, 3H), 3.57 (s, 3H), 3.35 (d, *J*= 102 Hz, 1H), 3.22 (dt, *J₁=* 2.7 Hz, *J₂=* 11.7 Hz, 1H), 2.98 (dd, *J₁=* 8.1 Hz, *J₂=*18.0 Hz, 1H), 2.80 (d, *J*= 13.5 Hz, 1H), 2.58 (d,*J*= 18.0 Hz, 1H), 2.33 (s, 3H), 2.30 (s, 3H), 2.21 (s, 3H), 2.02 (s, 3H), 1.89-1.76 (m, 2H), 1.72-1.66 (m, 1H), 1.37-1.25 (m, 2H), 0.78 (t, *J*= 7.5 Hz, 3H).
ESI-MS m/z: Calcd. for C₃₄H₄₁N₃O₉: 635.7. Found (M+1)⁺: 636.8.

### Example 100

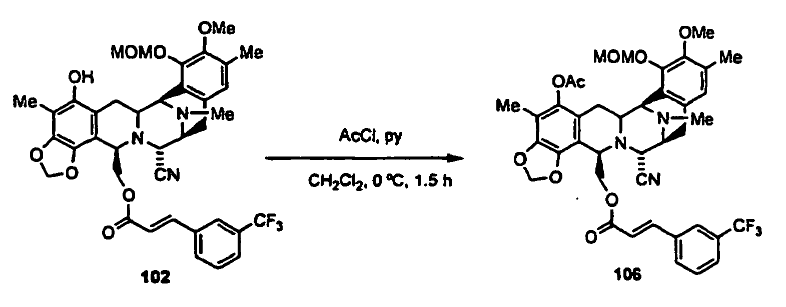

To a solution of **102** (16 mg, 0.022 mmol) in CH₂Cl₂ (0.2 mL), acetyl chloride (1.9 µL, 0.0266 mmol), and pyridine (2.15 µL, 0.0266 mmol ) were added at 0 °C. The reaction mixture was stirred for 1.5 h and then, the solution was diluted with CH₂Cl₂ (10 mL) and washed with 0.1 N HCl (7 mL). The organic layer was dried over sodium sulphate, filtered, and the solvent was eliminated under reduced pressure. The residue was purified by flash column chromatography (SiO₂, gradient Hex:EtOAc 4:1 toEtOAc) to afford 106 (12 mg, 71 %) as a white solid. Rf: 0.60 (Hex:EtOAc 1:1).
¹H NMR (300 MHz, CDCl₃) δ 7.83 (bs, 1H), 7.65-7.58 (m, 2H), 7.49-7.44 (m, 1H), 7.14 (d, *J=* 16.2 Hz, 1H), 6.62 (s, 1H), 6.06 (d, *J=* 16.2 Hz, 1H), 6.00 (d,*J=* 1.2 Hz, 1H), 5.95 (d, *J*= 1.2 Hz, 1H, 5.02 *(*d*,J=* 5.7 Hz, 1H), 4.96 (bs, 1H), 4.92 (d, *J*= 5.7 Hz. 1H). 4.15-4.11 (m, 3H), 3.88 (dd, *J₁=* 3.3 Hz, *J₂*= 11.1 Hz,1H), 3.08 (bs, 3H), 2.93 (dd, *J₁=* 8.1 Hz. *J₂*= 18.3 Hz, 1H), 2.80 (d,*J*= 13.2 Hz, 1H), 2.64 (d, *J=* 18.0 Hz, 1H), 2.31 (s, 3H), 2.27 (s. 3H). 2.08 (s, 3H), 1.91 (s, 3H), 1.69 (dd,*J₁=* 11.7 Hz, *J₂=* 15.9 Hz, 1H).).
ESI-MS m/z: Calcd. for C₄₀H₄₀F₃N₃O₉: 763.7. Found (M+1)⁺: 7642.

### Example 101

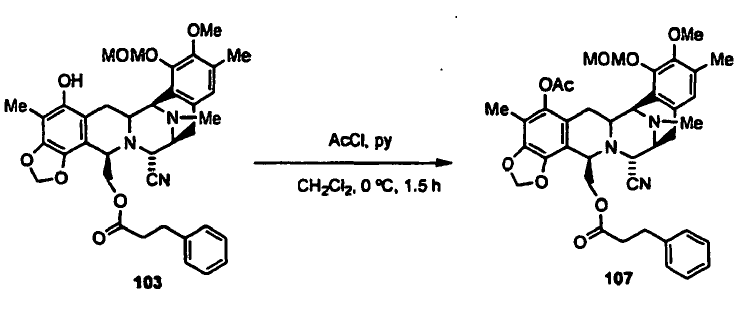

To a solution of **103** (34 mg, 0.052 mmol) in CH₂Cl₂ (0.2 mL), acetyl chloride (4.4 µL, 0.062 mmol), and pyridine (5 µL, 0.062 mmol) were added at 0 °C. The reaction mixture was stirred for 1.5 h and then, the solution was diluted with CH₂Cl₂ (10 mL) and washed with 0.1 N HCl (7 mL). The organic layer was dried over sodium sulphate, filtered, and the solvent was eliminated under reduced pressure. The residue was purified by flash column chromatography (SiO₂, gradient Hex:EtOAc 4:1 toEtOAc) to afford **107** (25.5 mg, 70 %) as a white solid. Rf: 0.48 (Hex:EtOAc 1:1).
¹H NMR (300 MHz, CDCl₃) δ 7.25-7.14 (m, 3H), 7.06-7.04 (m, 2H), 6.66 (s, 1H), 5.96 (d, *J=* 1.2 Hz, 1H), 5.91 (d,*J=* 1.2 Hz, 1H), 5.11 (d, *J*= 5.4 Hz, 1H), 4.14 (d, *J*=3.3 Hz, 1H), 4.07 (d,*J=* 3.6 Hz, 1H), 4.04 (d, *J=* 2.7Hz, 1H), 3.78 (dd, *J₁*= 3.3 Hz, *J₂*= 10.8 Hz, 1H), 3.55 (s, 3H), 3.51 (s, 3H), 3.33 (brd, *J=* 8.1 Hz, 1H), 3.23 (dt, *J₁*=2.7 Hz, *J₂=* 11.7 Hz,1H), 2.97 (dd, *J₁*= 8.1 Hz*, J₂=*18.0 Hz, 1H), 2.81 (d, *J*= 14.1 Hz, 1H), 2.63-2.52 (m, 3H), 2.33 (s, 3H), 2.29 (s, 3H), 2.26-202 (m, 2H), 2.09 (s, 3H), 2.04 (s, 3H), 1.74 (dd, *J₁=* 12.0 Hz, *J₂=* 15.6 Hz, 1H).
ESI-MS m/z: Calcd. for C₃₉H₄₃N₃O₉: 697.7. Found (M+1)⁺: 698.3.

### Example 102

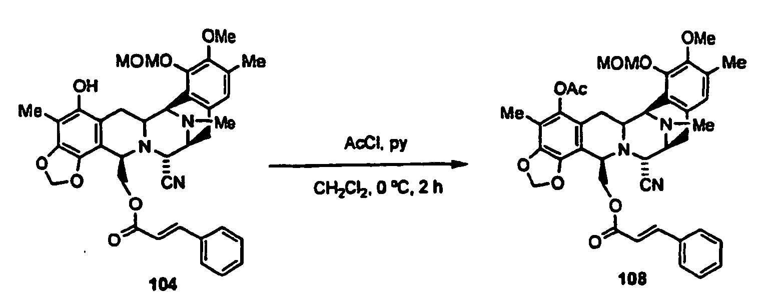

To a solution of 104 (29 mg, 0.0443 mmol) in CH₂Cl₂ (0.3 mL), acetyl chloride (3.77 µL, 0.053 mmol), and pyridine (4.3 µL, 0.053 mmol ) were added at 0 °C. The reaction mixture was stirred for 2 h and then, the solution was diluted with CH₂Cl₂ (15 mL) and washed with 0.1 N HCl (10 mL). The organic layer was dried over sodium sulphate, filtered, and the solvent was eliminated under reduced pressure. The residue was purified by flash column chromatography (SiO₂, gradient Hex:EtOAc 4:1 toEtOAc) to afford **108** (21.6 mg, 70 %) as a white solid. Rf: 0.58 (Hex:EtOAc 1:1).
¹H NMR (300 MHz, CDCl₃) δ 7.47-7.44 (m, 2H), 7.35-7.34 (m, 3H), 7.29 (d, *J*= 15.9 Hz, 1H), 6.62 (s, 1H), 5.99 (d, *J*= 1.2 Hz, 1H), 5.93 (d, *J*= 1.2 Hz, 1H), 5.05 (d, *J*= 5.7 Hz, 1H), 4.94 (d, *J*= 5.7Hz, 1H), 4.81 (d, *J=* 11.5 Hz, 1H), 4.16-4.11 (m, 3H), 3.34 (brd, *J=* 5.4 Hz, 1H), 3.24 (bs, 3H), 3.22-3.20 (m, 2H), 2.94 (dd, *J₁=* 8.1 Hz*, J₂=*18.0 Hz, 1H), 2.80 (d, *J*= 14.1 Hz, 1H), 2.64 (d, *J*= 18.0 Hz, 1H), 2.32 (s, 3H), 2.28 (s, 3H), 2.09 (s, 3H), 1.94 (s, 3H), 1.71 (dd, *J*₁= 11.7 Hz, *J*₂= 15.6 Hz, 1H).
ESI-MS m/z: Calcd. for C₃₉H₄₁N₃O₉: 695.7. Found (M+1)⁺: 696.2.

### Example 103

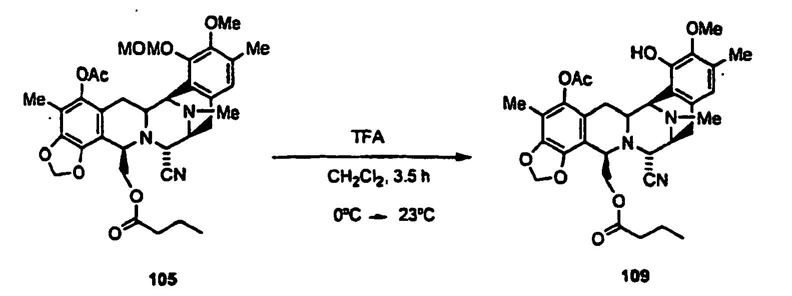

To a solution of 105 (16 mg, 0.025 mmol) in CH₂Cl₂ (0.2 mL), trifluoroacetic acid (77 µL, 1 mmol) was added at 0 °C and the reaction mixture was stirred for 3.5 h at 23°C. The reaction was quenched at 0°C with saturated aqueous sodium bicarbonate (15 mL) and extracted with ethyl acetate (2 x 10 mL). The combined organic layers were dried over sodium sulphate, filtered, and the solvent was eliminated under reduced pressure. The residue was purified by flash column chromatography (SiO₂, Hex:EtOAc 1:1) to afford **109** (12 mg, 81 %) as a white solid. Rf: 0.32 (Hex:EtOAc 1:1).
¹H NMR (300 MHz, CDCl₃) δ 6.43 (s, 1H), 5.97 (d, *J*= 1.5 Hz, 1H), 5.91 (d, *J=* 1.5 Hz, 1H), 5.69 (s, 1H), 4.51 (dd, *J₁=* 3.3 Hz, *J₂*=11.1 Hz, 1H), 4.10-4.05 (m, 3H), 3.78-3.77 (m, H), 3.75 (s, 3H), 3.33 (d, *J*= 8.1 Hz,1H), 3.22 (dt, *J₁*= 2.7 Hz, *J₂=* 12.0 Hz, H), 2.96 (dd, *J₁*= 8.4 Hz, *J*₂= 17.7 Hz, 1H), 2.80 (d, *J*= 15.6 Hz, 1H), 2.55 (d, *J*= 18.0 Hz, 1H), 2.33 (s, 3H), 2.24 (s, 3H), 2.01 (s, 3H), 1.87-1.66 (m, 3H), 1.37-1.27 (m, 2H), 0.77 (t, *J*= 7.5 Hz, 3H).
ESI-MS m/z: Calcd. for C₃₂H₃₁N₃O₈: 591.6. Found (M+1)⁺: 592.8.

### Example 104

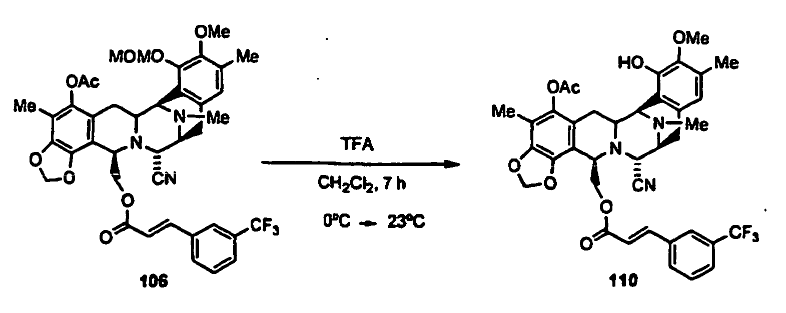

To a solution of 106 (90 mg, 0.1178 mmol) in CH₂Cl₂ (0.3 mL). trifluoroacetic acid (750 µl, 4.71 mmol) was added at 0 °C and the reaction mixture was stirred for 7 h at 23°C. The reaction was quenched at 0°C with saturated aqueous sodium bicarbonate (20 mL) and extracted with ethyl acetate (2 x 15 mL). The combined organic layers were dried over sodium sulphate, filtered, and the solvent was eliminated under reduced pressure. The residue was purified by flash column chromatography (SiO₂, Hex:EtOAc 1:1) to afford **110** (71 mg, 84 %) as a white solid. Rf: 0.6 (Hex:EtOAc 1:1).
H NMR (300 MHz, CDCl₃) δ 7.76 (bs, 1 H), 7.62-7.57 (m, 2H), 7.48-7.45 (m, 1H), 7.12 (d, *J*= 16.2 Hz, 1H), 6.37 (s, 1H), 6.00 (d, *J=* 16.2 Hz,1H), 5.98 (d,*J*= 1.2 Hz, 1H), 5.92 (d, *J*= 1.2 Hz,1H), 5.60 (bs,1H), 4.88 (d, *J*= 10.2 Hz,1H), 4.14 (bs, 1H), 4.10 (d,*J=* 2.4 Hz, 1H), 4.03 (d, *J*= 2.4 Hz, 1H), 3.89 (dd, *J₁=* 2.7 Hz, *J₂*=11.4 HzI H), 3.32 (d, *J*= 8.4 Hz, 1H), 3.26-3.21 (m, 4H), 2.91 (dd, *J₁=* 8.1 Hz, *J₂=* 18.0 Hz, 1H), 2.82 (d, *J=* 13.8 Hz, 1H), 2.58 (d, *J=* 18.0 Hz, 1H), 2.33 (s, 3H), 2.24 (s, 3H), 2.05 (s, 3H), 1.89 (s, 3H), 1.84 (dd, *J₁=* 12.0 Hz, J₁= 15.6 Hz,1H).
ESI-MS m/z: Calcd. for C₃₈H₃₆F₃N₃O₈: 719.7. Found (M+1)⁺: 720.3.

### Example 105

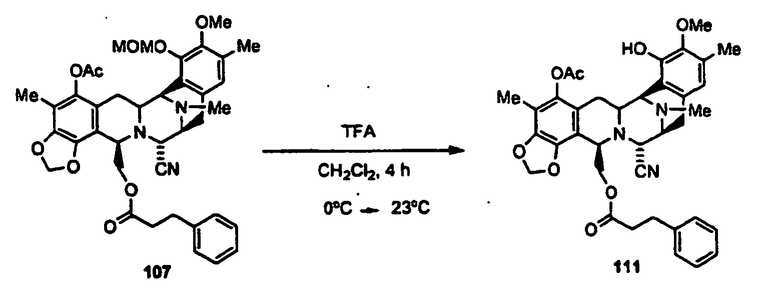

To a solution of **107** (20 mg, 0.286 mmol) in CH₂Cl₂ (0.2 mL), trifluoroacetic acid (88 µL, 1.144 mmol) was added at 0 °C and the reaction mixture was stirred for 4 h at 23°C. The reaction was quenched at 0°C with saturated aqueous sodium bicarbonate (15 mL) and extracted with ethyl acetate (2 x 10 mL). The combined organic layers were dried over sodium sulphate, filtered, and the solvent was eliminated under reduced pressure. The residue was purified by flash column chromatography (SiO₂, Hex:EtOAc 1:1) to afford **111** (18 mg, 96 %) as a white solid. Rf: 0.39 (Hex:EtOAc 1:1).
¹H NMR (300 MHz, CDCl₃) δ 7.23-7.16 (m, 3H), 7.06-7.04 (m, 2H), 6.43 (s, 1H). 5.96 (d, *J=* 1.5 Hz, 1H), 5.90 (d, *J=* 1.5 Hz,1H), 6.66 (s, 1H), 4.52 (dd, *J₁*= 3.3 Hz. *J*₂= 11.1 Hz, 1H), 4.07 (s, 1H), 4.05 (d, *J=* 3.3 Hz, 1H), 4.03 (d, *J=* 2.4 Hz, 1H), 3.76 (dd, *J₁=* 3.6 Hz, *J*₂= 11.1 Hz, 1H), 3.56 (s, 3H), 3.31 (d, *J*= 7.5 Hz, 1H), 3.23 (d, *J*=12.0 Hz, 1H), 2.95 (dd, *J₁=* 8.1 Hz, *J₂=* 18.0 Hz, 1H), 2.80 (d, *J*= 15.3 Hz, 1H), 2.63-2.58 (m, 2H), 2.53 (d, *J=* 18.0 Hz, 1H), 2.33 (s, 3H), 2.61 (s, 3H), 2.21-2.09 (m, 2H), 2.13 (s, 3H), 2.02 (s, 3H), 1.85 (dd, *J₁=* 11.7 Hz, *J*₂= 115.3Hz, 1H). ESI-MS m/z: Calcd. for C₃₇H₃₉N₃O₈: 653.7. Found (M+1)⁺: 654.3.

### Example 106

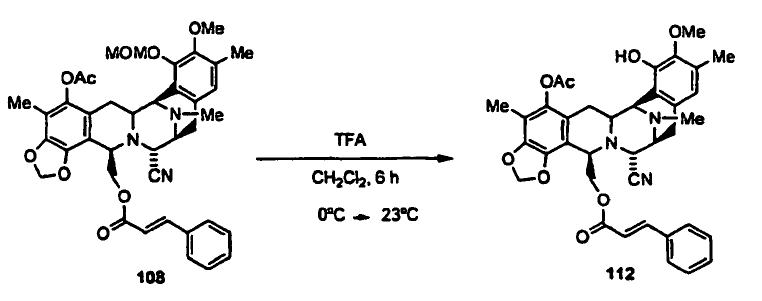

To a solution of **108** (14 mg, 0.02 mmol) in CH₂Cl₂ (0.4 mL), trifluoroacetic acid (61.5 µL, 0.8 mmol) was added at 0 °C and the reaction mixture was stirred for 6 h at 23°C. The reaction was quenched at 0°C with saturated aqueous sodium bicarbonate (15 mL) and extracted with ethyl acetate (2 x 10 mL). The combined organic layers were dried over sodium sulphate, filtered, and the solvent was eliminated under reduced pressure. The residue was purified by flash column chromatography (SiO₂, Hex:EtOAc 2:1) to afford **112** (12 mg, 92 %) as a white solid. Rf: 0.36 (Hex:EtOAc 1:1).
¹H NMR (300 MHz, CDCl₃) δ 7.46-7.45 (m, 2H), 7.35-7.20 (m, 4H), 6.38 (s, 1H), 6.05 (d, *J*= 15.9 Hz, 1H), 5.98 (d, *J*= 1.2 Hz, 1H), 5.93 (d, *J=* 1.2 Hz, 1H), 5.57 (s, 1H), 4.71 (d, *J=* 9.3 Hz,1H), 4.17-4.13 (m, 2H), 4.08 (d, *J*= 1.9 Hz, 1H), 3.89 (dd, *J₁=* 3.6 Hz, *J₂*= 11.4 Hz 1H), 3.33 (m, 5H), 3.26-3.22 (m, 1H), 2.93 (dd, *J₁=* 9.0 Hz, *J₂=* 17.4 Hz1 H), 2.34 (s. 3H), 2.25 (s, 3H), 2.05 (s, 3H), 1.97 (s, 3H), 1.81 (dd, *J₁*= 12.0 Hz, *J₂=*15.6 Hz, 1H).
ESI-MS m/z: Calcd. for C₃₇H₃₇N₃O₈: 651. Found (M+1)⁺: 652.2.

### Example 107

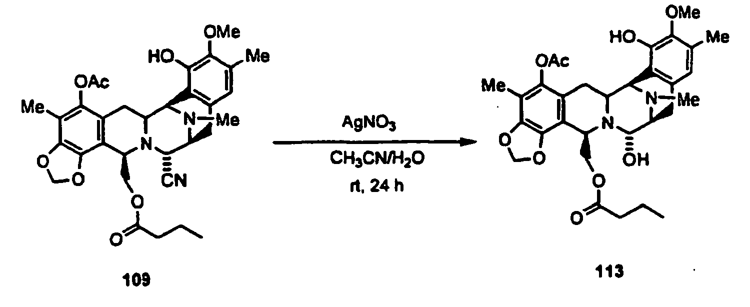

To a solution of **109** (10 mg, 0.017 mmol) in CH₃CN/H₂O (1.5 mL/1 mL), AgNO₃ (86 mg, 0.5 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂, gradient EtOAc to EtOAc:MeOH 3:1) to afford **113** (7 mg, 71 %) as a white solid.
Rf: 0.41 (EtOAc:MeOH 5:1).
¹H NMR (300 MHz, CDCl₃) δ 6.45 (s, 1H), 5.95 (d, *J*= 1.5 Hz, 1H), 5.88 (d, *J=* 1.5 Hz, 1H), 5.65 (bs, 1H), 4.50-4.48 (m, 2H), 4.44 (d, *J*= 2.1 Hz, 1H), 3.96 (d,*J*= 3.0 Hz, 1H), 3.76 (s, 3H), 3.74-3.70 (m, 1H), 3.30 (d,*J*= 12.3 Hz, 1H), 3.13 (d,*J*= 7.5 Hz, 1H), 2.86 (dd, *J₁*= 5.7 Hz, *J*₂=18.3 Hz, 1H), 2.73 (d,*J*=14.7 Hz, 1H), 2.48 (d,*J*= 17.7 Hz, 1H), 2.33 (s, 3H), 2.24 (s, 3H), 2.17 (s, 3H), 2.00 (s, 3H), 1.86-1.55 (m, 3H), 1.42-1.23 (m, 2H), 0.75 (t, *J*= 7.5 Hz, 3H).
ESI-MS m/z : Calcd. for C₃₁H₃₈N₂O₉ : 582.6. Found (M-17)⁺ : 565.3.

### Example 108

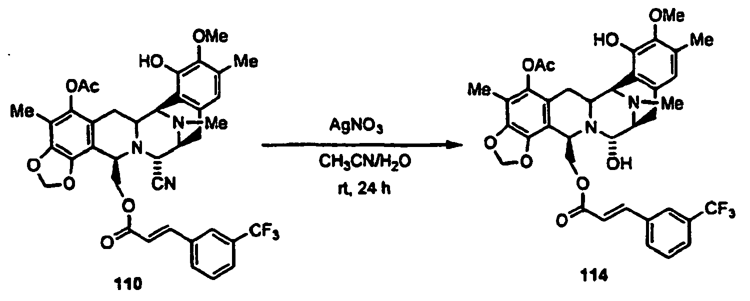

To a solution of **110** (42.8 mg. 0.059 mmol) in CH₃CN/H₂O (1.5 mL/1 mL). AgNO₃ (303 mg, 1.78 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (20 mL). The solution was extracted and the organic layer was dried over Na₂SO₄, filtered and concentrated *in vacuo.* The residue was purified by flash column chromatography (SiO₂, gradient EtOAc to EtOAc:MeOH 5:1) to afford **114** (30 mg, 71 %) as a white solid.
Rf: 0.30 (EtOAc:MeOH 5:1).
¹H NMR (300 MHz, CDCl₃) δ 7.75 (bs, 1H), 7.61-7.56 (m, 2H), 7.45-7.42 (m, 1H), 7.12 (d, *J*= 16.2 Hz, 1H), 6.38 (s, 1H), 6.02 (d, *J=* 16.2 Hz,1H), 5.97 (d, *J*= 1.5 Hz, 1H), 5.90 (d, *J=* 1.5 Hz, 1H), 5.50 (bs, 1H), 4.87 (bs, 1H), 4.56 (m, 1H), 4.45 (bs, 1H), 3.92 (d, *J=* 2.4 Hz, 1H), 3.31 (dt, *J₁=* 3.6 Hz, *J₂=* 12.9 Hz, 1H), 3.21 (bs, 3H), 3.13 (d, *J=* 7.8 Hz, 1H), 2.82 (dd, *J₁=* 8.1 Hz, *J₂=*18.0 Hz, 1H), 2.75 (d, *J=* 14.7 Hz, 1H), 2.49 (d, *J=* 18.0 Hz, 1H), 2.33 (s, 3H), 2.21 (s, 3H), 2.05 (s, 3H), 1.89 (s, 3H), 1.78 (dd, *J₁=* 12.0 Hz, *J₂=* 15.6 Hz, 1H).
ESI-MS m/z: Calcd. for C₃₇H₃₇F₃N₂O₉: 710.6. Found (M-17)⁺: 693.2.

### Example 109

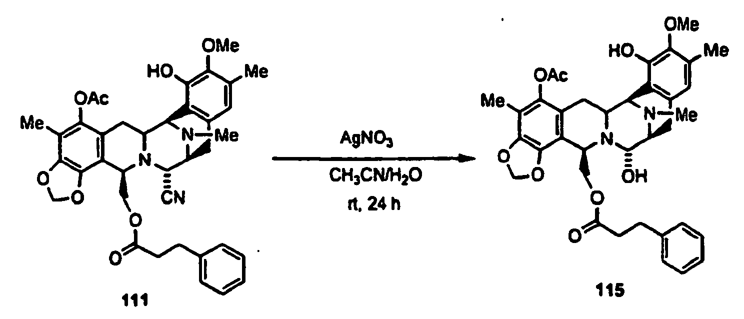

To a solution of 111 (12 mg, 0.018 mmol) in CH₃CN/H₂O (1.5 mL/1 mL). AgNO₃ (93.5 mg, 0.55 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂, gradient EtOAc to EtOAc:MeOH 1:1) to afford **115** (10 mg. 86 %) as a white solid.
Rf: 0.43 (EtOAc:MeOH 5:1).
¹H NMR (300 MHz, CDCl₃) δ 7.23-7.14 (m, 3H), 7.05-7.03 (m, 2H), 6.45 (s, 1H), 5.93 (d, *J*= 1.2 Hz, 1H), 5.88 (d, *J*= 1.2 Hz, 1H), 5.63 (brd, 1H), 4.55-4.49 (m, 2H), 4.43 (d, *J*= 2.7 Hz, 1H), 3.96 (d, *J*= 3.1 Hz, 1H), 3.80-3.73 (m, 1H), 3.56 (bs, 3H), 3.32 (dt, *J₁*= 3.3 Hz, *J₂=* 12.6 Hz, 1H), 3.13 (d, *J*= 6.0 Hz, 1H), 2.86 (dd, *J₁*= 7.5 Hz, *J₂=* 18.3 Hz, 1H), 2.74 (d, *J*= 14.7 Hz, 1H), 2.61-2.56 (m, 2H), 2.47 (d, *J=* 18.0 Hz, 1H), 2.33 (s, 3H), 2.23 (s, 3H), 2.13 (s, 3H), 2.01 (s, 3H), 1.99-1.94 (m, 2H), 1.78 (dd, *J₁*= 11.7 Hz, *J*₂= 15.0 Hz, 1H).
ESI-MS m/z: Calcd. for C₃₆H₄₀N₂O₉: 644.7. Found (M-17)⁺: 627.2.

### Example 110

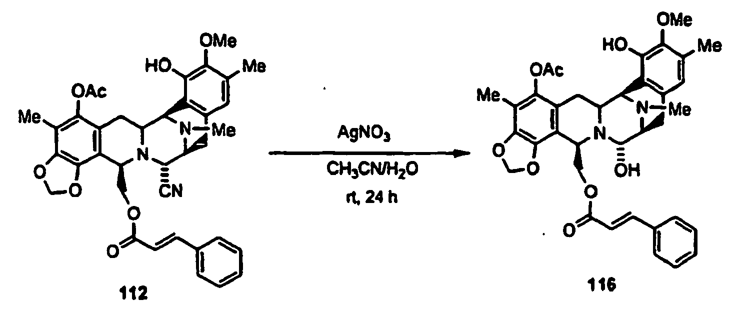

To a solution of **112** (12 mg, 0.018 mmol) in CH₃CN/H₂O (1.5 mL/1 mL), AgNO₃ (93 mg, 0.55 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂ gradient EtOAc to EtOAc:MeOH 1:1) to afford **116** (8 mg, 70 %) as a white solid.
Rf: 0.41 (EtOAc:MeOH 5:1).
¹H NMR (300 MHz, CDCl₃) δ 7.44-7.43 (m, 2H), 7.34-7.27 (m, 4H), 6.39 (s, 1H), 6.03 (d, *J*= 15.9 Hz, 1H), 5.96 (d,*J=* 1.5 Hz, 1H), 5.90 (d, *J*= 1.5 Hz, 1H), 5.55 (m, 1H), 4.47 (m, 1 H), 4.50 (m,1H), 3.94 (d,*J=* 3.6 Hz,1H), 3.85 (dd, J₁= 3.3 Hz, *J₂*= 11.1 Hz 1H), 3.66 (bs, 3H), 3.34-3.31 (m, 2H), 3.13 (d,*J*= 5.1 Hz, 1H), 2.93-2.73 (m, 2H). 2.53 (d,*J*= 18.0 Hz, 1H), 2.33 (s, 3H), 2.22 (s, 3H), 2.03 (s, 3H), 1.94-1.82 (m, 1H).
ESI-MS m/z: Calcd. for C₃₆H₃₈N₂O₉:642.7. Found (M-17)⁺: 625.2.

### Example 111

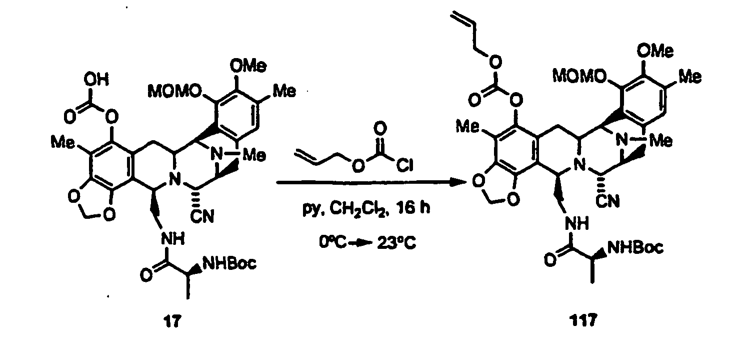

To a solution of 17 (6.28 g, 9.06 mmol) in CH₂Cl₂ (45.3 mL), allyl chloroformiate ( 3.85 mL, 36.24 mmol) and pyridine (2.93 mL, 36.24 mmol) were added at 0 °C. The reaction mixture was stirred for 16 h at 23°C and then, the solution was diluted with CH₂Cl₂ (150 mL) and washed with 0.1 N HCl (2 x 100 mL). The organic layer was dried over Na₂SO₄, filtered, and the solvent was eliminated under reduced pressure to give **117** (5.96 g, 84 %) which was used in following steps with no further purification.
Rf: 0.56 (CH₂Cl₂:EtOAc 1:1).
¹H NMR (300 MHz, CDCl₃) δ 6.72 (s, 1H), 6.05-5.94 (m, 1H), 6.01 (s, 1H), 5.91 (s, 1H), 5.44 (dd, *J1* = 1.2 *Hz, J2* = 17.1 Hz, 1H), 5.35 (dd, *J1* = 1.2 *Hz, J2* = 10.5 Hz1 H), 5.34 (m, 1H), 5.10 (d, *J* = 5.7 Hz, 1H), 5.05 (d, *J* = 5.7 Hz, 1H), 4.68 (d, *J* = 5.7 Hz, 1H), 4.65 (dt, *J1* = 1.2 Hz, *J2* = 6 Hz, 1H), 4.18 (brd, *J* = 9 Hz, 2H), 4.04 (bs, 1H), 3.70 (s, 3H), 3.67-3.60 (m, 1H), 3.55 (s, 3H), 3.43-3.41 (m, 2H), 3.29-3.25 (m, 2H), 3.00 (dd, *J1* = 8.7 Hz, *J2 =* 18.3 Hz, 1H), 2.90 (dd, *J1* = 2.4 Hz, *J₂=* 16.2 Hz, 1H), 2.75 (d, *J* = 18.3 Hz, 1H), 2.35 (s, 3H), 2.22 (s, 3H), 2.06 (s, 3H), 1.83 (dd, *J1* = 11.4 Hz, *J2* = 15.9 Hz, 1H), 1.39 (s, 9H). 0.73 (d, *J* = 6.9 Hz, 3H).
¹³C NMR (75 MHz, CDCl₃) δ 172.1, 152.8, 148.6, 148.3, 144.6, 140.7, 140.6, 131.5, 131.2, 131.1, 130.4, 125.3, 125.0, 123.3, 120.9, 119.1, 118.8, 117.6, 112.9, 112.0, 101.6, 99.2, 71.8, 69.0, 68.4, 59.7, 59.2, 57.6, 57.3, 56.7, 55.8, 55.2, 41.4, 39.9, 28.2, 26.0, 25.0, 18.6, 15.6, 9.0.
ESI-MS m/z: Calcd. for C₄₀H₅₁N₅O₁₁: 777.8. Found (M+1)⁺: 7783

### Example 112

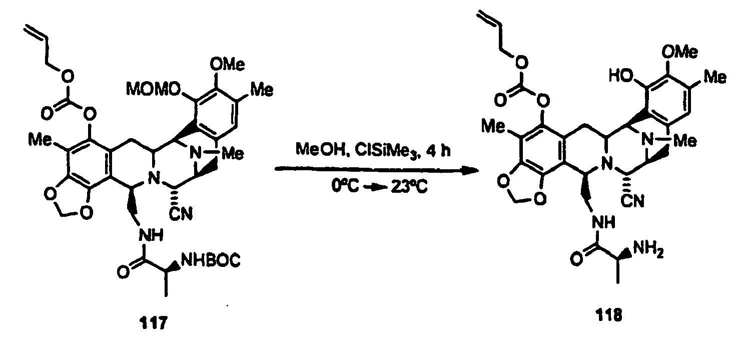

To a solution of **117** (3.96 g, 5.09 mmol) in MeOH (37.4 mL), trimetylchlorosilane ( 6.5 mL, 50.9 mmol) was added at 0 °C. The reaction mixture was stirred for 4 h at 23°C and then, the solvent was eliminated under reduced pressure. The residue was diluted with EtOAc (70 mL) and washed with a saturated aqueous solution of NaHCO₂ (2 x 45 mL). The organic layer was dried over Na₂SO₄, filtered, and the solvent was eliminated *in vacuo* to give **118** (2.77 g, 86 %) which was used in following steps with no further purification.
Rf: 0.61 (Hex:EtOAc 1:1).
¹H NMR (300 MHz, CDCl₃) δ 6.50 (s, 1H), 6.45 (m, 1H), 6.10-6.03 (m, 1H), 6.00 (s,1H), 5.93 (s, 1H), 5.47 (dd, *J1* = 1.2 Hz, *J2* = 17.1 Hz, 1H), 5.38 (dd, *J1* = 1.2 Hz, *J2* = 10.5 Hz, 1H), 4.81-4.64 (m, 2H), 4.10-4.03 (m, 3H), 3.75 (s, 3H), 3.70-3.44 (m, 2H), 3.35 (d, *J* = 8.1 Hz, 1H), 3.28 (dt, *J1* = 2.7 *Hz, J2* = 9 Hz, 1H), 2.98 (dd, *J1* = 7.8 Hz, *J2* = 18 Hz, 1H), 2.90 (dd, *J1* = 2.7 Hz, *J2* = 16.2 Hz, 1H), 2.78 (dd, *J1* = 6.9 Hz, *J2* = 14.1 Hz, 1H), 2.63 (d, *J =* 18.3 Hz, 1H), 2.30 (s, 3H), 2.25 (s, 3H), 2.04 (s, 3H), 1.88 (dd, *J1* = 13.2 Hz, *J2* = 15.6 Hz, 1H), 0.95 (d, *J* = 6.9 Hz, 3H).
¹³C NMR (75 MHz, CDCl₃) δ 175.8, 152.9, 146.6, 144.6, 142.5,140.8, 140.6, 131.5, 131.3, 128.5, 121.1, 120.8, 118.9, 117.8, 117.0, 113.2, 111.9, 101.7, 68.9, 60.6, 59.1, 56.6, 56.4, 55.7, 55.2, 50.5*,* 41.7, 39.4, 26.1, 25.0, 21.0, 15.6, 9.2.
ESI-MS m/z: Calcd. for C₃₃H₃₉N₅O₈: 633.6. Found (M+1)⁺: 634.2.

### Example 113

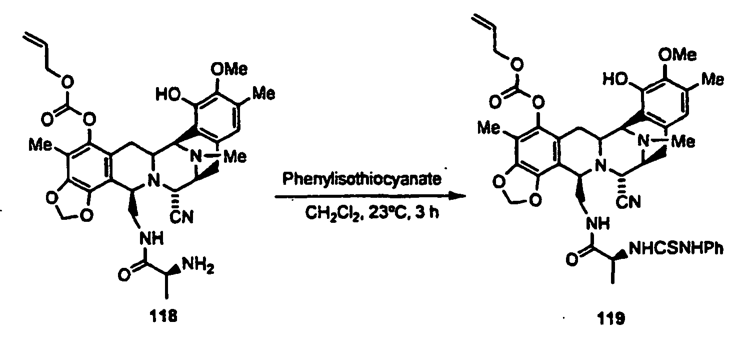

To a solution of **118** (3.52 g, 5.56 mmol) in CH₂Cl₂ (28 mL), phenylisothiocyanate ( 3.99 mL, 33.36 mmol) was added at 23 °C. The reaction mixture was stirred for 3 and then, the solvent was eliminated under reduced pressure. The residue was purified by flash column chromatography to afford **119** (3.5 g, 82 %) as a white solid.
Rf: 0.52 (CH₂Cl₂:EtOAc 1:5).
¹H NMR (300 MHz, CDCl₃) δ 7.69 (bs, 1H), 7.49-7.46 (m, 2H), 7.34-7.21 (m, 2H), 6.96 (d, *J =* 6.9 Hz, 1H), 6.06-5.97 (m, 1H), 6.03 (s, 1H), 5.96 (bs, 1H), 5.91 (s, 1H), 5.66 (s, 1H), 5.47 (dd, *J1* = 1.5 Hz, *J2* = 17.1 Hz, 1H), 5.37 (dd, *J1* = 1.5 Hz, *J2* = 10.5 Hz, 1H), 5.36 (s, 1H), 4.75-4.70 (m, 2H), 4.54-4-49 (m, 1H), 4.14 (d, *J* = 2.4 Hz, 1H), 4.07-4.06 (m, 2H), 3.70 (s, 3H), 3.44 (m, 1H), 3.35 (d, *J* = 8.1 Hz, 1H), 3.21 (dt, *J1* = 2.7 Hz, *J2* = 6.6 Hz, 1H), 2.94-2.82 (m, 2H), 2.63 (d, *J* = 18 Hz, 1 H), 2.24 (s, 3H), 2.06 (s, 3H), 2.06 (s, 3H), 1.90 (dd, *J1* =11.7 Hz, *J2*= 15.9 Hz, 1H), 0.71 (d, *J* = 6.9 Hz, 3H).
¹³C NMR (75 MHz, CDCl₃) δ 178.6, 171.9. 152.8, 146.7. 144.5, 142.6, 140.8, 140.5, 136.3, 131.3, 131.0, 129.9, 129.8, 128.9, 126.7, 125.2, 124.3, 121.1, 120.6, 118.9, 117.7, 116.5, 112.8, 112.1, 101.6, 68.9, 60.5, 58.9, 57.3, 56.1, 55.9, 55.1, 53.3, 41.5, 39.2, 25.9, 24.6, 20.9, 15.4, 9.1.
ESI-MS m/z: Calcd. for C₄₀H₄₄N₃O₈S: 768.8. Found (M+1)⁺: 769.3.

### Example 114

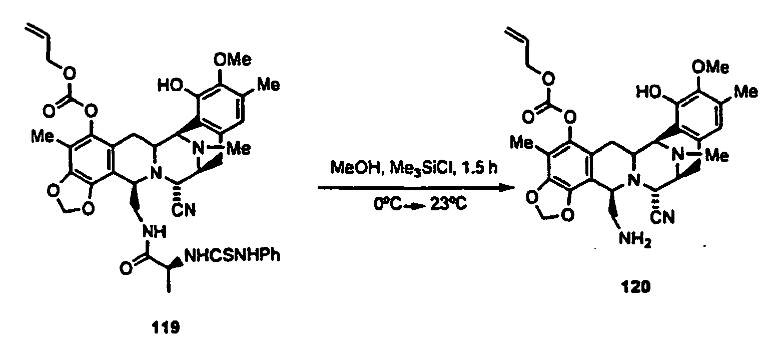

To a solution of **119** (3.38 g, 4.4 mmol) in MeOH (22 mL), trimetylchlorosilane (2.3 mL, 22 mmol) was added at 0 °C. The reaction mixture was stirred for 1.5 h at 23°C and then, the solvent was eliminated under reduced pressure. The residue was diluted with EtOAc (100 mL) and washed with 0.1 N HCl (2 x 75 mL). The aqueous phase was basified with a saturated aqueous solution of NaHCO₂ and extracted with CH₂Cl₂ (2 x 100 mL). The combined organic layers were dried over Na₂SO₄, filtered, and the solvent was eliminated under reduced pressure to afford **120** (2.47 g, 100 %) as a white solid which was used in following steps with no further purification.
Rf: 0.26 (EtOAc:MeOH 5:1).
¹H NMR (300 MHz, CDCl₃) δ 6.45 (s, 1H), 6.05-5.98 (m, 1H), 5.97 (d, *J* = 1.2 Hz, 1H), 5.90 (d, *J =* 1.2 Hz, 1H), 5.44 (dd, *J1* = 1.2 Hz, *J2* = 17.1 Hz, 1H), 5.35 (dd, *J1* = 1.2 Hz, *J2* = 10.2 Hz, 1H), 4.75-4.71 (m, 2H), 4.12-4.10 (m, 1H), 3.99 (d, *J* = 2.4 Hz, 1H), 3.92 (bs, 1H), 3.73 (s, 3H), 3.36-3.26 (m, 2H), 3.06 (dd, *J1* = 8.4 Hz, *J2* = 18 Hz, 1H), 2.89 (dd, *J1 =* 2.7 Hz, *J2* = 15.9 Hz, 1H), 2.75-2.73 (m, 2H), 2.48 (d, *J* = 18 Hz, 1H), 2.32 (s, 3H), 2.23 (s, 3H), 2.05 (s, 3H), 1.85 (dd, *J1*= 11.7 Hz, *J2* = 15.6 Hz, 1H).
¹³C NMR (75 MHz, CDCl₃) δ 153.0, 146.6, 144.5, 142.8, 140.7, 131.5, 130.5, 128.9, 121.3, 120.9, 119.1, 117.9, 116.7, 113.8, 111.6, 101.5, 69.0, 60.6, 59.8, 58.7, 56.5, 56.0, 55.3, 44.2, 41.8, 31.6, 26.1, 25.7, 15.7, 9.2.
ESI-MS m/z: Calcd. for C₃₀H₃₄N₄O₇: 562.6. Found (M+1)⁺: 563. 2.

### Example 115

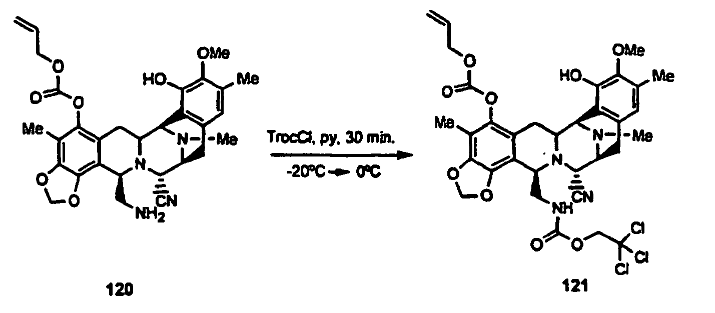

To a solution of **120** (2.57 g, 4.4 mmol) in CH₂Cl₂ (44 mL), TrocCl (0.91 mL, 6.6 mmol) and pyridine (0.53 mL, 6.6 mmol) were added at -20 °C. The reaction mixture was stirred for 30 min at 0°C and then, the solution was diluted with CH₂Cl₂ (50 mL) and washed with 0.1 N HCl (2 x 25 mL). The organic layer was dried over Na₂SO₄, filtered, and the solvent was eliminated under reduced pressure to give **121** (3.24 g, 100 %) which was used in following steps with no further purification.
Rf: 0.62 (EtOAc:MeOH 5:1).
¹H NMR (300 MHz, CDCl₃) δ 6.50 (s, 1H), 6.07-6.01 (m, 1H), 5.99 (d, *J* = 1.2 Hz, 1H), 5.93 (d, *J =* 1.2 Hz, 1H), 5.68 (s, 1H), 5.46 (dd, *J1* = 1.2 Hz*, J2* = 17.1 Hz, 1H), 5.37 (dd, *J1* = 1.2 Hz, *J2* = 10.5 Hz, 1H), 4.74 (t, *J* = 5.7 Hz, 2H), 4.63-4.62 (m, 1 H), 4.54 (d, *J* = 12 Hz, 1H), 4.30 (d, *J =* 12 Hz, 1H), 4.14-4.11 (m, 2H), 4.02-4.01 (m, 2H), 3.75 (s, 3H), 3.36-3.26 (m, 3H), 3.04 (dd, *J1* = 8.1 Hz, *J2* = 17.7 Hz, 1H), 2.91 (dd, *J1* = 2.4 Hz, *J2* = 15.6 Hz, 1H), 2.60 (d, *J* = 17.7Hz, 1H), 2.31 (s, 3H), 2.25 (s,3H), 2.04 (s, 3H), 1.84 (dd, *J1* = 12 Hz, *J2 =* 15.9 Hz, 1H).
ESI-MS m/z: Calcd. for C₃₃H₃₅Cl₃N₄O₉: 738.0. Found (M+1)⁺: 737.2.

### Example 116

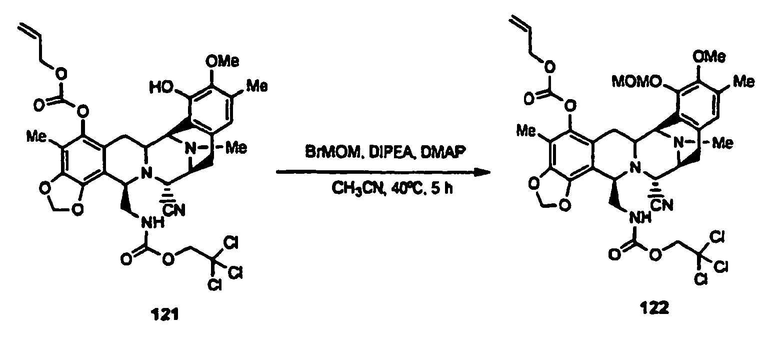

To a solution of **121** (0.45 g, 0.60 mmol) in CH₃CN (4 mL), diisopropylethylamine (2.17 mL, 12.46 mmol), bromomethyl methyl ether (0.76 mL, 9.34 mmol) and dimethylaminopyridine (8 mg, 0.062 mmol) were added at 0 °C. The reaction mixture was heated at 40°C for 5 h. Then, the reaction was diluted with CH₂Cl₂ (50 mL) and washed with 0.1 N HCl (2 x 25 mL). The organic layer was dried over Na₂SO₄, filtered, and the solvent was eliminated under reduced pressure to give **122** (0.453 g, 95 %) which was used in following steps with no further purification.
Rf: 0.31 (RP-18 CH₃CN-H₂O 8:2).
¹H NMR (300 MHz, CDCl₃) δ 6.70 (s, 1H), 6.05-5.99 (m, 1 H), 5.97 (s, 1H), 5.92 (s, 1H), 5.43 (dd, *J1* = 1.2 Hz, *J2* = 17.1 Hz1 H), 5.34 (dd, *J1* = 1.2 Hz, *J2* = 10.5 Hz, 1H), 5.10-5.04 (m, 2H), 4.72-4.68 (m, 2H), 4.60 (t, *J* = 5.7 Hz, 1H), 4.49 (d, *J* = 12.3 Hz, 1H), 4.38 (d, *J* = 12.3 Hz, 1H), 4.18 (d, *J* = 2.7 Hz, 1H), 4.03-4.00 (m, 2H), 3.71 (s, 3H), 3.54 (s, 3H), 3.38-3.22 (m, 4H), 3.04 (dd, *J1* = 7.8 Hz, *J2* = 18.3 Hz, 1H), 2.91 (dd, *J1* = 2.4 Hz, *J2* = 15.9 Hz, 1H), 2.61 (d, *J* = 18 Hz, 1H), 2.31 (s, 3H), 2.20 (s, 3H), 2.03 (s, 3H), 1.76 (dd, *J1* = 11.7 Hz, *J2* = 15.6 Hz, 1H).
ESI-MS m/z: Calcd. for C₃₁H₃₉Cl₃N₄O₁₀: 782.0. Found (M+1)⁺: 783.2.

### Example 117

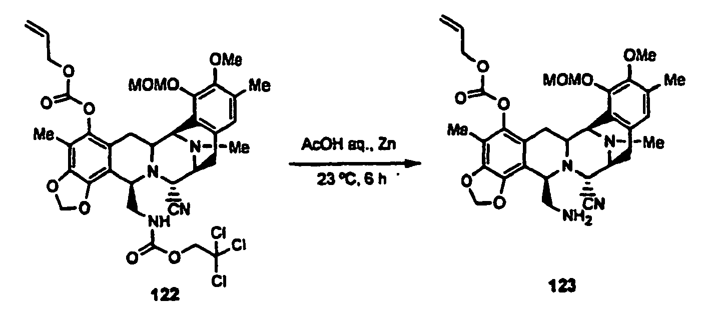

To a suspension of **122** (0.45 g, 0.579 mmol) in 90 % aqueous acetic acid (6 mL), powder zinc (0.283 g, 4.34 mmol) was added and the reaction was stirred for 6 h at 23 °C. Then, the mixture was filtered through a pad of celite which was washed with CH₂Cl₂ (25 mL). The organic layer was washed with an aqueous sat. solution of sodium bicarbonate (pH= 9) (2 x 15 mL), dried over Na₂SO₄ filtered, and the solvent was eliminated under reduced pressure to give **123** (0.351 g, 100 %) which was used in following steps with no further purification.
Rf: 0.38 (SiO₂, EtOAc:MeOH 5:1).
¹H NMR (300 MHz, CDCl₃) δ 6.68 (s, 1H), 6.06-5.99 (m, 1H), 5.97 (d, *J* = 1.5 Hz, 1H), 5.91 (d, *J* = 1.25 Hz, 1H), 5.44 (dd, *J1* = 1.5 Hz, *J2* = 17.4 Hz, 1H), 5.36 (dd, *J₁* = 1.5 Hz, *J2* = 10.2 Hz, 1H), 5.08 (q, *J* = 5.7 Hz, 2H), 5.74-4.70 (m, 2H), 4.02 (d, *J* = 3 Hz, 1H), 4.00 (d, *J =* 2.4 Hz. 1H), 3.91 (m, 1H), 3.71 (s, 3H), 3.56 (s, 3H), 3.37-3.35 (m, 1H), 3.29 (t, *J*= 2.7 Hz, 1H), 3.08 (dd, *J1* = 7.5 Hz, *J2* = 18 Hz, 1H), 2.90 (dd, *J1* = 2.7 Hz, *J2* = 15.9 Hz, 1H), 2.74 (dd, *J1* = 2.4 Hz, *J2* = 5.1 Hz, 2H), 2.48 (d, *J* = 18 Hz, 1H), 2.35 (s, 3H), 2.20 (s, 3H), 2.05 (s, 3H), 1.80 (dd, *J1* = 12 Hz, *J2* = 15.9 Hz, 2H).
ESI-MS m/z: Calcd. for C₃₂H₃₈N₄O₈: 606.6. Found (M+1)⁺: 607.3.

### Example 118

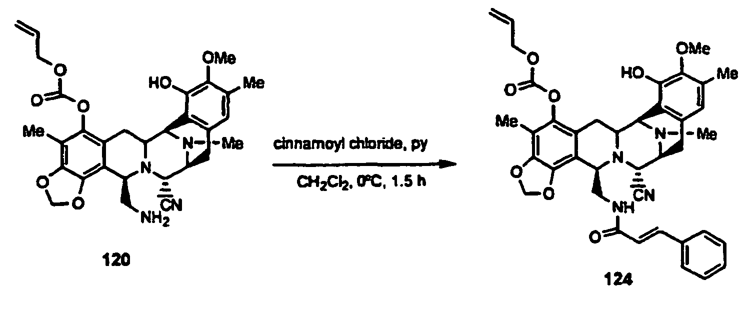

To a solution of 120 (100 mg, 0.177 mmol) in CH₂Cl₂ (0.7 mL), cinnamoyl chloride ( 29.5 mg, 0.177 mmol) and pyridine (14.37 µL, 0.177 mmol) were added at 0 °C. The reaction mixture was stirred for 1.5 h and then, the solution was diluted with CH₂Cl₂ (15 mL) and washed with 0.1 N HCl (10 mL). The organic layer was dried over Na₂SO₄. filtered, and the solvent was eliminated under reduced pressure. The residue was purified by flash column chromatography (SiO₂, gradient Hex:EtOAc 2:1 to Hex:EtOAc 1:3) to afford **124** (86 mg, 70 %) as a white solid.
Rf: 0.77 (EtOAc:MeOH 5:1).
¹H NMR (300 MHz, CDCl₃) δ 7.39-7.26 (m, 5H), 7.25 (d, J = 15.6 Hz 1H), 6.44 (s, 1H), 6.01 (d, J = 1.2 Hz, 1H), 5.94 (d, J = 1.2 Hz, 1H), 5.68 (s, 1H), 5.65 (d, J = 15.6 Hz, 1H), 5.44 (dd, J1 =1.2 Hz, J2 = 17.1 Hz, 1H), 5.35 (dd, J1 =1.2 Hz, J2 = 10.5 Hz, 1H), 5.18 (t, J = 6 Hz, 1H), 4.73-4.69 (m, 2H), 4.11-4.09 (m, 3H), 3.66-3.58 (m, 2H), 3.65 (s, 3H), 3.38-3.31 (m, 3H), 3.02 (dd, J1 = 8.4 Hz, J2 = 18.3 Hz, 1H), 2.92 (dd, J1 = 2.7 Hz, J2 = 15.6 Hz, 1H), 2.59 (d, J = 18.3 Hz, 1H), 2.31 (s, 3H), 2.05 (s, 3H), 2.02 (s, 3H), 1.89 (dd, J1 = 12.3 Hz, J2 = 16.2 Hz, 1H).
¹³C NMR (75 MHz, CDCl₃) δ 165.5, 152.7, 146.6, 144.4, 142.6, 140.7, 140.5, 140.1, 134.7, 131.2, 130.6, 129.3, 128.7, 128.4, 127.6, 120.8, 120.5, 120.3, 118.9, 117.6, 116.5, 113.2, 111.8, 101.6, 68.8, 60.4, 59.0, 56.2, 56.1, 55.7, 55.0, 41.5, 40.6, 25.9, 25.1, 15.5, 9.0.
ESI-MS m/z: Calcd. for C₃₉H₄₀N₄O₈: 692.7. Found (M+1)⁺: 693.2

### Example 119

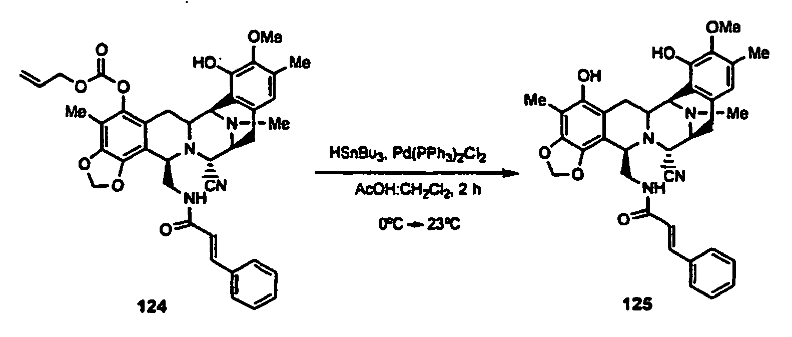

To a solution of **124** (495 mg, 0.713 mmol) in CH₂Cl₂ (28 mL), acetic acid (163 µL). Pd(PPh₃)₂Cl₂ (50 mg, 0.0713 mmol) and BU₃SnH (384 µL, 1.42 mmol) were added at 0 °C. The reaction mixture was stirred for 2 h at 23°C and then, the solution was poured into a pad of flash column (SiO₂, gradient Hex:EtOAc 1:1 to EtOAc) to afford **125** (435 mg, 100 %) as a white solid. Rf: 0.22 (Hex:EtOAc 1:2).
¹H NMR (300 MHz, CDCl₃) δ 7.36-7.33 (m, 5H), 7.28 (d, *J* = 15.9 Hz, 1H), 6.45 (s, 1H), 5.90 (s, 1H), 5.83 (s, 1H), 5.55 (d, *J* = 15.6 Hz, 1H), 5.24 (t, *J* = 12.9 Hz, 1H), 4.17 (d, *J* = 1.8 Hz, 1H), 4.10-4.07 (m, 2H), 3.72 (s, 3H), 3.46-3.32 (m, 3H), 3.14-3.00 (m, 2H), 2.54 (d, *J =* 18 Hz, 1H), 2.32 (s, 3H), 2.05 (s, 6H), 1.89 (dd, *J₁* = 12 *Hz, J2* = 15.3 Hz, 1H).
¹³C NMR (75 MHz, CDCl₃) δ 165.7, 146.9, 145.1, 144.2,143.0, 140.8, 136.5, 134.5, 130.6, 129.4, 128.9, 127.9, 127.7, 120.8, 119.8, 117.8, 114.1, 112.9, 107.1, 100.8, 60.5, 59.2, 56.4, 56.0, 55.1, 41.4, 3 0.7, 25.5, 25.3, 15.5, 8.9.
ESI-MS m/z: Calcd. for C₃₅H₃₆N₄O₆: 608. 6. Found (M+1)⁺: 609.2.

### Example 120

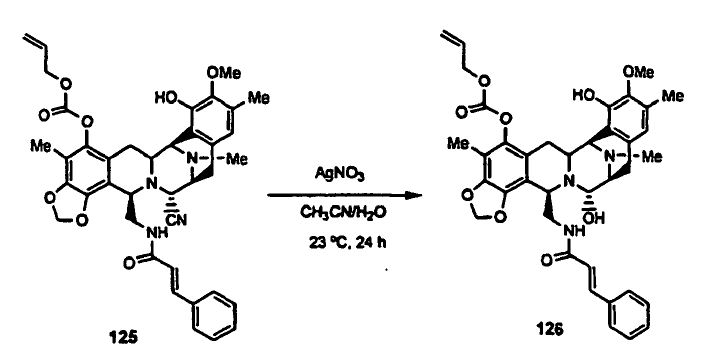

To a solution of 125 (86 mg, 0.124 mmol) in CH₃CN/H₂O (1.5 mL/1 mL). AgNO₃ (632 mg, 3.72 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (20 mL). The solution was extracted and the organic layer was dried over Na₂SO₄, filtered and concentrated *in vacuo.* The residue was purified by flash column chromatography (SiO₂, gradient EtOAc to EtOAc:MeOH 2:1) to afford **126** (70 mg, 83 %) as a white solid.
Rf: 0.07 (EtOAc:MeOH 5:1).
¹H NMR (300 MHz, CDCl₃) δ 7.40-7.28 (m, 5H), 7.25 (d, *J* = 15.6 Hz, 1H), 6.48 (s, 1H), 6.00-5.94 (m, 1H), 5.96 (s, 1H), 5.92 (s, 1H), 5.89 (s, 1H), 5.53 (d, *J* = 15.6 Hz, 1H), 5.42-5.36 (m, 2H), 5.31 (dd, *J*₁=1.2 Hz, *J*₂= 10.8 Hz, 1H), 4.71-4.65 (m, 2H), 4.51 (d, *J* = 3 Hz, 1H), 4.42 (bs, 1H), 4.07 (bs, 1H), 3.79 (dd, *J₁* = 6.9 Hz, *J2* = 12.9 Hz, 1H), 3.68 (s, 3H), 3.62-3.59 (m, 1H), 3.41-3.37 (m, 1H), 3.16 (d, *J =* 7.8 Hz, 1H), 2.95 (dd, *J*₁ = 7.5 Hz, *J*2 = 17.4 Hz, 1H), 2.88-2.83 (m, 1H), 2.43 (d, *J =* 18 Hz, 1H), 2.28 (s, 3H), 2.10 (s, 3H), 2.00 (s, 3H), 1.81 (dd, *J₁* = 11.7 Hz, *J2* = 15.3 Hz, 1H).
¹³C NMR (75 MHz, CDCl₃) δ 165.5, 152.9, 146.7, 144.5, 144.4, 142.7, 141.0, 140.0, 134.6, 131.4, 130.7, 129.2, 128.8, 128.5, 127.8, 127.7, 124.6, 121.2, 120.9, 118.9, 116.5, 114.9, 114.7, 111.3, 101.6, 93.3, 92.3, 83.2, 68.9, 60.6, 57.8, 56.8, 56.6, 56.3, 52.5, 52.2, 41.6, 26.1, 24.6, 15.6, 9.1.

ESI-MS m/z: Calcd. for C₃₈H₄₁N₃O₉: 683.7. Found (M-17)⁺: 666.3

### Example 121

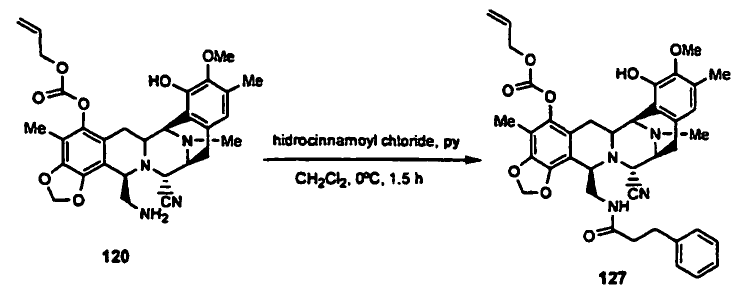

To a solution of **120** (1.61 g, 2.85 mmol) in CH₂Cl₂ (4 mL), hydrocinnamoyl chloride ( 423 µL, 2.85 mmol) and pyridine (230 µL, 2.85 mmol) were added at 0 °C. The reaction mixture was stirred for 1.5 h and then, the solution was diluted with CH₂Cl₂ (50 mL) and washed with 0.1 N HCl (30 mL). The organic layer was dried over Na₂SO₄, filtered, and the solvent was eliminated under reduced pressure. The residue was purified by flash column chromatography (SiO₂ gradient Hex:EtOAc 2:1 to EtOAc) to afford **127** (1.64 g, 83 %) as a white solid.
Rf: 0.63 (EtOAc:MeOH 5:1).
¹H NMR (300 MHz, CDCl₃) δ 7.26-7-14 (m, 3H), 7.04-7.01 (m, 2H), 6.44 (s, 1H), 6.07-5.99 (m, 1H), 5.97 (d, *J* = 1.5 Hz, 1H), 5.91 (d, *J =* 1.5 Hz, 1H), 5.75 (bs, 1H), 5.45 (dd, *J₁ =* 1.5 Hz, *J2* = 17.4 Hz, 1H), 5.36 (dd, *J₁* = 1.5 Hz, *J2* = 10.2 Hz, 1H), 5.03 (t, *J* = 5.7 Hz 1H), 5.74-5.66 (m, 2H), 4.09 (d, *J* = 2.4 Hz, 1H), 4.01 (bs, 1H), 3.97 (d, *J* = 2.7 Hz, 1H), 3.62 (dd, *J₁* = 8.4 Hz, *J*₂ = 13.5 Hz, 1H), 3.42 (s, 3H), 3.37-3.28 (m, 3H), 3.04-2.87 (m, 3H), 2.67-2.46 (m, 4H), 2.29 (s, 3H), 2.05 (s, 3H), 2.03 (s, 3H), 1.83-1.79 (m, 1H).
¹³C NMR (75 MHz, CDCl₃) δ 171.8, 152.8, 146.7, 144.5, 144.4, 142.7, 140.9, 140.8, 140.6, 131.4, 130.7, 128.9, 128.4, 128.2, 128.1, 126.0, 120.8, 120.4, 118.9, 117.6, 116.6, 113.0, 111.9, 101.6, 68.9, 60.3, 59.0, 56.3, 56.2, 55.6, 55.1, 41.6, 40.3, 37.7, 31.0, 25.9, 25.2, 15.5, 9.1.
ESI-MS m/z: Calcd. for C₃₉H₄₂N₄O₈: 694.3. Found (M+1)⁺: 695.3.

### Example 122

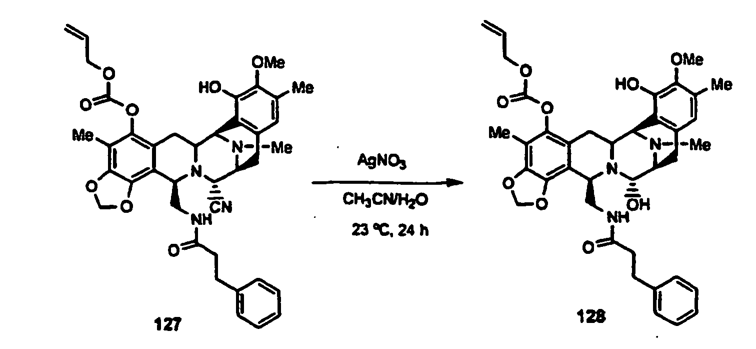

To a solution of 127 (50 mg, 0.072 mmol) in CH₃CN/H₂O (1.5 mL/1 mL), AgNO₃ (444 mg, 2.16 mmol) was added and the reaction was stirred at 23 °C for 24 h. Then, brine (10 mL) and Aq sat NaHCO₃ (10 mL) were added at 0 °C and the mixture was stirred for 15 min, filtered through a pad of celite and washed with CH₂Cl₂ (15 mL). The solution was extracted and the organic layer was dried over Na₂SO₄, filtered and concentrated *in vacuo.* The residue was purified by flash column chromatography (SiO₂, gradient EtOAc to EtOAc:MeOH 3:1) to afford **128** (30 mg, 61 %) as a white solid.
Rf: 0.65 (EtOAc:MeOH 5:1).
¹HNMR (300 MHz, CDCl₃) δ 7.22-7.11 (m, 3H), 7.06-7.03 (m, 2H), 6.43 (s, 1H), 6.08-5.98 (m, 1H), 5.96 (d, *J* = 1.5 Hz, 1H), 5.90 (d, *J* = 1.5 Hz, 1H), 5.66 (bs, 1H), 5.44 (dd, *J₁* = 1.5 Hz, *J2* = 17.4 Hz, 1H), 5.36 (dd, *J₁* = 1.5 Hz, *J2* = 10.5 Hz, 1H), 4.78-4.65 (m, 2H), 4.44 (d, *J* = 3 Hz, 1H), 4.3 6 (bs, 1H), 3.99 (td, *J1* = 2.1 Hz, *J2* = 9.9 Hz, 1H), 3.78-3.67 (m, 1H), 3.56 (dt, *J1* = 1.5 Hz, *J2* = 11.1 Hz, 1H), 3.43 (s, 3H), 3.30-3.12 (m, 2H), 3.02-2.89 (m, 1H), 2.83 (dd, *J1* = 2.7 Hz, *J2* = 15.9 Hz, 1H), 2.62-2.51 (m, 2H), 2.36 (d, *J* = 18.6 Hz, 1H), 2.27 (s, 3H), 2.02 (s, 3H), 2.00 (s, 3H), 1.86-1.66 (m, 3H).
¹³C NMR (75 MHz, CDCl₃) δ 171.6, 146.7, 141.2, 141.1, 131.5, 130.5, 128.9, 128.3, 128.2, 128.2, 125.9, 124.7, 121.1, 121.0, 118.8, 111.3, 101.6, 94.0, 83.2, 68.8, 60.3, 57.9, 56.6, 56.3, 52.3, 52.0, 41.7, 41.6, 41.1, 37.9, 31.1, 31.0, 26.1, 24.6, 15.5, 9.2.
ESI-MS m/z: Calcd. for C₃₈H₄₃N₃O₉: 685.7. Found (M-17)⁺: 668.3.

### Example 123

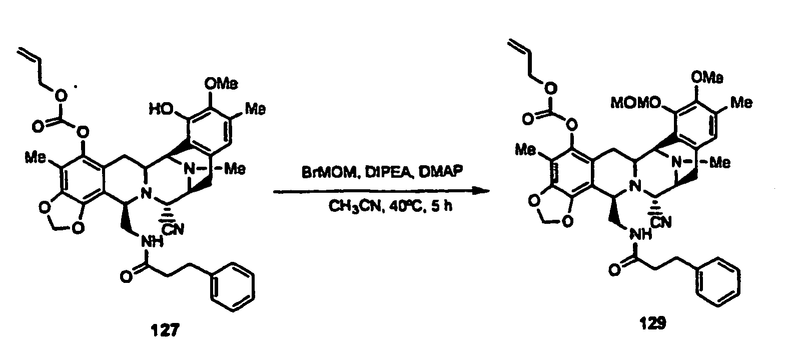

To a solution of **127** (1.64 g, 2.36 mmol) in CH₃CN (12 mL), diisopropylethylamine (8.22 mL, 47.2 mmol), bromomethyl methyl ether (2.89 mL, 35.4 mmol) and dimethylaminopyridine (29 mg, 0.236 mmol) were added at 0 °C. The reaction mixture was heated at 40°C for 5 h. Then, the reaction was diluted with CH₂Cl₂ (80 mL) and washed with 0.1 N HCl (3 x 25 mL). The organic layer was dried over Na₂SO_{4,} filtered, and the solvent was eliminated under reduced pressure to give **129** (1.46 g, 84 %) which was used in following steps with no further purification.
Rf : 0.24 (RP-18 CH₃CN-H₂O 8:2).
¹H NMR (300 MHz, CDCl₃) δ 7.27-7.11 (m, 3H), 7.05-7.02 (m, 2H), 6.67 (s, 1H), 6.08-5.98 (m, 1H), 5.96 (d, *J* = 1.2 Hz, 1H), 5.90 (d, *J* = 1.2 Hz, 1H), 5.44 (dd, *J1* = 1.2 Hz, *J2 =* 17.1Hz, 1H), 5.34 (dd, *J1* = 1.2 Hz, *J2* = 10.5 Hz, 1H), 5.05 (d, *J =* 6 Hz, 1H), 5.00 (d, *J*= 6 Hz,1H), 4.97 (t, *J* = 5.1 Hz, 1H), 4.75-4.68 (m, 2H), 4.16(d, *J* = 2.7 Hz, 1H), 3.98-3.97 (m, 1H), 3.68-3.67 (m, 1H), 3.65-3.61 (m, 1H), 3.52 (s, 3H), 3.35 (s, 3H), 3.32-3.26 (m, 3H), 3.05-2.86 (m, 3H), 2.59-2.48 (m, 2H), 2.30 (s, 3H), 2.02 (s, 3H), 1.94 (s, 3H), 1.91-1.67 (m, 3H).
¹³C NMR (75 MHz, CDCl₃) δ 171.4, 152.7, 148.5, 148.3, 144.5, 140.9, 140.8, 140.4, 131.1, 130.9, 130.4, 130.1, 128.4, 128.2, 126.0, 124.6, 123.7, 120.3, 119.0, 112.9, 111.8, 101.6, 99.1, 68.9, 59.4, 59.1, 57.5, 56.7, 56.3, 55.4, 55.1, 41.5, 40.2, 37.7, 30.9, 25.8, 25.2, 15.5, 9.0.
ESI-MS m/z: Calcd. for C₄₁H₄₆N₄O₉: 738.8. Found (M+ 23)⁺: 761.2.

### Example 124

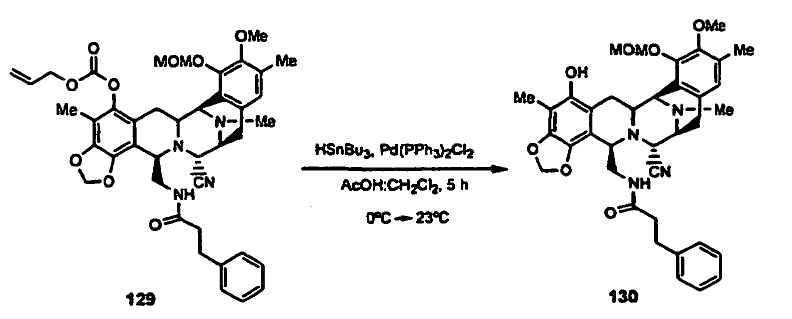

To a solution of **129** (1.46 g, 1.97 mmol) in CH₂Cl₂ (40 mL), acetic acid (450 µL), Pd(PPh₃)₂Cl₂ (138 mg, 0.197 mmol) and Bu₃SnH (1.06 mL, 3.95 mmol) were added at 0 °C. The reaction mixture was stirred for 5 h at 23°C and then, the solution was poured into a pad of flash column (SiO₂ gradient Hex:EtOAc 1:1 to EtOAc) to afford **130** (1.1 g, 85 %) as a white solid. Rf: 0.22 (Hex:EtOAc 1:2).
¹HNMR (300 MHz, CDCl₃,) δ 7.21-7.12 (m, 3H), 6.98-6.95 (m,2H), 5.86 (s. 1H), 5.84 (s, 1H), 5.79 (bs, 1H), 5.26 (d, *J* = 6 Hz, 1H), 5.11 (d, *J* = 6 Hz, 1H), 5.05 (t, *J* = 5.7 Hz, 1H), 4.19 (d, *J* = 2.4 Hz, 1H), 4.03 (d, *J* = 2.4 Hz, 1H), 3.99 (bs, 1H), 3.65 (s, 3H), 3.56 (s, 3H), 3.53-3.42 (m, 2H), 3.34 (d, *J* = 8.7 Hz, 1H), 3.27 (brd, *J* = 11.7 Hz, 1H), 3.11 (d, *J* = 15 Hz, 1H), 2.99 (dd, *J1* = 8.4 Hz, *J2* = 18.3 Hz, H), 2.64-2.52 (m, 3H), 2.29 (s, 3H), 2.08 (s, 3H), 2.06 (s, 3H), 1.84 (t, *J* = 7.8 Hz, 2H), 1.71 (dd, *J1* = 12.9 Hz, *J2* = 13.5 Hz, 1H).
¹³C NMR (75 MHz, CDCl₃) δ 171.7, 149.0, 147.6, 140.6, 132.1, 131.9, 130.9, 130.5, 128.5, 128.4, 128.3, 128.0, 126.0, 124.9, 124.6, 123.1, 117.6, 100.8, 99.6, 59.6, 58.9, 57.6, 56.6, 56.5, 55.6, 55.1, 41.5, 37.8, 31.5, 31.1, 25.9, 25.1, 22.6, 15.5, 8.8.
ESI-MS m/z: Calcd. for C₃₇H₄₂N₄O₇: 654.7. Found (M⁺+ Na): 655.1

### Example 125

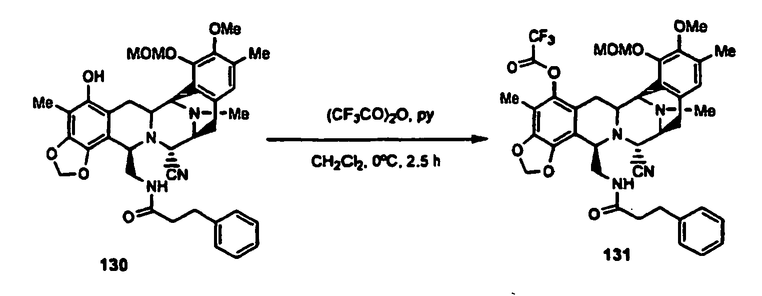

To a solution of **130** (130 mg, 0.198 mmol) in CH₂Cl₂ (1 mL), trifluoroacetyl anhydride (41.9 µL, 0.297 mmol) and pyridine (24 µL, 0.297 mmol) were added at 0 °C. The reaction mixture was stirred for 2.5 h and then, the solution was diluted with CH₂Cl₂ (10 mL) and washed with 0.1 N HCl (7 mL). The organic layer was dried over Na₂SO₄, filtered, and the solvent was eliminated under reduced pressure. The residue was purified by flash column chromatography (SiO₂, gradient Hex:EtOAc 4:1 to Hex:EtOAc 1:4) to afford **131** (93 mg, 62 %) as a white solid.
Rf: 0.30 (Hex:EtOAc 1:2).
¹H NMR (300 MHz, CDCl₃) δ 7.25-7.16 (m, 3H), 7.04-7.02 (m, 2H), 6.78 (s, 1H), 6.02 (d, J = 1.2 Hz, 1H), 5.95 (d, J = 1.2 Hz, 1H), 5.11 (d, J = 6.6 Hz, 1H), 4.98 (d, J = 6.6 Hz, 1H), 4.95 (t, J = 6.3 Hz, 1H), 4.61 (bs, 1H), 4.30 (s, 1H), 4.08 (s, 1H), 3.96 (d, J = 7.2 Hz, 1H), 3.66-3.54 (m, 1H), 3.50 (s, 3H), 3.39 (s, 3H), 3.19 (dd, J1 = 7.8 Hz, J2 = 18.3 Hz, 1H), 2.88 (d, J = 18.6 Hz, 1H), 2.79 (dd, J1 = 2.7 Hz, J2 = 15.9 Hz, 1H), 2.66-2.62 (m, 1H), 2.57 (s, 3H), 2.06 (s, 6H), 1.94-1.87 (m, 1H), 1.77-1.68 (m, 2H).
ESI-MS m/z: Calcd. for C₃₉H₄₁F₃N₄O₈: 750.7. Found (M+ Na)⁺: 751.2.

### Example 126

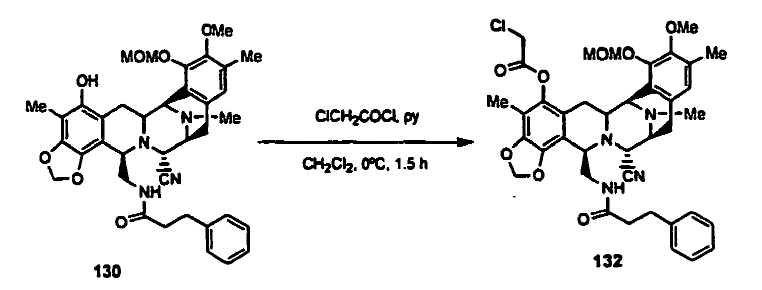

To a solution of **130** (130 mg, 0.198 mmol) in CH₂Cl₂ (2 mL), chloroacetyl chloride ( 23.65 µL, 0.297 mmol) and pyridine (24 µL, 0.297 mmol) were added at 0 °C. The reaction mixture was stirred for 1.5 h and then, the solution was diluted with CH₂Cl₂ (10 mL) and washed with 0.1 N HCl (7 mL). The organic layer was dried over Na₂SO₄. filtered, and the solvent was eliminated under reduced pressure. The residue was purified by flash column chromatography (SiO₂, gradient Hex:EtOAc 2:1 to Hex:EtOAc 1:1) to afford **132** (130 mg, 90 %) as a white solid.
Rf: 0.31 (Hex:EtOAc 1:2).
¹H NMR (300 MHz, CDCl₃) δ 7.24-7.15 (m, 3H), 7.07-7.05 (m, 2H), 6.69 (s, 1H). 6.00 (d, *J =* 1.5 Hz, 1H), 5.94 (d, *J =* 1.5 Hz,1H), 5.11 (d, *J* = 5.7 Hz, 1H), 5.04 (d, *J =* 5.7 Hz, 1H), 4.93 (m, 1H), 4.36 (s, 2H), 4.16 (d, *J* = 2.7 Hz, 1H), 4.01 (m, 2H), 3.64 (dd, *J1* = 6.9 *Hz, J2* = 12.3 Hz, 1H), 3.54 (s, 3H), 3.40 (s, 3H), 3.38-3.35 (m, 2H), 2.29 (dt, *J1* = 3 Hz, *J2* = 12 Hz, 1H), 3.03 (dd, *J1* = 7.8 Hz, *J2* = 18 Hz, 1H). 2.77 (dd, *J1* = 2.4 Hz, *J2* = 16.2 Hz, 1H), 2.58-2.52 (m, 3H), 2.32 (s, 3H), 2.02 (s, 3H), 1.92-1.85 (m, 1H), 1.76-1.65 (m, 2H).
¹³C NMR (75 MHz, CDCl₃) δ 171.6, 164.9, 148.3, 144.6, 140.9, 140.8, 139.8, 132.1, 131.9, 131.1, 130.0, 128.2, 126.0, 125.0, 124.6, 123.5, 120.1, 117.5, 113.0, 111.5, 101.7, 99.1, 64.9, 59.7, 58.9, 57.7, 56.6, 56.4, 55.2, 55.1, 41.5, 40.2, 39.9, 37.7, 30.9, 26.3, 25.1, 15.4, 9. 1.
ESI-MS m/z: Calcd. for C₃₉H₄₃ClN₄O₈: 730.2. Found (M+1)⁺: 731.1.

### Example 127

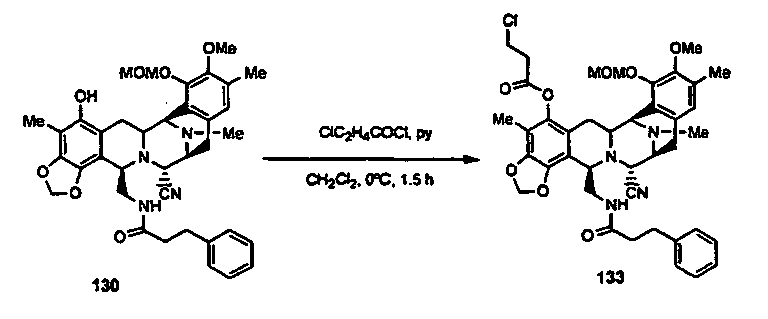

To a solution of **130** (130 mg, 0.198 mmol) in CH₂Cl₂ (2 mL), chloropropionyl chloride ( 28.35 µL, 0.297 mmol) and pyridine (24 µL, 0.297 mmol) were added at 0 °C. The reaction mixture was stirred for 1.5 h and then, the solution was diluted with CH₂Cl₂ (10 mL) and washed with 0.1 N HCl (7 mL). The organic layer was dried over Na₂SO₄, filtered, and the solvent was eliminated under reduced pressure. The residue was purified by flash column chromatography (SiO₂, Hex:EtOAc 1:1) to afford **133** (94 mg, 64 %) as a white solid.
Rf: 0.43 (Hex:EtOAc 1:2).
¹H NMR (300 MHz, CDCl₃) δ 7.23-7.12 (m, 3H), 7.06-7.04 (m, 2H), 6.69 (s, 1H), 5.97 (s, 1H), 5.92 (s, 1H), 5.08 (d, *J* = 6 Hz, 1H), 5.00 (d, *J =* 6 Hz, 1H), 4.97 (m, 1H), 4.16 (bs, 1H), 4.00 (m, 1H), 3.88 (t, *J* = 6.9 Hz, 2H), 3.75 (t, *J* = 6.9 Hz, 2H), 3.59 (dd, *J1* = 6.3 Hz, *J2* = 12.3 Hz, 1H), 3.53 (s, 3H), 3.37 (s, 3H), 3.03-3.26 (m, 1H), 3.17-2.97 (m, 3H), 2.83-2.73 (m, 2H), 2.58-2.52 (m, 3H), 2.31 (s, 3H), 2.03 (s, 6H), 1.93-1.86 (m, 1H), 1.79-1.64 (m, 2H).
¹³C NMR (75 MHz, CDCl₃) δ171.9,167.8,148.3, 144.7, 140.8, 132.1, 132.0,131.1,130.2, 128.2, 126.1, 125.2, 124.6, 123.7, 122.2, 120.2, 117.6, 114.7, 112.9, 111.8, 101.7, 99.3, 74.9, 65.0, 59.6, 59.0, 57.7, 56.7, 56.4, 55.4, 55.1, 41.5, 38.5, 37.8, 37.2, 31.0, 26.4, 25.2, 15.5, 9.3.
ESI-MS m/z: Calcd. for C₄₀H₄₅ClN₄O₈: 744.2. Found (M+1)⁺: 745.0.

### Example 128

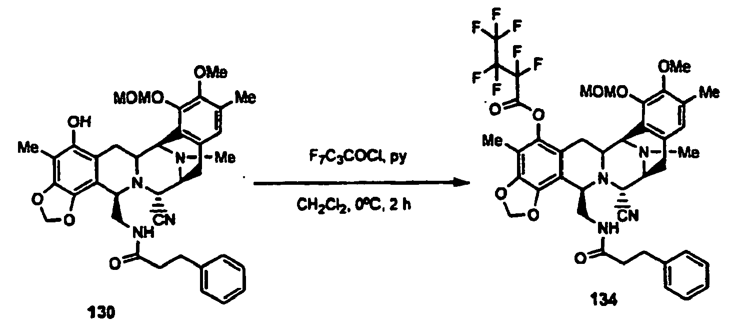

To a solution of **130** (160 mg, 0.244 mmol) in CH₂Cl₂ (2 mL), heptafluorobutyryl chloride (54.5 µL, 0.366 mmol) and pyridine (40 µL, 0.49 mmol) were added at 0 °C. The reaction mixture was stirred for 2 h and then, the solution was diluted with CH₂Cl₂ (15 mL) and washed with 0.1 N HCl (10 mL). The organic layer was dried over Na₂SO₄, filtered, and the solvent was eliminated under reduced pressure. The residue was purified by flash column chromatography (SiO₂, gradient Hex:EtOAc 2:1 to Hex:EtOAc 1:4) to afford **134** (120 mg, 63 %) as a white solid.
Rf: 0.40 (Hex:EtOAc 1:2).
¹H NMR (300 MHz, CDCl₃) δ 7.25-7.16 (m, 3H), 7.04-7.02 (m, 2H), 6.77 (s, 1H), 6.02 (d, *J* = 1.5 Hz, 1H), 5.96 (d, *J* = 1.5 Hz, 1H), 5.11 (d, *J* = 6.6 Hz, 1H), 4.95 (d, *J* = 6.6 Hz, 1H), 4.94 (m, 1H), 4.58 (m, 1H), 4.25 (bs, 1H), 4.06 (bs, 1H), 3.88 (d, *J* = 6.9 Hz, 1H), 3.64 (dd, *J1* = 7.5 Hz, *J2* = 12.9 Hz, 1H), 3.55-3.53 (m, 1H), 3.49 (s, 3H), 3.38 (s, 3H), 3.17 (dd, *J1* = 8.1 Hz, *J2* = 18.9 Hz, 1H), 2.85 (d, *J* = 18.3 Hz, 1H), 2.77 (dd, *J1* = 2.7 Hz, *J2* = 16.2 Hz, 1H), 2.60-2.57 (m, 3H), 2.56 (s, 3H), 2.06 (s, 3H), 2.03 (s, 3H), 1.96-1.88 (m, 1H), 1.79-1.69 (m, 2H).
ESI-MS m/z: Calcd. for C₄₁H₄₁F₇N₄O₈: 850.7. Found (M+1)⁺: 851.3.

### Example 129

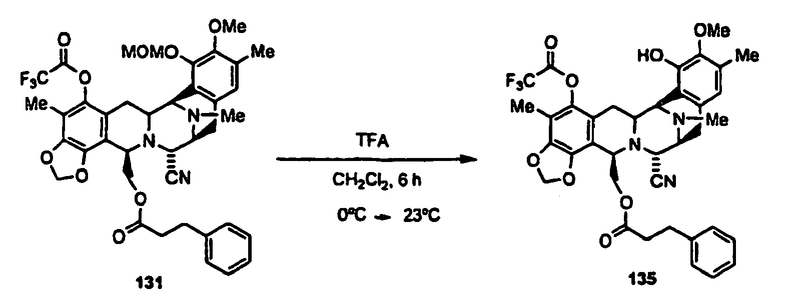

To a solution of **131** (93 mg, 0.123 mmol) in CH₂Cl₂ (1 mL), trifluoroacetic acid (381 µL, 4.95 mmol) was added at 0 °C and the reaction mixture was stirred for 6 h at 23°C. The reaction was quenched at 0°C with saturated aqueous sodium bicarbonate (15 mL) and extracted with ethyl acetate (2 x 10 mL). The combined organic layers were dried over sodium sulphate, filtered, and the solvent was eliminated under reduced pressure to give **135** (65 mg, 75 %) as a white solid which was used in following steps with no further purification. Rf: 0.26 (Hex:EtOAc 1: 1).
¹H NMR (300 MHz, CDCl₃) δ 7.24 - 7.15 (m, 3H), 7.04 - 7.01 (m, 2H), 6.45 (s, 1H), 6.03 (d, *J =* 1.5 Hz, 1H), 5.97 (d, *J =* 1.5 Hz, 1H), 5.62 (s, 1H), 4.97 (m, 1H), 4.09 (d, *J* = 1.8 Hz, 1H), 4.03 (bs, 1H), 3.99 (d, *J* = 2.4 Hz, 1H), 3.73 (dd, J1= 7.5 Hz, *J2* = 12 Hz, 1H), 3.38 (s, 3H), 3.34 - 3.28 (m, 3H), 3.05 (dd, J1= 8.4 Hz, *J2* = 18.3 Hz, 1H), 2.75 (dd, J1= 3.3 Hz, *J2* = 16.5 Hz, 1H), 2.60 - 2.47 (m, 3H), 2.30 (s, 3H), 2.05 (s, 3H), 2.02 (s, 3H), 1.91 - 1.65 (m, 3H).
ESI-MS m/z: Calcd, for C₃₇H₃₇F₃N₄O₇: 706.2. Found (M+1)⁺: 707.2.

### Example 130

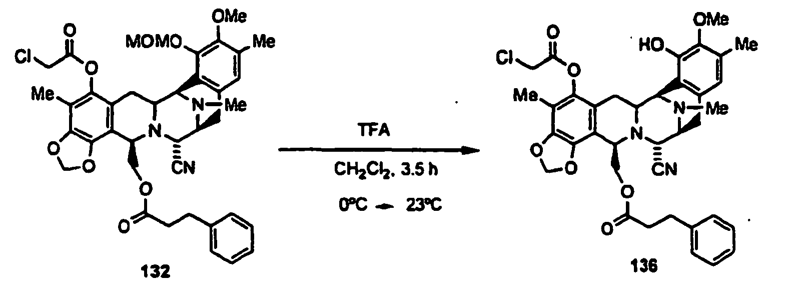

To a solution of **132** (130 mg, 0.177 mmol) in CH₂Cl₂ (1 mL), trifluoroacetic acid (545 µL, 7.08 mmol) was added at 0 °C and the reaction mixture was stirred for 3.5 h at 23°C. The reaction was quenched at 0°C with saturated aqueous sodium bicarbonate (15 mL) and extracted with ethyl acetate (2 x 10 mL). The combined organic layers were dried over sodium sulphate, filtered, and the solvent was eliminated under reduced pressure to give **136** (118 mg, 97 %) as a white solid which was used in following steps with no further purification. Rf: 0.27 (Hex:EtOAc 1:1).
H NMR (300 MHz, CDCl₃) δ 7.23 - 7.13 (m, 3H), 7.06 - 7.03 (m, 2H), 6.45 (s, 1H), 5.98 (d, *J =* 1.2 Hz, 1H), 5.91 (d, *J =* 1.2 Hz, 1H), 5.04 (t, *J =* 4.5 Hz, 1H), 4.37 (bs, 2H), 4.13 (d, *J* = 2.1 Hz, 1H), 4.03 (bs, 2H), 3.68 - 3.61 (dd, J1= 7.2 Hz, *J2* = 12.3 Hz 1H), 3.40 (s 3H), 3.37 - 3.28 (m, 3H), 3.02 (dd, J1= 8.4 Hz, *J2 =* 18.6 Hz 1H), 2.75 (dd, J1= 2.7 Hz, J2= 15.9 Hz 1H), 2.58 - 2.50 (m, 3H), 2.32 (s, 3H), 2.01 (s, 6H), 1.94 - 1.67 (m, 3H).
¹³C NMR (75 MHz, CDCl₃) δ 171.8, 165.0, 146.8, 144.6, 142.9, 141.0, 140.9, 139.8, 132.0, 130.3, 129.4, 128.5, 128.3, 126.0, 120.8, 120.1, 117.4, 116.1, 113.0, 111.5, 101.7, 60.5, 58.7, 56.3, 56.2, 55.2, 55.0, 41.5, 40.4, 39.5, 37.7, 31.0, 29.6, 26.4, 25.3, 15.5, 9.2.
ESI-MS m/z: Calcd. for C₃₇H₃₉CIN₄O₇: 686.2. Found (M+1)⁺: 687.2.

### Example 131

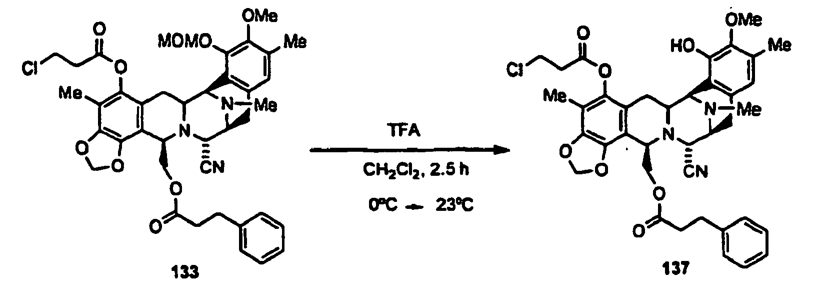

To a solution of **133** (94 mg, 0.126 mmol) in CH₂Cl₂ (1 mL), trifluoroacetic acid (385 µL, 5.0 mmol) was added at 0 °C and the reaction mixture was stirred for 2.5 h at 23°C. The reaction was quenched at 0°C with saturated aqueous sodium bicarbonate (15 mL) and extracted with ethyl acetate (2 x 10 mL). The combined organic layers were dried over sodium sulphate, filtered, and the solvent was eliminated under reduced pressure to give **137** (118 mg, 97 %) as a white solid which was used in following steps with no further purification. Rf: 0.24 (Hex:EtOAc 1:1).
¹H NMR (300 MHz, CDCl₃) δ 7.25-7.14 (m, 3H), 7.05-7.03 (m, 2H), 6.44 (s, 1H), 5.98 (d, *J* = 1.5 Hz, 1H), 5.92 (d, *J* = 1.5 Hz, 1H), 5.82 (s, 1H), 5.20 (t, *J* = 4.8 Hz, 1H), 4.07 (d, *J* = 2.1 Hz, 1H), 5.82 (s, 1H), 5.20 (t, *J* = 4.8 Hz, 1H), 4.07 (d, *J* = 2.1 Hz, 1H), 4.01 (bs, 1H), 3.98 (d, *J* = 2.4 Hz, 1H), 3.93-3.84 (m, 2H), 3.63 (ddd, *J1* = 1.5 Hz, *J2* = 6.9 Hz, *J3* = 12 Hz, 1H), 3.44 (bs, 3H), 3.37-3.26 (m, 3H), 3.11-3.06 (m, 2H), 3.01 (dd, *J1* = 8.4 Hz, *J2* = 18.3 Hz, 1H), 2.80 (brd, *J* = 13.8 Hz, 1H), 2.58-2.47 (m, 3H), 2.29 (s, 3H), 2.03 (s, 3H), 2.01 (s, 3H), 1.93-1.68 (m, 3H).
¹³CNMR (75 MHz, CDCl₃) δ 171.7, 168.0, 146.7, 144.6, 142.8, 142.1, 141.0, 140.8, 140.1, 130.7, 129.0, 128.2, 126.0, 122.2, 120.9, 116.7, 114.7, 113.1, 111.7, 102.3, 101.7, 72.0, 60.4, 59.1, 56.4, 56.3, 55.7, 55.2, 41.7, 40.3, 38.8, 37.8, 37.1, 31.0, 26.4, 25.2, 15.5, 9.4.
ESI-MS m/z : Calcd. for C₃₈H₄₁CIN₄O₇ : 700.2. Found (M+23)⁺: 723.1.

### Example 132

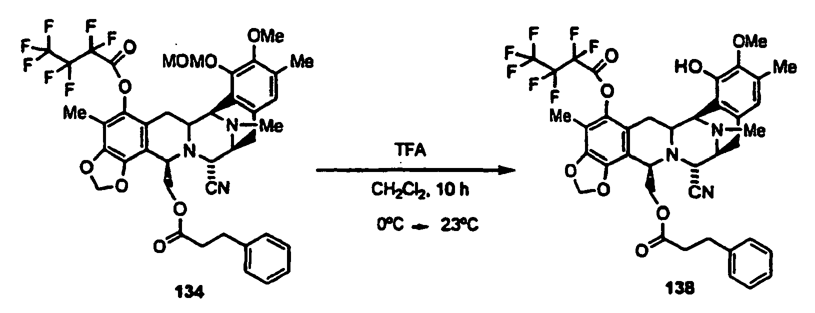

To a solution of **134** (46 mg, 0.054 mmol) in CH₂Cl₂ (1 mL), trifluoroacetic acid (166 µL, 2.16 mmol) was added at 0 °C and the reaction mixture was stirred for 10 h at 23°C. The reaction was quenched at 0°C with saturated aqueous sodium bicarbonate (15 mL) and extracted with ethyl acetate (2 x 10 mL). The combined organic layers were dried over sodium sulphate, filtered, and the solvent was eliminated under reduced pressure to give **138** (35 mg, 80 %) as a white solid which was used in following steps with no further purification. Rf: 0.26 (Hex:EtOAc 1:1).
¹H NMR (300 MHz, CDCl₃) δ 7.23 - 7.12 (m, 3H), 7.04 - 7.01 (m, 2H), 6.45 (s, 1H), 6.03 (d, *J* = 1.5 Hz, 1H), 5.97 (d, *J* = 1.5 Hz, 1H), 5.64 (s, 1H), 4.98 (m, 1H), 4.09 (d, *J* = 2.1 Hz, 1H), 4.03 (bs, 1H), 3.98 (d, *J* = 2.4 Hz, 1H), 3.75 (dd, J1 = 9.6 Hz, *J2* = 14.1 Hz, 1H), 3.35 (s, 3H), 3.29 - 3.24 (m, 3H), 3.04 (dd, J1= 7.8 Hz, *J2* = 18.0 Hz, 1H), 2.74 (dd, J1= 3.0 Hz, *J2* = 16.8 Hz, 1H), 2.57 - 2.45 (m, 3H), 2.30 (s, 3H), 2.03 (s, 6H), 1.92 - 1.64 (m, 3H). ESI-MS m/z : Calcd. for C₃₉H₃₇F₇N₄O₇ : 806.7. Found (M+1)⁺ : 807.3.

### Example 133

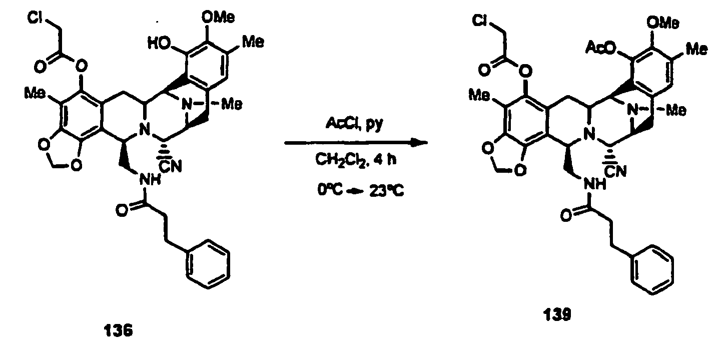

To a solution of **136** (45 mg, 0.065 mmol) in CH₂Cl₂ (0.3 mL), acetyl chloride (4.65 µL, 0.065 mmol), and pyridine (5.2 µL, 0.065 mmol ) were added at 0 °C. The reaction mixture was stirred for 4 h and then, the solution was diluted with CH₂Cl₂ (15 mL) and washed with 0.1 N HCl (7 mL). The organic layer was dried over sodium sulphate, filtered, and the solvent was eliminated under reduced pressure. The residue was purified by flash column chromatography (SiO₂, gradient Hex:EtOAc 5:1 to EtOAc) to afford **139** (27 mg, 57 %) as a white solid.
Rf: 0.36 (Hex:EtOAc 1:2).
¹H NMR (300 MHz, CDCl₃) δ 7.26 - 7.14 (m, 3H), 7.07 - 7.04 (m, 2H), 6.84 (s, 1H), 6.00 (d, *J*= 1.2 Hz, 1H), 5.94 (d, *J*= 1.2 Hz, 1H), 4.94 (t, *J*= 5.1 Hz, 1H), 4.39 - 4.38 (m, 2H), 4.02 (bs, 2H), 3.67 (d, *J*= 3 Hz, 1H), 3.60-3.54 (m, 1H), 3.47-3.35 (m, 3H), 3.42 (s, 3H), 3.26 (dt, *J₁* = 4.8 Hz, *J₂ =* 8.7 Hz 1H), 3.02 (dd, *J₁* = 8.1 Hz, *J₂* = 18.3 Hz, 1H), 2.64 - 2.38 (m, 3H), 2.35 (s, 3H), 2.25 (s, 3H), 2.06 (s, 3H), 2.03 (s, 3H), 1.95 - 1.69 (m, 3H).
ESI-MS m/z: Calcd. for C₃₉H₄₁ClN₄O₈: 729.2. Found (M+23)⁺: 752.3.

### Example 134

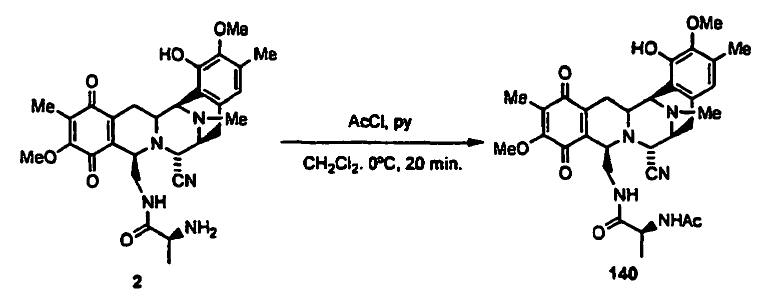

To a solution of **2** (15 mg, 0.0273 mmol) in CH₂Cl₂ (0.2 mL), acetyl chloride (1.94 µL, 0.0273 mmol), and pyridine (2.20 µL, 0.0273 mmol ) were added at 0 °C. The reaction mixture was stirred for 20 minutes and then, the solution was diluted with CH₂Cl₂ (15 mL) and washed with 0.1 N HCl (5 mL). The organic layer was dried over sodium sulphate, filtered, and the solvent was eliminated under reduced pressure. The residue was purified by flash column chromatography (SiO_{2,} gradient EtOAc to EtOAcMeOH 5:1) to afford **140** (9 mg, 56 %) as a light yellow solid. Rf: 0.56 (EtOAc:MeOH 5:1).
¹H NMR (300 MHz, CDCl₃) δ 6.52 (s, 1H), 6.40 (s, 1H), 5.73 (d, *J*= 7.5 Hz,1H), 4.95 (d, *J*= 6.9 Hz, 1H), 4.20 (d, *J*= 1.5 Hz, 1H), 4.00 (s, 3H), 3.86 (d, *J=* 4.5 Hz, 1H), 3.79 (s, 3H), 3.78-3.77 (m, 1H), 3.40-3.35 (m, 2H), 3.24 (dt, *J₁=* 3.6 Hz, *J₂=* 11.4 Hz, 1H), 3.17 (d, *J*= 7.8 Hz, 1H), 3.11 (d, *J=* 7.5 Hz,1H), 3.04 (dd, *J₁*= 3.6 *Hz, J₂=* 18.6 Hz, 1H), 2.92 (dt, *J₁=* 3.3 Hz, *J*₂= 14.1 Hz, 1H), 2.43 (d, *J*= 18.0 Hz, 1H), 2.37 (s, 3H), 2.29 (s, 3H), 1.89 (s, 3H), 1.79 (s, 3H), 1.75 (dd, *J₁=* 2.7 Hz, *J₂=* 6.9 Hz, 1H), 0.99 (d, *J*= 7.5 Hz, 3H).
ESI-MS m/z: Calcd. for C₃₁H₃₇N₅O₇: 591.6. Found (M+1)⁺: 592.3.

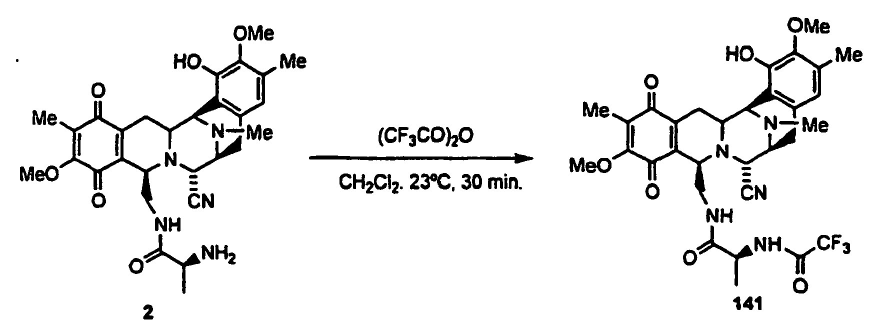

### Example 135

To a solution of **2** (15 mg, 0.0273 mmol) in CH₂Cl₂ (0.2 mL), trifluoroacetyl anhydride (3.85 µL, 0.0273 mmol was added at 23 °C. The reaction mixture was stirred for 30 minutes and then, the solution was diluted with CH₂Cl₂ (15 mL) and washed with 0.1 N HCl (5 mL). The organic layer was dried over sodium sulphate, filtered, and the solvent was eliminated under reduced pressure. The residue was purified by flash column chromatography (SiO₂, gradient EtOAc to EtOAcMeOH 4:1) to afford **141** (12.1 mg, 69 %) as a light yellow solid. Rf: 0.73 (EtOAc:MeOH 5:1).
¹HNMR (300 MHz, CDCl₃) δ 6.90 (d, *J=* 6.6 Hz, 1H), 6.56 (s, 1H), 5.11 (d, *J*= 6.6 Hz, 1H), 4.47 (bs, 1H), 4.23 (bs, 1H), 3.97 (s, 3H), 3.93 (bs, 1H), 3.85-3.81 (m, 1H), 3.77 (s, 3H), 3.40-3-36 (m, 2H), 3.23 (dd, *J₁=* 7.2 Hz, *J₂*= 18.6 Hz, 1H), 3.13-3.08 (m, 3H), 1.86 (s, 3H), 1.74 (dd, *J₁=* 10.8 Hz, *J₂=* 16.8 Hz, 1 H), 1.07 (d, *J*= 6.9 Hz, 3H).
ESI-MS m/z: Calcd. for C₃₁H₃₄F₃N₅O₇: 645.6. Found (M+1)⁺: 646.3.

### Example 136

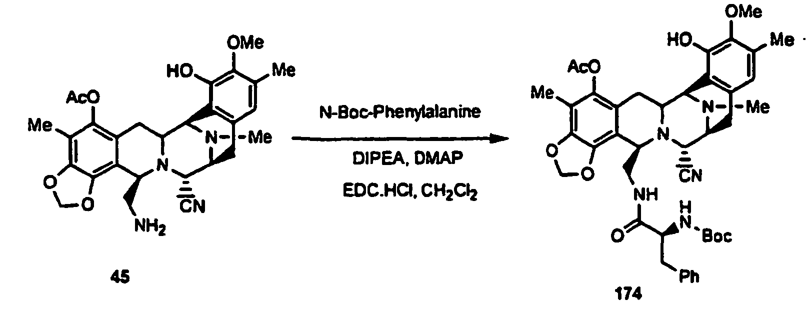

To a solution of **45** (30 mg, 0.058 mmol) in CH₂Cl₂ (0.87 mL), DIPEA (15.0 mL, 0.086 mmol), EDC·HCl (27.6 mg, 0.145 mmol), N-Boc-Phenylalanine (22.9 mg, 0.086mmol) and DMAP (0.7 mg, 0.006 mmol) were added at room temperature and the reaction mixture was stirred for 4h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (SiO₂, Hex: EtOAc 1:2) to afford **174** (17 mg, 38%) as a white solid.
Rf = 0.35 Hex:AcOEt 1:2.
¹H NMR (300 MHz, CDCl₃) 7.24-7.15 (m, 3H), 7.05-7.02 (m, 2H), 6.43 (s, 1H), 5.88 (s, 1H), 5.78 (s, 1H), 5.64 (s, 1H), 5.63 (bs, 1H), 4.80 (bs, 1H), 3.98 (s, 1H), 3.85 (bs, 2H), 3.75 (bs, 1H), 3.58 (bs, 1H), 3.53 (bs, 3H), 3.38 (m, 1H), 3.17-3.10 (m, 3H) 2.90 (dd,*J₁* = 8.7 Hz, *J₂=* 17.7 Hz, 1H), 2.73 (d, *J*= 14.4 Hz,1H), 2.57 (m, 1H), 2.43-2.37 (m, 1H), 2.25 (s, 3H), 2.24 (s, 3H), 2.10 (s, 3H), 1.94 (s, 3H), 1.76 (dd, *J₁* = 12.3 Hz,*J₂* = 15.6 Hz, 1H), 1.19 (bs, 9H). ¹³C NMR (75 MHz, CDCl₃) 171.2, 168.8, 146.6, 144.6, 142.8, 140.6, 137.0, 130.7, 129.5, 129.0, 128.4, 126.8, 121.1, 121.0, 117.8, 116.7, 113.3, 111.8, 101.5, 60.5, 59.7, 57.0, 56.4, 55.3, 41.9, 41.6, 38.7, 31.6, 29.7, 28.2, 26.5, 25.2, 22.6, 20.3, 15.7, 14.1, 9.3.
ESI-MS m/z: Calcd. for C₄₂H₄₉N₅O₉: 767.87. Found (M+1)⁺: 768.3.

### Example 137

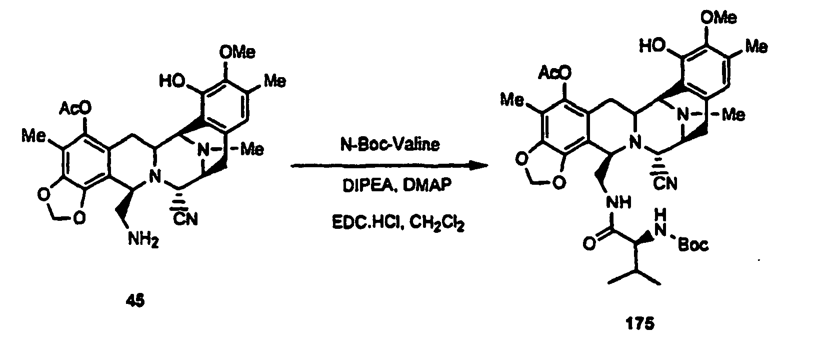

To a solution of **45** (30 mg, 0.058 mmol) in CH₂Cl₂ (0.87 mL), DIPEA (15.0 mL, 0.086 mmol), EDC-HCl (27.6 mg, 0.145 mmol), N-Boc-Valine (18.8 mg, 0.086 mmol) and DMAP (0.7 mg, 0.006 mmol) were added at room temperature and the reaction mixture was stirred for 4 h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (SiO₂. Hex: EtOAc 1:2) to afford **175** (18 mg, 43%) as a white solid.
Rf=0.25 Hex:EtOAc 1:1.
¹H NMR (300 MHz, CDCl₃) δ 6.42 (s, 1H), 5.97 (s, 1H), 5.82 (s, 1H), 5.73 (bs, 1H), 5.50 (bs, 1H), 4.82 (bs, 1H), 4.15 (bs, 1H), 4.03 (bs, 1H), 3.96 (bs, 1H), 3.72 (s, 3H), 3.61 (m, 1H), 3.41-3.15 (m, 3H), 2.96 (dd, *J₁* = 8.4 Hz, *J₂ =* 18.3 Hz, 1H), 2.72 (d,*J*= 16.5 Hz, 1H), 2.53 (d, *J=* 18 Hz, 1H), 225 (s, 3H), 2.21 (s, 3H), 1.93 (s, 3H), 1.8 1 (dd, *J₁* = 14.1 Hz, *J₂ =* 14.7 Hz, 1H), 1.34 (s, 9H), 0.83-0.76 (m, 2H), 0.61 (d, *J*= 6.3 Hz, 3H), 0.54 (d, *J*= 6.3 Hz, 3H).
¹³C NMR(75 MHz, CDCl₃) δ 171.6.168.7,155.4,146.8.144.5,142.9,140.7.130.7.128.8, 121.0,120.6.117.7,116.8,113.3,111.9,101.4,60.6,60.0,59.3,57.2,56.3,55.2,41.7,29.7, 29.3,28.2.26.2,25.2,22.6.20.3,18.9,17.7.15.7,14.1,9.3.
ESI-MS m/z: Calcd. for C₃₈H₄₉N₅O₉: 719.82. Found (M+1)⁺: 720.3.

### Example 138

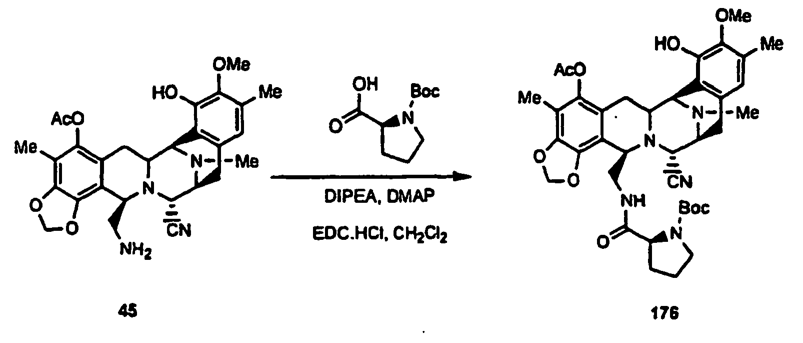

To a solution of **45** (38 mg, 0.073 mmol) in CH₂Cl₂ (1.09 mL), DIPEA (19.0 mL, 0.109 mmol), EDC·HCl (34.9 mg, 0.182 mmol), N-Boc-Proline (23.5 mg, 0.109 mmol) and DMAP (0.8 mg, 0.007 mmol) were added at 23 °C and the reaction mixture was stirred for 4.5 h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (SiO₂, Hex: EtOAc 1:1) to afford **176** (33 mg, 63%) as a white solid.
Rf = 0.14 Hex:EtOAc 1:2.
¹H NMR (300 MHz, CDCl₃) δ 6.49 (s, 1H), 6.02 (bs, 1H), 5.90 (s, 1H), 5.74 (s, 1H), 4.19 (bs, 1H), 4.09 (bs, 1H), 3.98 (bs, 1H), 3.76 (s, 3H), 3.38 (d, *J* = 6 Hz, 2H). 3.22 (d, *J =* 11.7 Hz, 1H), 3.15-2.99 (m, 2H), 2.80 (d, *J* = 15.3 Hz, 1H), 2.63-2.58 (m, 1H), 2.32 (s. 3H), 2.26 (s, 6H), 1.99 (s, 3H), 1.78-1.62 (m, 1H), 1.50-0.83 (m, 7H), 1.21 (s, 9H).
ESI-MS m/z: Calcd. for C₃₈H₄₇N₅O₉: 717.81. Found (M+1)⁺: 718.3.

### Example 139

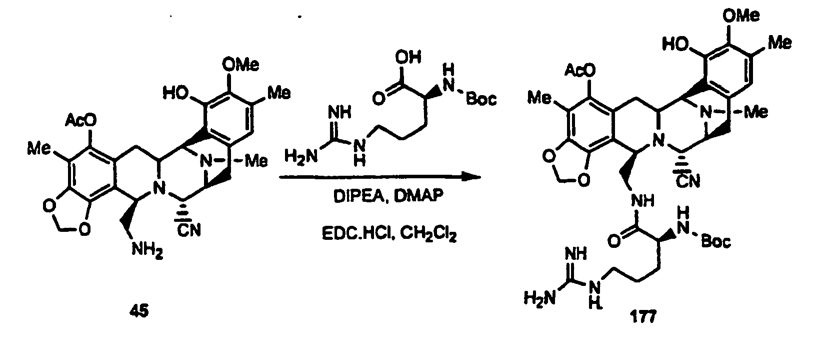

To a solution of **45** (50 mg, 0.144 mmol) in CH₂Cl₂ (0.96 mL), DIPEA (41.8 mL. 0.240 mmol), EDC·HCl (46.0 mg, 0.240 mmol), N-Boc-Arginine hidrochloride hydrate (47.2 mg, 0.144 mmol) and DMAP (1.1 mg, 0.01 mmol) were added at 23 °C and the reaction mixture was stirred for 4 h. Then, the solvent was removed under vacuum and the residue was purified by flash column chromatography (SiO₂ Hex: EtOAc 1:2) to afford **177** (58 mg, 78%) as a white solid.
Rf = 0.40 MeOH:EtOAc 1:5.
¹H NMR (300 MHz, CDCl₃) δ 7.53 (bs, 1H), 6.95 (bs, 3H), 6.54 (bs, 1H), 6.48 (s, 1H), 6.07 (s, 1H), 6.00 (bs, 1H), 5.88 (s, 1H), 5.11 (bs, 1H), 4.23 (s, 1H), 4.08 (s, 1H), 4.02 (s, 1H), 3.76 (s, 3H), 3.70 (bs, 1H), 3.48 (bs, 1H), 3.37 (d, *J* = 6.9 Hz, 1H), 3.18 (d,*J*= 10.2 Hz, 1H), 3.00-2.94 (m, 3H), 2.82-2.70 (m, 2H), 2.34 (s, 3H), 2.25 (s, 6H), 1.99 (s, 3H), 1.73 (brt, *J*= 14.1 Hz, 1H), 1.40 (s, 9H), 1.25 (bs, 3H), 0.95-0.85 (m, 2H).
ESI-MS m/z: Calcd. for C₃₉H₅₂N₈O₉: 776.88. Found (M+1)⁺: 777.3.

### Example 140

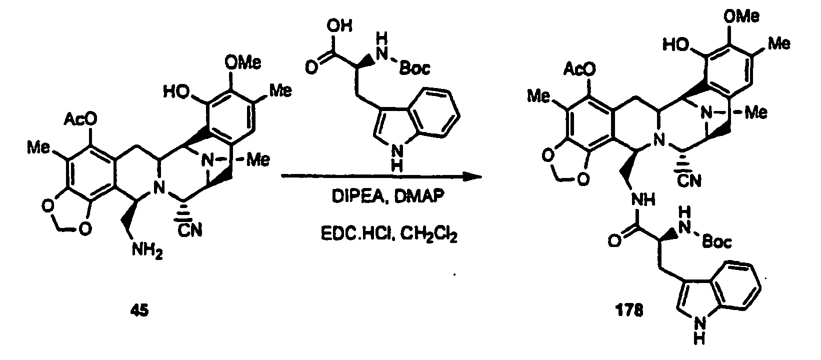

To a solution of **45** (50 mg, 0.096 mmol) in CH₂Cl₂ (1.44 mL), DIPEA (25.8 mL, 0.144 mmol), EDC·HCl (46.0 mg, 0.240 mmol), N-Boc-Tryptophan (43.8 mg, 0.144 mmol) and DMAP (1.2 mg, 0.009 mmol) were added at 23 °C and the reaction mixture was stirred for 4 h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (SiO₂, Hex: EtOAc 1:2) to afford **178** (57 mg, 74%) as a white solid.
Rf = 0.12 Hex:EtOAc 1: 1.
H NMR (300 MHz, CDCl₃) δ 8.50 (bs, 1H), 7.73-7.71 (m, 1H), 7.13-7.12 (m. 3H), 6.51 (s, 1H), 5.72 (s, 1H), 5.36 (bs,1H), 5.28 (bs,1H), 4.95 (bs, 1H), 4.41 (bs, 1H), 4.05 (s, 1H), 3.70 (s, 3H), 3.50 (bs, 2H), 3.30-3.17 (m, 4H), 2.89-2.82 (m, 3H), 2.40 (s, 3H), 2.29 (s, 3H) 2.19 (s, 3H), 2.03 (s, 3H), 1.49 (s, 9H), 1.26-1.25 (m, 2H).
ESI-MS m/z: Calcd. for C₄₄H₅₀N₆O₉: 806.90. Found (M+1)⁺: 807.3.

### Example 141

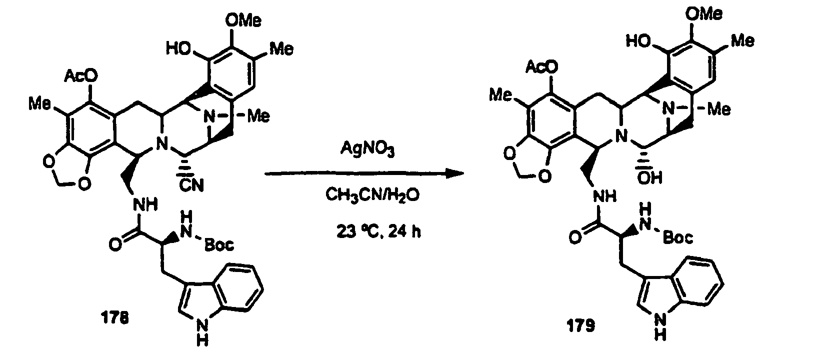

To a solution of **178** (43 mg, 0.053 mmol) in CH₃CN/H₂O (3 mL/2 mL), AgNO₃ (271 mg, 1.60 mmol) was added and the reaction was stirred at 23°C for 17 h. Then, Aq sat NaCl (10 mL) and Aq sat NaHCO₃ (10 mL) were added at 0°C and the mixture was stirred for 15 min, filtered through a pad of celite and washed with CH₂Cl₂ (20 mL). The solution was decanted and the organic layer was dried and concentrated *in vacuo.* The residue was purified by flash column chromatography (SiO₂, EtOAc:MeOH 5:1) to afford **179** (24 mg, 56%) as a white solid.
Rf= 0.38 EtOAc:MeOH 5:1.
¹H NMR (300 MHz, CDCl₃) δ 8.40 (s, 1H), 7.66 (bs, 1H), 7.25-7.21 (m, 1H), 7.16-7.09 (m, 2H), 6.45 (s, 1H), 5.75 (bs, 1H), 5.55 (bs, 1H), 5.45 (s, 1H), 5.25 (bs, 1H), 4.36 (bs, 1H), 4.16 (bs, 1H), 4.05 (bs, 1H), 3.95 (s, 1H), 3.69 (s, 3H), 3.35-3.02 (m, 6H), 2.83-2.73 (m, 3H), 2.35 (s, 3H), 2.24 (s, 3H), 2.19 (s, 3H), 1.99 (s, 3H), 1.77 (dd, *J*₁ = 12 Hz, *J*₂ = 15.3 Hz 1H). ESI-MS m/z: Calcd. for C₄₃H₅₁N₅O₁₀: 797.89. Found (M-17)⁺: 780.

### Example 142

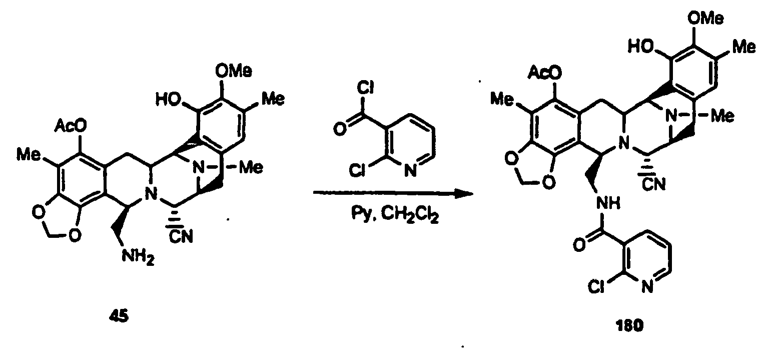

To a solution of 45 (50 mg, 0.0960 mmol) in CH₂Cl₂ (0.7 mL), 2-Chloronicotinoyl chloride (17.7 mg, 0.101 mmol) and pyridine (8.1mL, 0.101 mmol) were added at 0°C. The reaction mixture was stirred for 1.5 h and then, the solution was diluted with CH₂Cl₂ (5 mL) and washed with 0.1 N HCl (3 mL). The organic layer was dried over Na₂SO₄, filtered, and the solvent was eliminated under reduced pressure. The residue was purified by flash column chromatography (SiO₂, Hex: EtOAc 1:1) to afford **180** (45 mg, 71%) as a white solid.
Rf = 0.18 Hex:EtOAc 1:2.
¹H NMR (300 MHz, CDCl₃) δ 8.32-8.29 (m, 1H), 7.38-7.34 (m, 1H), 7.14-7.09 (m, 1H), 6.14 (s, 1H), 5.97 (d, *J*= 1.2 Hz, 1H), 5.92-5.91 (m, 2H), 5.75 (d, *J* = 2.1 Hz. 1H), 4.18 (d, *J* = 2.1 Hz, 1H), 4.15 (s, 1H), 4.07 (s, 1H), 3.91-3.73 (m, 2H), 3.68 (s, 3H), 3.36 (d, *J*= 7.5 Hz, 1H), 3.31 (dt, *J*₁ = 2.4 Hz, *J₂*= 11.7 Hz, 1H), 2.92 (dd, *J₁* = 8.1 Hz, *J₂* = 18 Hz, 1H), 2.80 (d, *J=* 16.2 Hz, 1H), 2.58 (d, *J*= 18 Hz, 1H), 2.31 (s, 3H), 2.27 (s, 3H), 1.99 (s, 3H), 1.91 (s, 3H) 1.97-1.83 (m, 1H).
¹³C NMR (75 MHz, CDCl₃) δ 168.6, 164.8, 150.3, 147.2, 146.5, 144.6, 142.5, 140.6, 139.0, 130.9, 130.5, 128.8, 122.3, 120.8, 120.3, 117.6, 116.3, 112.7, 112.1, 101.6, 60.6, 58.8, 56.5, 56.3, 55.6, 55.1, 41.6, 39.8, 31.5, 26.2, 24.9, 20.3, 15.5, 9.3.
ESI-MS m/z: Calcd. for C₃₄H₃₄ClN₅O₇: 659.2. Found (M+1)⁺: 660.1.

### Example 143

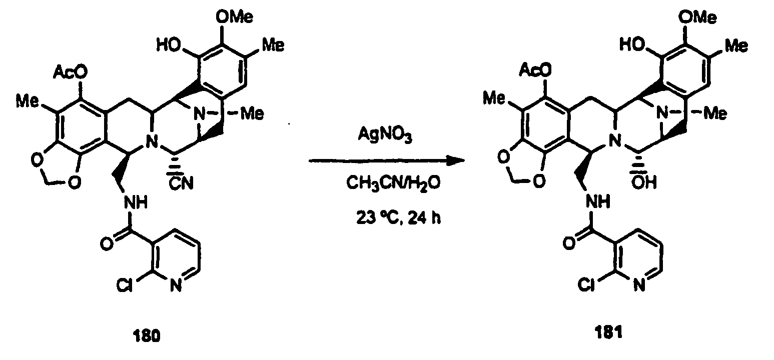

To a solution of **180** (39 mg, 0.059 mmol) in CH₃CN/H₂O (3 mL/2 mL), AgNO₃ (301 mg,1.77 mmol) was added and the reaction was stirred at 23°C for 17 h. Then, Aq sat NaCl (10 mL) and Aq sat NaHCO3 (10 mL) solutions were added at 0°C and the mixture was stirred for 15 min, filtered through a pad of celite and washed with CH₂Cl₂ (20 mL). The solution was decanted and the organic layer was dried and concentrated *in vacuo.* The residue was purified by flash column chromatography (SiO₂, EtOAc:MeOH 5:1) to afford **181** (28 mg, 73%) as a white solid.
Rf = 0.24, EtOAc:MeOH 5:1.
¹H NMR (300 MHz, CDCl₃) δ 8.33-8.31 (m, 1H), 7.40-7.35 (m, 1H), 7.16-7.09 (m, 2H), 6.20 (s, 1H), 5.98 (d,*J*= 1.2 Hz,1H), 5.96 (s, 1H), 5.92 (d, *J* = 1.2 Hz, 1H). 5.63 (bs, 1H), 4.60 (bs, 1H), 4.47 (bs, 1H), 4.02-3.95 (m, 2H), 3.69 (s, 3H), 3.65-3.56 (m, 1H), 3.48 (s, 3H), 3.43-3.38 (m, 1H), 3.17 (brd, *J*= 7.2 Hz, 1H), 2.88 (dd, *J₁* = 8.7 Hz, *J₂ =* 18.3 Hz, 1H), 2.74 (d, *J*= 15.3 Hz, 1H), 2.40 (d, *J*= 18.3 Hz, 1H), 2.32 (s, 3H), 2.26 (s, 3H), 2.00 (s, 3H), 1.99 (s, 3H), 1.77 (dd, *J₁* = 12 Hz, *J₂* = 15 Hz, 1H).
¹³C NMR (75 MHz, CDCl₃) δ 168.1, 165.0, 150.0, 147.2, 146.5, 144.4, 142.5, 140.9, 138.7, 131.5, 130.2, 128.9, 122.3, 121.1, 120.7, 116.1, 114.4, 111.4, 101.5, 82.6, 60.6, 57.8, 56.2, 52.1, 41.6, 31.5, 26.4, 24.5, 22.6, 20.3, 15.6, 14.1, 9.3.
ESI-MS m/z: Calcd. for C₃₃H₃₅ClN₄O₈: 650.2 Found (M-17)⁺: 633.3.

### Example 144

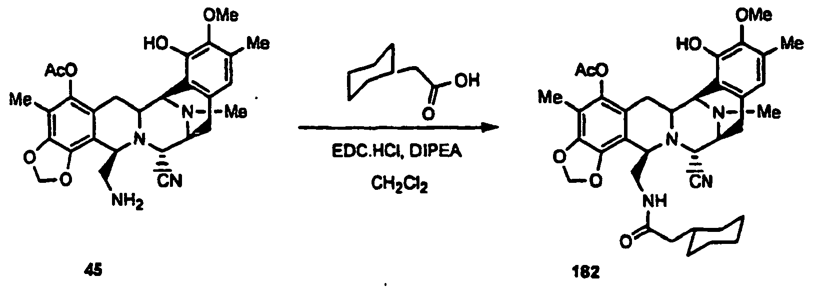

To a solution of **45** (30 mg, 0.058 mmol) in CH₂Cl₂ (0.87 mL). DIPEA (15.0 mL, 0.086 mmol), EDC·HCl (27.6 mg, 0.145 mmol), cyclohexylacetic acid (12.2 mg, 0.086 mmol) and DMAP (0.7 mg, 0.006 mmol) were added at 0°C and the reaction mixture was stirred for 5 h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (SiO₂, Hex: EtOAc 1:2) to afford **182** (10 mg, 27%) as a white solid.
Rf = 0.11 Hex:EtOAc 1:1.
¹H NMR (300 MHz, CDCl₃) δ 6.50 (s, 1H), 5.98 (d, *J*=1.2 Hz, 1H), 5.91 (d, *J*=1.2 Hz, 1H), 5.75 (s, 1H), 5.02-4.91 (m, 1H), 4.11 (bs, 1H), 4.04 (d, *J*= 2.1 Hz, 1H), 4.01 (bs, 1H), 3.78 (s, 3H), 3.72-3.69 (m, 1H), 3.38-3.29 (m, 3H), 3.05 (dd, *J₁* = 7.8 Hz, *J₂* = 18.0 Hz, 1H), 2.77 *(d,J=* 15.6 Hz, 1H), 2.54 (d, *J=* 18.6 Hz, 1H), 2.33 (s, 3H), 2.32 (s, 3H), 2.27 (s, 3H), 1.98 (s, 3H), 1.79 (dd, *J₁* = 11.7 Hz, *J₂*= 15.6 Hz, 1H), 1.59-0.61 (m, 13H).
ESI-MS m/z: Calcd. for C₃₆H₄₄N₄O₇: 644.76. Found (M+1)⁺: 645.3.

### Example 145

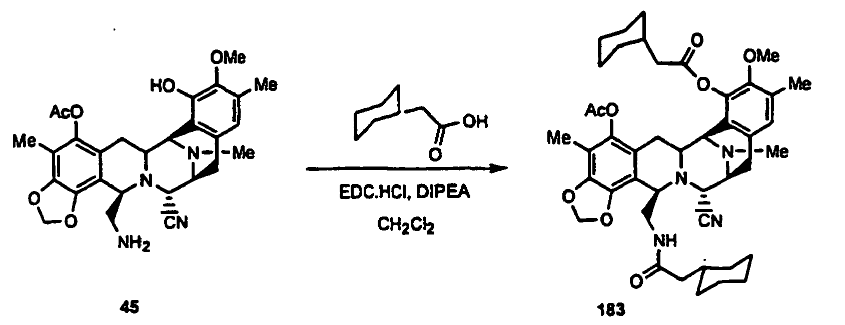

To a solution of **45** (30 mg, 0.058 mmol) in CH₂Cl₂ (0.87 mL), DIPEA (15.0 mL. 0.086 mmol), EDC·HCl (27.6 mg, 0.145 mmol), cyclohexylacetic acid (12.2 mg, 0.086 mmol) and DMAP (0.7 mg, 0.006 mmol) were added at 0°C and the reaction mixture was stirred for 5 h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (SiO₂ Hex: EtOAc 1:2) to afford **183** (17 mg, 38%) as a white solid.
Rf = 0.13 Hex:EtOAc 1:1.
¹H NMR (300 MHz, CDCl₃) δ 6.87 (s, 1H), 5.99 (d, *J*= 1.2 Hz, 1H), 5.92 (d, *J*= 1.2 Hz, 1H), 4.95 (t,*J*= 5.7 Hz, 1H), 4.08 (bs, 1H), 4.00 (bs, 1H), 3.71 (s, 3H), 3.64 (d, *J*= 1.8 Hz, 2H), 3.38 (d, *J*= 6.6 Hz, 1H), 3.33-3.32 (m, 1H), 3.27 (d, *J=* 11.7 Hz, 1H), 3.06 (dd, *J₁* = 7.8 Hz,*J₂* = 18.0 Hz, 1H), 2.65-2.59 (m, 1H), 2.50-2.47 (m, 1H), 2.35 (s, 3H), 2.27 (s, 6H), 1.99 (s, 3H), 1.78-1.74 (m, 1H) 1.60-0.62 (m, 26H).
ESI-MS m/z: Calcd. for C₄₄H₅₆N₄O₈: 768.94. Found (M+1)⁺: 769.3.

### Example 146

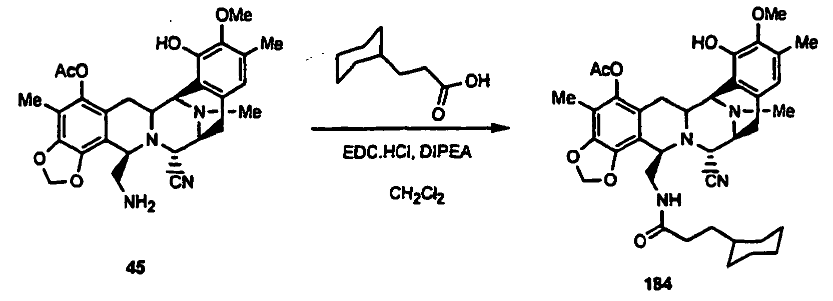

To a solution of **45** (30 mg, 0.058 mmol) in CH₂Cl₂ (0.87 mL), DIPEA (15.0 mL, 0.086 mmol), EDC.HCl (27.6 mg, 0.145 mmol), cyclohexylpropionic acid (13.5 mg, 0.086 mmol) and DMAP (0.7 mg, 0.006 mmol) were added at 0°C and the reaction mixture was stirred at 23 °C for 6 h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (SiO₂, Hex: EtOAc 1:2) to afford **184** (15 mg, 39%) as a white solid.
Rf = 0.15 Hex:EtOAc 1:1.
¹H NMR (300 MHz, CDCl₃) δ 6.50 (s, 1H), 5.98 (s, 1H), 5.91 (s, 1H), 5.74 (s, 1H), 5.01 (t, *J*= 5.1 Hz, 1H), 4.09 (bs, 1H), 4.06 (s, 1H), 4.02 (bs, 1H), 3.76 (s, 3H), 3.64-3.58 (m, 1H), 3.42-3.41 (m, 1H), 3.36 (d, *J* = 7.5 Hz, 1H), 3.28 (d, *J* = 12.3 Hz, 1H), 3.05 (dd, *J*₁ = 8.6 Hz, *J₂=* 18 Hz, 1H), 2.79 (d, *J*= 14.7 Hz, 1H), 2.57 (d, *J* = 18 Hz, 1H), 2.32 (s, 3H), 2.30 (s, 3H), 2.25 (s, 3H), 1.99 (s, 3H), 1.77 *(dd, J₁* = 12.0 Hz, *J₂* = 15.9 Hz, 1H), 1.62-0.71 (m, 15H). ESI-MS m/z : Calcd. for C₃₇H₄₆N₄O₇ : 658.78. Found (M+1)⁺: 659.3.

### Example 147

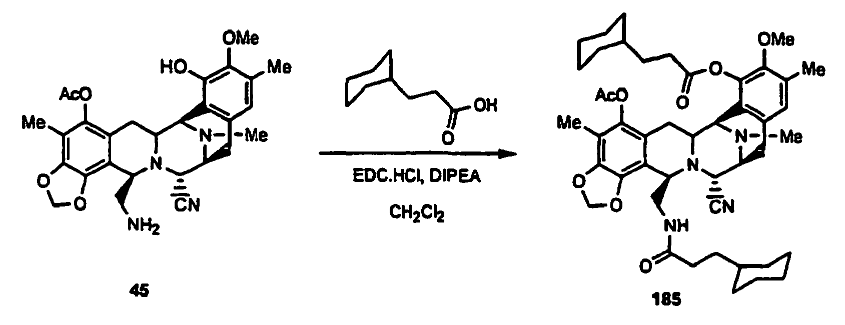

To a solution of **45** (30 mg, 0.058 mmol) in CH₂Cl₂ (0.87 mL), DIPEA (15.0 mL, 0.086 mmol), EDC.HCI (27.6 mg, 0.145 mmol), cyclohexylpropionic acid (13.5 mg, 0.086 mmol) and DMAP (0.7 mg, 0.006 mmol) were added at 0°C and the reaction mixture was stirred for 6 h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (SiO₂, Hex: EtOAc 1:2) to afford **185** (21 mg, 46%) as a white solid.
Rf = 0.17 Hex:EtOAc 1:1.
H NMR (300 MHz, CDCl₃) δ 6.86 (s, 1H), 5.99 (s, 1H), 5.92 (s, 1H), 4.97 (t, *J*= 5.4 Hz, 1H), 4.10 (d, *J*= 2.4 Hz, 1H), 4.01 (bs, 1H), 3.70 (s, 3H), 3.64 (d, *J*= 2.4 Hz, 1H), 3.51 (bs, 1H), 3.37 (d, *J*= 8.1 Hz, 1H), 3.23 (d, *J*=11.1 Hz, 1H), 3.02 (dd, *J*₁ = 7.8 Hz, *J₂* = 18 Hz, 1H), 2.69-2.59 (m, 4H), 2.35 (s, 3H), 2.26 (s, 6H), 2.00 (s, 3H), 1.76-0.72 (m, 30H).
¹³C NMR (75 MHz, CDCl₃) δ 173.1,171.5,169.2,147.9,144.7,142.5,140.7,140.3,130.9, 130.6, 127.7, 123.3, 120.0, 117.5, 113.1, 111.9, 101.6, 60.5, 59.0, 57.3, 56.7, 55.2, 55.0, 41.6, 39.9, 37.2, 33.5, 33.0, 32.9, 32.9, 32.8, 32.5, 32.4, 31.9, 31.7, 29.7, 29.3, 26.6, 26.5, 26.2, 24.9, 20.3, 15.8, 14.1, 9.4.
ESI-MS m/z: Calcd. for C₄₆H₆₀N₄O₈: 796.4. Found (M+1)⁺: 797.5.

### Example 148

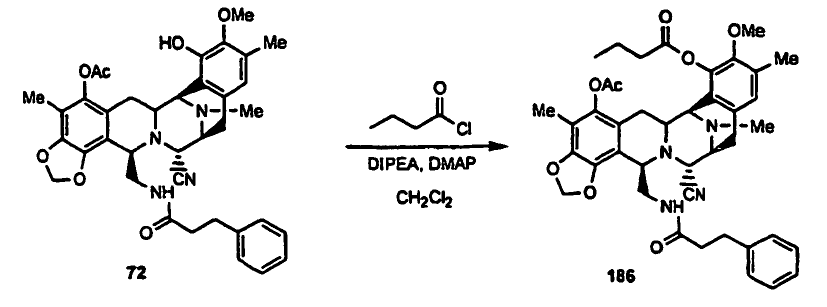

To a solution of 72 (111 mg, 0.162 mmol) in CH₂Cl₂ (0.81 mL), DIPEA (56.3 mL. 0.324 mmol), butyryl chloride (33.6 mL, 0.324 mmol) and DMAP (1.96 mg, 0.016 mmol) were added at 0 °C and the reaction mixture was stirred for 5 h at this temperature. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (RP-18, CH₃CN: H₂O 1:1) to afford **186** (65.4 mg. 54%) as a white solid.
Rf = 0.21 Hex:EtOAc 1:2.
¹H NMR (300 MHz, CDCl₃) δ 7.24-7.15 (m, 3H), 7.12-7.04 (m, 2H), 6.84 (s, 1H), 5.98 (d, *J* = 1.2 Hz, 1H), 5.92 (d, *J*=1.2 Hz, 1H), 4.97 (t, *J* = 5.7 Hz, 1H), 4.03 (m, 3H), 3.63 (d, *J* = 2.7 Hz, 1H), 3.50 (m, 2H), 3.44 (s, 3H), 3.37 (d, *J* = 8.4 Hz, 1H), 3.24 (dt, *J*₁ = 2.7 Hz, *J₂* = 11.7 Hz, 1H), 3.02 (dd, *J₁* = 8.1 Hz, *J₂* = 18.3 Hz, 1H), 2.65-2.54 (m, 7H), 2.35 (s, 3H), 2.25 (s, 3H), 2.07 (s, 3H), 2.02 (s, 3H), 1.87-1.75 (m, 3H), 1.08 (t, *J*= 7.5 Hz, 3H).
¹³C NMR (75 MHz, CDCl₃) δ 171.7, 170.8, 168.2, 147.8, 144.7, 142.5, 140.8, 140.6, 140.3, 131.1, 130.5, 128.3, 128.2, 127.6, 126.0, 123.2, 117.5, 112.9, 111.8, 101.6, 60.2, 59.0, 57.3, 56.6, 55.1, 54.9, 41.5, 39.9, 37.8, 36.0, 31.0, 26.5, 24.8, 22.6, 20.2, 18.5, 15.6, 13.7, 9.3.
ESI-MS m/z: Calcd. for C₄₁H₄₆N₄O₈: 722.83. Found (M+1)⁺: 723.2.

### Example 149

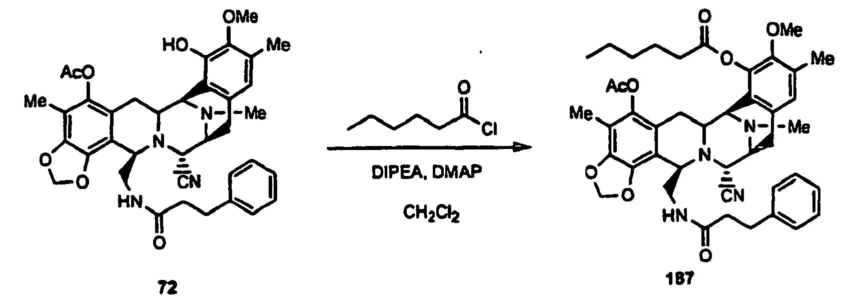

To a solution of **72** (80 mg, 0.122 mmol) in CH₂Cl₂ (0.61 mL), DIPEA (64.0 mL, 0.367 mmol), hexanoyl chloride (49.5 mL, 0.367mmol) and DMAP (1.50 mg. 0.012 mmol) were added at 0 °C and the reaction mixture was stirred at this temperature for 5h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (RP-18, CH₃CN: H₂O 6:4) to afford 187 (86.1 mg, 94%) as a white solid.
Rf= 0.25 Hex:EtOAc 1:2
¹H NMR (300 MHz, CDCl₃) δ 7.20-7.06 (m, 3H), 6.99-6.97 (m, 2H), 6.77 (s, 1H), 5.91 (s, 1H), 5.85 (s, 1H), 4.90 (m, 1H), 3.96 (d, J = 3 Hz, 2H), 3.57-3.55 (m, 1H), 3.43 (bs, 2H), 3.36 (bs, 3H), 3.29 (brd, *J =* 10.5 Hz,1 H), 3.18 (d, *J =* 11.7 Hz, 1H), 2.97 (dd, *J*₁= 4.8 Hz, *J₂* = 12 Hz, 1H), 2.58-2.46 (m, 6H), 2.28 (s, 3H), 2.18 (s, 3H), 2.00 (s, 3H), 1.95 (s, 3H), 1.86-1.66 (m, 7H), 1.41-1.38 (m, 2H), 0.86-0.81 (m, 3H).
¹³C NMR (75 MHz, CDCl₃) δ 171.7, 171.0, 168.2, 147.8, 144.7, 142.5, 140.8, 140.6, 140.3, 131.1, 130.5, 128.3, 128.2, 127.6, 126.0, 117.5, 112.9, 111.8, 101.6, 60.2, 59.0, 57.3, 56.6, 55.1, 55.0, 41.5, 39.9, 37.8, 34.1, 31.3, 31.1, 29.6, 24.8, 24.7, 22.3, 20.2, 15.6, 13.8.
ESI-MS m/z: Calcd. for C₄₃H₅₀N₄O₈: 750.88. Found (M+1)⁺: 751.3.

### Example 150

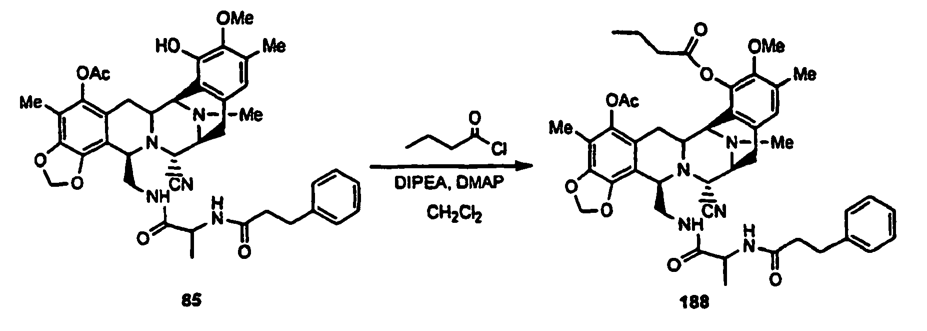

To a solution of 85 (80 mg, 0.110 mmol) in CH₂Cl₂ (0.55 mL), DIPEA (57.7 mL, 0.331 mmol), butyryl chloride (34.4 mL, 0.331mmol) and DMAP (1.30 mg, 0.011 mmol) were added at 0 °C and the reaction mixture was stirred at 23 °C for 5 h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (RP-18, CH₃CN: H₂O 1:1) to afford **188** (70.1 mg, 80%) as a white solid.
Rf = 0.54 MeOH:EtOAc 1:5.
¹H NMR (300 MHz, CDCl₃) δ 7.28-7.14 (m, 5H), 6.80 (s, 1H), 6.07 (d, *J*= 6.6 Hz, 1H), 6.00 (d, *J*= 1.5 Hz, 1H), 5.90 (d, *J* = 1.5 Hz, 1H), 5.35 (t, *J* = 5.4 Hz, 1H), 4.12 (d, *J*= 2.4 Hz, 1H), 4.05 (bs, 1H), 3.89 (brt, *J*= 6.9 Hz, 1H), 3.66 (s, 3H), 3.64-3.63 (m, 1H), 3.59-3.45 (m, 2H), 3.40 (brd, *J*= 7.8 Hz, 1H), 3.20 (dt, *J₁* = 2.7 Hz, *J*₂ = 12 Hz, 1H), 3.00 (dd, *J*₁ = 8.1 Hz, *J*₂=18Hz, 1H), 2.87 (t, *J =* 8.1 Hz, 2H), 2.71 (d, *J =* 18.6 Hz, 1H), 2.66-2.61 (m, 1H), 2.58 (t, *J=* 7.2 Hz, 2H), 2.41-2.35 (m, 2H), 2.33 (s, 3H), 2.23 (s, 3H), 2.21 (s, 3H), 2.00 (s, 3H), 1.90-1.77 (m, 3H), 1.08 (t, *J* = 7.2 Hz, 3H), 0.69 (d, *J =* 6.9 Hz, 3H).
¹³C NMR (75 MHz, CDCl₃) δ 172.0, 171.3, 170.8, 168.5, 147.7, 144.7, 142.5, 140.6, 140.5, 140.3,131.0,130.7,128.4,128.2,127.7,126.1,123.1,120.3,117.5,112.7,111.8,101.6, 60.3, 59.1, 57.3, 57.2, 55.4, 54.9, 48.2, 41.5, 39.5, 38.0, 36.0, 31.4, 26.8, 26.6, 24.6, 20.1, 18.5,18.1,15.7,13.7,9.2.
ESI-MS m/z: Calcd. for C₄₄H₅₁N₅O₉: 793.9. Found (M+1)⁺: 7943.

### Example 151

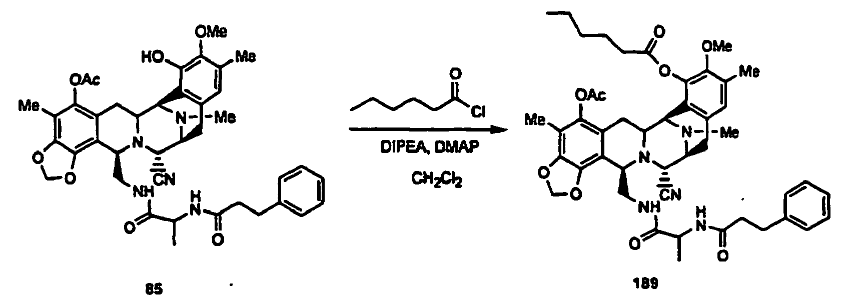

To a solution of **85** (80 mg, 0.110 mmol) in CH₂Cl₂ (0.55 mL), DIPEA (57.7 mL, 0.331 mmol), hexanoyl chloride (46.3 mL, 0.331mmol) and DMAP (1.30 mg, 0.011 mmol) were added at 0 °C and the reaction mixture was stirred at 23°C for 5 h. Then, the solution was diluted with CH₂Cl₂ (10 mL) and washed succesively with 0.1 N HCl (5 mL) and a solution of 10% NaHCO₃ (5 ml). The organic layer was dried over Na₂SO₄. filtered, and the solvent was eliminated under reduced pressure. The residue was purified by column chromatography (RP-18, CH₃CN: H₂O 1:1) to afford **189** (80 mg, 88%) as a white solid.
Rf= 0.23 Hex:EtOAc 1:3.
¹H NMR (300 MHz, CDCl₃) δ 7.21-7.08 (m, 5H), 6.74 (s, 1H), 6.00 (d,*J*= 6.9 Hz, 1H), 5.94 (d, *J*=1.5 Hz, 1H), 5.84 (*d*,*J*= 1.5 Hz, 1H), 5.24 (*t*,*J=* 5.4 Hz,1H), 4.06 (bs, 1H), 4.00 (bs, 1H), 3.83 (t, *J*= 6 Hz, 1H), 3.59 (s, 3H), 3.57 (m, 1H), 3.53-3.40 (m, 2H), 3.33 (d, *J* = 7.8 Hz, 1H), 3.14 *(d,J=* 11.7 Hz, 1 H), 2.94 (dd, *J₁* = 8.4 Hz, *J₂=* 18 Hz,1H), 2.81 (t, *J* = 7.5 Hz, 2H), 2.65 (d, *J*= 18 Hz, 1H), 2.60-2.54 (m, 1H), 2.52 (t, *J*= 7.2 Hz, 2H), 2.35-2.29 (m, 2H), 2.27 (s, 3H), 2.17 (s, 3H), 2.15 (s, 3H), 1.95 (s, 3H), 1.76-1.60 (m, 3H), 1.35-1.29 (m, 2H), 1.84 (m, 2H), 0.85-0.78 (m, 3H), 0.62 (t, *J*= 6.6 Hz, 3H).
¹³C NMR (75 MHz, CDCl₃) δ 172.0, 171.3,171.1, 168.4, 147.8, 144.8, 142.6, 140.7, 140.5, 131.2, 130.6, 128.4, 128.3, 127.7, 126.2, 123.1, 120.3, 117.5, 112.6, 112.0, 101.7, 60.4, 59.1, 57.4, 57.2, 55.4, 54.9, 48.3, 41.5, 39.6, 38.1, 34.1, 33.6, 31.5, 31.3, 26.7, 24.7, 22.3, 20.2, 18.2,15.7,13.9,9.3.
ESI-MS m/z: Calcd. for C₄₆H₅₅N₅O₉: 821.96. Found (M+1)⁺: 822.3.

### Example 152

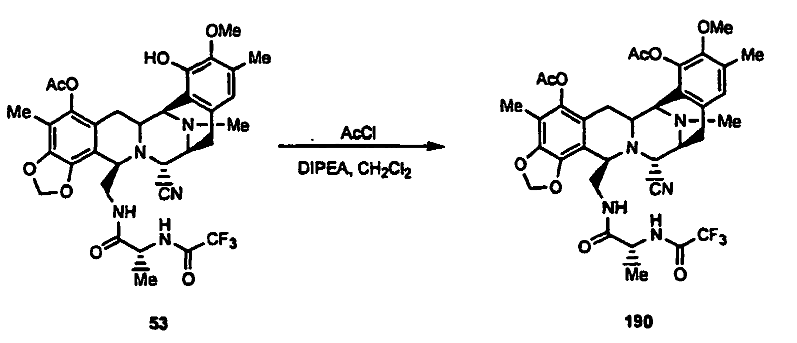

To a solution of **53** (100 mg, 0.145 mmol) in CH₂Cl₂ (0.72 mL), DIPEA (50.6 mL, 0.291 mmol) and acetyl chloride (20.7 mL, 0.291 mmol) were added at 0 °C and the reaction mixture was stirred for 4 h at 23 °C. Then, the solution was diluted with CH₂Cl₂ (10 mL), and washed successively with 0.1 N HCl (5 mL), and a solution of 10% NaHCO₃ (5 ml). The organic layer was dried over Na₂SO₄, filtered, and the solvent was eliminated under reduced pressure. The residue was purified by flash column chromatography (SiO₂, Hex: EtOAc 1:2) to afford **190** (27 mg, 25%) as a white solid.
Rf = 0.24 Hex:EtOAc 1:1.
¹H NMR (300MHz, CDCl₃) δ 6.82 (s, 1H), 6.02 (d, *J*= 0.9 Hz, 1H), 5.92 (d, *J* = 0.9 Hz, 1H), 5.30 (bs, 1H), 4.14 (d, *J*= 2.7 Hz, 1H), 4.10 (s, 1H), 3.90-3.73 (m, 2H), 3.68 (s, 3H), 3.67 (bs, 1H), 3.49 (bs, 1H), 3.42 (brd, *J*= 8.1 Hz, 1H), 3.24-3.20 (m, 1H), 3.01 (dd, *J₁*= 8.4 *Hz, J₂ =* 18.3 Hz, 1H), 2.78 (d, *J*=18 Hz, 1H), 2.64 (brd, *J*=15.6Hz, 1H), 2.36 (s, 3H), 2.34 (s, 3H), 2.24 (s, 3H), 2.20 (s, 3H), 2.02 (s, 3H), 1.77 (dd, *J₁* = 11.7 Hz, *J₂ =* 15.6 Hz, 1H), 0.65 (d, *J=* 6.6 Hz, 3H).
¹³C NMR (75 MHz, CDCl₃) δ 170.2, 168.6, 168.1, 167.6, 147.9, 144.9, 142.8, 140.5, 131.5, 131.0, 127.7, 123.2, 120.3, 117.5, 112.3, 112.2, 101.7, 60.4, 59.0, 57.4, 57.2, 55.2, 54.9, 48.6, 41.5, 39.1, 36.6, 29.7, 26.7, 24.6, 20.7, 20.2, 17.6, 15.5, 9.2,
ESI-MS m/z: Calcd. for C₃₅H₃₈F₃N₅O₉: 729.70. Found (M+1)⁺: 730.3.

### Example 153

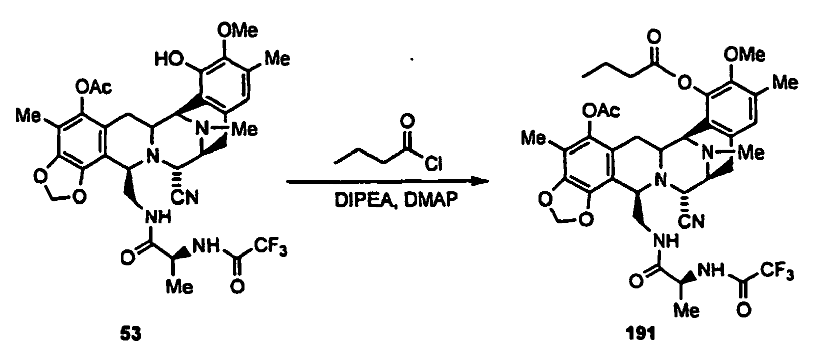

To a solution of **53** (150 mg, 0.218 mmol) in CH₂Cl₂ (1.09 mL). DIPEA (151.9 mL, 0.87 mmol), butyryl chloride (90.6 mL, 0.87 mmol) and DMAP (2.70 mg, 0.02 mmol) were added at 0 °C and the reaction mixture was stirred at 23 °C for 4h.. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (RP-18, CH₃CN: H₂O 4:1) to afford **191** (20.2 mg, 12%) as a white solid.
Rf = 0.3 Hex:EtOAc 1:1.
¹H NMR (300 MHz, CDCl₃) δ 6.81 (s,1H), 6.03 (d, *J=* 1.2 Hz, 1H), 5.92 (d, *J* = 1.2 Hz, 1H), 5.16 (t, *J* = 5.4 Hz, 1H), 4.13 (d, *J* = 2.1 Hz, 1H), 4.10 (bs, 1H), 3.87-3.82 (m, 1H), 3.80-3.74 (m, 1), 3.68 (s, 3H), 3.64 (d, *J*= 3 Hz, 1H), 3.52-3.47 (m, 1H), 3.42 (brd, *J* = 7.2 Hz, 1H), 3.24-3.20 (m, 1H), 3.02 (dd, *J₁* = 8.1 Hz, *J₂*=18.3 Hz, 1H), 2.77 (d, *J*=17.7 Hz, 1H), 2.64 (brd, *J*= 16.2 Hz, 1H), 2.58 (t, *J*= 7.2 Hz, 2H), 2.33 (s, 3H), 2.25 (s, 3H), 2.22 (s, 3H), 2.02 (s, 3H), 1.87-1.73 (m, 3H), 1.08 (t, *J*= 7.2 Hz, 3H), 0.68 (d, *J*= 6.6 Hz, 3H)
¹³C NMR (75 MHz, CDCl₃) δ 172.8, 172.1, 170.4, 157.8, 150.0, 146.9, 144.8, 142.6, 142.5, 133.3, 132.8, 129.6, 125.3, 122.3, 119.5, 118.4, 115.7, 114.3, 114.2, 103.8, 62.4, 61.0, 59.4, 59.2, 57.2, 57.0, 50.6, 43.6, 41.2, 38.1, 31.7, 28.7, 26.6, 22.2, 20.6, 19.7, 17.5, 15.7, 11.2.
ESI-MS m/z: Calcd. for C₃₇H₄₂F₃N₅O₉: 757.75. Found: 758.5 (M+1)⁺, 780.5 (M+23)⁺.

### Example 154

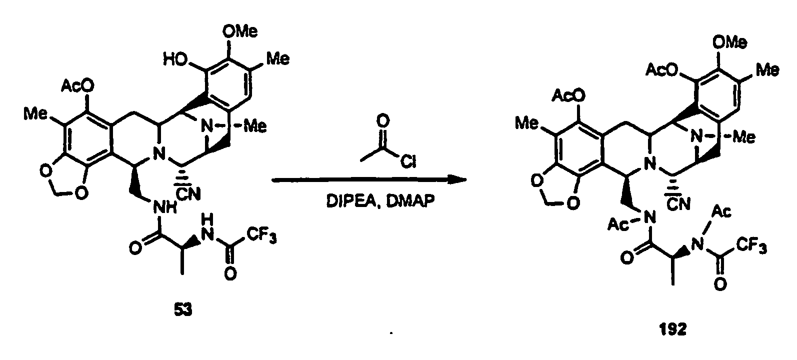

To a solution of **53** (150 mg, 0.218 mmol) in CH₂Cl₂ (1.09 mL), DIPEA (151.9 mL, 0.87 mmol), acetyl chloride (62.0 mL, 0.87 mmol) and DMAP (2.70 mg, 0.02 mmol) were added at 0 °C and the reaction mixture was stirred at 23 °C for 5 h. Then, the solution was diluted with CH₂Cl₂ (10 mL) and washed successively with 0.1 N HCl (5 mL) and a solution of 10% NaHCO₃ (5 ml). The organic layer was dried over Na₂SO₄. filtered, and the solvent was eliminated under reduced pressure. The residue was purified by flash column chromatography (RP-18, CH₃CN: H₂O 1:1) to afford 192 (111 mg, 62%) as a white solid.
Rf= 0.25 Hex:EtOAc 1:1.
¹H NMR (300 MHz, CDCl₃) δ 6.80 (s, 1H), 5.87 (s, 1H), 5.81 (s, 1H), 4.70 (dd, *J₁* = 2.4 Hz, *J*₂ = 9.9 Hz, 1H), 4.20 (d,*J*= 6.3 Hz,1H), 4.09 (s, 1H), 3.74 (s, 3H), 3.60 (s, H), 3.28 (d, *J* = 7.5 Hz,1H), 3.17 (d, *J* =12 Hz, 1H), 3.07 *(dd, J₁* = 7.2 Hz, *J₂* = 18.3 Hz,1H), 2.93 (d, *J=* 13.2 Hz, 1H), 2.66 (d, *J*= 15.3 Hz, 1H), 2.53 (d, *J*= 17.7 Hz, 1H), 2.47-2.20 (m, 1H), 2.37 (s, 1H), 2.33 (s, 3H), 2.26 (s, 3H), 2.24 (s, 3H), 2.08 (s, 3H), 2.00 (s, 3H), 1.96 (s, 3H), 1.72 (t, *J=* 14.4 Hz, 1H), 1.53 (d, *J*= 6.9 Hz, 3H).
¹³C NMR (75 MHz, CDCl₃) δ 174.1, 168.6, 168.4, 167*.*5*,* 147*.*7*,* 144.8, 142.2, 140.4, 131.1, 130.5, 126.9, 123.3, 120.4, 117.5, 112.4, 111.8, 101.1, 60.7, 60.6, 57.6, 57.2, 56.6, 55.3, 52.7, 48.3, 41.5, 31.6, 29.7, 26.4, 25.5, 23.0, 22.6, 20.7, 20.5, 20.2,17.8, 15.9, 14.1, 9.5.
ESI-MS m/z: Calcd. for C₃₉H₄₂F₃N₅O₁₁: 813.7. Found (M+1)⁺: 814.3.

### Example 155

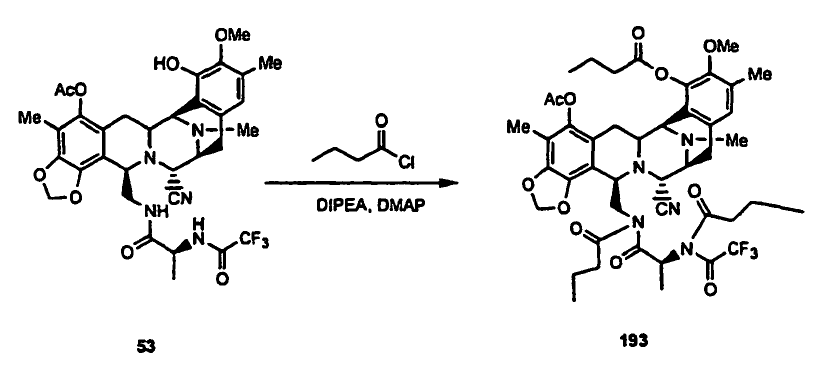

To a solution of **53** (150 mg, 0.218 mmol) in CH₂Cl₂ (1.09 mL), DIPEA (151.9 mL, 0.87 mmol), butyryl chloride (90.6 mL, 0.87 mmol) and DMAP (2.70 mg, 0.02 mmol) were added at 0 °C and the reaction mixture was stirred at 23 °C for 4h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (RP-18, CH₃CN: H₂O 4:1) to afford **193** (58 mg, 30%) as a white solid.
Rf = 0.38 Hex:EtOAc 1:1.
¹H NMR (300 MHz, CDCl₃) δ 6.85 (s, 1H), 5.99 (d,*J*= 1.2 Hz, 1H), 5.90 (d, *J*= 1.2 Hz, 1H), 5.47-5.42 (m, 2H), 4.09-4.08 (m, 2H), 3.69 (s, 3H), 3.66 (m, 1H), 3.41 (d, *J*= 7.5 Hz, 1H), 3.28-3.18 (m, 2H), 3.07 (dd, *J₁* = 8.1 Hz*, J₂* = 18 Hz,1H), 2.66 (d, *J*= 18.6 Hz, 1H), 2.61-2.39 (m, 3H), 2.34 (s, 3H), 2.26 (s, 3H), 2.21 (s, 3H), 2.01 (s, 3H), 1.95-1.79 (m, 6H), 1.72-1.59 (m, 6H) 1.09 (t, *J*= 7.5 Hz, 3H), 0.99-0.94 (m, 6H), 0.85 (d, *J*= 6.9 Hz, 3H).
¹³C NMR (75 MHz, CDCl₃) δ 171.2,170.7,169.1,168.4,148.1,145.0,142.7,140.9,140.6, 131.2,130.5,128.4,123.4,119.9,117.6,113.0,112.1,101.9,60.7,59.5,57.6,56.5,55.7. 55.2,41.8,41.4,36.3,35.8,29.9,27.0,25.3,20.5,20.0,18.8,18.3,15.8,14.0,13.8,13.4, 12.7, 9.6.
ESI-MS m/z: Calcd. for C₄₅H₅₄F₃N₅O₁₁: 897.93. Found (M+1)⁺: 898.3.

### Example 156

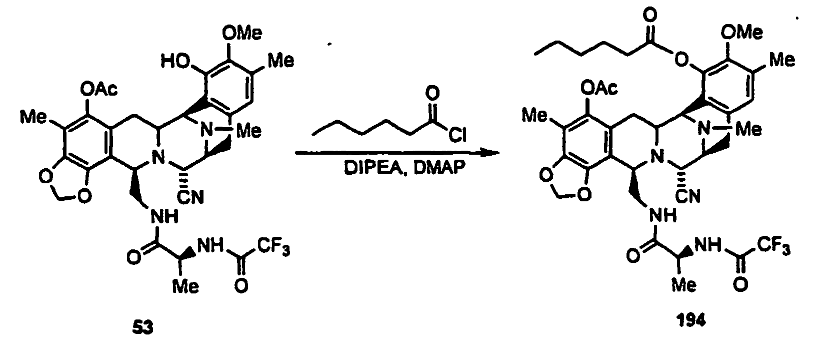

To a solution of **53** (150 mg, 0.218 mmol) in CH₂Cl₂ (1.09 mL), DIPEA (151.9 mL, 0.87 mmol), hexanoyl chloride (121.9 mL, 0.87 mmol) and DMAP (2.70 mg, 0.02 mmol) were added at 0 °C and the reaction mixture was stirred at 23 °C for 4h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (RP-18, CH₃CN: H₂O 4:1) to afford **194** (37.5 mg, 22%) as a white solid.
Rf = 0.32 Hex:EtOAc 1:1.
¹H NMR (300 MHz, CDCl₃) δ 6.80 (s, 1H), 6.02 (d, *J* = 1.2 Hz, 1H), 5.92 (d, *J*= 1.2 Hz, 1H), 5.22 (t, *J*= 5.7 Hz, 1H), 4.13 (d, *J*= 2.4 Hz, 1H), 4.09 (s, 1H), 3.88-3.81 (m, 1H), 3.80-3.71 (m, 1H), 3.67 (s, 3H), 3.64 (d, *J* = 3 Hz, 1H), 3.52-3.43 (m, 1H), 3.41 (brd, *J* = 6.6 Hz, 1H), 3.23-3.19 (m, 1H), 3.00 (dd, *J₁* = 8.7 Hz, *J₂=* 18.6 Hz, 1H), 2.77 (d, *J=*18Hz, 1H), 2.67-2.56 (m, 3H), 2.33 (s, 3H), 2.24 (s, 3H), 2.22 (s, 3H), 2.01 (s, 3H), 1.82-1.74 (m, 4H), 1.43-1.38 (m, 3H), 0.97-0.88 (m, 3H), 0.67 (d, *J*= 6.9Hz, 3H).
¹³C NMR (75 MHz, CDCl₃) δ 171.2, 170.3, 168.6, 148.2, 145.1, 143.0, 140.8, 140.7, 131.7, 131.1, 127.8, 123.5, 120.6, 117.7, 112.5, 102.0, 60.7, 59.2, 57.6, 57.4, 55.4, 55.2, 48.9, 41.8, 34.4, 31.8, 31.6, 29.9, 26.9, 25.0, 24.8, 22.9, 22.5, 20.4, 17.9, 15.8, 14.3, 14.1, 9.5.
ESI-MS m/z: Calcd. for C₃₉H₄₆F₃N₅O₉: 785.81. Found: 786 (M+1)⁺, 805.5 (M+23)⁺.

### Example 157

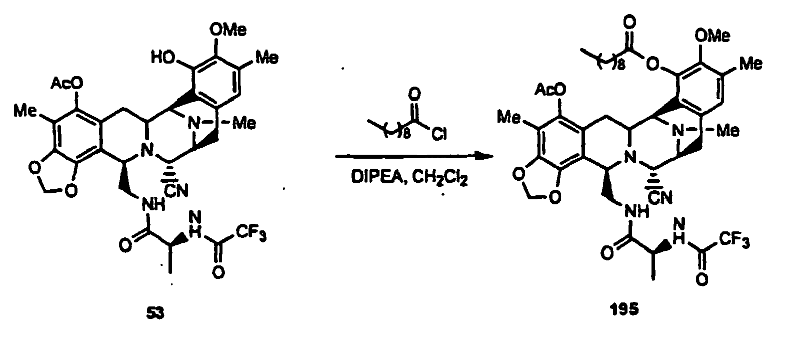

To a solution of **53** (150 mg, 0.218 mmol) in CH₂Cl₂ (1.09 mL), DIPEA (75.9 mL, 0.436 mmol), and decanoyl chloride ( 92.7mL, 0.436 mmol) were added at 0 °C and the reaction mixture was stirred at 23 °C for 4h. Then, the solution was diluted with CH₂Cl₂ (10 mL) and washed successively with 0.1 N HCl (5 mL), and a solution of 10% NaHCO₃ (5 ml). The organic layer was dried over Na₂SO₄, filtered, and the solvent was eliminated under reduced pressure. The residue was purified by flash column chromatography (RP-18, CH₃CN: H₂O 1:1) to afford **195** (75 mg, 41%) as a white solid.
Rf = 0.32 Hex:EtOAc 1:1.
¹H NMR (300 Hz, CDCl₃) δ 6.82 (s, 1H), 6.03 (d, *J=* 1.5 Hz, 1H), 5.93 (d, *J =* 1.5 Hz, 1H), 5.26 (bs, 1H), 4.15 (s, 1H), 4.11 (s, 1H), 3.89-3.75 (m, 2H), 3.68 (s, 3H), 3.65 (bs, 1H), 3.52-3.44 (m, 1H), 3.43 (d, *J* = 8.1 Hz, 1H), 3.22 (brd, *J* = 11.4 Hz, 1H), 3.03 (dd, *J₁*= 7.8 Hz, *J₂* = 17.4 Hz, 1H), 2.78 (d, *J=* 17.7 Hz, 1H), 2.69-2.56 (m, 3H), 2.34 (s, 3H), 2.26 (s, 3H), 2.23 (s, 3H), 2.03 (s, 3H), 1.83-1.74 (m, 3H), 1.83-1.74 (m, 12H), 0.90-8.88 (m, 3H), 0.68 (d, *J* = 6Hz, 3H).
¹³C NMR (75 Hz, CDCl₃) δ 171.0, 170.1, 168.4, 148.0, 144.8, 142.8, 140.5, 131.5, 130.8, 127.5, 123.3, 120.3, 117.5, 112.3, 112.2, 101.7, 60.4, 59.0, 57.4, 57.2, 55.1, 55.0, 48.6, 41.5, 39.1, 34.2, 31.8, 29.4, 29.2, 26.7, 25.0, 24.6, 22.6, 20.2, 17.6, 15.5, 14.0, 9.2.
ESI-MS m/z: Calcd. for C₄₃H₅₄F₃N₅O₉: 841.91. Found (M+1)⁺: 842.3.

### Example 158

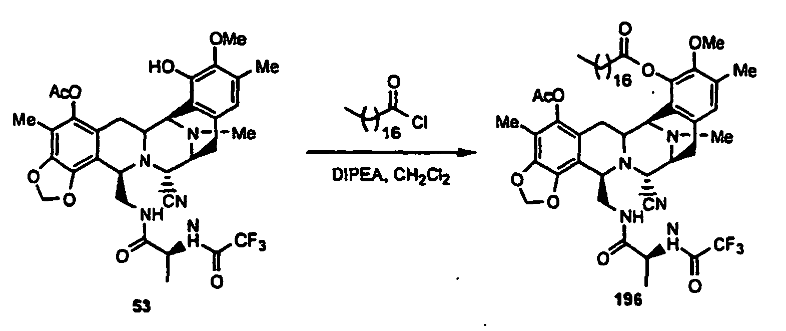

To a solution of **53** (150 mg, 0.218 mmol) in CH₂Cl₂ (1.09 mL), DIPEA (75.9 mL, 0.436 mmol), and stearoyl chloride (147.3 mL, 0.436 mmol) were added at 0 °C and the reaction mixture was stirred at 23 °C for 4h. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (RP-18, CH₃CN: H₂O 1:1) to afford **196** (86 mg, 41%) as a white solid.
Rf = 0.42 Hex:EtOAc 1:1.
¹H NMR (300 MHz, CDCl₃) δ 6.81 (s, 1H), 6.03 (s, 1H), 5.92 (s, 1H), 5.21 (bs, 1H), 4.14 (s, 1H), 4.10 (s, 1H), 3.88-3.74 (m, 2H), 3.67 (s, 3H), 3.64 (d, *J*= 3 Hz, 1H), 3.49 (brd, *J=* 14.7 Hz, 1H), 3.42 (d, *J* = 8.1 Hz, 1H), 3.22 (brd, *J =* 11.4 Hz, 1H), 3.02 (dd, *J₁* = 8.7 Hz, *J*₂= 18.6 Hz, 1H), 2.78 (d, *J*= 18Hz, 1H), 2.68-2.56 (m, 3H), 2.33 (s, 3H), 2.25 (s, 3H), 2.02 (s, 3H), 1.82-1.73 (m, 3H), 1.42-1.19 (m, 28H), 0.87 (t, *J*= 7.2 Hz, 3H), 0.67 (d, *J*= 6.6 Hz, 3H).
¹³C NMR (75 MHz, CDCl₃) δ 171.0, 170.2, 168.5, 147.9, 144.8, 142.8, 140.4, 131.4, 130.9, 127.5, 123.3, 120.4, 117.5, 112.4, 112.1, 101.7, 60.4, 58.9, 57.4, 57.2, 55.2, 55.0, 48.6, 41.5, 39.0, 34.2, 31.9, 29.7, 29.6, 29.4, 29.3, 29.2, 26.7, 25.1, 24.6, 22.7, 20.2, 17.6, 15.5, 14. 1, 9.2. ESI-MS m/z: Calcd. for C₅₁H₇₀F₃N₅O₉: 953.5. Found (M+1)⁺: 954.4.

### Example 159

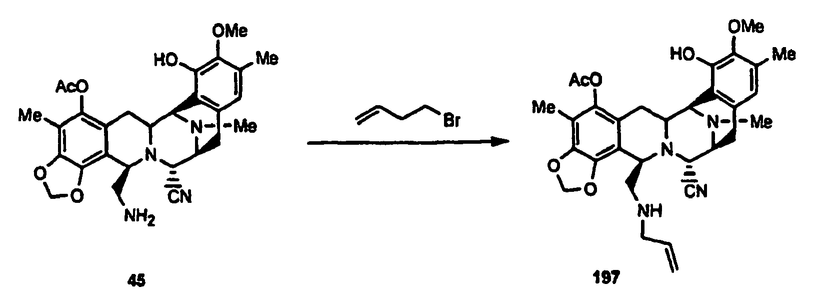

To a solution of **45** (10 mg, 0.019 mmol) in CH₂Cl₂ (0.095 mL), triethylamine (2.94 mL, 0.021 mmol) and allyl bromide (2.0 mL, 0.023 mmol) were added at 23 °C. The reaction mixture was stirred for 6 h and then, the solvent was removed under reduced pressure. The residue was purified by flash column chromatography (SiO₂, MeOH: EtOAc 1:5) to afford **197** (3.8 mg, 35%) as a white solid.
Rf = 0.19 EtOAc:MeOH 5:1.
¹H NMR (300 MHz, CDCl₃) δ 6.43 (s, 1H), 5.95 (s, 1H), 5.89 (s, 1H), 5.62-5.59 (m,1H), 4.94-4.84 (m,, 2H), 4.19 (s, 1H), 4.08 (s, 1H), 3.98 (t, *J* = 4.5 Hz, 1H), 3.76 (s, 3H), 3.32-3.26 (m, 2H), 3.07 (dd, *J₁* = 7.5 Hz, *J₂* = 17.4 Hz, 1H), 2.89 (d, *J*= 6 Hz, 2H), 2.80 (d, *J*= 3.9 Hz, 1H), 2.76 (d, *J*= 3.3 Hz, 1H), 2.57-2.52 (m, 2H), 2.33 (s, 6H), 2.24 (s, 3H), 1.99 (s, 3H), 1.88-1.79 (dd, *J₁* = 12.9 Hz, *J₂* = 15.9 Hz, 1H).
ESI-MS m/z: Calcd. for C₃₁H₃₆N₄O₆: 560.64. Found (M+1)⁺: 561.3.

### Example 160

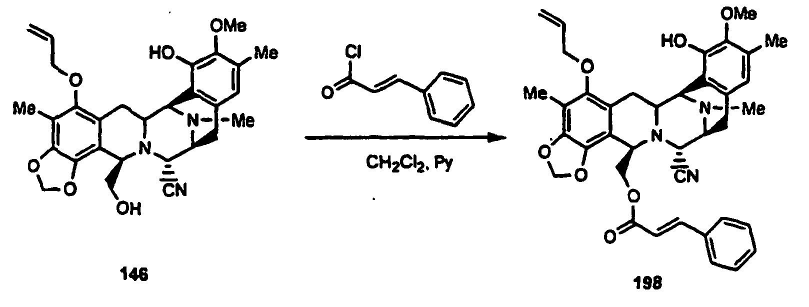

To a solution of 146 (50 mg, 0.096 mmol) in CH₂Cl₂ (0.96 mL), pyridine (11.7 mL, 0.144 mmol), and cinnamoyl chloride (24.0 mg, 0.144 mmol) were added at 23 °C and the reaction mixture was stirred for 18 h at that temperature. Then, the solution was diluted with CH₂Cl₂ (10 mL) and washed successively with 0.1 N HCl (5 mL) and a solution of 10% NaHCO₃ (5 ml). The organic layer was dried over Na₂SO₄, filtered, and the solvent was eliminated under reduced pressure. The residue was purified by flash column chromatography (SiO₂ Hex:EtOAc 1:2) to afford **198** (54 mg, 86%) as a white solid.
Rf = 0.45 Hex:EtOAc 1:1.
¹H NMR (300 MHz, CDCl₃) δ 7.41-7.37 (m, 6H), 6.38 (s, 1H), 6.19-6.03 (m, 1H), 6.08 (d, *J* = 15.9 Hz, 1H), 5.93 (d, *J* = 1.5 Hz, 1H), 5.88 (d, *J*= 1.5 Hz, 1H), 5.62 (s, 1H), 5.38 (dd, *J₁* = 1.5 Hz, *J₂* = 17.1 HZ, 1H), 5.26 (dd, *J₁* = 1.5 Hz, *J₂* = 10.5 Hz, 1H), 4.47 (dd, *J₁* = 3.6 Hz, *J₂* = 10.8 Hz, 1H), 4.23-4.11 (m, 5H), 3.89 (dd, *J₁* = 4.8 Hz, *J₂* = 11.1 Hz, 1H), 3.51 (s, 3H), 3.34 (brd,*J*= 8.4 Hz, 1H), 3.27-3.21 (m, 2H), 2.97 (dd, *J₁* = 7.8 Hz, *J₂* = 17.7 Hz, 1H), 2.28 (s, 3H), 2.15 (s, 3H), 2.04 (s, 3H), 1.91 (dd, *J₁* = 12 Hz, *J₂*= 15.6 Hz, 1H).
¹³C NMR (75 MHz, CDCl₃) δ 166.5, 148.8, 146.7, 144.7, 144.5, 142.7, 139.5, 134.4, 134.1, 131.1, 130.6, 129.1, 128.7, 128.2, 121.9, 121.2, 118.5, 117.8, 116.8, 112.9, 112.7, 101.5, 74.7, 65.2, 60.7, 60.6, 57.4, 56.8, 56.6, 55.7, 41.9, 31.8, 26.7, 25.5, 22.9, 15.9, 14.4, 9.7.
ESI-MS m/z: Calcd. for C₃₈H₃₉N₃O₇: 649.7. Found (M+1)⁺: 650.3.

### Example 161

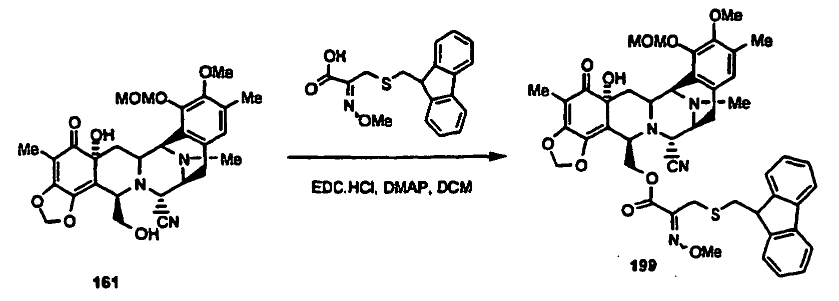

To a solution of 161 (78.5 mg, 0.146 mmol) and the cysteine derivative (81.1 mg, 0.247 mmol) in anhydrous CH2Cl2 (7.3 mL), DMAP (50 mg, 0.41 mmol) and EDC.HCl (78.1 mg, 0.41 mmol) were added at 23 oC. The reaction mixture was stirred at 23 oC under Argon atmosphere for 1.5 h. The mixture was diluted with CH2Cl2 (20 mL) and extracted with an aqueous saturated solution of sodium bicarbonate (25 mL). The aqueous phase was extracted with additional CH2Cl2 (20 mL) and the combined organic extracts were dried over Na2SO4, filtered and the solvent was eliminated under reduced pressure. The crude of the reaction was purified by flash column chromatography (inner diameter of the column 2 cm, height of silica 10 cm) with mixtures of ethyl acetate/hexane in a gradient manner, from 1:4 to 3:1 as eluent. Compound 199 (113 mg, 88%) was obtained as a pale yellow solid.
Rf = 0.36 Hex:EtOAc 1:1.
¹H NMR (300 MHz, CDCl₃) δ: 7.76 (d, *J =* 7.8 Hz, 2H), 7.63 (d, *J* = 7.8 Hz, 2H), 7.40 (t, J = 7.6 Hz, 2H), 7.29 (t, J = 7.6 Hz, 2H), 6.54 (s, 1H), 5.80 (s, 1H), 5.74 (s, 1H), 5.10 (d, J = 5.7 Hz, 1H), 5.08 (d, *J* = 5.7 Hz, 1H), 4.50 (dd, J = 4.9 Hz, J = 11.8 Hz, 1H), 4.20-4.05 (m, 4H), 4.02 (s, 3H), 3.81 (s, 3H), 3.61 (d, J = 13.8 Hz, 1H), 3.55 (d, *J* = 13.8 Hz, 1H), 3.50 (s, 3H), 3.21 (m, 1H), 3.06 (m, 1H), 3.00 (d, J = 6.0 Hz, 2H), 2.90 (dd, J = 8.9 Hz, J = 17.4 Hz, 1H), 2.79 (s, 1H), 2.56 (m, 1H), 2.50 (dd, J = 4.8 Hz, J = 14.9 Hz, 1H), 2.21 (s, 3H), 2.18 (s, 3H), 1.80 (s, 3H), 1.75 (m, 2H).
ESI-MS m/z: Calcd. for C₄₆H₄₈N₄O₁₀S: 848.3. Found: 849.3 (M+1)⁺, 871.3 (M+23)⁺. HPLC: Conditions: Column: Simmetry C18, Mobile phase: CH₃CN/H₂O in gradient from 50 to 100% in 25 minutes. ∅ = 1 mL/min, t= 40 °C. Retention time: 16.04 minutes. HPLC purity in area: 89.29%.

### Example 162

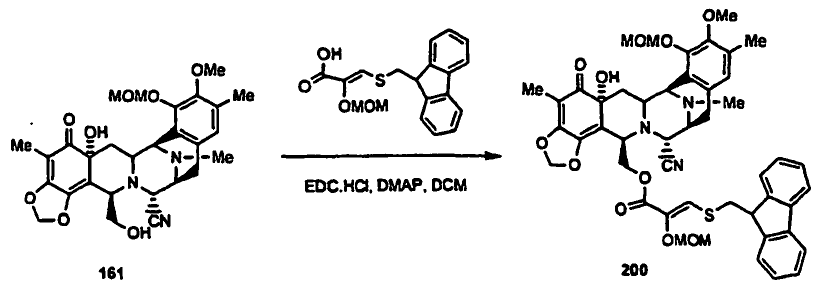

To a solution of **161** (80 mg, 0.148 mmol) and the cysteine derivative (76 mg, 0.223 mmol) in anhydrous CH₂Cl₂ (6.8 mL), DMAP (45 mg, 0.37 mmol) and EDC.HCl (71 mg, 0.37 mmol) were added at 23 °C. The reaction mixture was stirred at 23 °C under Argon atmosphere for 2.5 h Then, the mixture was diluted with CH₂Cl₂ (20 mL) and extracted with an aqueous saturated solution of sodium bicarbonate (25 mL). The aqueous phase was extracted with additional CH₂Cl₂ (20 mL) and the combined organic extracts were dried over Na₂SO₄, filtered and the solvent was eliminated under reduced pressure. The crude of the reaction was purified by flash column chromatography (inner diameter of the column 2 cm, height of silica 10 cm) with mixtures of ethyl acetate/hexane in gradient from 1:4 to 3:1 as eluent. Compound **200** (83 mg, 65%) was obtained as a pale yellow solid.
Rf = 0.5 Hex:EtOAc 1:1.
¹H NMR (300 MHz, CDCl₃) δ: 7.71 (m, 3H), 7.49 (d, *J* = 7.3 Hz, 1H), 7.36 (t, *J* = 7.3 Hz, 2H), 7.32- 7.23 (m, 2H), 6.65 (s, 1H), 5.80 (s, 1H), 5.79 (s, 1H), 5.13 (d, *J* = 6.1 Hz, 1H), 5.11 (d, *J* = 6.1 Hz, 1H), 5.05 (d, *J* = 6.1 Hz, 1H), 5.01 (d, *J* = 6.3 Hz, 1H), 4.76 (dd, *J =* 3.9 Hz, *J* = 11.9 Hz, 1H), 4.15- 4.03 (m, 4H), 3.96 (t, *J* = 4.0 Hz, 1H), 3.87 (s, 3H), 3.55 (s, 3H), 3.51 (s, 3H), 3.34-3.29 (m, 2H), 3.24 (dd, *J* = 5.5 Hz, *J* = 13.5 Hz, 1H), 3.03 (m, 1H), 2.97 (t, *J* = 7.5 Hz, 1H), 2.44-2.35 (m, 3H), 2.29 (s, 3H), 2.14 (s, 3H), 1.98 (dd, *J* = 8.06, *J* =15.1 Hz, 2H), 1.75 (s, 3H)
¹³C NMR (75 MHz, CDCl₃) δ 196.98, 161.13, 158.21, 149.01, 148.78, 145.05, 144.91, 141.01, 140.69, 140.07, 137.53, 132.76, 131.15, 129.41, 127.70, 127.67, 127.21, 126.83, 125.28, 125.05, 124.94, 122.51, 119.84, 119.73, 116.61, 110.26, 104, 57, 101.40, 99.23, 96.70, 70.25, 63.15, 60.40, 58.89, 57.52, 56.98, 56.72, 56.15, 55.06, 47.22, 41.37, 38.26, 35.22, 29.57, 25.34, 15.62, 7.26.
ESI-MS m/z: Calcd, for C₄₇H₄₉N₃O₁₁S: 863.97. Found: 865.0 (M+1)⁺, 887.1 (M+23)⁺. HPLC: Conditions: Column: Simmetry C18, Mobile phase: CH₃CN/H₂O in gradient from 50 to 100% in 25 minutes. Ø = 1 mL/min, t= 40 °C. Retention time: 15.36 minutes. HPLC purity in area: 91.56%.

### Example 163

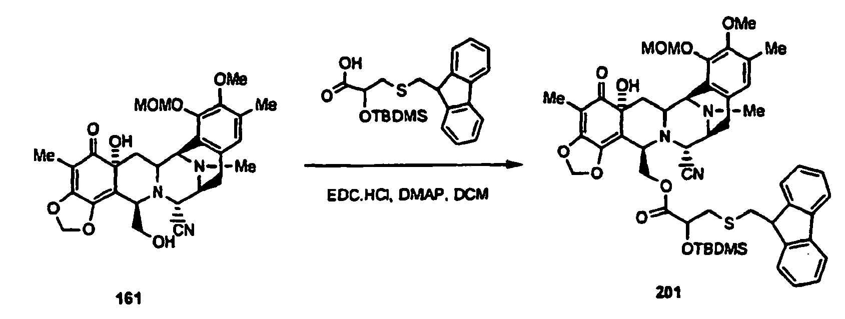

To a solution of **161** (418 mg, 0.77 mmol) and the cysteine derivative (321 mg, 0.77 mmol) in anhydrous CH₂Cl₂ (35 mL), DMAP (235 mg, 1.92 mmol) and EDC.HCl (369 mg, 1.92 mmol) were added at 23 °C and the reaction was stirred under Argon atmosphere for 2 h. The mixture was diluted with CH₂Cl₂ (20 mL) and extracted with an aqueous saturated solution of sodium bicarbonate (25 mL). The aqueous phase was extracted with additional CH₂Cl₂ (20 mL) and the combined organic extracts were dried over Na₂SO₄, filtered and the solvent was eliminated under reduced pressure. The crude of the reaction was purified by flash column chromatography (inner diameter of the column 3 cm, height of silica 11 cm) with mixtures of ethyl acetate/hexane in a gradient manner, from 1:3 to 3:1 as eluent. Compound **201** (372 mg, 52%) was obtained as a pale yellow solid.
Rf= 0.41 Hex:EtOAc 1:1.
¹H-RMN (CDCl₃, 300 MHz) δ 7.76-7.64 (m, 4H), 7.41-7.30 (m, 4H), 6.54 (s, 1H major isomer), 6.51 (s, 1H, minor isomer), 5.69 (s, 1H, minor isomer), 5.67 (s, 1H, major isomer), 5.60 (s, 1H minor isomer), 5.57 (s, 1H major isomer), 5.08 (s, 2H), 4.26 (t, *J* = 5.1 Hz, 1H minor isomer), 4.23 (t, *J=* 4.9 Hz, 1H major isomer), 4.07-4.03 (m, 3H), 3.98- 3.88 (m, 3H), 3.84 (s, 3H), 3.71 (dt, *J₁*= 5.6 Hz, *J*₂ = 10.0 Hz, 1H), 3.49 (s, 3H, major isomer), 3.49 (s, 3H, minor isomer), 3.40 (dt, *J₁* = 5.6 Hz, *J*₂ = 9.5 Hz, 1H), 3.18 (m, 3H), 3.11 (m, 1H). 2.91-2.82 (m, 1H), 2.48-2.28 (m, 2H), 2.24 (s, 3H), 2.16 (s, 3H, major isomer), 2.14 (s. 3H. minor isomer), 2.03 (s, 3H), 1.91 (dt, *J₁* = 8.8 Hz, *J₂* = 14.4 Hz, 1H), 1.76 (s, 3H, minor isomer). 1.76 (s, 3H major isomer), 0.85 (s, 9H minor isomer), 0.85 (s, 9H major isomer). 0.04 and 0.01 (s, 6H both isomers).
ESI-MS m/z: Calcd. for C₅₁H₆₁N₃O₁₀SSi: 935.4. Found: 936.4 (M+1)⁺, 958.3 (M+23)⁺.

### Example 164

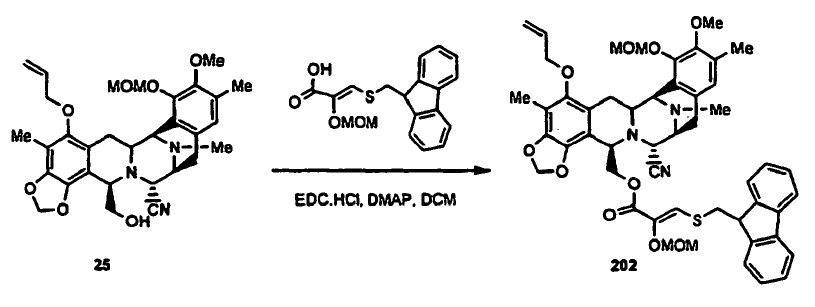

To a solution of **25** (2 mg, 0.0035 mmol) and an excess amount of the cysteine derivative in anhydrous CH₂Cl₂ (0.2mL), an excess amounts of DMAP and EDC.HCl were added at 23 °C. The reaction mixture was stirred at 23 °C under Argon atmosphere for 14 h. Then, the mixture was diluted with CH₂Cl₂ (10 mL) and washed with a saturated aqueous solution of sodium bicarbonate (10 mL). The aqueous phase was extracted with additional CH₂Cl₂ (10 mL). The combined organic layers were dried over Na₂SO₄, filtered and the solvent was eliminated under reduced pressure. The crude of the reaction was purified by flash column chromatography (SiO₂, Hex:EtOAc 4:1) to afford **202** as a pale yellow solid.
¹H NMR (300 MHz, CDCl₃) (poor resolution) δ 7.78.7,62 (m, 4H), 7.41-7.26 (m, 4H), 6.73 (s, 1H), 6.10 (m, 1H), 5.92 (d, *J* = 1.3 Hz, 1H), 5.88 (d, *J* = 1.3 Hz, 1H), 5.40-5.22 (m, 2H), 5.11 (s, 3H), 5.02 (d, *J* = 13.8 Hz, 1H). 429-4.02 (m, 6H), 3.97 (m, 1H), 3.72 (d, *J* = 12.5 Hz, 2H), 3.70 (s, 3H), 3.58 (s, 3H), 3.51 (d, *J* = 12.3 Hz, 2H), 3.50 (s, 3H), 3.49-3.20 (m, 4H), 2.54-2.28 (m, 4H), 2.40 (s, 3H), 2.21 (s, 3H), 2.16 (s, 3H).

Fermentation Procedures

### Example A

Seed medium YMP3 containing 1% glucose; 0.25% beef extract; 0.5% bacto-peptone; 0.25% NaCl; 0.8% CaCO₃ was inoculated with 0.1% of a frozen vegetative stock of the microorganism, strain A2-2 of *Pseudomonas fluorescens,* and incubated on a rotary shaker (250 rpm) at 27°C. After 30 h of incubation, the seed culture was added to a agitated-vessel fermentor with a production medium composed of 2% dextrose; 4% mannitol, 2% dried brewer's yeast *(Vitalevor® Biolux, Belgium);* 1% (NH₄)₂SO₄: 0.04% K₂HPO₄; 0.8 KCI; 0.001% FeCl₃; 0.1% L-Tyr; 0.8% CO₃Ca; 0.05% PPG-2000; 0.2% antifoam silicone (ASSAF-100, RHODIA UK). The sterilisation was carried out at 122°C 30 minutes. The volume inoculated was a 2% (v/v). The temperature was 27°C (0 to 16h) and 24°C from 16h to final process (41 hours). The dissolve oxygen-pressure was upper to 25%. The pH was controlled at 6.0 with diluted sulphuric acid since 28 hours till final process. The overpressure was 0.5 bar. A 1% mannitol or sorbitol was added from 16 h to final process (for two days running) and 2% for three days fermentation-process.

After 41 or 64 hours, the fermentation broth must be extracted for recovery safracin B or KCN treatment in the clarified broth for recovery safracin B - cyano.

### Example B

Obtention of safracin B cyano from the crude extract.

A clarification or filtration from the fermentation broth at pH 6 removes the solids. The clarified broth was adjusted a pH 9.5 with diluted sodium hydroxide and extracted twice with 2:1 (v/v) ethyl acetate, methylene chloride or butyl acetate. The extraction was carried out into an agitated-vessel during 20', the temperature of the mixture was maintained at 8 to 10°C. The two phases were separated by a liquid-liquid centrifuge. The organic phase was dried with sodium sulphate anhydrous or frozen and then filtered for removing ice. This organic phase (ethyl acetate layer) was evaporated until obtention of an oil-crude extract.

### Example C

### Obtention of safracin B cyano from the clarified broth.

A clarification or filtration from the fermentation broth at pH 6 removes the solids. The clarified broth was adjusted at pH 3.9 with concentrated acetic acid. 0.5 grams per litre of KCN are added to the clarified broth an incubated at 20°C during 1 hour with agitation. Then, the temperature was decreased at 15°C and the pH was adjusted at 9.5 with diluted sodium hydroxide and extracted with 2:1.5 (v/v) ethyl acetate. The extraction was carried out into an agitated-vessel during 20 minutes, the temperature of the mixture was maintained at 8 to 10°C. The two phases were separated by a liquid-liquid centrifuge. The organic phase was dried with sodium sulphate anhydrous. This organic phase (ethyl acetate layer) was evaporated until obtention of an oil-crude extract. This extract was purified by flash column chromatography (SiO₂, gradient 20:1 to 10: to 5:1 ethyl acetate:methanol) to afford quantitatively compound 2 as a light yellow solid.
Rf: 0.55 (ethyl acetate:methanol5:1); .t_{R}= 19.9 min [HPLC, Delta Pack C4, 5µm, 300 A, 150x3 mm, λ=215 nm, flow= 0.7 ml/min, temp= 50°C, grad.: CH₃CN-aq. NaOAc (10mM) 85% - 70% (20')];
¹H NMR (300 Mhz, CDCl₃): δ 6.54 (dd, *J₁*= 4.4Hz, *J₂=* 8.4 Hz, 1H),6.44 (s, 1H), 4.12 (d, *J* = 2.4 Hz, 1H), 4.04 (d, *J* = 2.4 Hz, 1H), 4.00 (s, 3H), 3.87 (bs, 1H), 3.65 (ddd, *J₁* = 1.5 Hz, *J₂* = 8.7 Hz, *J₃*=9.9 Hz, 1H), 3.35 (br. D, *J*= 8.4 Hz, 1H), 3.15-2.96 (m, 4H), 2.92 (q, *J=* 7.2 Hz, 1H), 2.47 (d, *J=* 18.3 Hz, 1H), 2.29 (s, 3H), 2.18 (s, 3H) 1.83 (s, 3H), 1.64 (ddd, *J₁* = 2.7 *Hz, J₂* = 11.1 Hz, *J*₃=14.1 Hz, 1H), 0.79 (d, *J*= 7.2 Hz, 3H);
¹³C NMR (75 Mhz, CDCl₃): δ 186.0 (q), 175.9 (q), 156.2 (q), 146.8 (q), 142.8 (q), 140.7 (q), 136.6 (q), 130.5 (q), 128.8 (q), 127.0 (q), 120.5 (s), 117.4 (q), 116.5 (q), 60.8 (t), 60.4 (s), 58.7 (t), 56.2 (s), 55.7 (s), 54.8 (s), 54.8 (s), 54.4 (s), 50.0 (s), 41.6 (t), 39.8 (d), 25.2 (d), 24.4 (d), 21.2 (t), 15.5 (t), 8.4 (t).
ESI-MS m/z: Calcd for C₂₉H₃₅N₅O₆: 549.6. Found (M+Na)⁺: 572.3.

### Example D

A medium (501) composed of dextrose (2%), mannitol (4%), dry brewer's yeast (2%), ammonium sulphate (1%), potassium secondary phosphate (0.04%), potassium chloride (0.8%), iron (III) chloride 6-hydrate (0.001%), L-tyrosine (0.1%), calcium carbonate (0.8%), poly- (propylene glycol) 2000 (0.05%) and antifoam ASSAF 1000 (0.2%) was poured into a jar-fermentor with 751 total capacity and, after sterilisation, inoculated with seed culture (2%) of A2-2 strain (FERM BP-14) and aerated cultivation under agitation was carried out at 27°C to 24°C for 64 hours (aeration of 75 l per minute and agitation from 350 to 500 rpm). The pH was controlled by automatic feeding of diluted sulphuric acid from 27 hours to final process. A 2% mannitol was added from 16 hours to final process. The cultured medium (45 l) thus obtained was, after removal of cells by centrifugation, adjusted to pH 9.5 with diluted sodium hydroxide, extracted with 25 litres of ethyl acetate twice. The mixture was carried out into an agitated-vessel at 8°C for 20 minutes. The two phases were separated by a liquid-liquid centrifuge. The organic phases were frozen at -20°C and filtered for removing ice and evaporated ice and evaporated until obtention of a 40 g oil-dark-crude extract. After introduction of the cyanide group and purification, 3.0 grams of safracin B cyano were obtained.

### Example E

A medium (501) composed of dextrose (2%), mannitol (4%), dry brewer's yeast (2%), ammonium sulphate (1%), potassium secondary phosphate (0.02%, potassium chloride (0.2%), Iron (III) chloride 6-hydrate (0.001%, L-tyrosine (0.1%), calcium carbonate (0.8%, poly- (propylene glycol) 2000 (0.05%) and antifoam ASSAF 1000 (0.2%) was poured into a jar-fermentor with 751 total capacity and, after sterilisation, inoculated with seed culture (2%) of A2-2 strain (FERM BP-14) and aerated cultivation under agitation was carried out at 27°C to 24°C for 41 hours (aeration of 75 l per minute and agitation from 350 to 500 rpm). The pH was controlled by automatic feeding of diluted sulphuric acid from 28 hours to final process. A 1% mannitol was added from 16 hours to final process. The cultured medium (451) thus obtained was, after removal of cells by centrifugation, adjusted to pH 3.9 with 200 ml of conc. acetic acid. 25 grams of potassium cyanide 97% were added and after 1 hour of agitation at 20°C, the pH was adjusted to 9.5 with 1500 ml of a solution 10% sodium hydroxide. Then, extracted with 35 litres of ethyl acetate. The mixture was carried out into an agitated-vessel at 8°C for 20 minutes. The two phases were separated by a liquid-liquid centrifuge. The organic phase was dried by sodium sulphate anhydrous and evaporated until obtention of a 60 g oil-dark-crude extract.

After chromatography, 4.9 grams of safracin B cyano were obtained.

### REFERENCES

European Patent 309,477.
US Patent 5,721,362.
Sakai, R., Jares-Erijman, E.A., Manzanares, I., Elipe, M.V.S., and Rinehart. K.L. J. Am. Chem. Soc. (1996) 118, 9017-9023
Martinez, E.J., Owa, T., Schreiber, S.L. and Corey, EJ. Proc. Natl. Acad. Sci. USA, 1999, 96, 3496-3501.
Japanese Kokai JP-A2 59/225189.
Japanese Kokai JP-A2 60/084288.
Arai, T,; Kubo, A. In The Alkaloids, Chemistry and Pharmacology; Brossi, A. Ed.; Academic: New York, 1983, Vol 21; pp 56-110.
Remers, W. A.: In The Chemistry of Antitumor Antibiotics; Vol. 2; Wiley; New York, 1988, pp 93-118.
Gulavita N. K.; Scheuer, P. J.: Desilva, E. D. Abst. Indo-United States Symp. on Bioactive Compounds from Marine Organisms, Goa, India, Feb. 23-27, 1989, p 28.
Arai, T; Takahashi, K; Kubo, A. J. Antibiot, 1977, 30, 1015-1018*.*
Arai. T.; Takahashi, K.; Nakahara, S.; Kubo, A. Experientia 1980, 36, 1025-1028.
Mikami, Y.; Takahashi, K; Yazawa, K.; Hour-Young, C.; Arai, T.; Saito, N.; Kubo, A. J. Antibiot. 1988, 41, 734-740.
Arai, T.; Takahashi, K.; Ishiguro, K.; Yazawa, K. J. Antibiot. 1980, 33, 951-960.
Yazawa, K.; Takahashi, K.; Mikami, Y.; Arai, T.; Saito, N.; Kubo, A. J. Antibiot. 1986, 39, 1639-1650.
Arai, T.; Yazawa, K.; Takahashi, K.; Maeda, A.; Mikami, Y. Antimicrob. Agent Chemother. 1985,28,5-11.
Takahashi, K.; Yazawa, K.; Kishi, K.; Mikami, Y.; Arai, T.; Kubo, A. J. Antibiot. 1982, 35, 196-201.
Yazawa, K.; Asaoka, T.; Takahashi, K.; Mikami, Y.; Arai, T. J. Antibiot. 1982, 35, 915-917. Frincke, J. M.; Faulkner, D. J. J. Am. Chem. Soc. 1982, 104, 265-269.
He, H. -Y.; Faulkner, D. J. J. Org. Chem. 1989, 54, 5822-5824.
Kubo, A.; Saito, N.; Kitahara, Y.; Takahashi, K.; Tazawa, K.; Arai, T. Chem Pharm. Bull. 1987, 35, 440-442.
Trowitzsch-Kienast, W.; Irschik, H.; Reichenback, H.; Wray, V.; Höfle, G. Liebigs Ann. Chem. 1988,475-481.
Ikeda, Y.; Idemoto, H.; Hirayama, F.; Yamamoto, K.; Iwao, K.; Asano, T.; Munakata, T. J. Antibiot. 1983, 36, 1279-1283.
Asaoka, T.; Yazawa, K.; Mikami, Y. Arai, T.; Takahashi, K. J. Antibiot. 1982, 35, 1708-1710.
Lown, J. W.; Hanstock, C. C.; Joshua, A. V.; Arai, T; Takahashi, K. J. Antibiot. 1983, 36, 1184-1194.
Munakata et al. United States Patent 4, 400, 752, 1984.
Y. Ikeda et al. The Journal of Antibiotics. VOL XXXVI, N°10, 1284, 1983.
R. Cooper, S. Unger. The Journal of Antibiotics. VOL XXXVIII, N°1, 1985.
Corey et al. United States Patent 5, 721, 362. 1998.
Corey et al. J. Am. Chem. Soc. vol 118 pp 9202-92034, 1996.
Proc. Natl. Acad. Sci. USA. Vol. 96, pp 3496-3501, 1999.

## Claims

1. A compound of the formula: 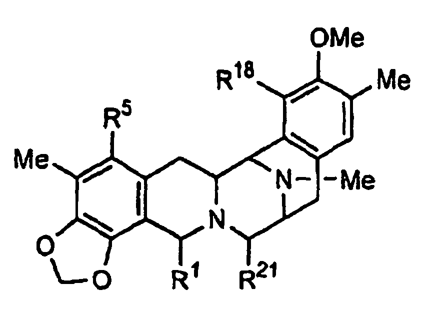 wherein:
R¹ is selected from the group consisting of -CH₂-NHR^{a} and -CH₂-OR^{a}, where R^{a} is selected from the group consisting of alkyl-CO-; haloalkyl-CO-; haloalkyl-O-CO-; arylalkenyl-CO- wherein the aryl group is selected from phenyl, biphenyl and naphthyl; heteroaryl-CO- wherein the heteroaryl contains one, two or three heteroatoms selected from nitrogen, sulphur and oxygen; alkenyl-CO-; and amino acid acyl;
R⁵ is OH; acetyloxy or other acyloxy group up to 4 carbon atoms;
R¹⁸ is -OH; and
R²¹ is -CN.

2. A compound according to claim 1, which is of the formula: 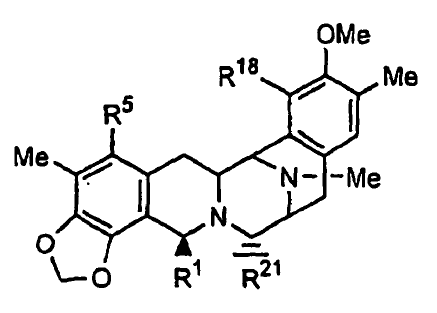 wherein R¹, R⁵, R¹⁸, and R²¹ are as defined in claim 1.

3. A compound according to claim 1, which is of the formula: 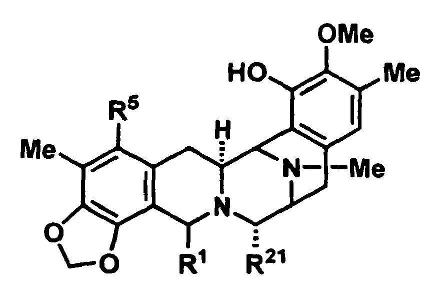 wherein R¹, R⁵ and R²¹ are as defined in claim 1.

4. A compound according to any preceding claim, wherein R¹ is -CH₂-NHR^{a}.

5. A compound according to any preceding claim, wherein R^{a} is -aa-R^{b} where aa is amino acid acyl and R^{b} is selected from the group consisting of H; alkyl-CO-; haloalkyl-CO-; haloalkyl-O-CO-; arylalkyl-CO- wherein the aryl group is selected from phenyl, biphenyl and naphthyl; arylalkenyl-CO- wherein the aryl group is selected from phenyl, biphenyl and naphthyl; heteroaryl-CO- wherein the heteroaryl contains one, two or three heteroatoms selected from nitrogen, sulphur and oxygen; alkenyl-CO-; amino acid acyl; and a protecting group.

6. A compound according to claim 5, wherein said amino acid acyl is further substituted with at least one R^{a} group.

7. A compound according to any preceding claim, wherein R¹ is -CH₂-NH-aa-R^{b} where aa is an amino acid and R^{b} is selected from the group consisting of hydrogen; protecting group; arylalkenyl-CO- wherein the aryl group is selected from phenyl, biphenyl and naphthyl; haloalkyl-CO-; alkyl-CO-; arylalkyl-CO- wherein the aryl group is selected from phenyl, biphenyl and naphthyl; and amino acid acyl.

8. A compound according to claim 7, wherein R¹ is -CH₂-NH-aa-R^{b} where aa is alanine and R^{b} is selected from the group consisting of hydrogen, Boc, PhNHCS-, CF₃CO-, trans(trifluoromethyl)cinnamoyl, cinnamoyl, C₃F₇CO-, butyryl, 3-chloropropionoyl, hydrocinnamoyl, phenylacetyl, and acetyl; aa is valine and R^{b} is selected from the group consisting of Cbz and Boc; aa is phenylalanine and R^{b} is Boc; aa is proline and R^{b} is Boc; aa is arginine and R^{b} is Boc; or aa is tryptophan and R^{b} is Boc.

9. A compound according to any of claims 1 to 6, wherein R¹ is -CH₂-NHR^{a} where R^{a} is selected from the group consisting of alkyl-CO-; alkenyl-CO-; arylalkenyl-CO wherein the aryl group is selected from phenyl, biphenyl and naphthyl; and heteroaryl-CO- wherein the heteroaryl contains one, two or three heteroatoms selected from nitrogen, sulphur and oxygen -.

10. A compound according to claim 9, wherein R¹ is -CH₂-NHR^{a} where R^{a} is selected from the group consisting of acetyl, isovaleryl, decanoyl, and cinnamoyl.

11. A compound according to claim 9 wherein R¹ is -CH₂-NHR^{a} where R^{a} is selected from the group consisting of propionyl, myristoyl, stearoyl, hexanoyl, crotonyl, and chloronicotinoyl.

12. A compound according to claim 1, 2 or 3, wherein R¹ is -CH₂-OR^{a} where R^{a} is selected from the group consisting of a protected cysteine; alkyl-CO- and arylalkenyl-CO- wherein the aryl group is selected from phenyl, biphenyl and naphthyl.

13. A compound according to claim 12, wherein R¹ is-CH₂-OR^{a} where R^{a} is selected from the group consisting of butyryl, trans(trifluoromethyl) cinnamoyl and cinnamoyl,

14. A compound according to any preceding claim wherein R⁵ is -OCOCH₃.

15. A compound of a formula selected from the following general structure I: 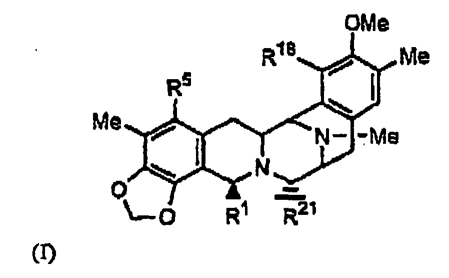 wherein R^{a}, R⁵, R¹⁸, R¹, and R²¹ are each independently selected from the groups defined below:
| R³ | R⁵ | R¹⁸ | R¹ | R²¹ |
|---|---|---|---|---|
| | OH | OH | CH₂NHR^{a} | CN |
| COCH₂CH₂CH₃ | OAc | | CH₂OR^{a} | |
| | | | | |
| COCH=CHPh | | | | |
| 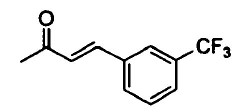 | | | | |
| COCH(CH₃)NHCOCH₂CH₂Ph | | | | |
| CO-(S)-CH(CH₃)NHCOCF₃ | | | | |
| CO-(R)-CH(CH₃)NHCOCF₃ | | | | |
| CO-(S)-CH(NHCbz)CH(CH₃)₂ | | | | |
| CSNHPh | | | | |
| 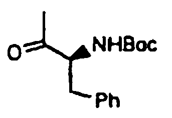 | | | | |
| 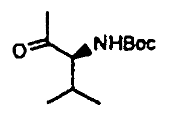 | | | | |
| 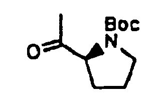 | | | | |
| 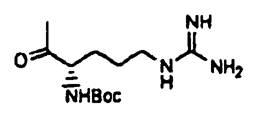 | | | | |
| 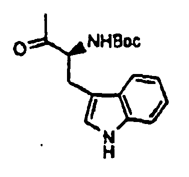 | | | | |

16. A compound of a formula selected from the following general structure (II): 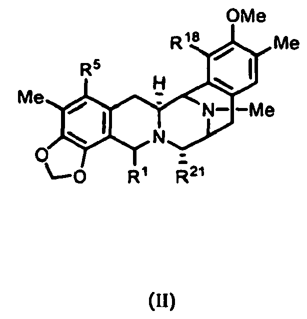 wherein R^{a}, R⁵, R¹⁸, R¹, and R²¹ are each independently selected from the groups defined below:
| R^{a} | R⁵ | R¹⁸ | R¹ | R²¹ |
|---|---|---|---|---|
| COCH₂CH₂CH₃ | OH | OH | CH₂NHR^{a} | CN |
| COCH=CHPh | OAc | | CH₂OR^{a} | |
| 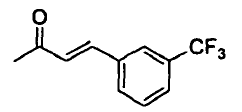 | | | | |
| COCH(CH₃)NHCOCH₂CH₂Ph | | | | |
| CO-(S)-CH(CH₃)NHCOCF₃ | | | | |
| CO-(R)-CH(CH₃)NHCOCF₃ | | | | |
| CO-(S)-CH(NHCbz)CH(CH₃)₂ | | | | |
| CSNHPh | | | | |
| 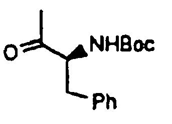 | | | | |
| 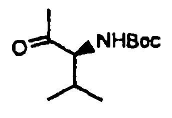 | | | | |
| 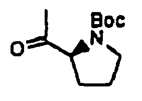 | | | | |
| 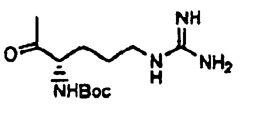 | | | | |
| 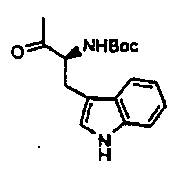 | | | | |

17. A compound of a formula of the following general structure I: 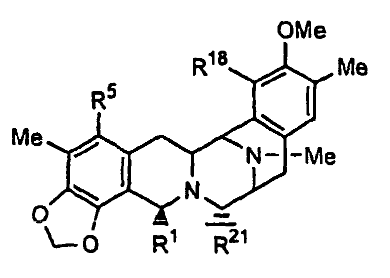 wherein R^{b}, R⁵, R¹, R¹⁸ and R²¹ are each independently selected from the groups defined below:
R¹ is -CH₂-NH-CO-CHCH₃-NHR⁶
| R^{b} | R⁵ | R¹⁸ | R²¹ |
|---|---|---|---|
| H | OH | OH | CN |
| | OAc | | |
| COCH₂CH₂CH₃ | | | |
| COCH₂Ph | | | |
| COCH₂CH₂Ph | | | |
| COCH=CHPh | | | |
| 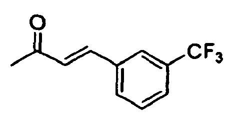 | | | |
| Boc | | | |
| CSNHPh | | | |

18. A compound of a formula of the following general structure II: 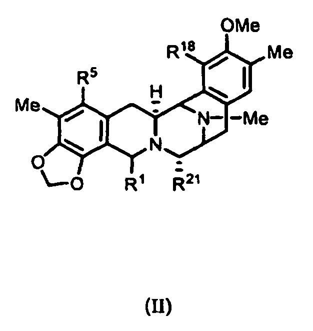 wherein R^{b}, R⁵, R¹, R¹⁸ and R²¹ are each independently selected from the groups defined below:
R¹ is -CH₂-NH-CO-CHCH₃-NHR^{b}
| R^{b} | R⁵ | R¹⁸ | R²¹ |
|---|---|---|---|
| H | OH | OH | CN |
| COCH₂CH₂CH₃ | OAc | | |
| COCH₂Ph | | | |
| COCH₂CH₂Ph | | | |
| COCH=CHPh | | | |
| 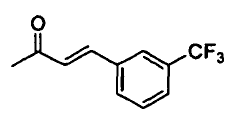 | | | |
| Boc | | | |
| CSNHPh | | | |

19. A compound of a formula selected from the following general structures (III) and (IV): 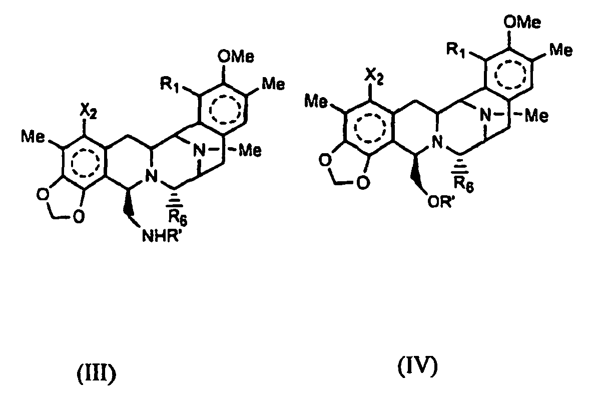 wherein R', X₂, R₁, and R₆ are each independently selected from the groups defined below:
| R' | X₂ | R₁ | R₆ |
|---|---|---|---|
| CH₂CH=CH₂ | OH | OH | CN |
| COCH₂CH₃ | OAc | OAc | |
| CO(CH₂)₄CH₃ | OCOOCH₂CH=CH₂ | OCOCH₂C₆H₁₁ | |
| CO(CH₂)₁₂CH₃ | OCOCF₃ | OCOCH₂CH₂C₆H₁₁ | |
| CO(CH₂)₁₆CH₃ | OCOCH₂Cl | OCOCH₂CH₂CH₃ | |
| COCH₂C₆H₁₁ | OCOCH₂CH₂Cl | OCO(CH₂)₄CH₃ | |
| COCH₂CH₂C₆H₁₁ | OCOCF₂CF₂CF₃ | OCO(CH₂)₈CH₃ | |
| | | OCO(CH₂)₁₆CH₃ | |
| COCH=CHCH₃ | | | |
| COCH(CH₃)NHCOCH₂CH₂Ph | | | |
| CO-(S)-CH(NHCbz)GH(CH₃)₂ | | | |
| 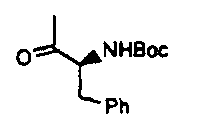 | | | |
| 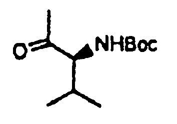 | | | |
| 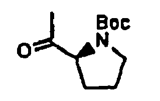 | | | |
| 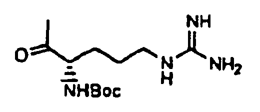 | | | |
| 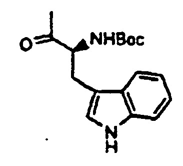 | | | |
| 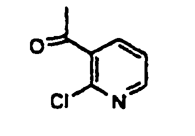 | | | |

20. A compound of a formula of the following general structure V: 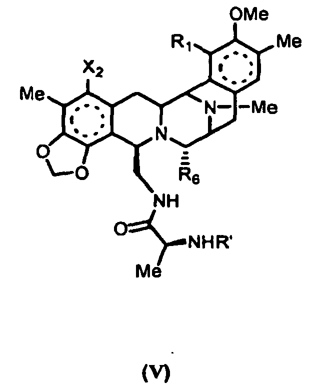 wherein X₂, R₁, R₆, and R are each independently selected from the groups defined below:
| R' | X₂ | R₁ | R₆ |
|---|---|---|---|
| - | OH | OH | CN |
| CH₂CH=CH₂ | OAc | OAc | |
| COCH₂CH₃ | OCOOCH₂CH=CH₂ | OCOCH₂C₆H₁₁ | |
| CO(CH₂)₄CH₃ | OCOCF₃ | OCOCH₂CH₂C₆H₁₁ | |
| CO(CH₂)₁₂CH3 | OCOCH₂Cl | OCOCH₂CH₂CH₃ | |
| CO(CH₂)₁₆CH₃ | OCOCH₂CH₂Cl | OCO(CH₂)₄CH₃ | |
| COCH₂C₆H₁₁ | OCOCF₂CF₂CF₃ | OCO(CH₂)₈CH₃ | |
| COCH₂CH₂C₆H₁₁ | | OCO(CH₂)₁₆CH₃ | |
| COCH₂Ph | | | |
| COCH₂CH₂Ph | | | |
| COCH=CHCH₃ | | | |
| COCH(CH₃)NHCOCH₂CH₂Ph | | | |
| CO-(S)-CH(CH₃)NHCOCF₃ | | | |
| CO-(R)-CH(CH₃)NHCOCF₃ | | | |
| CO-(S)-CH(NHCbz)CH(CH₃)₂ | | | |
| Boc | | | |
| 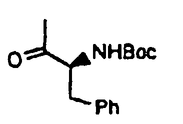 | | | |
| 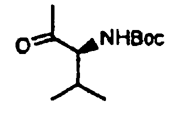 | | | |
| 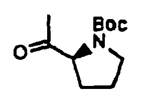 | | | |
| 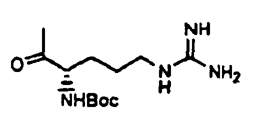 | | | |
| 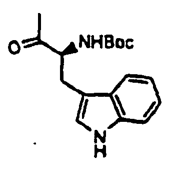 | | | |
| 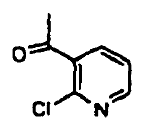 | | | |

21. A compound which is selected from the following formulae:

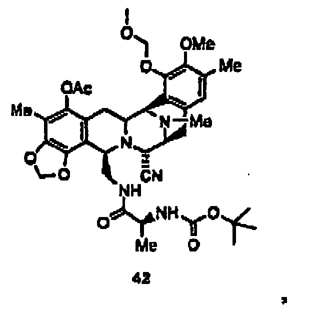

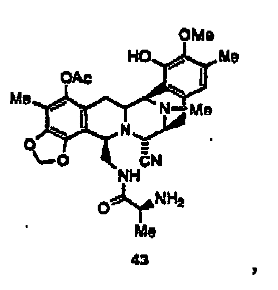

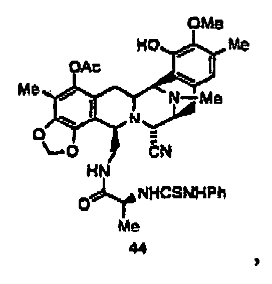

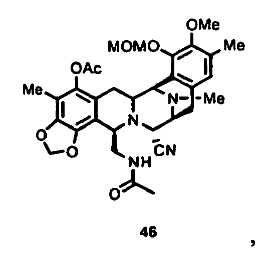

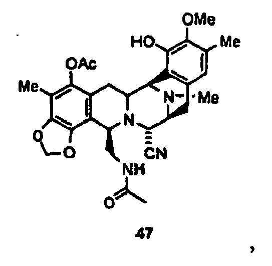

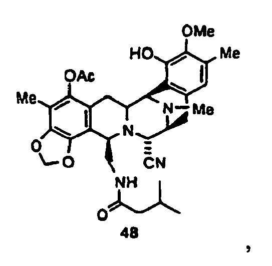

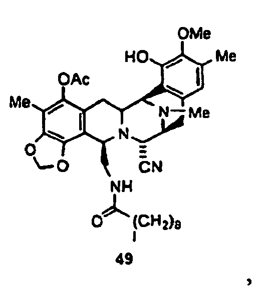

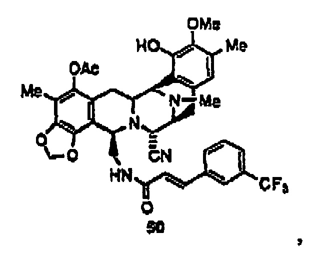

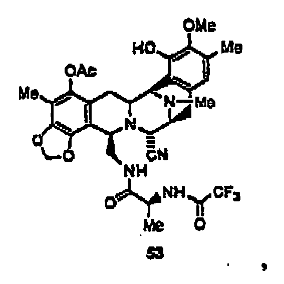

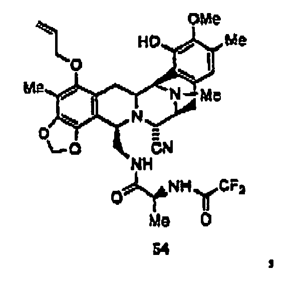

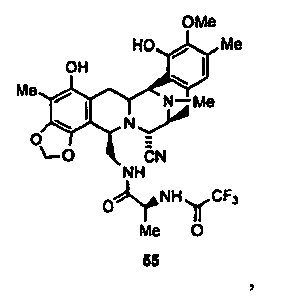

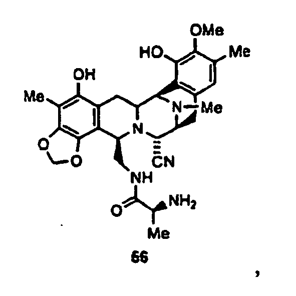

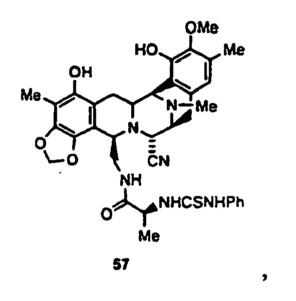

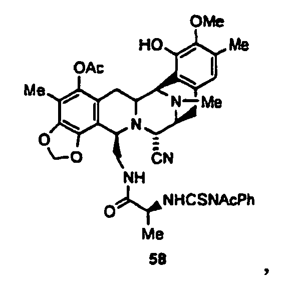

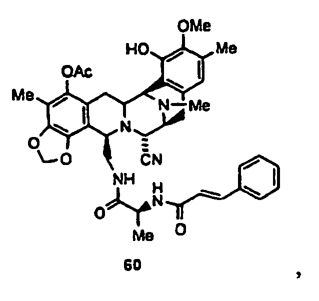

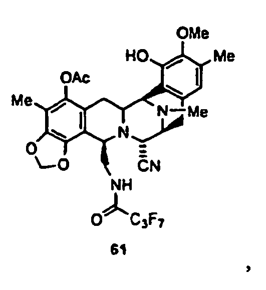

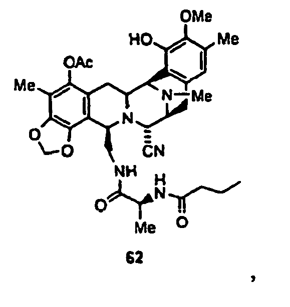

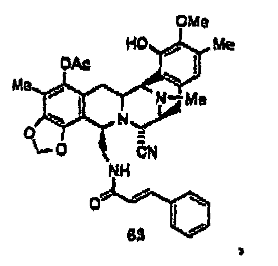

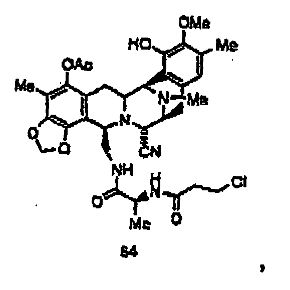

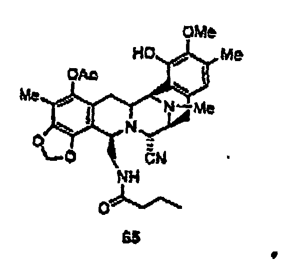

22. A compound which is selected from the following formulae: 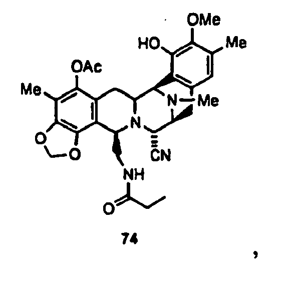 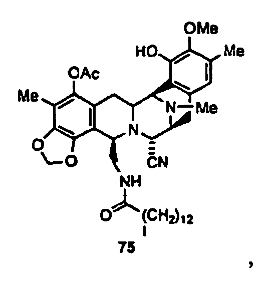 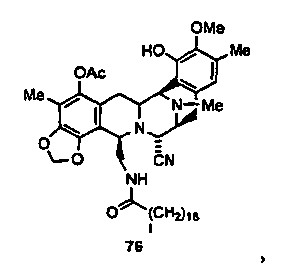 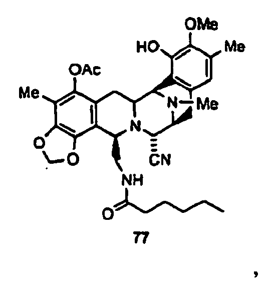 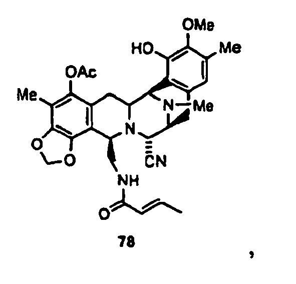 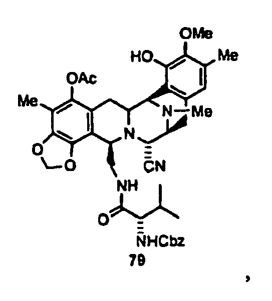 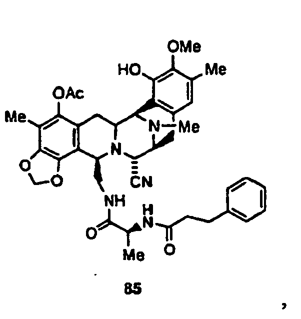 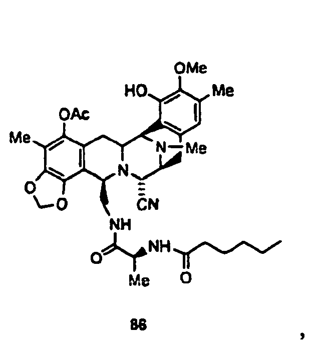 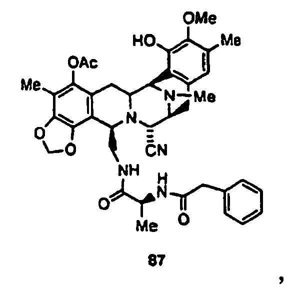 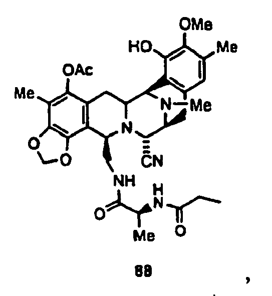 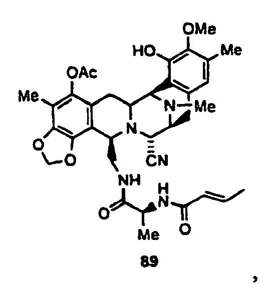 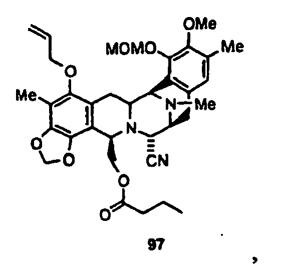 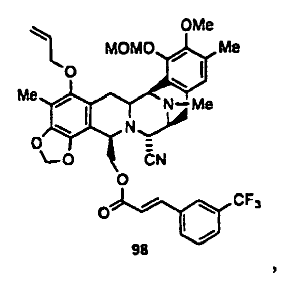 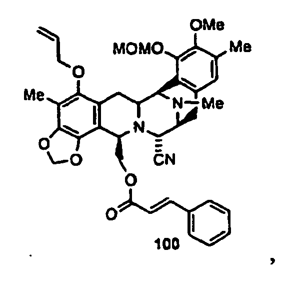 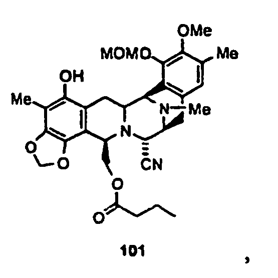 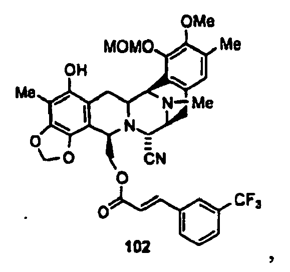 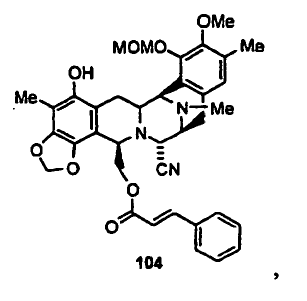 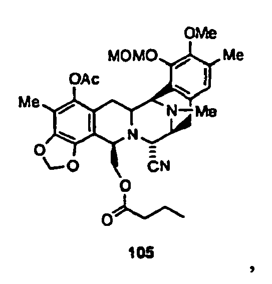 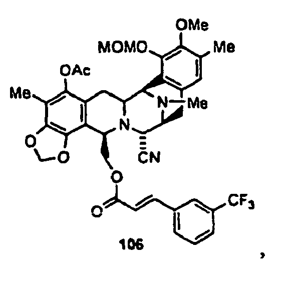 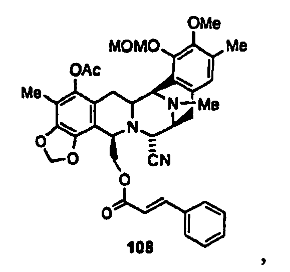 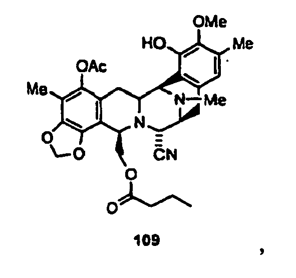 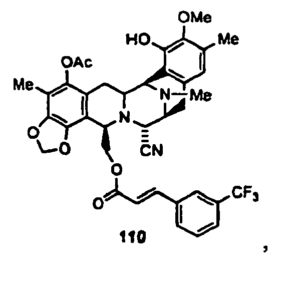 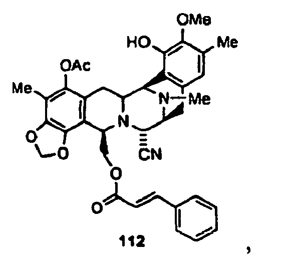 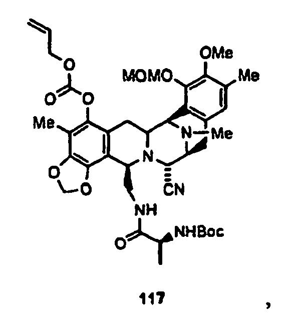 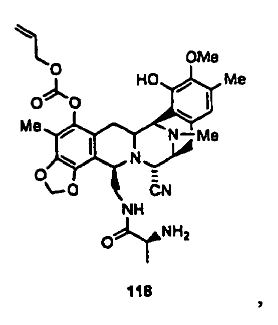 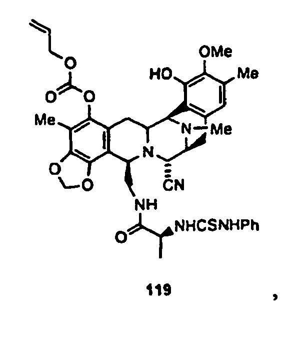 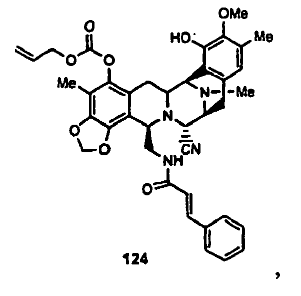 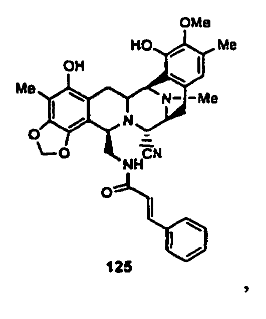 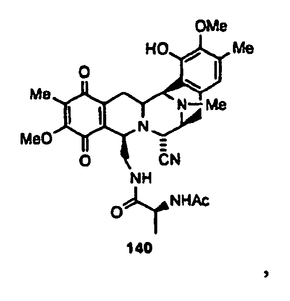 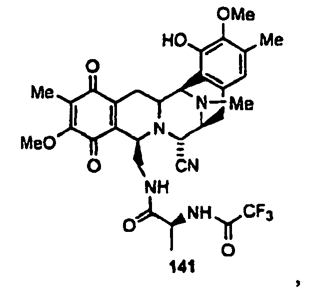 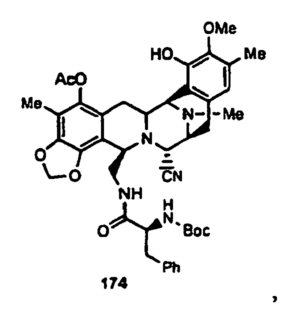 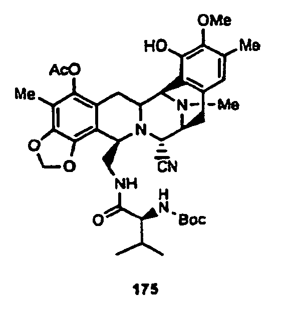 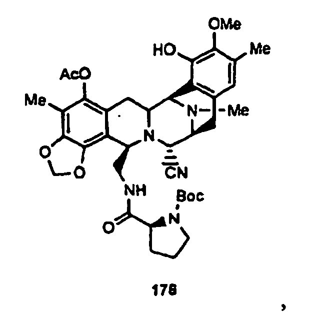 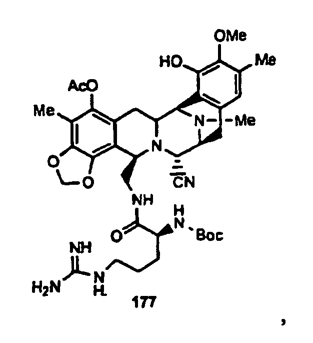 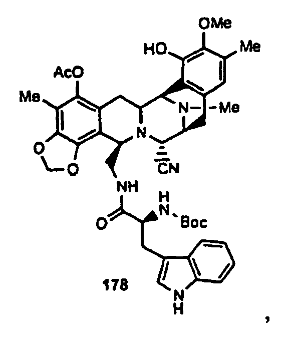 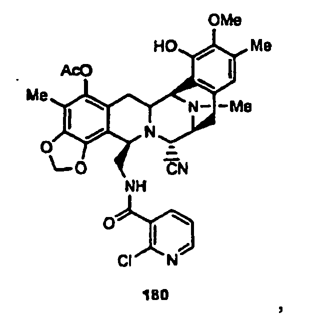 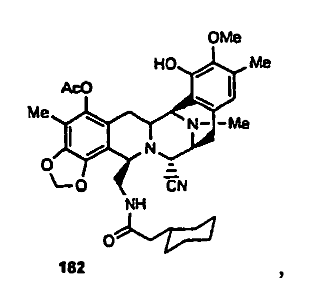 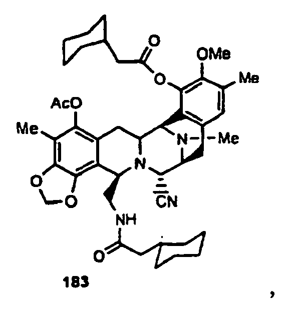 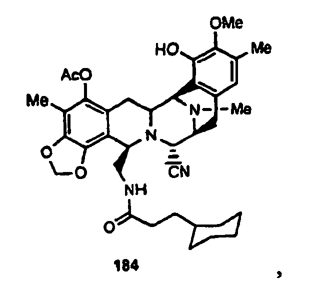 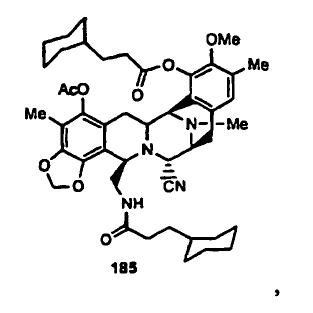 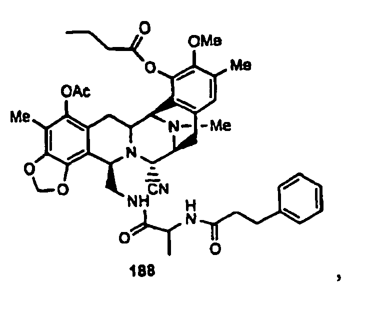 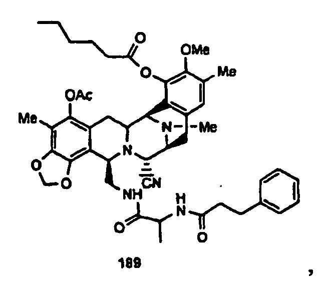 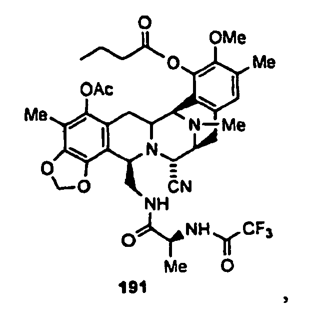 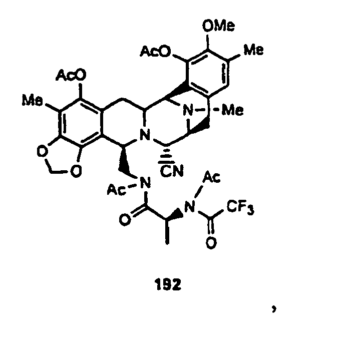 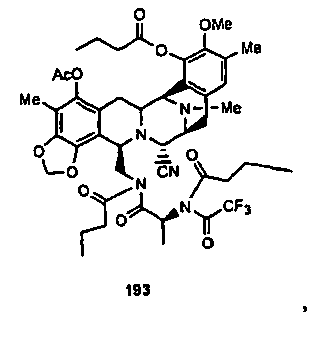 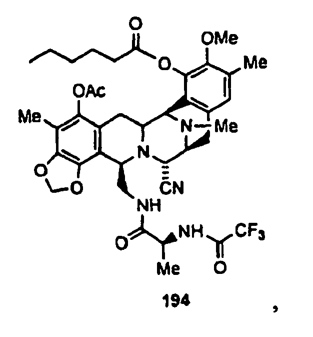 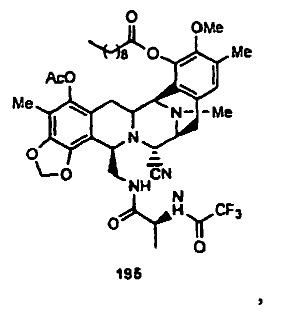 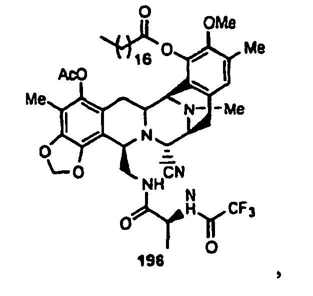 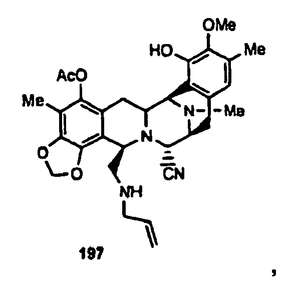 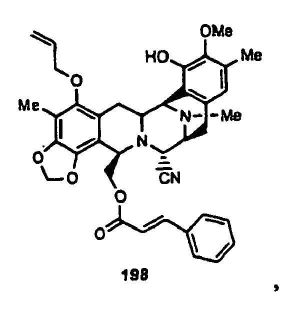 and

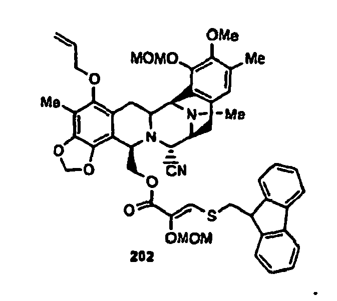

23. A compound of the formula:

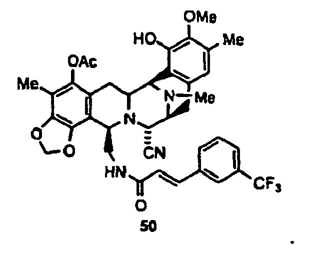

24. A compound of the formula:

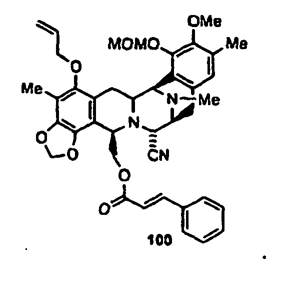

25. A compound of the formula:

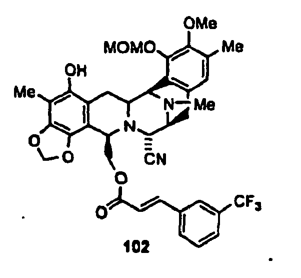

26. A compound of the formula:

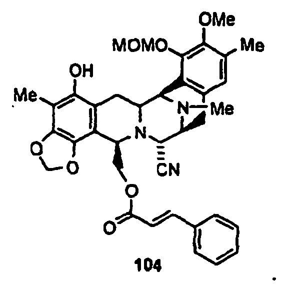

27. A compound of the formula:

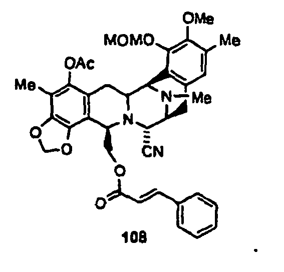

28. A compound of the formula:

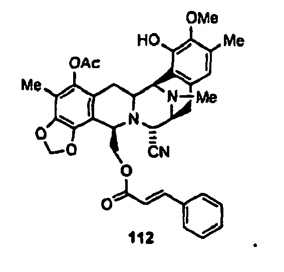

29. A compound of the formula:

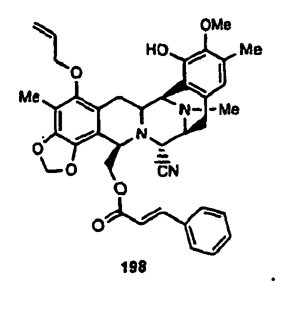

30. A compound of the formula: 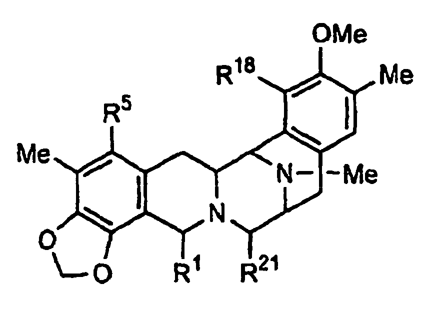 wherein:
R¹ is -CH₂-OR^{a}, where R^{a} is selected from the group consisting of alkyl-CO-; haloalkyl-CO-; cycloalkylalkyl-CO-; haloalkyl-O-CO-; arylalkenyl-CO- wherein the aryl group is selected from phenyl, biphenyl and naphthyl; heteroaryl-CO- where the heteroaryl contains one, two or three heteroatoms selected from nitrogen, sulphur and oxygen; alkenyl-CO-; alkenyl; and amino acid acyl;
R⁵ is -OR", where R" is selected from the group consisting of H; alkyl-CO-; cycloalkyl-CO-; and haloalkyl-CO-; R¹⁸ is -OR, where R is selected from the group consisting of H, alkyl-CO-; cycloalkylalkyl-CO-; and
R²¹ is -CN.

31. A compound according to claim 30, which is of the formula: 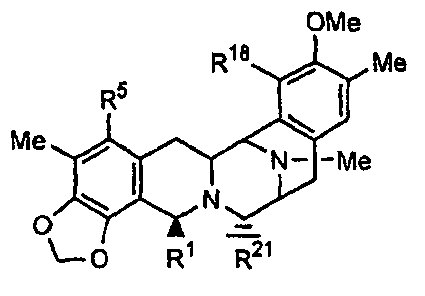 wherein R¹, R⁵, R¹⁸, and R²¹ are as defined in claim 30.

32. A compound according to claim 30, which is of the formula: 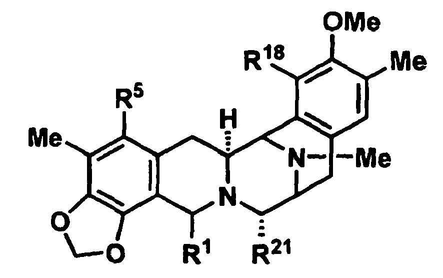 wherein R¹, R⁵, R¹⁸, and R²¹ are as defined in claim 30.

33. A compound according to claim 30, 31 or 32, wherein R' is -CH₂-OR^{a} where R^{a} is selected from the group consisting of a protected cysteine; alkyl-CO-; arylalkenyl-CO- wherein the aryl group is selected from phenyl, biphenyl and naphthyl; a cysteine derivative of the formula Prot^{SH}-S-CH₂-C(=NOProt^{OH})-CO- where Prot^{SH} is a protecting group for thiol and Prot^{OH} is a protecting group hydroxy; and a cysteine derivative of formula Prot^{SH}-S-CH=C(-OProt^{OH})-CO-, where Prot^{SH} is a protecting group for thiol and Prot^{OH} is a protecting group for hydroxy.

34. A compound according to claim 33, wherein R¹ is -CH₂-OR^{a} where R^{a} is selected from the group consisting of S-Fm-O-TBDMS-cysteine; butyryl; (trifluoromethyl)cinnamoyl; cinnamoyl; a cysteine derivative of the formula Prot^{SH}-S-CH₂-C(=NOProt^{OH})-CO- where Prot^{SH} is Fm and Prot^{OH} is methoxy; and a cysteine derivative of formula Prot^{SH}-S-CH=C(-OProt^{OH})-CO-, where Prot^{SH} is Fm and Prot^{OH} is MOM.

35. A pharmaceutical composition comprising a compound according to any preceding claim.

36. The use of a compound as defined in any of claims 1 to 34 in the preparation of a pharmaceutical composition for a method of treating a mammal affected by cancer which comprises administering to the affected mammal a therapeutically effective amount of the compound.

37. The use according to claim 36, wherein said mammal is a human.

38. The use according to claim 37, wherein said compound is administered by intravenous infusion.

39. The use according to any of claims 36 to 38, wherein said compound is administered as part of a combination therapy.

## Patentansprüche

1. Verbindung der Formel: 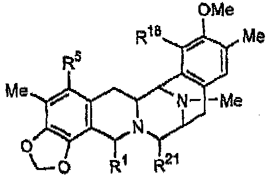 worin;
R¹ ausgewählt ist aus der Gruppe, bestehend aus -CH₂-NHR² und -CH₂-OR^{a}; worin R^{a} ausgewählt ist aus der Gruppe, bestehend aus Alkyl-CO-; Halogenalkyl-CO-; Halogenalkyl-O-CO-; Arylalkenyl-CO-, wobei die Aryl-Gruppe ausgewählt ist aus Phenyl, Biphenyl und Naphthyl; Heteroaryl-CO-, wobei das Heteroaryl ein, zwei oder drei Heteroatome enthält, die ausgewählt sind aus Stickstoff, Schwefel und Sauerstoff; Alkenyl-CO-; und Aminosäureacyl;
R⁵ ist OH; Acetyloxy- oder eine andere Acyloxy-Gruppe mit bis zu 4 Kohlenstoffatomen;
R¹⁸ ist -OH und
R²¹ ist -CN.

2. Verbindung nach Anspruch 1, welche die Formel hat: 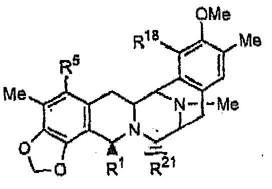 worin R¹, R⁵, R¹⁸ und R²¹ wie in Anspruch 1 festgelegt sind.

3. Verbindung nach Anspruch 1, welche die Formel hat: 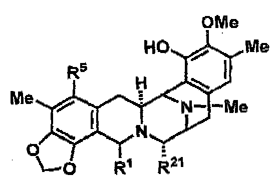 worin R¹, R⁵ und R²¹ wie in Anspruch 1 festgelegt sind.

4. Verbindung nach einem der vorgenannten Ansprüche, worin R¹ -CH₂-NHR^{a} ist.

5. Verbindung nach einem der vorgenannten Ansprüche, worin R^{a} -aa-R^{b} ist, worin "aa" Aminosäureacyl ist und R^{b} ausgewählt ist aus der Gruppe, bestehend aus: H; Alkyl-CO-; Halogenalkyl-CO-; Halogenalkyl-O-CO-; Arylalkyl-CO-, wobei die Aryl-Gruppe ausgewählt ist aus Phenyl, Biphenyl und Napthyl, Arylalkenyl-CO-, wobei die Aryl-Gruppe ausgewählt ist aus Phenyl, Biphenyl und Naphthyl; Heteroaryl-CO-, wobei das Heteroaryl ein, zwei oder drei Heteroatome enthält, die ausgewählt sind aus Stickstoff, Schwefel und Sauerstoff; Alkenyl-CO-; Aminosäureacyl; und einer schützenden Gruppe.

6. Verbindung nach Anspruch 5, worin das Aminosäureacyl ferner substituiert ist mit mindestens einer R^{a}-Cruppe.

7. Verbindung nach einem der vorgenannten Ansprüche, worin R¹ -CH₂-NH-aa-R^{b} ist, wobei "aa" eine Aminosäure ist und R^{b} ausgewählt ist aus der Gruppe, bestehend aus Wasserstoff; einer schützenden Gruppe; Arylalkenyl-CO-, wobei die Aryl-Gruppe ausgewählt ist aus Phenyl, Biphenyl und Naphthyl; Halogenalkyl-CO-; Alkyl-CO-; Arylalkyl-CO-, wobei die Aryl-Gruppe ausgewählt ist aus Phenyl, Biphenyl und Naphthyl ; und Aminosäureacyl.

8. Verbindung nach Anspruch 7, worin R¹ -CH₂-NH-aa-R^{b} ist, wobei "aa" Alanin ist und R^{b} ausgewählt ist aus der Gruppe, bestehend aus Wasserstoff, Boc, PhNHCS-, CF₃CO-, *trans*(Trifluormethyl)cinnamoyl, Cinnamoyl, C₃F₇CO-, Butyryl, 3-Chlorpropionyl, Hydrocinnamoyl, Phenylacetyl und Acetyl; "aa" ist Valin und R^{b} ist ausgewählt aus der Gruppe, bestehend aus Cbz und Boc; "aa" ist Phenylalanin und R^{b} ist Boc; "aa" ist Prolin und R_{b} ist Boc; "aa" ist Arginin, und R_{b} ist Boc; oder "aa" ist Tryptophan und R^{b} ist Boc.

9. Verbindung nach einem der Ansprüche 1 bis 6, worin R¹ -CH₂-NHR^{a} ist, wobei R^{a} ausgewählt ist aus der Gruppe, bestehend aus Alkyl-CO-; Alkenyl-CO-; Arylalkenyl-CO, wobei die Aryl-Gruppe ausgewählt ist aus Phenyl, Biphenyl und Naphthyl; und Heteroaryl-CO-, wobei das Heteroaryl ein, zwei oder drei Heteroatome enthält, die ausgewählt sind aus Stickstoff, Schwefel und Sauerstoff.

10. Verbindung nach Anspruch 9, worin R¹ -CH₂-NHR^{a} ist, worin R^{a} ausgewählt ist aus der Gruppe, bestehend aus Acetyl, Isovaleryl, Decanoyl und Cinnamoyl.

11. Verbindung nach Anspruch 9, worin R¹ -CH₂-NHR^{a} ist, worin R^{a} ausgewählt ist aus der Gruppe, bestehend aus Propionyl, Myristoyl, Stearoyl, Hexanoyl, Crotonyl und Chlornicotinoyl.

12. Verbindung nach Anspruch 1, 2 oder 3, worin R¹ -CH₂-OR^{a} ist, worin R^{a} ausgewählt ist aus der Gruppe, bestehend aus geschütztem Cystein; Alkyl-CO- und Arylalkenyl-CO-, worin die Aryl-Gruppe ausgewählt ist aus Phenyl, Biphenyl und Naphthyl.

13. Verbindung nach Anspruch 12, worin R¹ -CH₂-OR^{a} ist, worin R^{a} ausgewählt ist aus der Gruppe, bestehend aus Butyryl, *trans*(Trifluormethyl)cinnamoyl und Cinnamoyl.

14. Verbindung nach einem der vorgenannten Ansprüche, worin R⁵ -OCOCH₃ ist.

15. Verbindung einer Formel, ausgewählt aus der folgenden allgemeinen Struktur I: 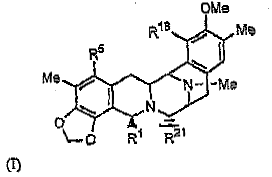 worin R^{a}, R⁵, R¹⁸, R¹ und R²¹ jeweils unabhängig ausgewählt sind aus den nachfolgend festgelegten Gruppen:
| R^{a} | R⁵ | R¹⁸ | R¹ | R²¹ |
|---|---|---|---|---|
| | OH | OH | CHCN₂NHR^{a} | CN |
| | OAc | | CH₂OR^{a} | |
| COCH₂CH₂CH₃ | | | | |
| COCH=CHPh | | | | |
| 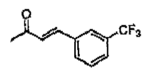 | | | | |
| COCH(CH₃)NHCOCH₂CH₂Ph | | | | |
| CO-(S)-CH(CH₃)NHCOCF₃ | | | | |
| CO-(R)-CH(CH₃)NHCOCF₃ | | | | |
| CO-(S)-CH(NHCbz)CH(CH₃)₂ | | | | |
| CSNHPh | | | | |
| 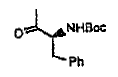 | | | | |
| 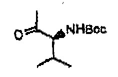 | | | | |
| 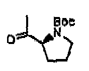 | | | | |
| 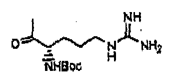 | | | | |
| 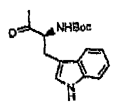 | | | | |

16. Verbindung einer Formel, ausgewählt aus der folgenden allgemeinen Struktur (II): 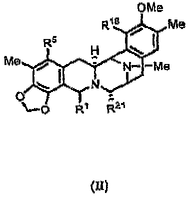 worin R^{a}, R⁵, R¹⁸, R¹ und R²¹ jeweils unabhängig ausgewählt sind aus den Gruppen, die nachfolgend festgelegt sind:
| R^{a} | R⁵ | R¹⁸ | R¹ | R²¹ |
|---|---|---|---|---|
| COCH₂CH₂CH₃ | OH | OH | CH₂NHR^{a} | CN |
| | OAc | | CH₂OR^{a} | |
| COCH=CHPh | | | | |
| 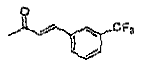 | | | | |
| COCH(CH₃)NHCOCH₂CH₂Ph | | | | |
| COCH(CH₃)NHCOCH₂CH₂Ph | | | | |
| CO-(S)-CH(CH₃)NHCOCF₃ | | | | |
| CO-(R)-CH(CH₃)NHCOCF₃ | | | | |
| CO-(S)-CH(NHCbz)CH(CH₃)₂ | | | | |
| CSNHPh | | | | |
| 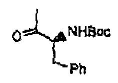 | | | | |
| 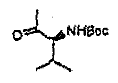 | | | | |
| 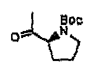 | | | | |
| 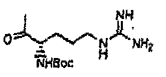 | | | | |
| 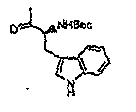 | | | | |

17. Verbindung einer Formel der folgenden allgemeinen Struktur I: 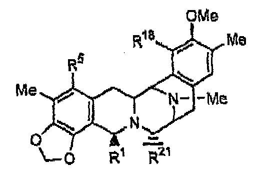 worin R^{b}, R⁵, R¹, R¹⁸ und R²¹ jeweils unabhängig ausgewählt sind aus den Gruppen, die nachfolgend festgelegt sind:
R¹ ist -CH₂-NH-CO-CHCH₃-NH_{R}^{b}
| R^{b} | R⁵ | R¹⁸ | R²¹ |
|---|---|---|---|
| H | OH | OH | CN |
| | OAc | | |
| COCH₂CH₂CH₃ | | | |
| COCH₂Ph | | | |
| COCH₂CH₂Ph | | | |
| COCH=CHPh | | | |
| 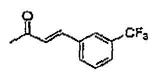 | | | |
| Boc | | | |
| CSNHPh | | | |

18. Verbindung einer Formel der folgenden allgemeinen Struktur II: 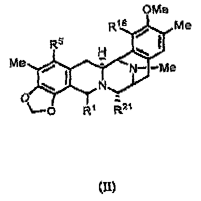 worin R^{b}, R⁵, R¹, R¹⁸ und R²¹ jeweils unabhängig ausgewählt sind aus den Gruppen, die nachfolgend festgelegt sind:
R¹ ist -CH₂-NH-CO-CHCH₃-NHR^{b}
| R^{b} | R⁵ | R¹⁸ | R²¹ |
|---|---|---|---|
| H | OH | OH | CN |
| COCH₂CH₂CH₃ | OAc | | |
| COCH₂Ph | | | |
| COCH₂CH₂Ph | | | |
| COCH=CHPh | | | |
| 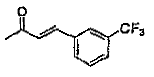 | | | |
| Boc | | | |
| CSNHPh | | | |

19. Verbindung einer Formel, ausgewählt aus den folgenden allgemeinen Strukturen (III) und (IV): 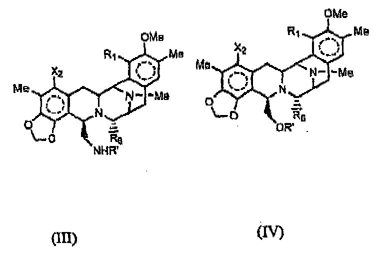 worin R', X₂, R₁ und R₆ jeweils unabhängig ausgewählt sind aus den Gruppen, die nachfolgend festgelegt sind:
| R' | X₂ | R₁ | R₆ |
|---|---|---|---|
| CH₂CH=CH₂ | OH | OH | CN |
| COCH₂CH₃ | OAc | OAc | |
| CO(CH₂)₄CH₃ | OCOOCH₂CH=CH₂ | OCOCH₂C₆H₁₁ | |
| CO(CH₂)₁₂CH₃ | OCOCF₃ | OCOCH₂CH₂C₆H₁₁ | |
| CO(CH₂)₁₆CH₃ | OCOCH₂Cl | OCOCH₂CH₂CH₃ | |
| COCH₂C₆H₁₁ | OCOCH₂CH₂Cl | OCO(CH₂)₄CH₃ | |
| COCH₂CH₂C₆H₁₁ | OCOCF₂CF₂CF₃ | OCO(CH₂)₃CH₃ | |
| | | OCO(CH₂)₁₈CH₃ | |
| COCH-CHCH₃ | | | |
| COCH(CH₃)NHCOCH₂CH₂Ph | | | |
| CO-(S)-CH(NHCbz)CH(CH₃)₂ | | | |
| 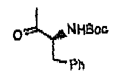 | | | |
| 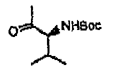 | | | |
| 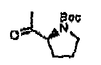 | | | |
| 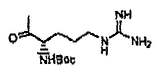 | | | |
| 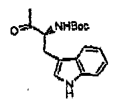 | | | |
| 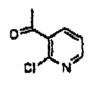 | | | |

20. Verbindung einer Formel der folgenden allgemeinen Struktur V: 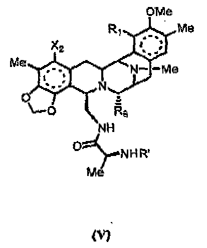 worin X₂, R₁, R₆ und R' jeweils unabhängig ausgewählt sind aus den Gruppen, die nachfolgend festgelegt sind:
| R' | X₂ | R₁ | R₆ |
|---|---|---|---|
| - | OH | OH | CN |
| CH₂CH=CH₂ | OAc | OAc | |
| COCH₂CH₃ | OCOOCH₂CH=CH₂ | OCOCH₂C₆H₁₁ | |
| CO(CH₂)₄CH₃ | OCOCF₃ | OCOCH₂CH₂C₆H₁₁ | |
| CO(CH₂)₁₂CH₃ | OCOCH₂Cl | OCOCH₂CH₂CH₃ | |
| CO(CH₂)₁₆CH₃ | OCOCH₂CH₂Cl | OCO(CH₂)₄CH₃ | |
| COCH₂C₆H₁₁ | OCOCF₂CF₂CF₃ | OCO(CH₂)₈CH₃ | |
| COCH₂CH₂C₆H₁₁ | | OCO(CH₂)₁₆CH₃ | |
| COCH₂Ph | | | |
| COCH₂CH₂Ph | | | |
| COCH=CHCH₃ | | | |
| COCH(CH₃)NHCOCH₂CH₂Ph | | | |
| CO-(S)-CH(CH₃)NHCOCF₃ | | | |
| CO-(R)-CH(CH₃)NHCOCF₃ | | | |
| CO-(S)-CH(NHCbz)CH(CH₃)₂ | | | |
| Boc | | | |
| 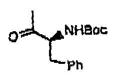 | | | |
| 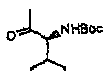 | | | |
| 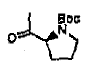 | | | |
| 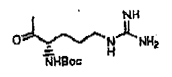 | | | |
| 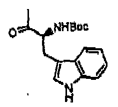 | | | |
| 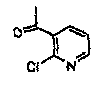 | | | |

21. Verbindung, ausgewählt aus den folgenden Formeln:

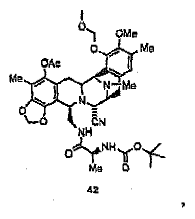

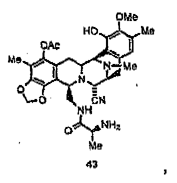

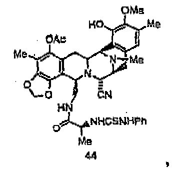

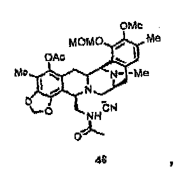

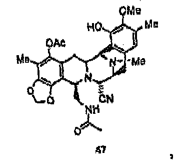

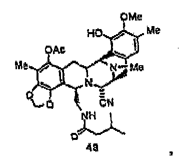

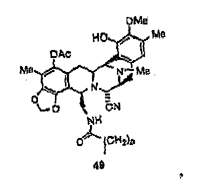

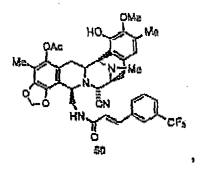

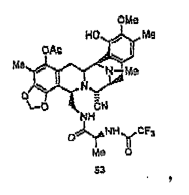

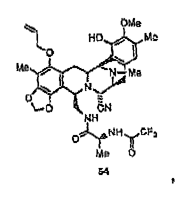

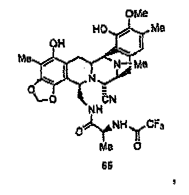

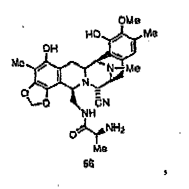

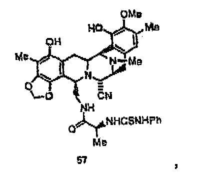

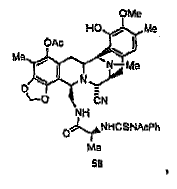

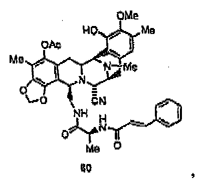

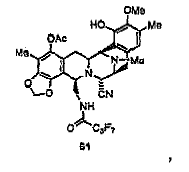

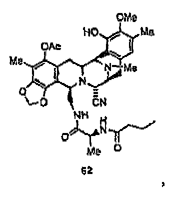

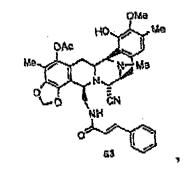

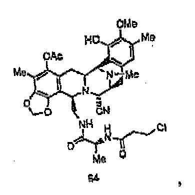

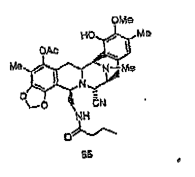

22. Verbindung, ausgewählt aus den folgenden Formeln; 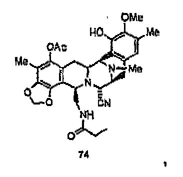 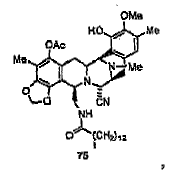 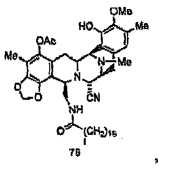 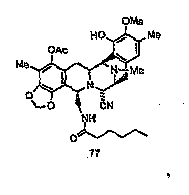 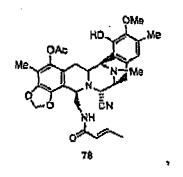 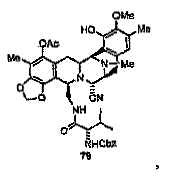 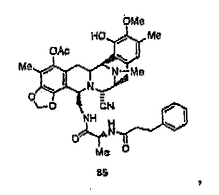 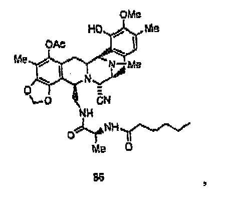 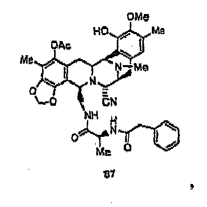 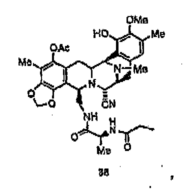 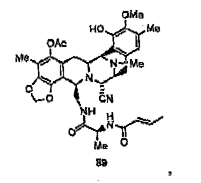 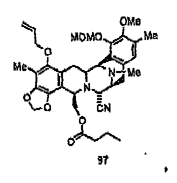 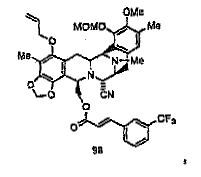 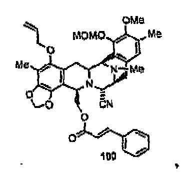 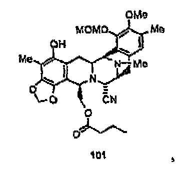 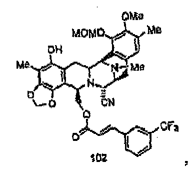 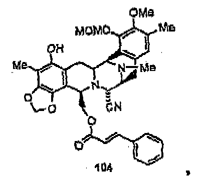 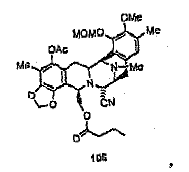 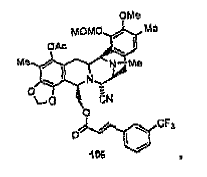 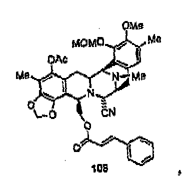 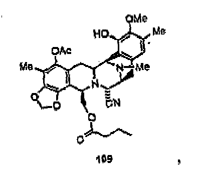 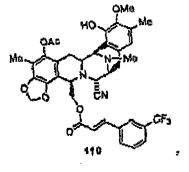 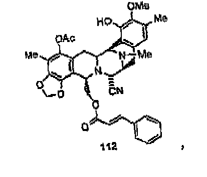 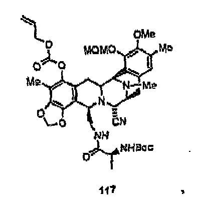 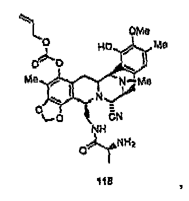 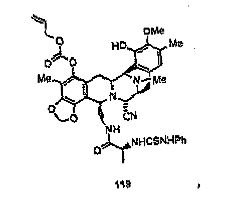 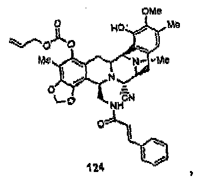 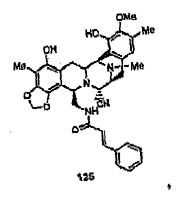 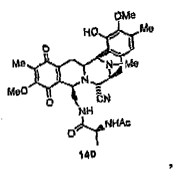 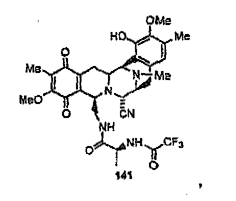 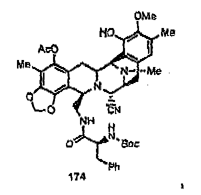 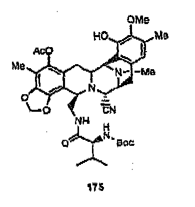 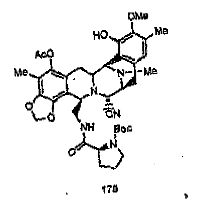 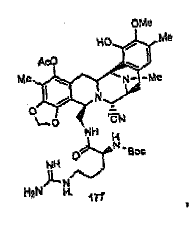 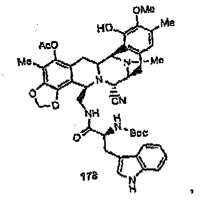 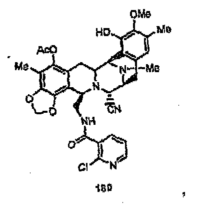 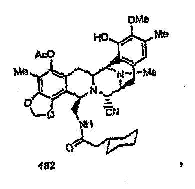 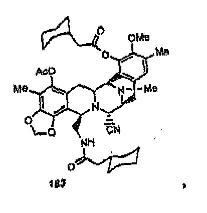 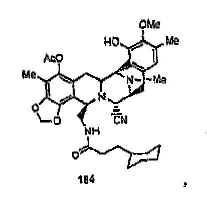 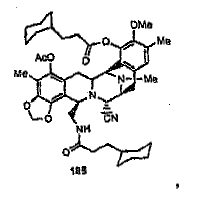 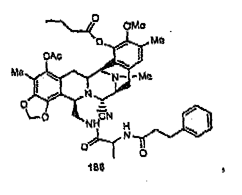 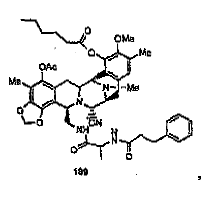 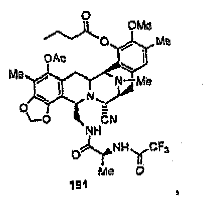 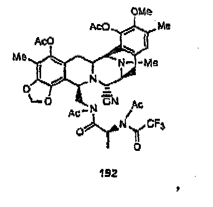 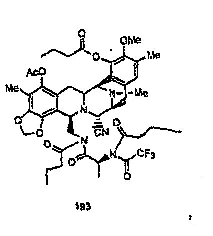 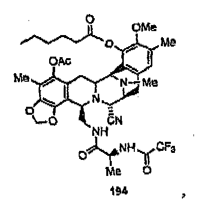 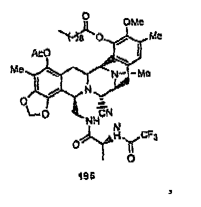 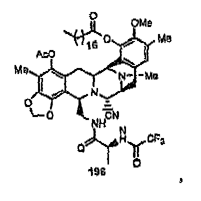 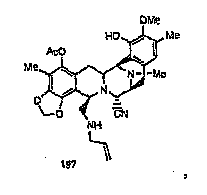 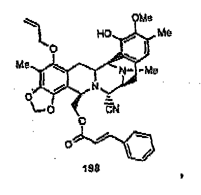 und

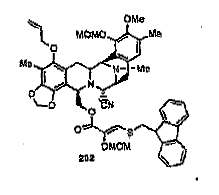

23. Verbindung der Formel :

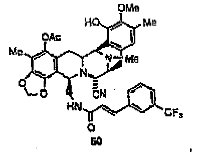

24. Verbindung der Formel:

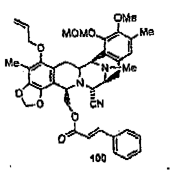

25. Verbindung der Formel:

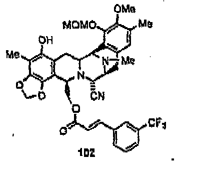

26. Verbindung der Formel:

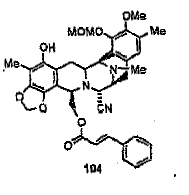

27. Verbindung der Formel:

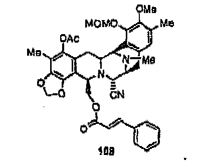

28. Verbindung der Formel:

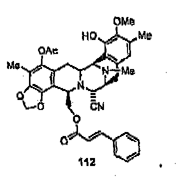

29. Verbindung der Formel:

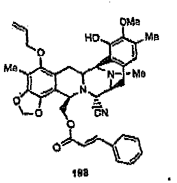

30. Verbindung der Formel: 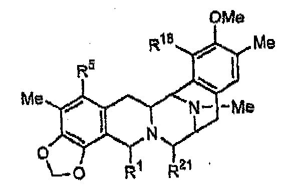 worin:
R¹ ist -CH₂-OR^{a},
worin R^{a} ausgewählt ist aus der Gruppe, bestehend aus Alkyl-CO-; Halogenalkyl-CO-; Cycloalkylalkyl-CO-; Halogenalkyl-O-CO-; Arylalkenyl-CO-, wobei die Aryl-Gruppe ausgewählt ist aus Phenyl, Biphenyl und Naphthyl; Heteroaryl-CO-, wobei das Heteroaryl ein, zwei oder drei Heteroatome enthält, die ausgewählt sind aus Stickstoff, Schwefel und Sauerstoff; Alkenyl-CO-; Alkenyl; und Aminosäureacyl;
R⁵ ist -OR", worin R" ausgewählt ist aus der Gruppe, bestehend aus H; Alkyl-CO-; Cycloalkyl-CO- und Halogenalkyl-CO-; R¹⁸ ist -OR, worin R ausgewählt ist aus der Gruppe, bestehend aus H, Alkyl-CO-; Cycloalkylalkyl-CO-; und
R²¹ ist-CN.

31. Verbindung nach Anspruch 30, die die Formel hat: 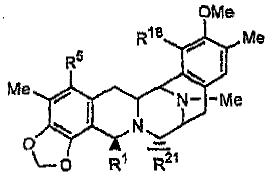 worin R¹, R⁵, R¹⁸ und R²¹ in Anspruch 30 festgelegt sind.

32. Verbindung nach Anspruch 30, die die Formel hat: 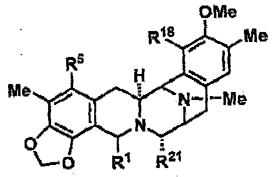 worin R¹, R⁵, R¹⁸ und R²¹ in Anspruch 30 festgelegt sind.

33. Verbindung nach Anspruch 30, 31 oder 32, worin R' -CH₂-OR^{a} ist, worin R^{a} ausgewählt ist aus der Gruppe, bestehend aus einem geschützten Cystein; Alkyl-CO-; Arylalkenyl-CO-, worin die Aryl-Gruppe ausgewählt ist aus Phenyl, Biphenyl und Naphthyl; ein Cystein-Derivat der Formel Prot^{SH}-S-CH₂-C(=NOProt^{OH})-CO-, worin Prot^{SH} eine schützende Gruppe für Thiol ist und Prot^{OH} eine schützende Gruppe für Hydroxy ist; und ein Cystein-Derivat der Formel Prot^{SH}-S-CH=C(-OProt^{OH})-CO-, worin Prot^{SH} eine schützende Gruppe für Thiol ist und Prot^{OH} eine schützende Gruppe für Hydroxy ist.

34. Verbindung nach Anspruch 33, worin R¹ -CH₂-OR^{a} ist, worin R^{a} ausgewählt ist aus der Gruppe, bestehend aus S-Fm-O-TBDMS-Cystein; Butyryl; (Trifluormethyl)cinnamoyl; Cinnamoyl; einem Cystein-Derivat der Formel Prot^{SH}-S-CH₂-C(=NOProt^{OH})-CO-, worin Prot^{SH} Fm ist und Prot^{OH} Methoxy ist; und einem Cystein-Derivat der Formel Prot^{SH}-S-CH=C(-OPro^{OH})-CO-, worin Prot^{SH} Fm ist und Prot^{OH} MOM ist.

35. Pharmazeutische Zusammensetzung, aufweisend eine Verbindung nach einem der vorgenannten Ansprüche.

36. Verwendung einer Verbindung nach einem der Ansprüche 1 bis 34 in der Herstellung einer pharmazeutischen Zusammensetzung für eine Methode zum Behandeln eines an Krebs erkrankten Säugertiers, wobei die Methode das Verabreichen einer therapeutisch wirksamen Menge der Verbindung an das erkrankte Säugetier umfasst.

37. Verwendung nach Anspruch 36, wobei das Säugetier ein Mensch ist.

38. Verwendung nach Anspruch 37, wobei die Verbindung durch intravenöse Infusion verabreicht wird.

39. Verwendung nach einem der Ansprüche 36 bis 38, wobei die Verbindung als Teil einer Kombinationstherapie verabreicht wird.

## Revendications

1. Composé de la formule: 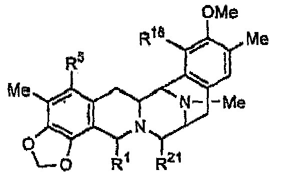 dans laquelle:
R¹ est choisi dans le groupe constitué de -CH₂-NHR^{a} et -CH₂-OR^{a}, où R^{a} est choisi dans le groupe constitué de groupes alkyle-CO-; haloalkyle-CO-; haloalkyle-O-CO-; arylalcényle-CO-, où le groupe aryle est choisi parmi des groupes phényle, biphényle et naphtyle; hétéroaryle-CO-, où le groupe hétéroaryle contient un, deux ou trois hétéroatomes choisis parmi l'azote, le soufre et l'oxygène; alcényle-CO-; et acyle d'acide aminé;
R⁵ est OH, un groupe acétyloxy ou un autre groupe acyloxy jusqu'à 4 atomes de carbone;
R¹⁸ est -OH; et
R²¹ est -CN.

2. Composé suivant la revendication 1, qui est de la formule: 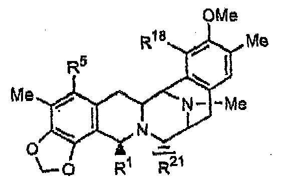 dans laquelle R¹, R⁵, R¹⁸ et R²¹ sont tels que définis dans la revendication 1.

3. Composé suivant la revendication 1, qui est de la formule: 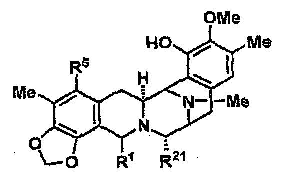 dans laquelle R¹, R⁵ et R²¹ sont tels que définis dans la revendication 1.

4. Composé suivant l'une quelconque des revendications précédentes, dans lequel R¹ est -CH₂-NHR^{a}.

5. Composé suivant l'une quelconque des revendications précédentes, dans lequel R^{a} est -aa-R^{b}, où aa est un groupe acyle d'acide aminé et R^{b} est choisi dans le groupe constitué de H; de groupes alkyle-CO-; haloalkyle-CO-; haloalkyle-O-CO-; arylalkyle-CO-; où le groupe aryle est choisi parmi des groupes phényle, biphényle et naphtyle; arylalcényle-CO-, où le groupe aryle est choisi parmi des groupes phényle, biphényle et naphtyle; hétéroaryle-CO-, où le groupe hétéroaryle contient un, deux ou trois hétéroatomes choisis parmi l'azote, le soufre et l'oxygène; alcényle-CO-; acyle d'acide aminé; et d'un groupe protecteur.

6. Composé suivant la revendication 5, dans lequel ledit groupe acyle d'acide aminé est en outre substitué avec au moins un groupe R^{a},

7. Composé suivant l'une quelconque des revendications précédentes, dans lequel R¹ est -CH₂-NH-aa-R^{b}, où aa est un groupe acide aminé et R^{b} est choisi dans le groupe constitué d'hydrogène; d'un groupe protecteur; de groupes arylalcényle-CO-, où le groupe aryle est choisi parmi des groupes phényle, biphényle et naphtyle; haloalkyle-CO-; alkyle-CO-; arylalkyle-CO-; où le groupe aryle est choisi parmi des groupes phényle, biphényle et naphtyle; et acyle d'acide aminé.

8. Composé suivant la revendication 7, dans lequel R¹ est -CH₂-NH-aa-R^{b}, où aa est un groupe alanine et R^{b} est choisi dans le groupe constitué d'hydrogène, de groupes Boc, PhNHCS-, CF₃CO-, trans(trifluorométhyl)cinnamoyle, cinnamoyle, C₃F₇CO-, butyryle, 3-chloropropionoyle, hydrocynnamoyle, phénylacétyle et acétyle; aa est un groupe valine et R^{b} est choisi dans le groupe constitué de Cbz et Boc; aa est un groupe phénylalanine et R^{b} est un groupe Boc; aa est un groupe proline et R^{b} est un groupe Boc; aa est un groupe arginine et R^{b} est un groupe Boc; ou aa est un groupe tryptophane et R^{b} est un groupe Boc.

9. Composé suivant l'une quelconque des revendications 1 à 6, dans lequel R¹ est - CH₂-NHR^{a}, où R^{a} est choisi dans le groupe constitué de groupes alkyle-CO-; alcényle-CO-; arylalcényle-CO-, où le groupe aryle est choisi parmi des groupes phényle, biphényle et naphtyle; et hétéroaryle-CO-, où le groupe hétéroaryle contient un, deux ou trois hétéroatomes choisis parmi l'azote, le soufre et l'oxygène.

10. Composé suivant la revendication 9, dans lequel R¹ est -CH₂-NHR^{a}, où R^{a} est choisi dans le groupe constitué de groupes acétyle, isovaléryle, décanoyle et cinnamoyle,

11. Composé suivant la revendication 9, dans lequel R¹ est -CH₂-NHR^{a}, où R^{a} est choisi dans le groupe constitué de groupes propionyle, myristoyle, stéaroyle, hexanoyle, crotonyle et chloronicotinoyle.

12. Composé suivant la revendication 1, 2 ou 3, dans lequel R¹ est -CH₂-OR^{a}, où R^{a} est choisi dans le groupe constitué d'un groupe cystéine protégé; de groupes alkyle-CO- et arylalcényle-CO-, où le groupe aryle est choisi parmi des groupes phényle, biphényle et naphtyle.

13. Composé suivant la revendication 12, dans lequel R¹ est -CH₂-OR^{a}, où R^{a} est choisi dans le groupe constitué de groupes butyryle, trans(trifluorométhyl)cinnamoyle et cinnamoyle,

14. Composé suivant l'une quelconque des revendications précédentes, dans lequel R⁵ est -OCOCH₃.

15. Composé d'une formule choisie parmi la structure générale (I) suivante: 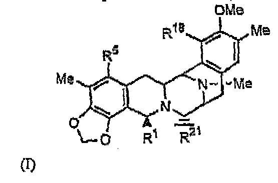 dans laquelle R^{a}, R⁵, R¹⁸, R¹ et R²¹ sont chacun indépendamment choisis parmi les groupes définis ci-dessous:
| R^{a} | R⁵ | R¹⁸ | R¹ | R²³ |
|---|---|---|---|---|
| | OH | OH | CH₂NHR^{a} | CN |
| | OAc | | CH₂OR^{a} | |
| COCH₂CH₂CH₃ | | | | |
| COCH=CHPh | | | | |
| 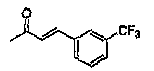 | | | | |
| COCH(CH₃)NHCOCH₂CH₂Ph | | | | |
| CO-(S)-CH(CH₃)NHCOCF₃ | | | | |
| CO-(R)CH(CH₃)NHCOCF₃ | | | | |
| CO-(S)-CH(NHCbz)CH(CH₃)₂ | | | | |
| CSNHPh | | | | |
| 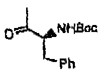 | | | | |
| 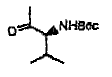 | | | | |
| 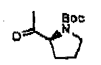 | | | | |
| 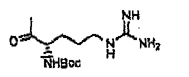 | | | | |
| 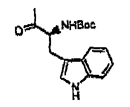 | | | | |

16. Composé d'une formule choisie parmi la structure générale (II) suivante: 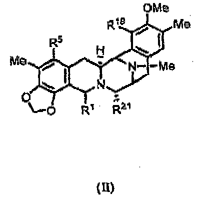 dans laquelle R^{a}, R⁵, R¹⁸, R¹ et R²¹ sont chacun indépendamment choisis parmi les groupes définis ci-dessous:
| R^{a} | R⁵ | R¹⁸ | R¹ | R²¹ |
|---|---|---|---|---|
| COCH₂CH₂CH₃ | OH | OH | CH₂NHR^{a} | CN |
| | OAc | | CH₂OR^{a} | |
| COCM=CHPh | | | | |
| 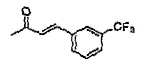 | | | | |
| COCH(CH₃)NHCOCH₂CH₂Ph | | | | |
| COCH(CH₃)NHCOCH₂CH2Ph | | | | |
| CO(S)-CH(CH₃)NHCOCF₃ | | | | |
| CO-(R)-CH(CH₃)NHCOCF₃ | | | | |
| CO-(S)-CH(NHCbz)CH(CH₃)₂ | | | | |
| CSNHPh | | | | |
| 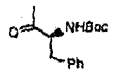 | | | | |
| 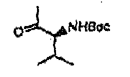 | | | | |
| 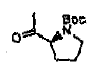 | | | | |
| 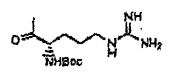 | | | | |
| 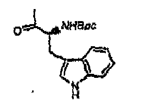 | | | | |

17. Composé d'une formule de la structure générale (I) suivante: 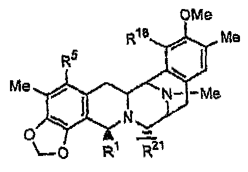 dans laquelle R^{b}, R⁵, R¹⁸, R¹ et R²¹ sont chacun indépendamment choisis parmi les groupes définis ci-dessous:
R¹ est -CH₂-NH-CO-CHCH₃-NHR^{b}
| R^{b} | R⁵ | R¹⁸ | R²¹ |
|---|---|---|---|
| H | OH | OH | CN |
| | OAc | | |
| COCH₂CH₂CH₃ | | | |
| COCH₂Ph | | | |
| COCH₂CH₂Ph | | | |
| COCH=CHPh | | | |
| 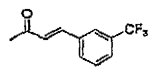 | | | |
| Boc | | | |
| CSNHPh | | | |

18. Composé d'une formule de la structure générale (II) suivante: 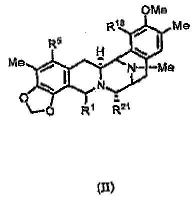 dans laquelle R^{b}, R⁵, R¹, R¹⁸ et R²¹ sont chacun indépendamment choisis parmi les groupes définis ci-dessous:
R¹ est -CH2-NH-CO-CHCH₃-NHR^{b}
| R^{b} | R⁵ | R¹⁸ | R²¹ |
|---|---|---|---|
| H | OH | OH | CN |
| COCH₂CH₂CH₃ | OAc | | |
| COCH₂Ph | | | |
| COCH₂CH₂Ph | | | |
| COCH=CHPh | | | |
| 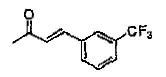 | | | |
| Boc | | | |
| CSNHPh | | | |

19. Composé d'une formule choisie parmi les structures générales (III) et (IV) suivantes: 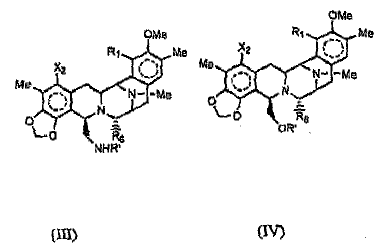 dans lesquelles R', X₂, R₁ et R₆ sont chacun indépendamment choisis parmi les groupes définis ci-dessous:
| R' | X₂ | R₁ | R₆ |
|---|---|---|---|
| CH₂CH=CH₂ | OH | OH | CN |
| COCH₂CH₃ | OAc | OAc | |
| CO(CH₂)₄CH₃ | OCOOCH₂CH=CH₂ | OCOCH₂C₆H₁₁ | |
| CO(CH₂)₁₂CH₃ | OCOCF₃ | OCOCH₂CH₂C₆H₁₁ | |
| CO(CH₂)₁₆CH₃ | OCOCH₂Cl | OCOCH₂CH₂CH₃ | |
| COCH2C₆H₁₁ | OCOCH₂CH₂Cl | OCO(CH₂)₄CH₃ | |
| COCH₂CH₂C₆H₁₁ | OCOCF₂CF₂CF₃ | OCO(CH₂)₈CH₃ | |
| | | OCO(CH₂)₁₆CH₃ | |
| COCH=CHCH₃ | | | |
| COCH(CH₃)NHCOCH₂CH₂Ph | | | |
| CO-(S)-CH(NHCbz)CH(CH₃)₂ | | | |
| 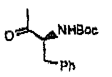 | | | |
| 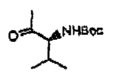 | | | |
| 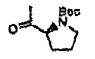 | | | |
| 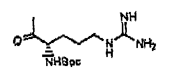 | | | |
| 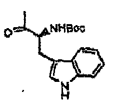 | | | |
| 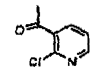 | | | |

20. Composé d'une formule de la structure générale (V) suivante ; 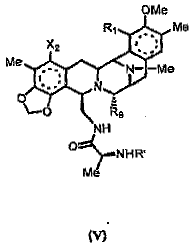 dans laquelle X₂, R₁, R₆ et R' sont chacun indépendamment choisis parmi les groupes définis ci-dessous:
| R' | X₂ | R₁ | R₆ |
|---|---|---|---|
| | OH | OH | CN |
| CH₂CH=CH₂ | OAc | OAc | |
| COCH₂CH₃ | OCOOCH₂CH=CH₂ | OCOCH₂C₆H₁₁ | |
| CO(CH₂)₄CH₃ | OCOCF₃ | OCOCH₂CH₂C₆H₁₁ | |
| CO(CH₂)₁₂CH₃ | OCOCH₂Cl | OCOCH₂CH₂CH₃ | |
| CO(CH₂)₁₆CH₃ | OCOCH₂CH₂Cl | OCO(CH₂)₄CH₃ | |
| COCH₂C₆H₁₁ | OCOCF₂CF₂CF₃ | OCO(CH₂)₈CH₃ | |
| COCH₂CH₂C₆H₁₁ | | OCO(CH₂)₁₆CH₃ | |
| COCH₂Ph | | | |
| COCH₂CH₂Ph | | | |
| COCH=CHCH₃ | | | |
| COCH(CH₃)NHCOCH₂CH₂Ph | | | |
| CO-(S)-CH(CH₃)NHCOCF₃ | | | |
| CO-(R)-CH(CH₃)NHCOCF₃ | | | |
| CO-(S)-CH(NHCb₂)CH(CH₃)₂ | | | |
| Boc | | | |
| 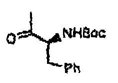 | | | |
| 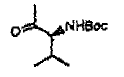 | | | |
| 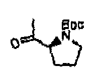 | | | |
| 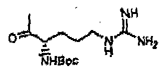 | | | |
| 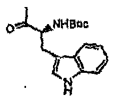 | | | |
| 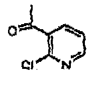 | | | |

21. Composé qui est choisi parmi les formules suivantes:

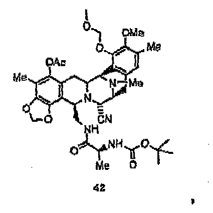

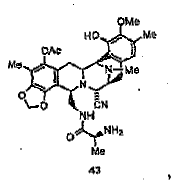

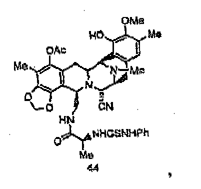

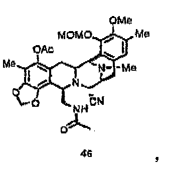

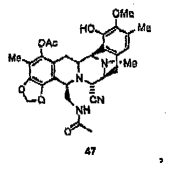

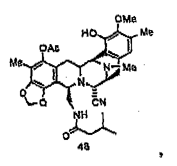

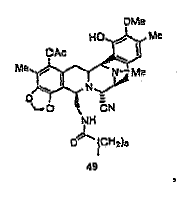

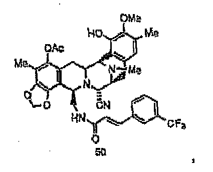

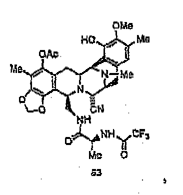

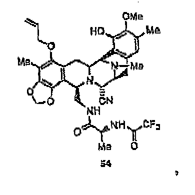

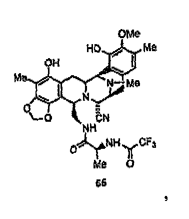

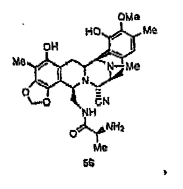

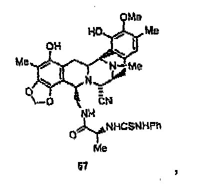

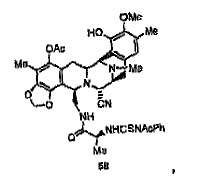

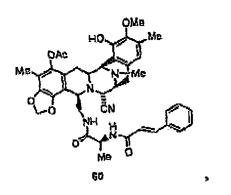

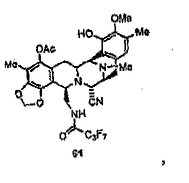

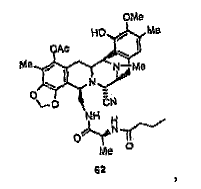

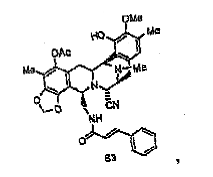

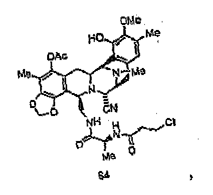

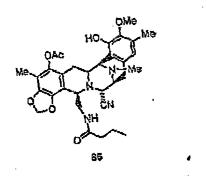

22. Composé qui est choisi parmi les formules suivantes: 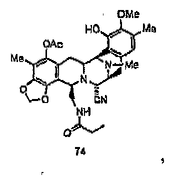 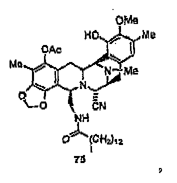 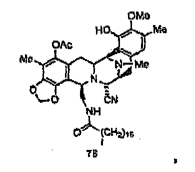 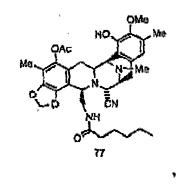 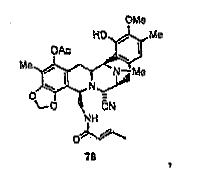 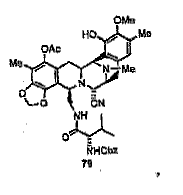 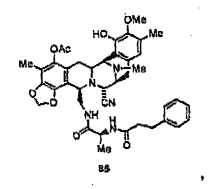 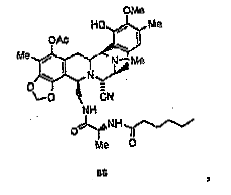 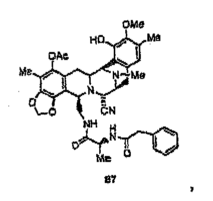 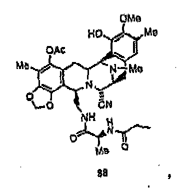 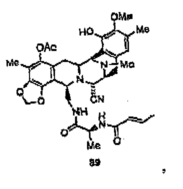 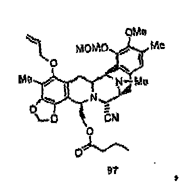 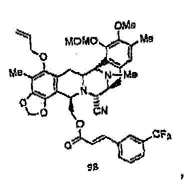 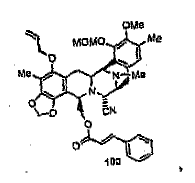 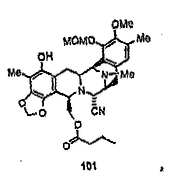 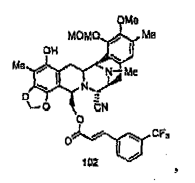 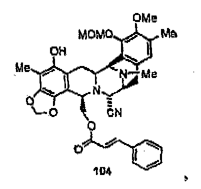 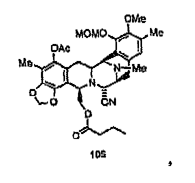 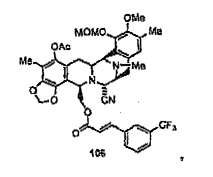 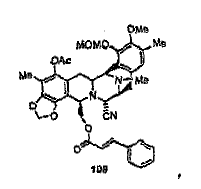 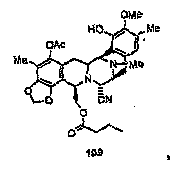 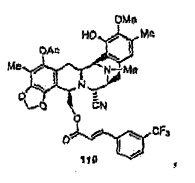 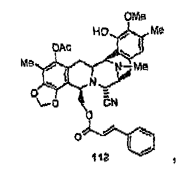 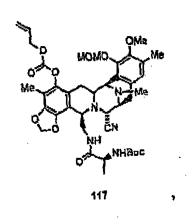 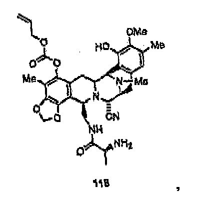 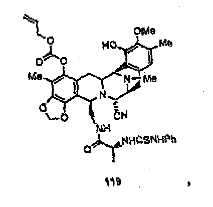 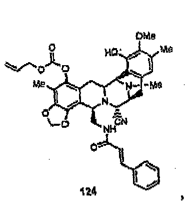 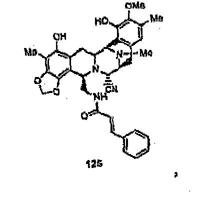 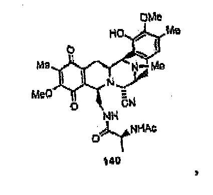 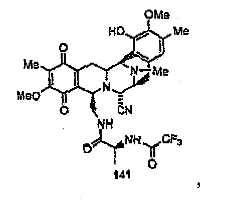 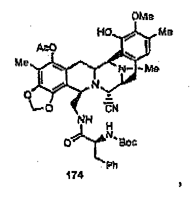 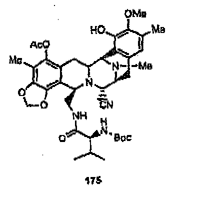 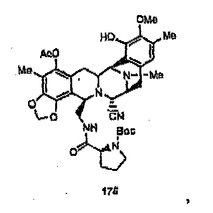 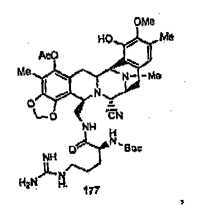 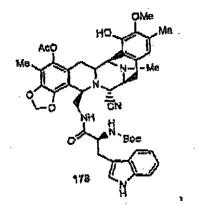 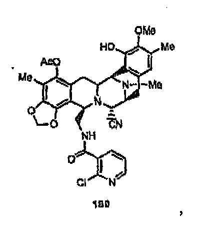 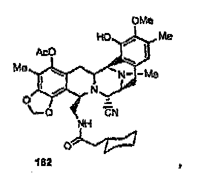 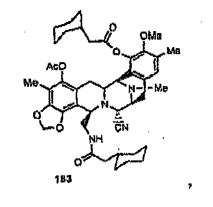 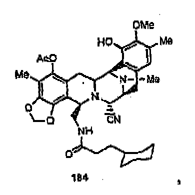 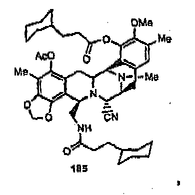 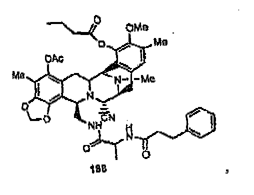 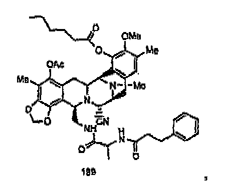 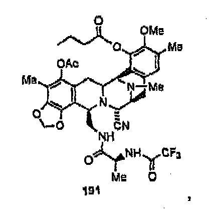 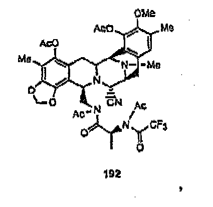 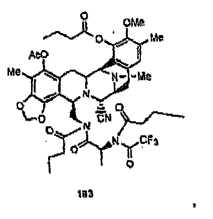 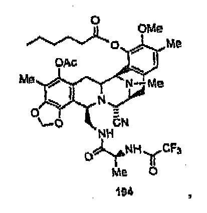 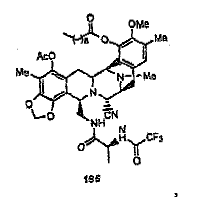 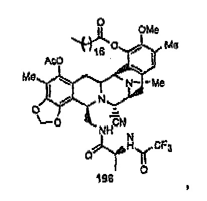 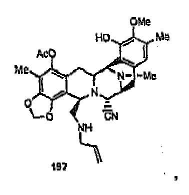 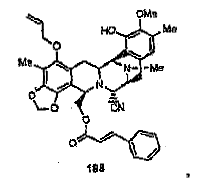 et

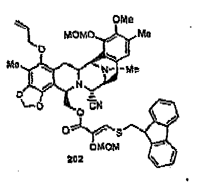

23. Composé de la formule:

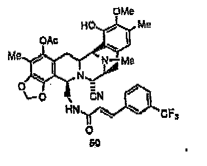

24. Composé de la formule:

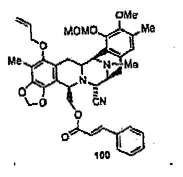

25. Composé de la formule:

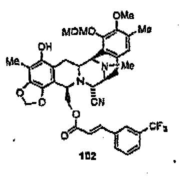

26. Composé de la formule:

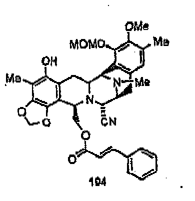

27. Composé de la formule:

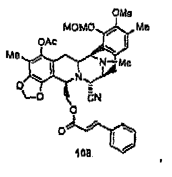

28. Composé de la formule:

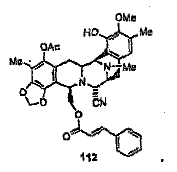

29. Composé de la formule;

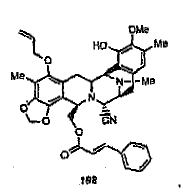

30. Composé de la formule: 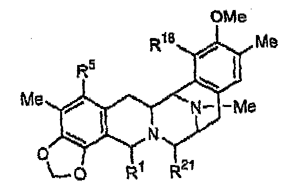 dans laquelle:
R¹ est -CH₂OR^{a}, où R^{a} est choisi dans le groupe constitué de groupes alkyle-CO-; haloalkyle-CO-; cycloalkylalkyle-CO-; haloalkyle-O-CO-; arylalcényle-CO-, où le groupe aryle est choisi parmi des groupes phényle, biphényle et naphtyle; hétéroaryle-CO-, où le groupe hétéroaryle contient un, deux ou trois hétéroatomes choisis parmi l'azote, le soufre et l'oxygène; alcényle-CO-; alcényle; et acyle d'acide aminé;
R⁵ est -OR", où R" est choisi dans le groupe constitué de H; de groupes alkyle-CO-; cycloalkyle-CO-; et haloalkyle-CO-;
R¹⁸ est -OR, où R est choisi dans le groupe constitué de H; de groupes alkyle-CO-; cycloalkylalkyle-CO-; et
R²¹ est -CN.

31. Composé suivant la revendication 30, qui est de la formule: 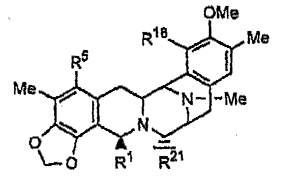 dans laquelle R¹, R⁵, R¹⁸ et R²¹ sont tels que définis dans la revendication 30.

32. Composé suivant la revendication 30, qui est de la formule; 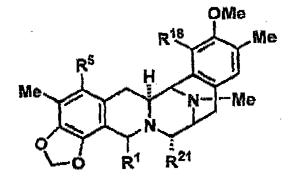 dans laquelle R¹, R⁵, R¹⁸ et R²¹ sont tels que définis dans la revendication 30.

33. Composé suivant la revendication 30, 31 ou 32, dans lequel R' est -CH₂-OR^{a}, où R^{a} est choisi dans le groupe constitué d'un groupe cystéine protégé; de groupes alkyle-CO-; arylalcényle-CO-, où le groupe aryle est choisi parmi des groupes phényle, biphényle et naphtyle; un dérivé de cystéine de la formule Prot^{SH}-S-CH₂-C(=NOProt^{OH})-CO-, où Prot^{SH} est un groupe protecteur pour un groupe thiol et Prot^{OH} est un groupe protecteur pour un groupe hydroxyle; et un dérivé de cystéine de la formule Prot^{SH}-S-CH=C(-OProt^{OH})-CO-, où Prot^{SH} est un groupe protecteur pour un groupe thiol et Prot^{OH} est un groupe protecteur pour un groupe hydroxyle,

34. Composé suivant la revendication 33, dans lequel R¹ est -CH₂-OR^{a}, où R^{a} est choisi dans le groupe constitué de groupes S-Fm-O.-TBDMS-cystéine; butyryle; (trifluorométhyl)cinnamoyle; cinnamoyle; un dérivé de cystéine de la formule Prot^{SH}-S-CH₂-C(=NOProt^{OH})-CO-, où Prot^{SH} est Fm et Prot^{OH} est un groupe méthoxy; et un dérivé de cystéine de la formule Prot^{SH}-S-CH=C(-OProt^{OH})-CO-, où Prot^{SH} est Fm et Prot^{OH} est MOM.

35. Composition pharmaceutique comprenant un composé suivant l'une quelconque des revendications précédentes.

36. Utilisation d'un composé tel que défini dans l'une quelconque des revendications 1 à 34 dans la préparation d'une composition pharmaceutique pour une méthode de traitement d'un mammifère affecté par un cancer qui comprend l'administration au mammifère affecté d'une quantité thérapeutiquement efficace du composé.

37. Utilisation suivant la revendication 36, dans laquelle ledit mammifère est un humain.

38. Utilisation suivant la revendication 37, dans laquelle ledit composé est administré par une perfusion intraveineuse.

39. Utilisation suivant l'une quelconque des revendications 36 à 38, dans laquelle ledit composé est administré sous forme d'une partie d'une thérapie de combinaison.
